(19) Europäisches Patentamt
European Patent Office
Office européen des brevets

(11) EP 4 406 538 A1

(12) **EUROPEAN PATENT APPLICATION**
published in accordance with Art. 153(4) EPC

(43) Date of publication:
**31.07.2024 Bulletin 2024/31**

(21) Application number: **22872104.9**

(22) Date of filing: **23.09.2022**

(51) International Patent Classification (IPC):
***A61K 31/495*** (2006.01) ***A61K 31/496*** (2006.01)
***A61K 31/472*** (2006.01) ***A61K 31/551*** (2006.01)
***A61K 31/395*** (2006.01) ***A61K 31/4375*** (2006.01)
***A61K 31/445*** (2006.01) ***A61K 31/5375*** (2006.01)
***A61K 45/06*** (2006.01) ***A61P 35/00*** (2006.01)
***A61P 35/02*** (2006.01)

(52) Cooperative Patent Classification (CPC):
**A61K 31/395; A61K 31/4375; A61K 31/445; A61K 31/472; A61K 31/495; A61K 31/496; A61K 31/5375; A61K 31/551; A61K 31/675; A61K 45/06; A61P 35/00; A61P 35/02**

(86) International application number:
**PCT/CN2022/120817**

(87) International publication number:
**WO 2023/046060 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.09.2021 CN 202111130589**

(71) Applicant: **Ascentawits Pharmaceuticals, Ltd.**
**Shenzhen, Guangdong 518118 (CN)**

(72) Inventors:
- **DUAN, Jianxin**
  **Shenzhen, Guangdong 518118 (CN)**
- **LI, Anrong**
  **Shenzhen, Guangdong 518118 (CN)**
- **MENG, Fanying**
  **San Francisco, CA 94121 (US)**
- **QI, Tianyang**
  **Shenzhen, Guangdong 518118 (CN)**

(74) Representative: **Studio Torta S.p.A.**
**Via Viotti, 9**
**10121 Torino (IT)**

(54) **TREATMENT OF CANCER PATIENTS HAVING KRAS MUTATIONS**

(57) A method for treating cancer and tumor patients having KRAS mutations, and pharmaceutical use.

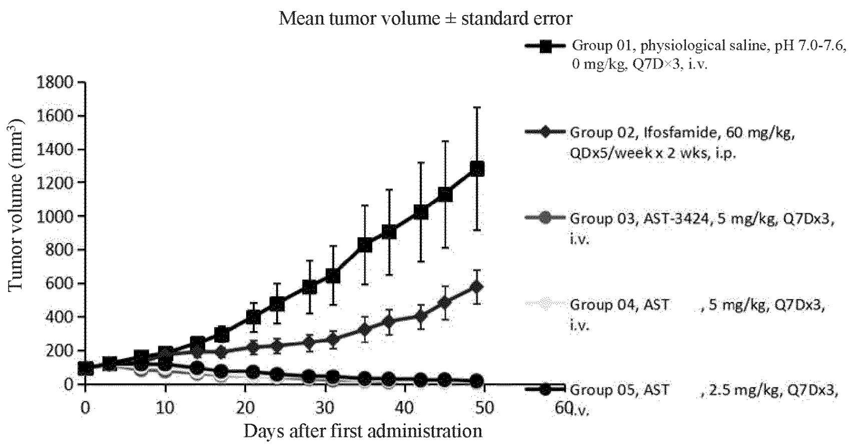


Fig. 1

EP 4 406 538 A1

## Description

## TECHNICAL FIELD

The present invention relates to a treatment method of cancer, and in particular to a treatment method of cancer patients having KRAS mutations.

## BACKGROUND

The full name of the KRAS gene is Kirsten ratsarcoma viral oncogene homolog. The protein encoded by the KRAS gene is a small GTPase, which belongs to the RAS protein superfamily. The RAS genes include three types of genes: KRAS, NRAS, and HRAS. The proteins encoded by these genes are GTPases, which act as molecular switches in the pathways that regulate cell proliferation and survival. Based on current COSMIC data, KRAS is the most commonly mutated gene of the three genes (22%), followed by NRAS (8%), and finally HRAS (3%). KRAS gene has a significant impact on human cancers, with KRAS mutations present in approximately 30% of all cancer patients including 90% of pancreatic cancer, 50% of colon cancer, and 25% of lung cancer. In non-small cell lung cancer, KRAS gene mutations account for 20-30% of the cases, mostly present in lung adenocarcinoma, and rarer in lung squamous carcinoma. KRAS gene mutations are mainly concentrated in codon positions 12, 13 and 61, wherein mutations at codon position 12 account for more than 80% of all mutations, including G12A, G12C, G12D, G12R, G12S and G12V. The most common way in which the KRAS gene is activated is point mutation, with 95% of KRAS mutations occurring mainly at codon 12 (>80%) and codon 13 of exon 2. Among the common forms of mutation are KRAS-G12C mutation (accounting for 13% of all KRAS mutations), KRAS-G12V (20%) and KRAS-G12D (29%) mutations (Loong HHF, Du N, Cheng C, Lin H,Guo J, Lin G, Li M, Jiang T, Shi Z, Cui Y, Jin X, Yao J, Xing Y, Yao M, Wang K, Mok TSK, Liu L. KRAS G12C mutations in Asia: A landscape analysis of 11,951 Chinese tumor samples. Transl Lung Cancer Res 2020. doi: 10.21037/tlcr-20-455).

G12C mutation in the KRAS gene, whose mutants have a cysteine residue (glycine at position 12 is changed to cysteine), has been used to design covalent inhibitors with preclinical activity. For example, AMG-510 (the first KRAS-G12C inhibitor in clinical development) and MRTX849 have shown strong anti-tumor activity.

On May 29, 2021, Sotorasib (AMG-510, Lumakras), known as a "revolutionary anti-cancer drug" in the industry and effective against KRAS mutations, was approved by the FDA for the treatment of non-small cell lung cancer patients with KRAS-G12C mutations who have received at least one prior systemic therapy. AMG510 is specifically designed for the mutant subtype KRAS-G12C and has high selectivity. AMG510 can specifically bind to KRAS-G12C in over 6000 proteins, locking in and inactivating it.

On June 24, 2021, the U.S. Food and Drug Administration (FDA) approved breakthrough therapy designation to Adagrasib (MRTX849) for the treatment of patients with non-small cell lung cancer (NSCLC) harboring the KRAS-G12C mutation who have received prior systemic therapy. The drug is an oral inhibitor optimized specifically for the KRAS-G12C mutant. By irreversibly and selectively binding to KRAS-G12C in its inactive state, preventing it from sending cell growth signals and causing cancer cell death, MRTX849 has shown promising safety and anticancer activity in the treatment of non-small cell lung cancer (NSCLC) and colorectal cancer (CRC) harboring the KRAS-G12C mutation, as well as other solid tumors, as demonstrated in preliminary human clinical trials.

However, no drugs have been marketed for the broader G12D subtype of the KRAS mutation.

## SUMMARY OF THE INVENTION

AST-3424 (OBI-3424) is a first-in-class DNA alkylating cancer therapeutic drug targeting overexpression of aldo-keto reductase 1C3 (AKR1C3), which selectively targets cancers overexpressing AKR1C3 and selectively releases a potent DNA alkylating agent in the presence of AKR1C3 enzyme. This selective mode of activation distinguishes AST-3424 from conventional alkylating agents (such as cyclophosphamide and ifosfamide). AKR1C3 overexpression is present in a wide range of treatment-resistant and refractory cancers, including HCC, castration-resistant prostate cancer (CRPC), and T-cell acute lymphoblastic leukemia (T-ALL). AKR1C3 is highly expressed in up to 15 solid and hematologic tumors. Currently, the drug is undergoing phase II clinical trials in China and the United States (US OBI-3424-NCT03592264-phase II, castration prostate cancer and liver cancer; US OBI-3424-NCT04315324-phase II, T-lymphoblastic acute leukemia (T-ALL); China AST-3424-CTR20191399-phase II, solid tumors; China AST-3424-CTR20201915-II phase, T-lymphocytic acute leukemia (T-ALL) and B-lymphocytic acute leukemia (B-ALL)).

AST-3424 and compound

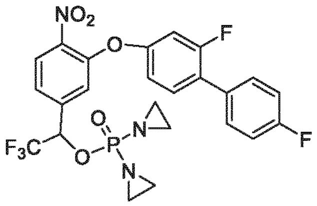

(hereinafter referred to as AST) have been found in an animal *in vivo* model study of this drug and similar drugs to have good therapeutic efficacy on a KRAS mutant G12D subtype tumor model with high expression of AKR1C3.

Based on the experimental results, the present application provides the following method of treating cancer and pharmaceutical use of the compounds.

A treatment method which uses a drug monotherapy containing an AKR1C3-activated DNA alkylating agent prodrug compound or in combination with other therapeutic drugs for treating cancer and tumor patients having KRAS mutations.

**Pharmaceutical use** of an AKR1C3-activated DNA alkylating agent prodrug compound, wherein the compound is used in the manufacture of a drug monotherapy or in combination with other therapeutic drugs for treating cancer and tumor patients having KRAS mutations.

An AKR1C3-activated DNA alkylating agent prodrug compound means that the compound is a prodrug, and the prodrug molecule reacts with the AKR1C3 enzyme to release a cytotoxic DNA alkylating agent upon reaction.

Specifically, taking AST-3424 as an example, these compounds as aldo-keto reductase AKR1C3 specific substrates, can be quickly and effectively reduced only in cancer cells with high expression of AKR1C3, thereby releasing cytotoxic-DNA alkylating agent AST-2660, and AST-2660 is crosslinked with the DNA, subsequently leads to cancer cells death:

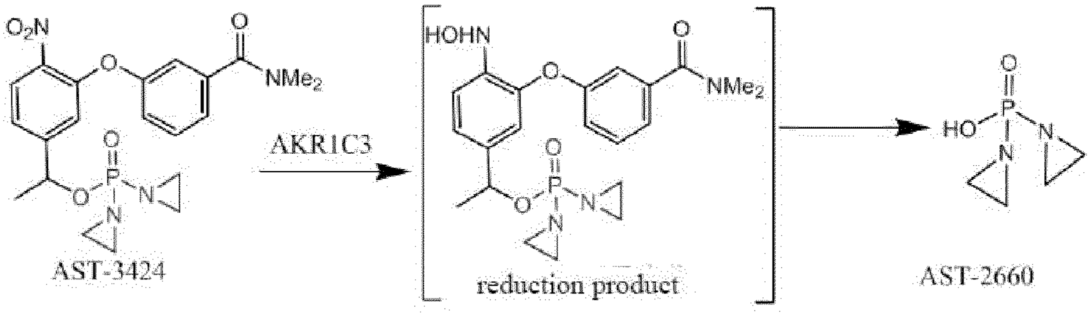


A treatment method which uses a drug monotherapy containing an AKR1C3-activated DNA alkylating agent prodrug compound or in combination with other therapeutic drugs for treating cancer and tumor patients having KRAS mutations, wherein the compound is selected from structural Formula (1) or (2) and salts, esters, solvates, and isotopic isomers thereof:

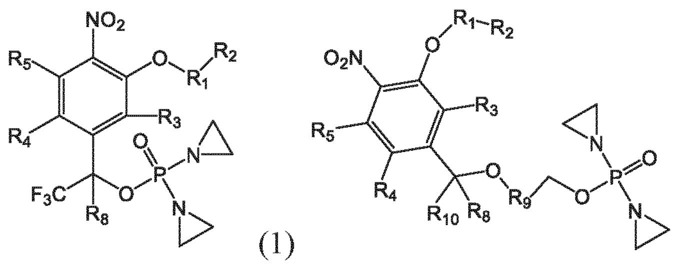
(1) (2),

**Pharmaceutical use** of an AKR1C3-activated DNA alkylating agent prodrug compound, wherein the compound is used in the manufacture of a drug monotherapy or in combination with other therapeutic drugs for treating cancer and tumor patients having KRAS mutations, wherein the compound is selected from the structural Formula (1) or (2) and salts, esters, solvates, and isotopic isomers thereof.

Wherein the definitions of $R_1$, $R_2$, $R_3$, $R_4$, $R_5$, $R_8$, $R_9$, and $R_{10}$ are described in the claims of Patent Application PCT/CN2020/089692 with Publication No. WO2020228685A1.

Specifically, the groups are defined as follows:
wherein,

$R_1$ is $C_6$-$C_{10}$ aryl or Z-substituted aryl, 4-15 membered heterocycle or Z-substituted heterocycle, 5-15 membered heteroaryl or Z-substituted heteroaryl, a 7-15 membered fused ring or Z-substituted fused ring;

$R_2$ is hydrogen, a halogen atom, cyano or isocyano, hydroxy, sulfhydryl, amino, OTs, OMS, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, $C_6$-$C_{10}$ aryl or Z-substituted aryl, 4-15 membered heterocycle or Z-substituted heterocycle, 5-15 membered heteroaryl or Z-substituted heteroaryl, ether having from 1 to 6 carbon atoms or Z-substituted alkoxy having from 1 to 6 carbon atoms, $-CONR^6R^7$, $-SO_2NR^6R^7$, $-SO_2R^6$, $-OCOO-R^6$, $-COOR^6$, $-NR^6COR^7$, $-OCOR^6$, $-NR^6SO_2R^7$ or $-NR^6SO_2NR^6R^7$, or $R^2$ together with the atom in the group $R_1$ to which it is bonded to form a 7-15 membered fused ring or Z-substituted fused ring;

$R_3$ is hydrogen, halogen, cyano or isocyano, hydroxy, sulfhydryl, amino, OTs, OLCMS, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, $C_6$-$C_{10}$ aryl or Z-substituted aryl, 4-15 membered heterocycle or Z-substituted heterocycle, 5-15 membered heteroaryl or Z-substituted heteroaryl, $C_1$-$C_6$ alkoxy or Z-substituted $C_1$-$C_6$ alkoxy, $-CONR^6R^7$, $-SO_2NR^6R^7$, $-SO_2R^6$, $-OCO-R^6$, $-OCOO-R^6$, $-COOR^6$, $-NR^6COR^7$, $-OCOR^6$, or $-NR^6SO_2R^7$;

$R_4$ and $R_5$ are each independently hydrogen, a halogen atom, cyano or isocyano, hydroxy, sulfhydryl, amino, OTs, OLCMS, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, $C_6$-$C_{10}$ aryl or Z-substituted aryl, 4-15 membered heterocycle or Z-substituted heterocycle, 5-15 membered heteroaryl or Z-substituted heteroaryl, $C_1$-$C_6$ alkoxy or Z-substituted $C_1$-$C_6$ alkoxy, $-CONR^6R^7$, $-SO_2NR^6R^7$, $-SO_2R^6$, $-OCOO-R^6$, $-COOR^6$, $-NR^6COR^6$, $-OCOR^6$ or $-NR^6SO_2R^7$, or $R^4$ and $R^5$ together with the atom in the benzene ring to which they are bonded to form a 7-15 membered fused ring or Z-substituted fused ring;

$R_6$ and $R_7$ are each independently hydrogen, cyano or isocyano, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, $C_6$-$C_{10}$ aryl or Z-substituted aryl, 4-15 membered heterocycle or Z-substituted heterocycle, 5-15 membered heteroaryl or Z-substituted heteroaryl, $C_1$-$C_6$ alkoxy or Z-substituted $C_1$-$C_6$ alkoxy, or $R_6$ and $R_7$ together with the atom to which they are bonded to form 5-7 membered heterocyclyl or Z-substituted 5-7 membered heterocyclyl;

$R_8$ and $R_{10}$ are each independently hydrogen, deuterium, aryl or Z-substituted aryl, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, and at least one of $R^8$ and $R^{10}$ must be hydrogen or deuterium;

$R_9$ is substituted $C_6$-$C_{10}$ aryl which is substituted with at least one fluorine atom or nitro group, substituted 4-15 membered heterocycle which is substituted with at least one fluorine atom or nitro group, or substituted 5-15 membered heteroaryl which is substituted with at least one fluorine atom or nitro group;

the substituent Z is a halogen atom, cyano or isocyano, hydroxy, sulfhydryl, amino, OTs, OMS, $C_1$-$C_3$ alkyl or substituted alkyl, $C_1$-$C_3$ alkoxy or substituted alkoxy, $C_2$-$C_3$ alkenyl or substituted alkenyl, $C_2$-$C_3$ alkynyl or substituted alkynyl, $C_3$-$C_8$ cycloalkyl or substituted cycloalkyl, an aromatic ring, heterocycle, a heteroaromatic ring and fused ring or a substituted aromatic ring, heterocycle, a heteroaromatic ring and fused ring, the pattern of substitution being mono- or di-substitution;

the substitution in the substituted $C_6$-$C_{10}$ aryl, substituted 4-15 membered heterocycle or substituted 5-15 membered heteroaryl in $R_9$ is a halogen atom, nitro, cyano or isocyano, hydroxy, amino, $C_1$-$C_3$ alkyl or alkoxy, alkenyl, alkynyl, cycloalkyl or benzene ring, substituted benzene ring, $C_1$-$C_3$ alkoxy or halogen atom-substituted alkoxy.

Specifically, the compounds of Formulae (1) and (2) are selected from the group consisting of:

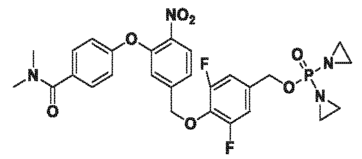
,
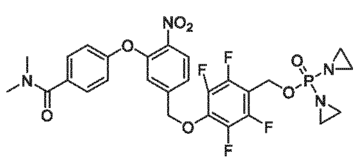
,
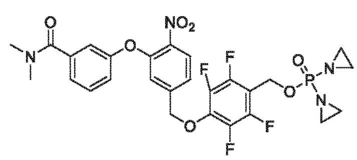

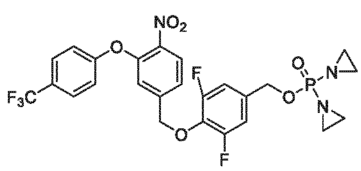 , 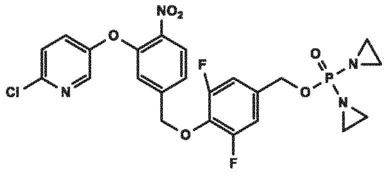 , 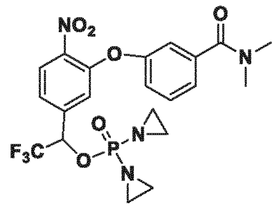

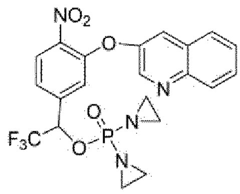 , 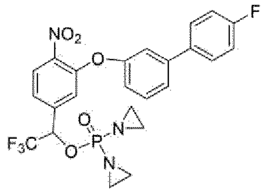 , 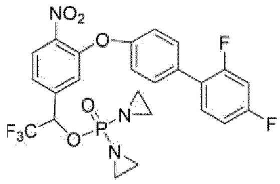

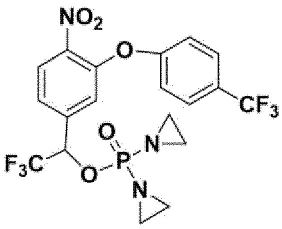 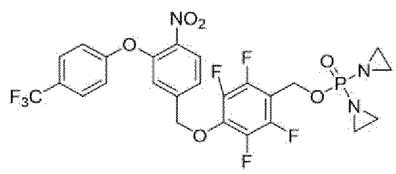 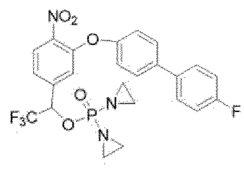

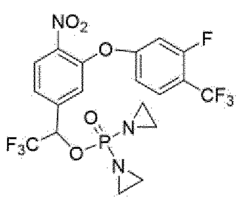 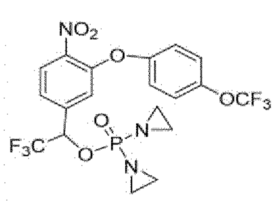 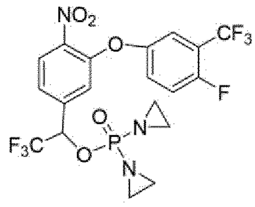

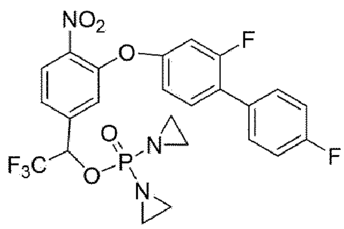 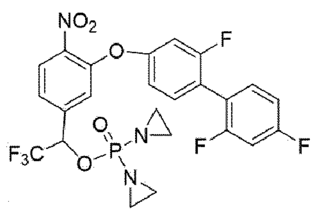 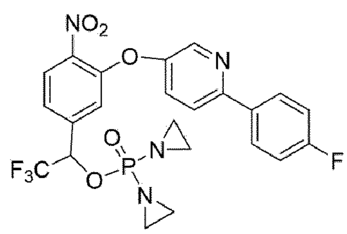

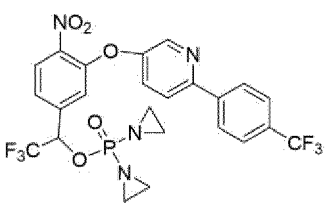 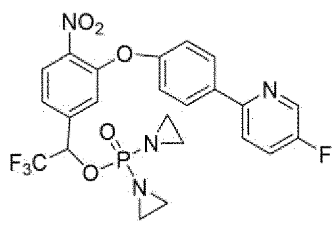 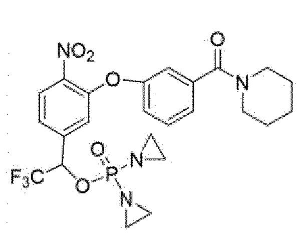

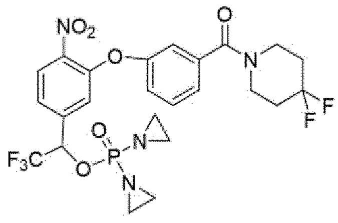 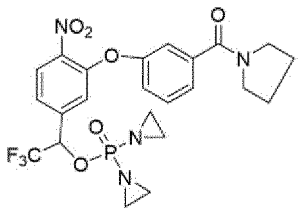 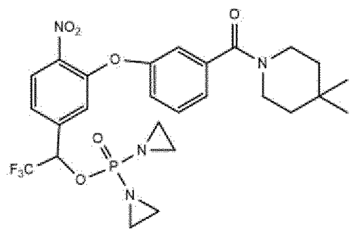

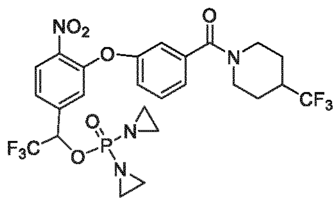 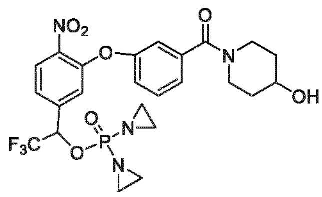 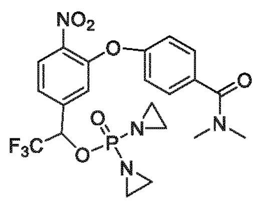

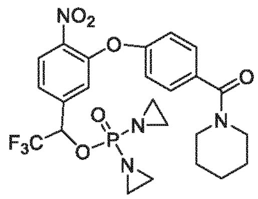 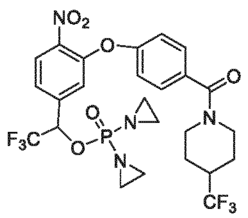 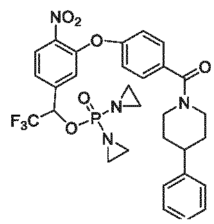

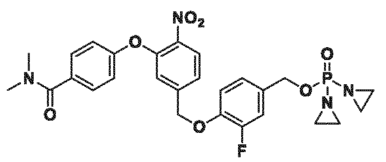 , 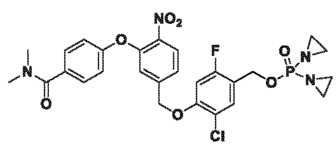 , 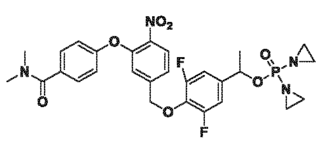 ,

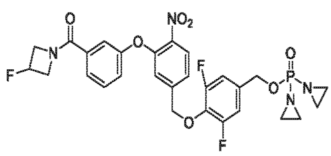 , 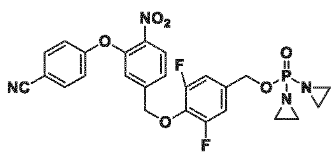 , 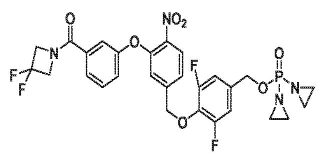 ,

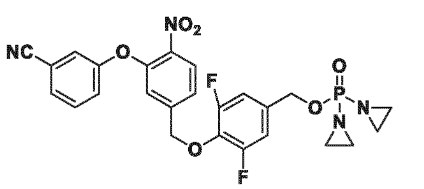 , 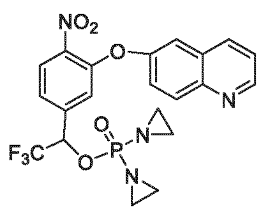 , 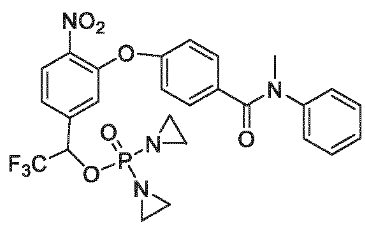 ,

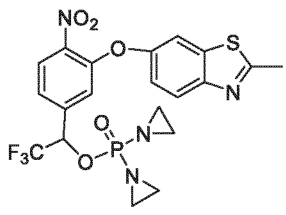, 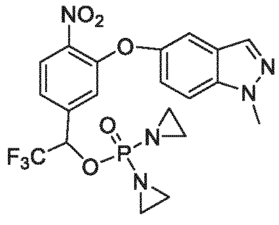, 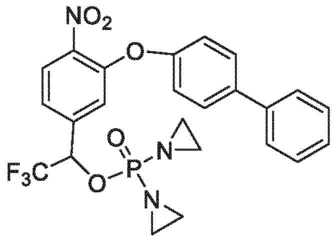

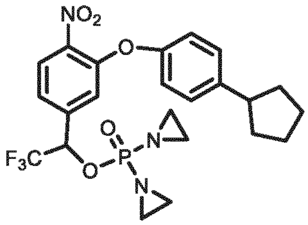, 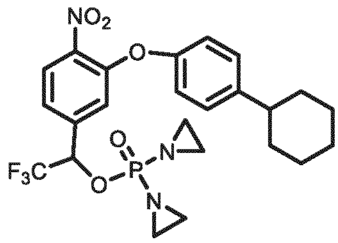, 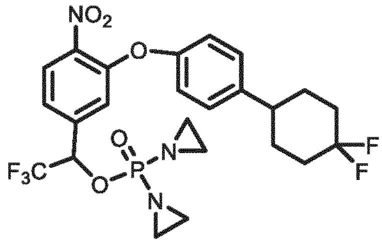.

Specific definitions and meanings of the groups, and the preparation methods and spectral data of the compounds have been described in Patent Application PCT/CN2020/089692 with Publication No. WO2020228685A1, which is incorporated herein by reference in its entirety.

Evidently, the compounds of structural Formula (1) or (2), similar to AST-342 or AST, are prodrugs of AST-2660, and can be activated by the AKR1C3 enzyme to form AST-2660 (an alkylating agent) to exhibit its anti-cancer efficacy:

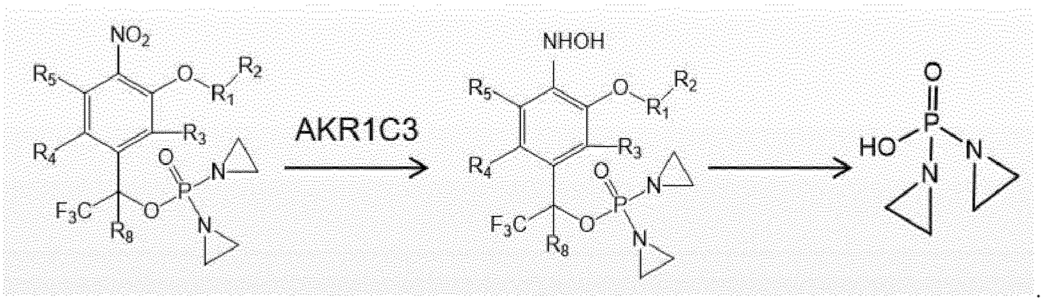
.

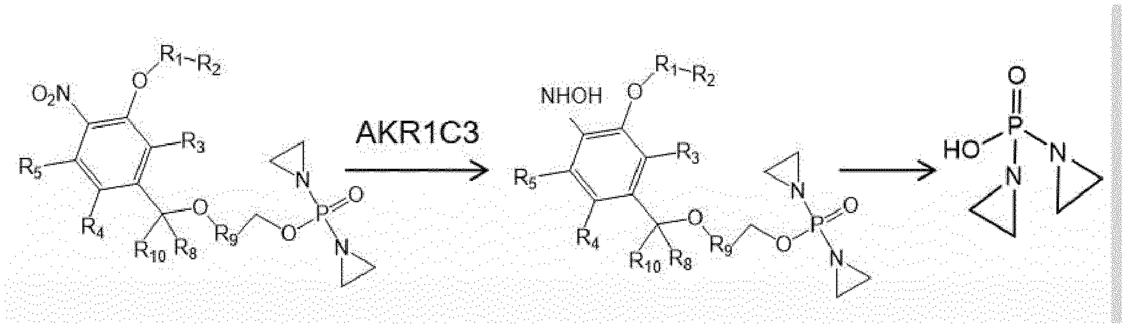


A treatment method which uses a drug monotherapy containing an AKR1C3-activated DNA alkylating agent prodrug compound or in combination with other therapeutic drugs for treating cancer and tumor patients having KRAS mutations, wherein the compound is selected from structural Formula (3) and salts, esters, solvates, and isotopic isomers thereof:

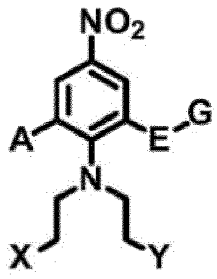

(3)

**Pharmaceutical use** of an AKR1C3-activated DNA alkylating agent prodrug compound, wherein the compound is used in the manufacture of a drug monotherapy or in combination with other therapeutic drugs for treating cancer and tumor patients having KRAS mutations, wherein the compound is selected from the structural Formula (3) and salts, esters, solvates, and isotopic isomers thereof:

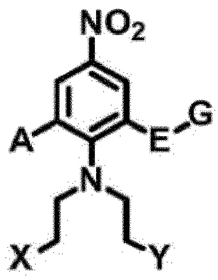

(3)

wherein the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A).

Specifically, the groups are defined as follows:
wherein,

A is H, $C_1$-$C_6$ alkyl, $C_1$-$C_6$ alkenyl, $C_1$-$C_6$ alkynyl, $CFH_2$, $CF_2H$, $CF_3$, F, Cl, Br, I, $OCF_3$, COR or $CON(R)_2$;
E is SO or $SO_2$;
X is Cl, Br, I or $OSO_2R$;
Y is Cl, Br, I or $OSO_2R$;
Each R is independently H or $C_1$-$C_6$ alkyl;
G is a radical group selected from the group consisting of Formulae (B)-(AA):

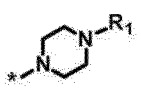 (B)
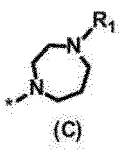 (C)
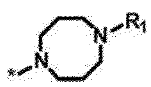 (D)
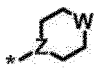 (F)
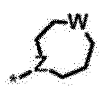 (H)
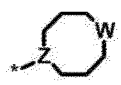 (J)

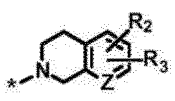 (K)
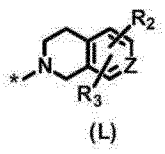 (L)
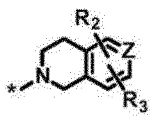 (M)
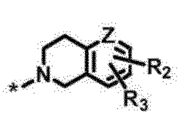 (N)
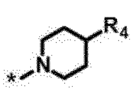 (O)

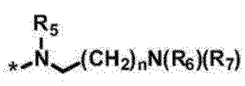 (P)
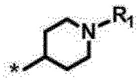 (Q)
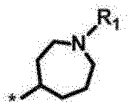 (S)
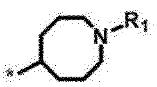 (T)
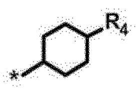 (U)
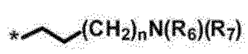 (AA)

wherein,

$R_1$ is H, $C_1$-$C_6$ alkyl, $CH_2(CH_2)_nOH$, $CH_2CH(OH)CH_2OH$, phenyl, pyridyl, benzyl, or pyridylmethyl, provided that when $R_1$ is phenyl, pyridyl, benzyl or pyridylmethyl, $R_1$ is optionally substituted at any available position with $C_1$-$C_6$ alkyl, $C_1$-$C_6$ alkenyl, $C_1$-$C_6$ alkynyl, $OR_6$, $N(R_6)(R_7)$, $CFH_2$, $CF_2H$, $CF_3$, F, Cl, Br, I, $OCF_3$, $COR_6$, $CON(R_6)(R_7)$, $SOR_6$, $SON(R_6)(R_7)$, $SO_2R_6$, $SO_2N(R_6)(R_7)$, CN, or $NO_2$;

$R_2$ and $R_3$ are each independently H, $C_1$-$C_6$ alkyl, $C_1$-$C_6$ alkenyl, $C_1$-$C_6$ alkynyl, $OR_6$, $N(R_6)(R_7)$, $CFH_2$, $CF_2H$, $CF_3$, F, Cl, Br, I, $OCF_3$, $COR_6$, $CON(R_6)(R_7)$, $SOR_6$, $SON(R_6)(R_7)$, $SO_2Re$, $SO_2N(R_6)( R_7)$, CN or $NO_2$;

$R_4$ is $N(R_6)(R_7)$, OH, $OCH_2(CH_2)_nN(R_6)(R_7)$ or $CH_2(CH_2)_nN(R_6)(R_7)$;

$R_5$ is H or a $C_1$-$C_6$ alkyl group;

$R_6$ and $R_7$ are each independently H or $C_{1-6}$ alkyl, or $R_6$ and $R_7$ together to form substituted or unsubstituted 5-membered or 6-membered heterocycle;

Z isCH orN;

W is $CH_2$, O, S, SO or $SO_2$;

n is 0 to 6;

* represents a point of attachment to Formula (I).

Specifically, the compound of Formula (3) is selected from the group consisting of:

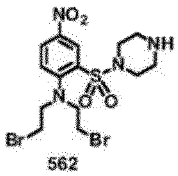
562
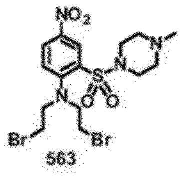
563
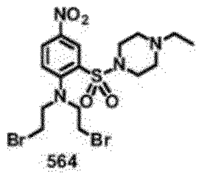
564
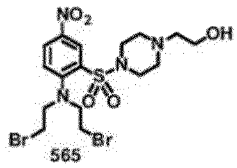
565
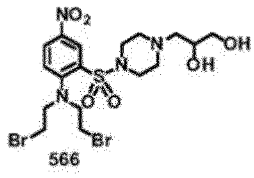
566

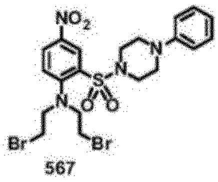
567
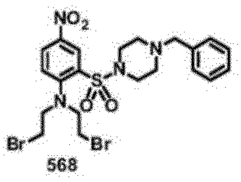
568
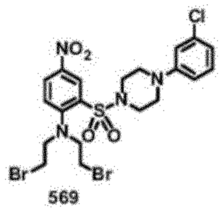
569
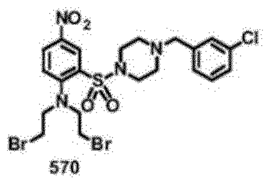
570

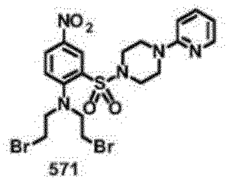
571
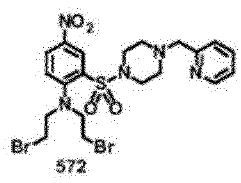
572
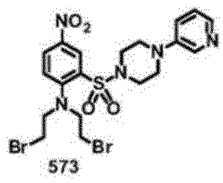
573
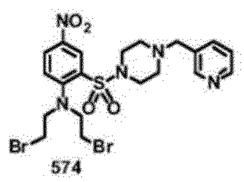
574

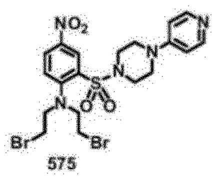
575
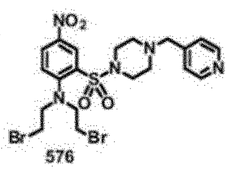
576
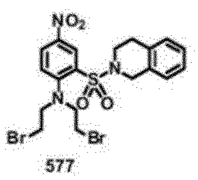
577
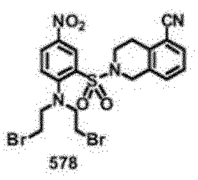
578
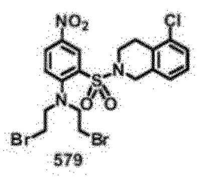
579

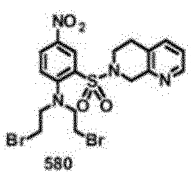
580
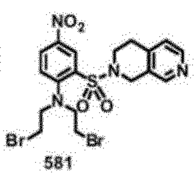
581
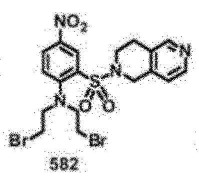
582
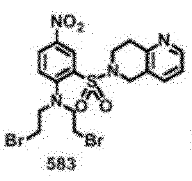
583
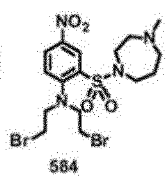
584
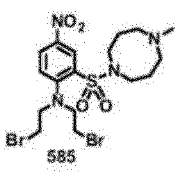
585

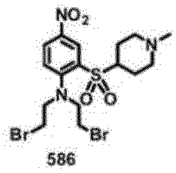
586
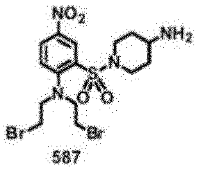
587
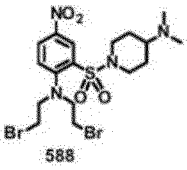
588
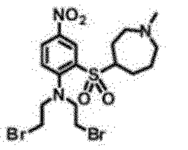
589
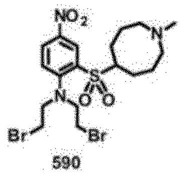
590
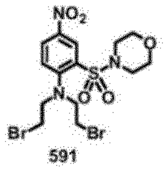
591

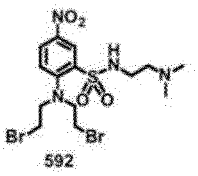
592
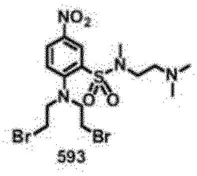
593
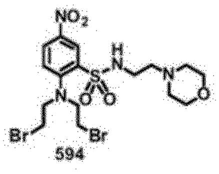
594
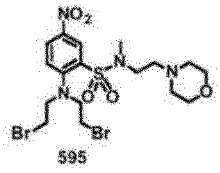
595
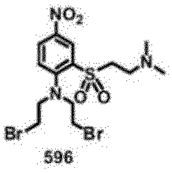
596

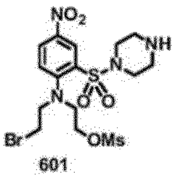
601
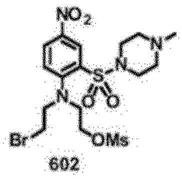
602
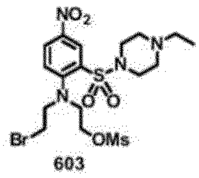
603
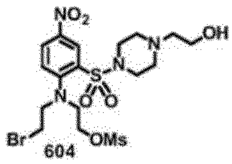
604
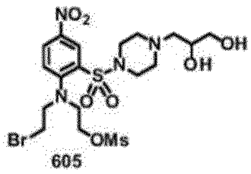
605

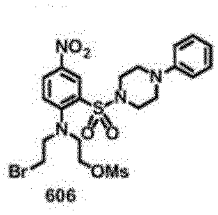
606
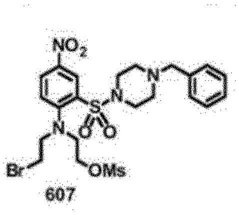
607
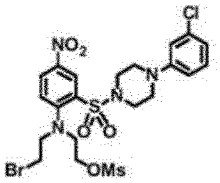
608
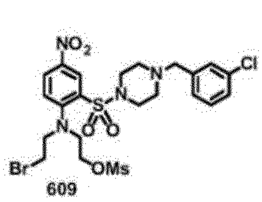
609

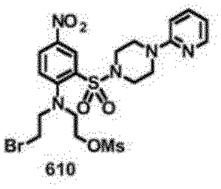
610
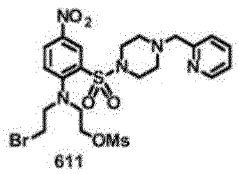
611
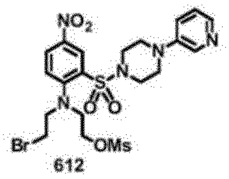
612
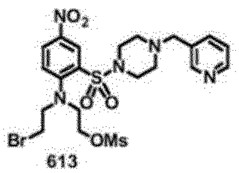
613

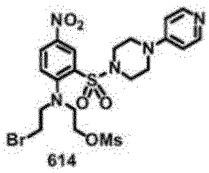
614
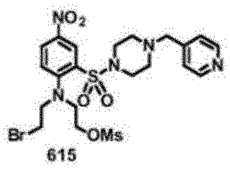
615
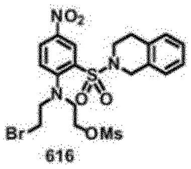
616
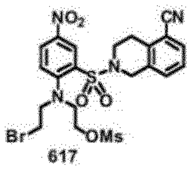
617
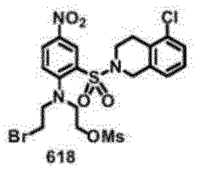
618

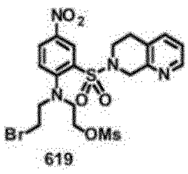
619
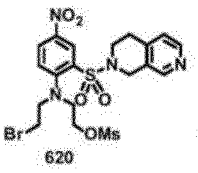
620
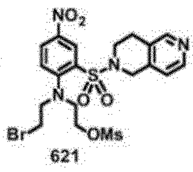
621
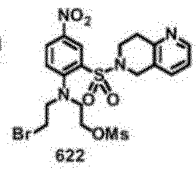
622
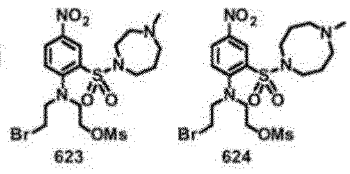
623
624

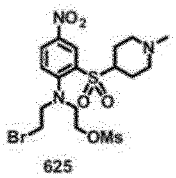
625
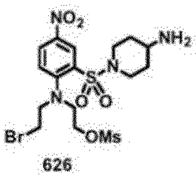
626
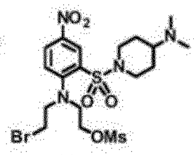
627
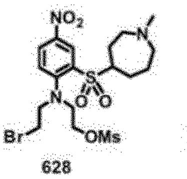
628
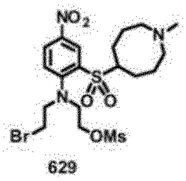
629
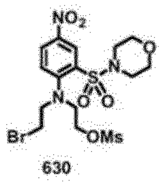
630

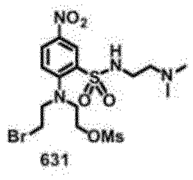
631
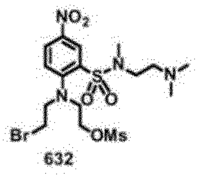
632
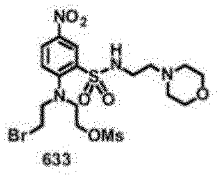
633
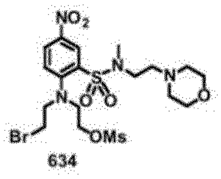
634
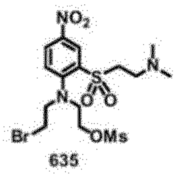
635

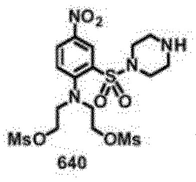
640
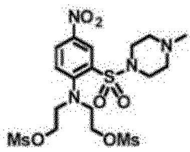
641
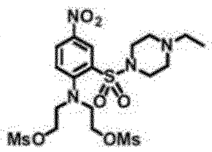
642
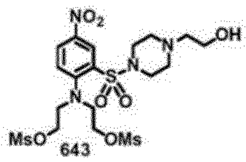
643
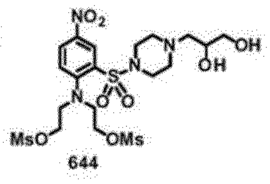
644

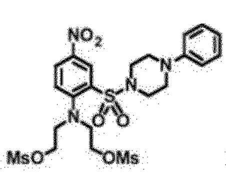
645
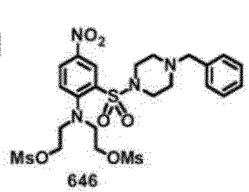
646
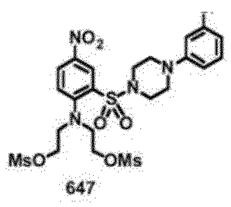
647
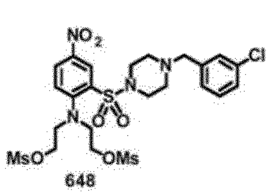
648

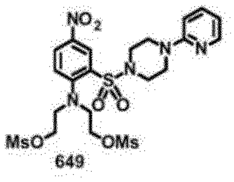
649
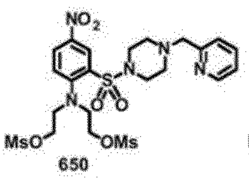
650
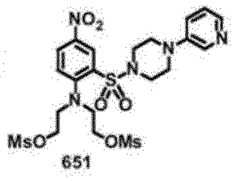
651
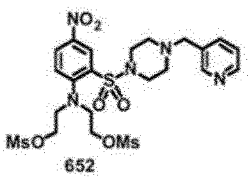
652

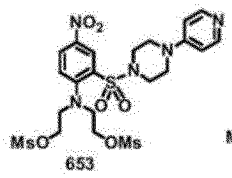
653
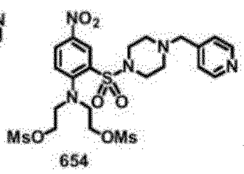
654
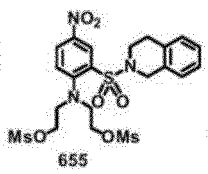
655
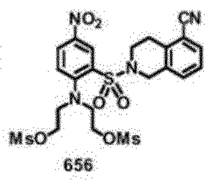
656
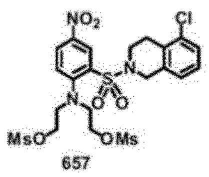
657

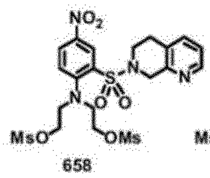
658
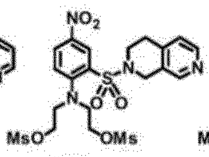
659
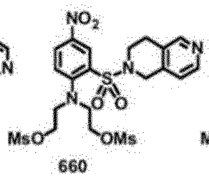
660
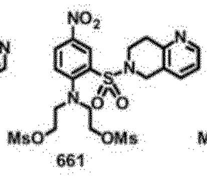
661
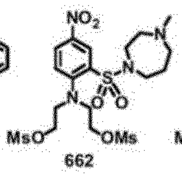
662
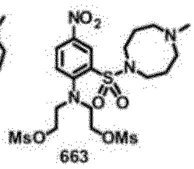
663

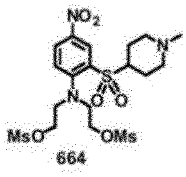
664
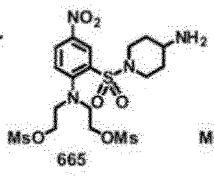
665
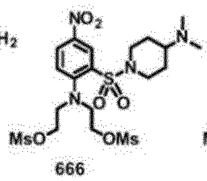
666
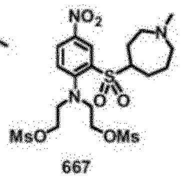
667
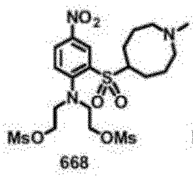
668
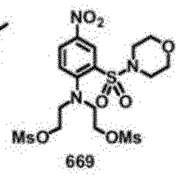
669

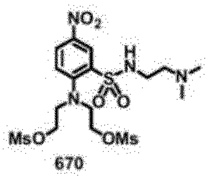
670
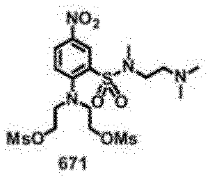
671
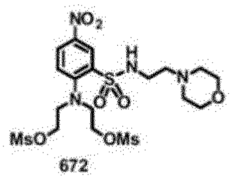
672
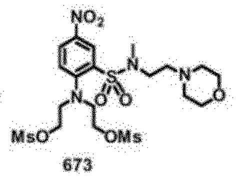
673
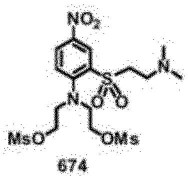
674

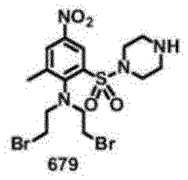
679
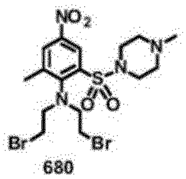
680
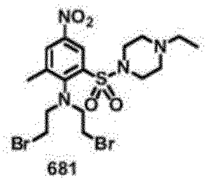
681
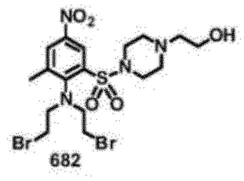
682
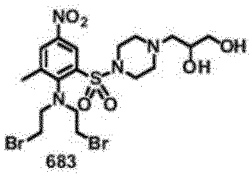
683

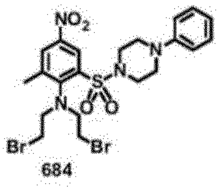
684
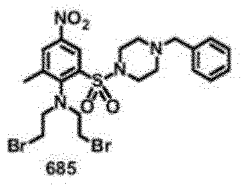
685
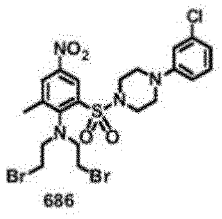
686
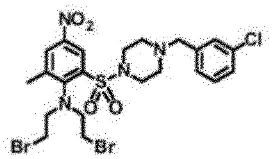
687

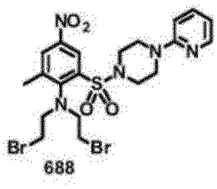
688
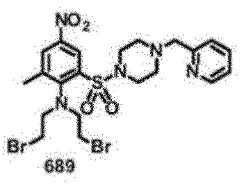
689
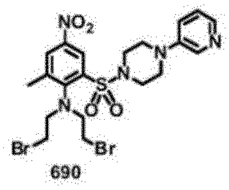
690
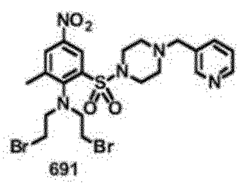
691

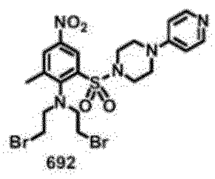
692
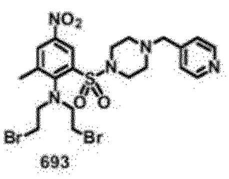
693
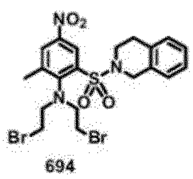
694
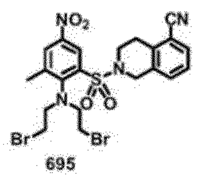
695
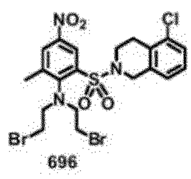
696

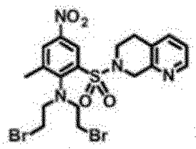
697
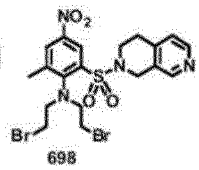
698
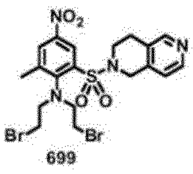
699
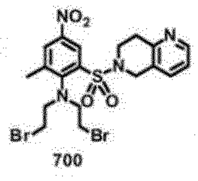
700
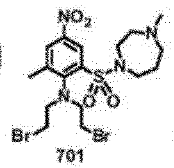
701
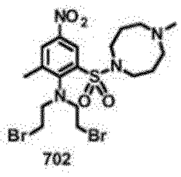
702

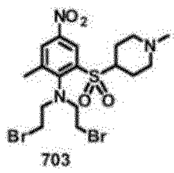
703
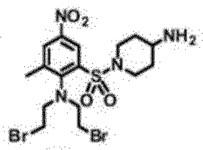
704
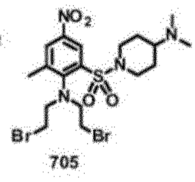
705
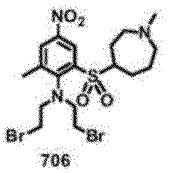
706
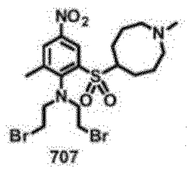
707
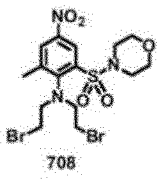
708

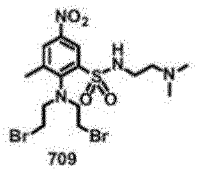
709
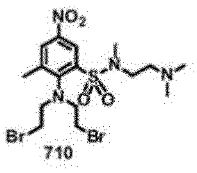
710
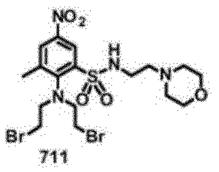
711
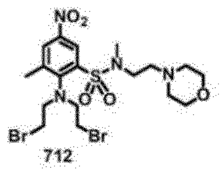
712
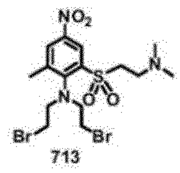
713

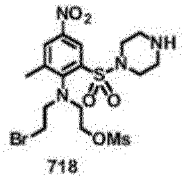
718
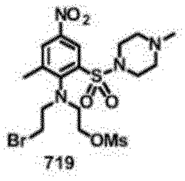
719
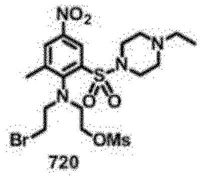
720
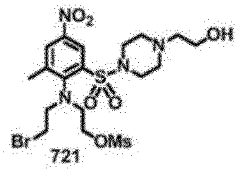
721
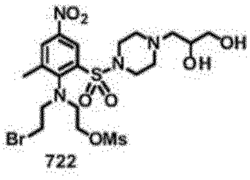
722

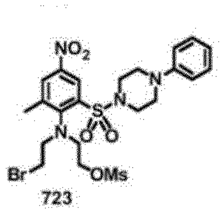
723
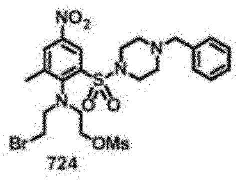
724
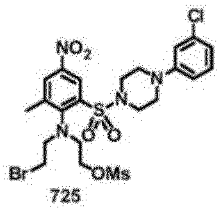
725
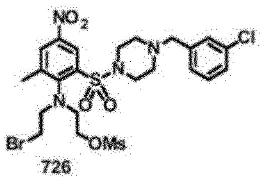
726

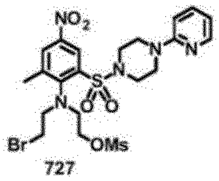
727
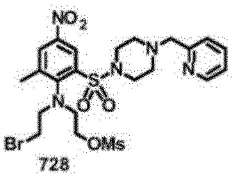
728
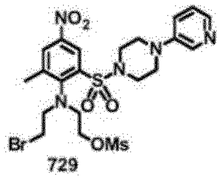
729
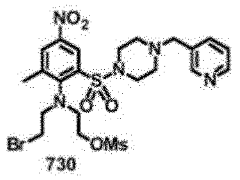
730

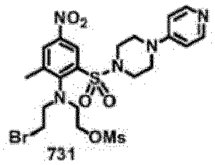
731
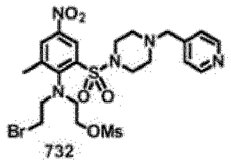
732
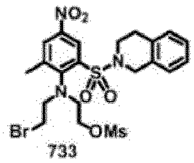
733
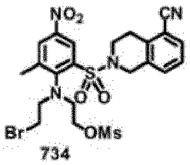
734
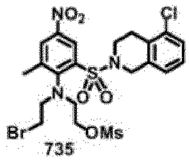
735

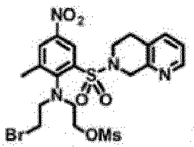
736
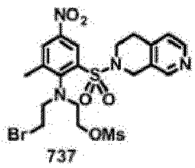
737
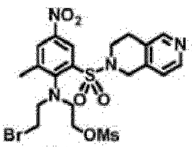
738
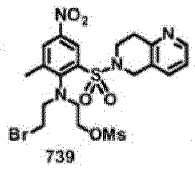
739
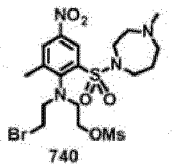
740
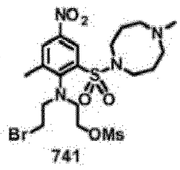
741

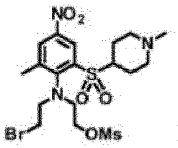
742
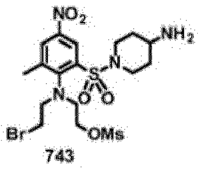
743
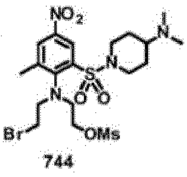
744
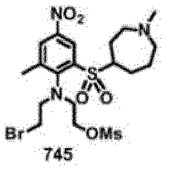
745
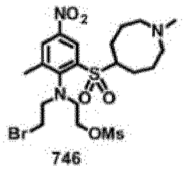
746
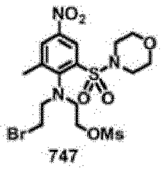
747

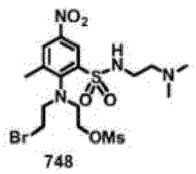
748
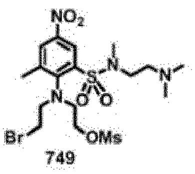
749
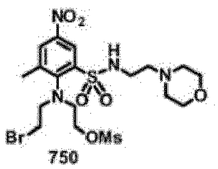
750
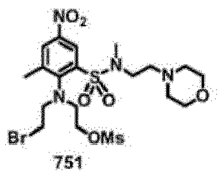
751
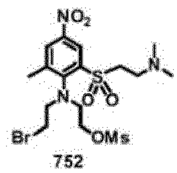
752

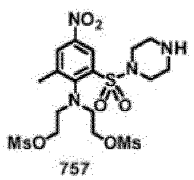
757
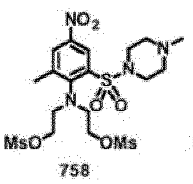
758
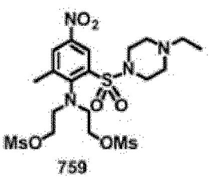
759
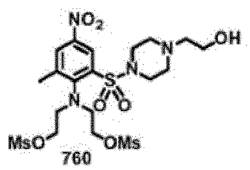
760
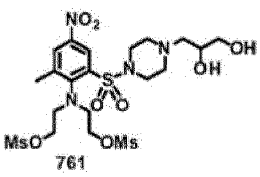
761

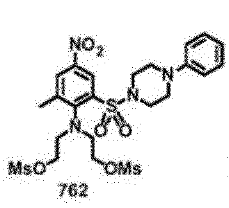
762
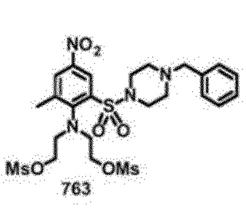
763
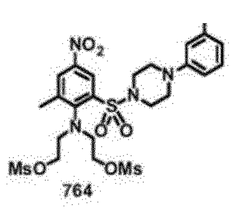
764
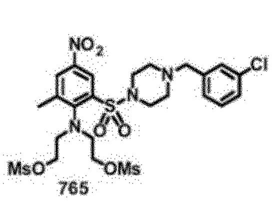
765

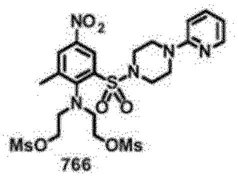
766
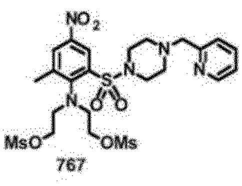
767
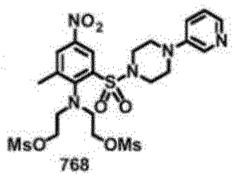
768
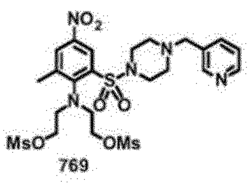
769

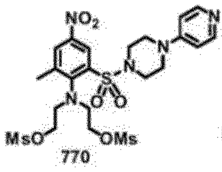
770
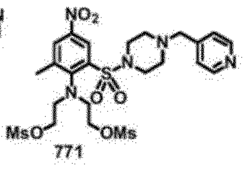
771
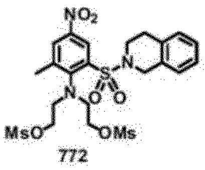
772
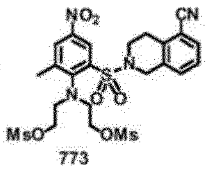
773
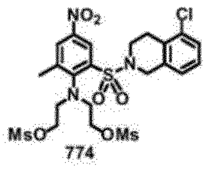
774

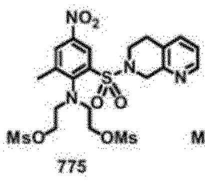
775
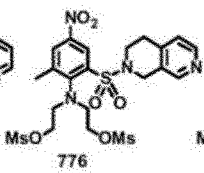
776
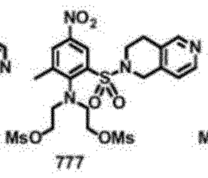
777
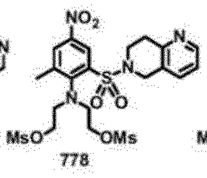
778
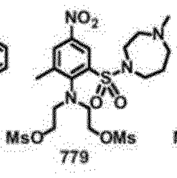
779
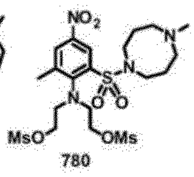
780

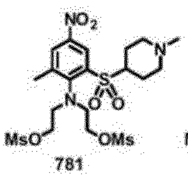
781
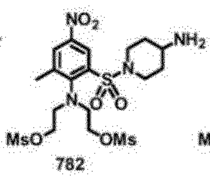
782
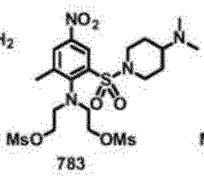
783
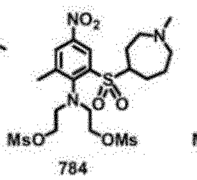
784
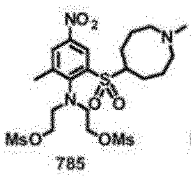
785
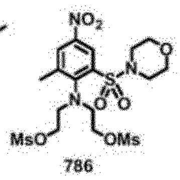
786

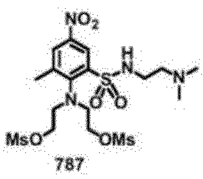
787
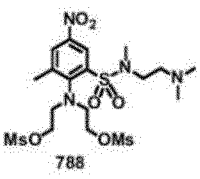
788
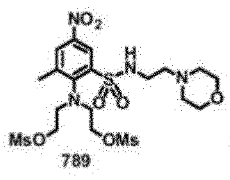
789
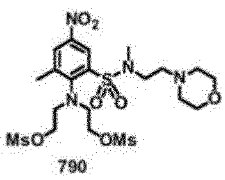
790
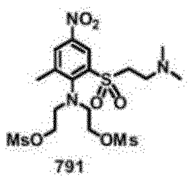
791

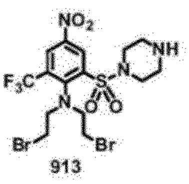
913
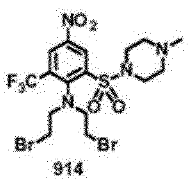
914
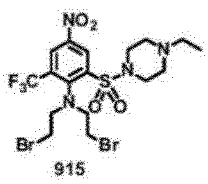
915
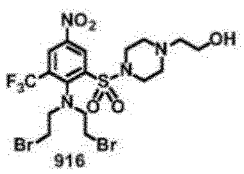
916
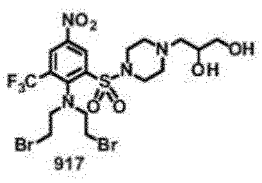
917

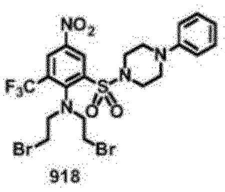
918
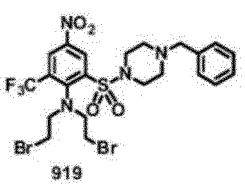
919
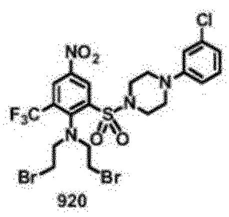
920
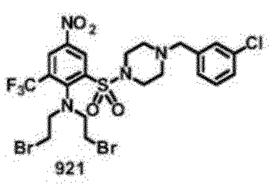
921

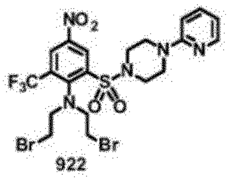
922
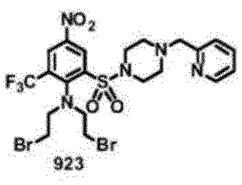
923
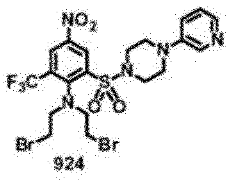
924
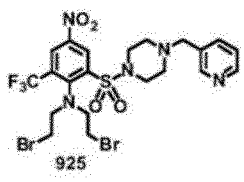
925

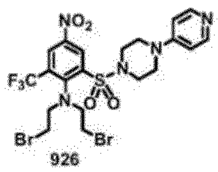
926
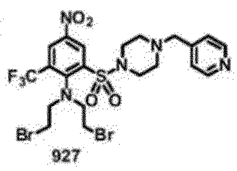
927
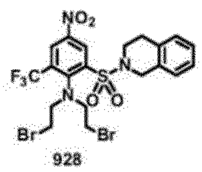
928
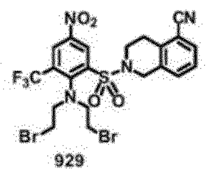
929
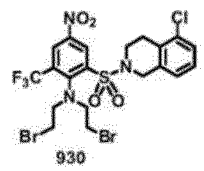
930

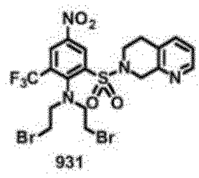
931
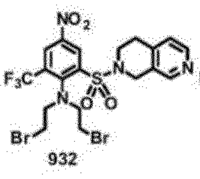
932
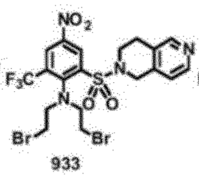
933
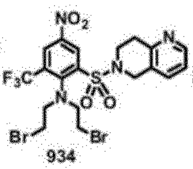
934
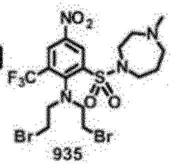
935
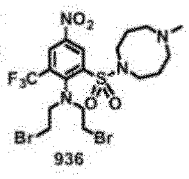
936

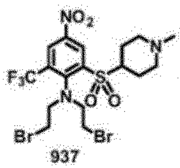
937
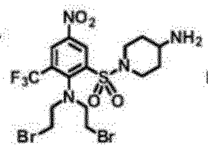
938
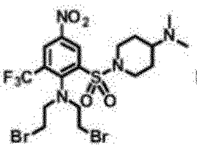
939
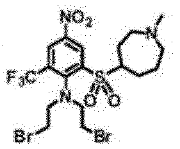
940
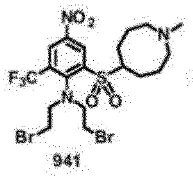
941
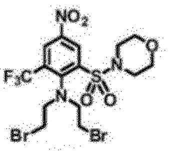
942

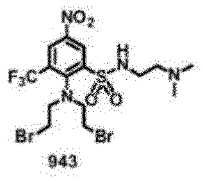
943
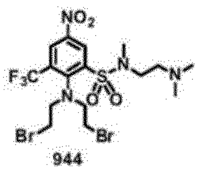
944
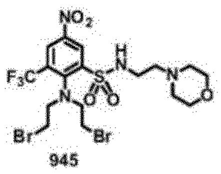
945
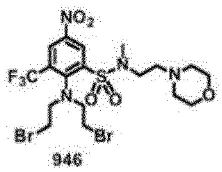
946
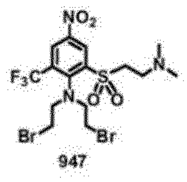
947

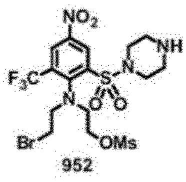
952
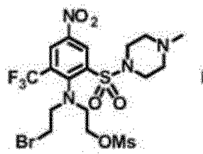
953
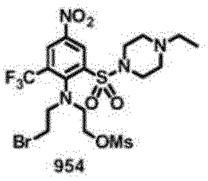
954
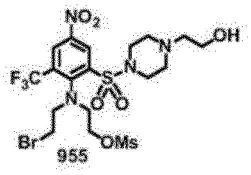
955
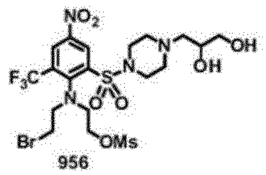
956

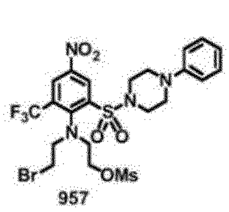
957
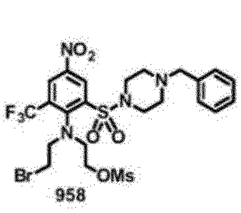
958
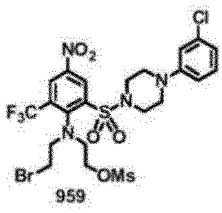
959
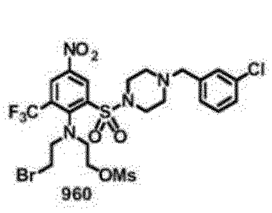
960

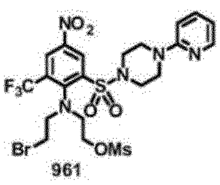
961
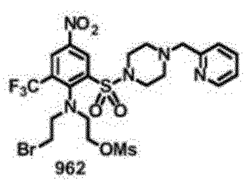
962
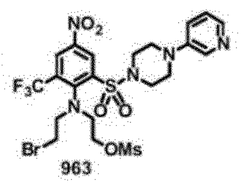
963
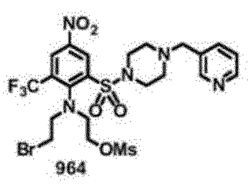
964

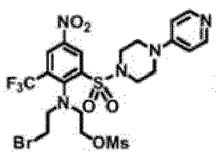
965
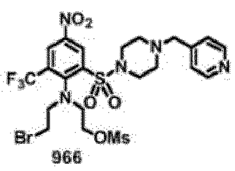
966
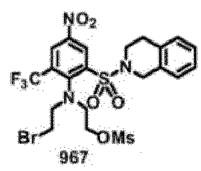
967
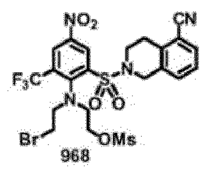
968
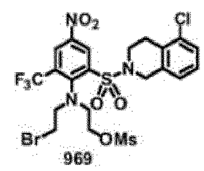
969

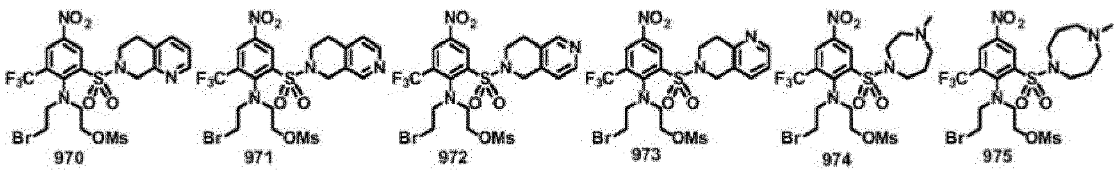

970 971 972 973 974 975

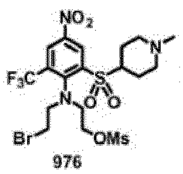
976
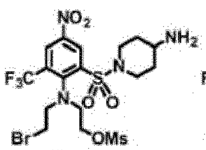
977
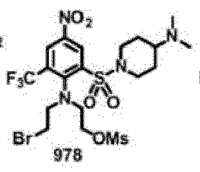
978
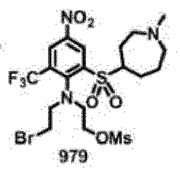
979
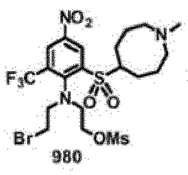
980
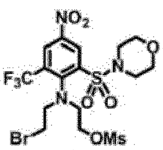
981

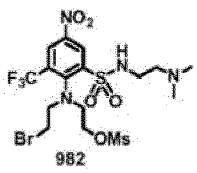
982
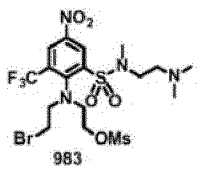
983
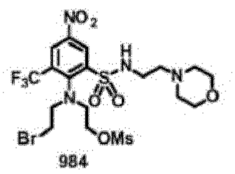
984
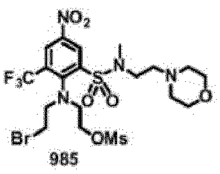
985
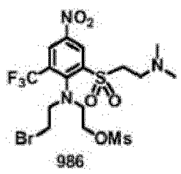
986

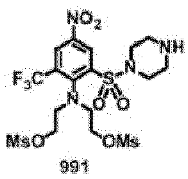
991
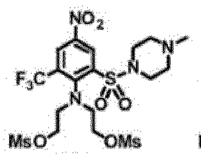
992
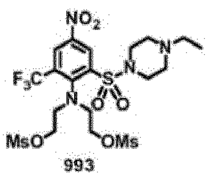
993
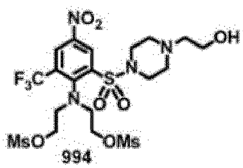
994
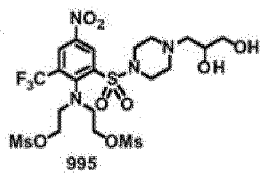
995

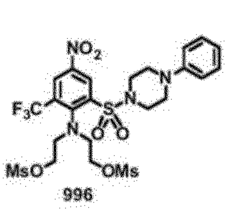
996
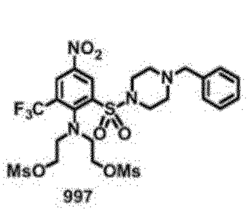
997
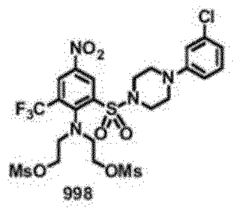
998
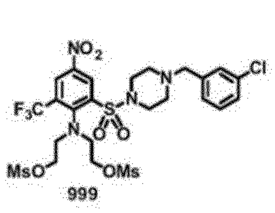
999

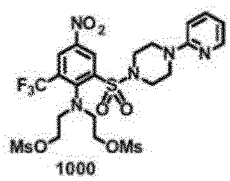
1000
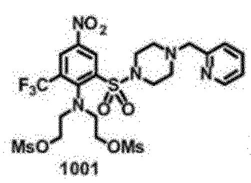
1001
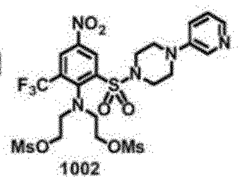
1002
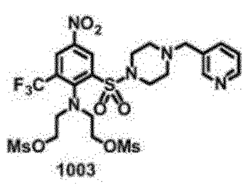
1003

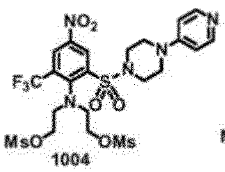
1004
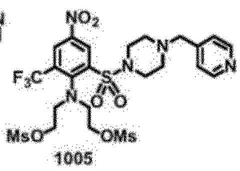
1005
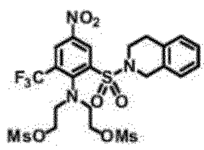
1006
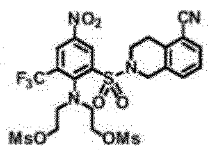
1007
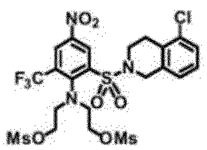
1008

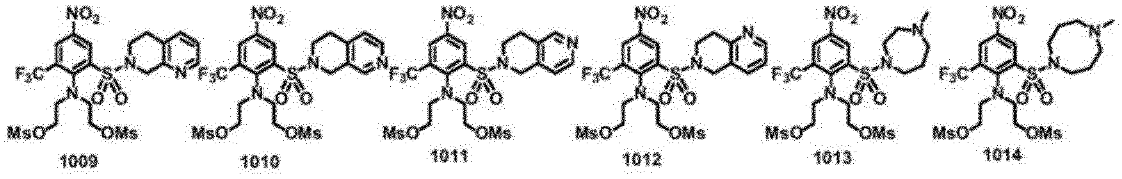

1009 1010 1011 1012 1013 1014

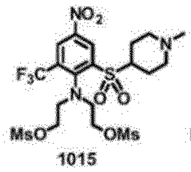
1015
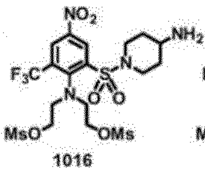
1016
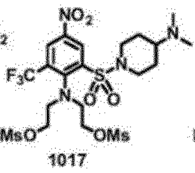
1017
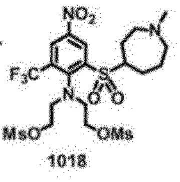
1018
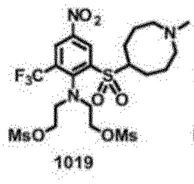
1019
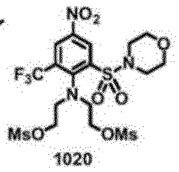
1020

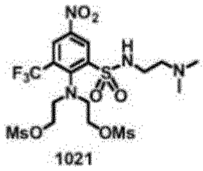
1021
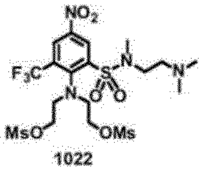
1022
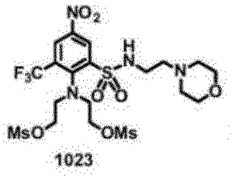
1023
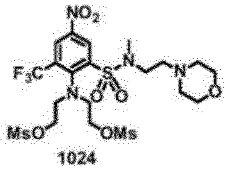
1024
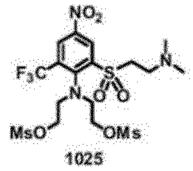
1025

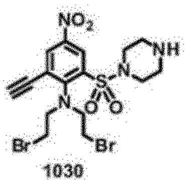
1030
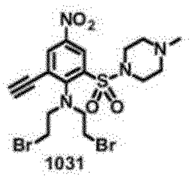
1031
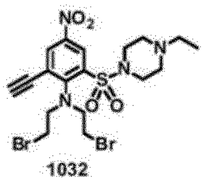
1032
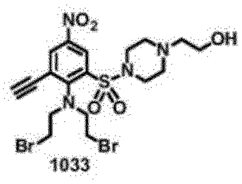
1033
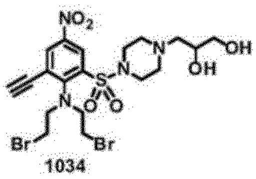
1034

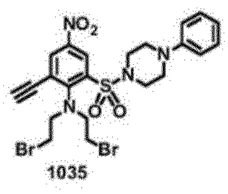
1035
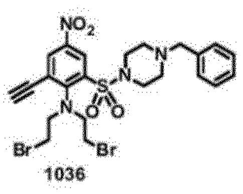
1036
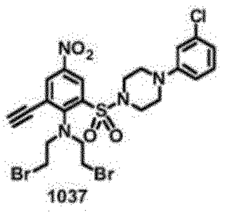
1037
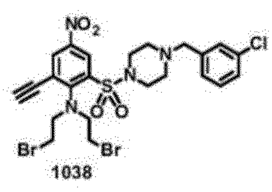
1038

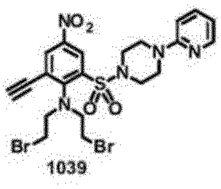
1039
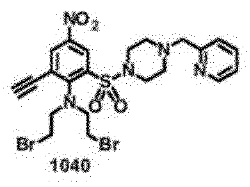
1040
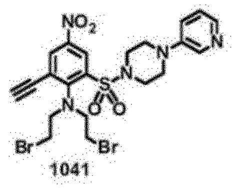
1041
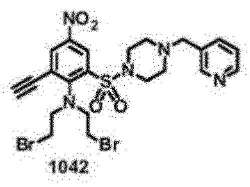
1042

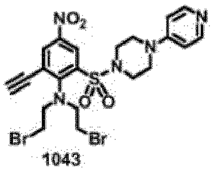
1043
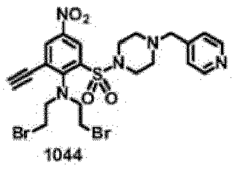
1044
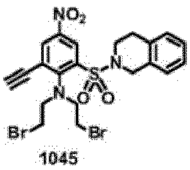
1045
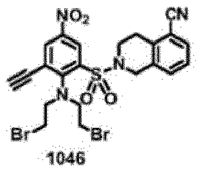
1046
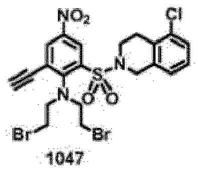
1047

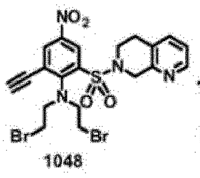
1048
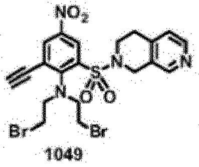
1049
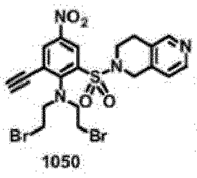
1050
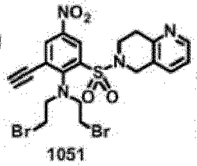
1051
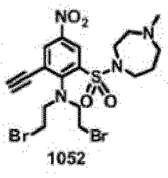
1052
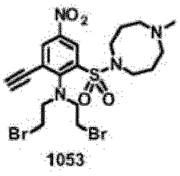
1053

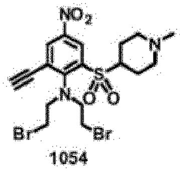
1054
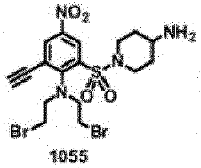
1055
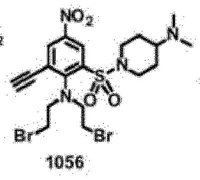
1056
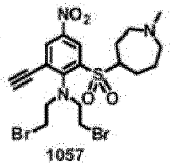
1057
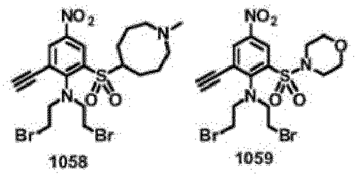
1058
1059

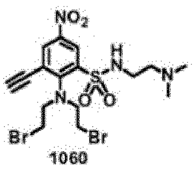
1060

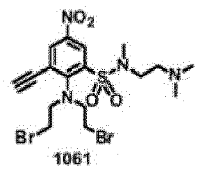
1061

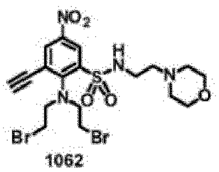
1062

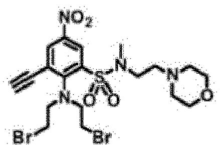
1063

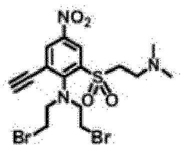
1064

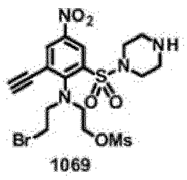
1069

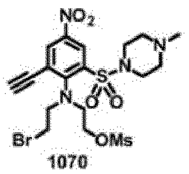
1070

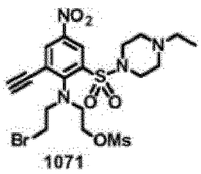
1071

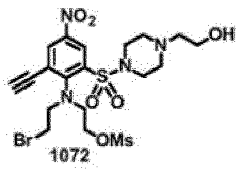
1072

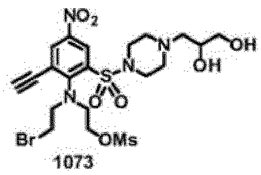
1073

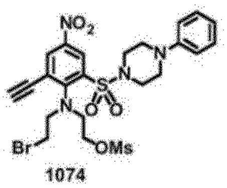
1074

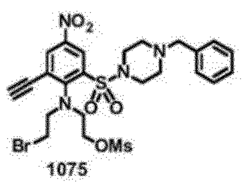
1075

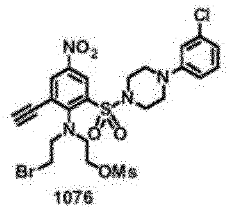
1076

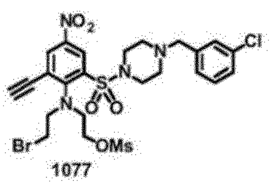
1077

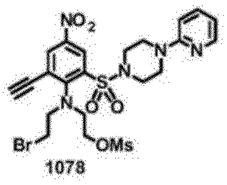
1078

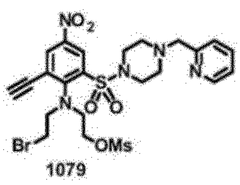
1079

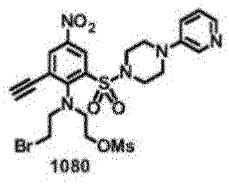
1080

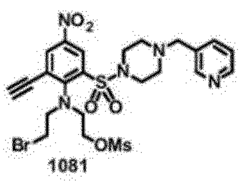
1081

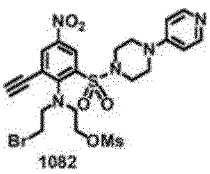
1082

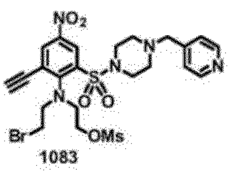
1083

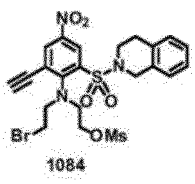
1084

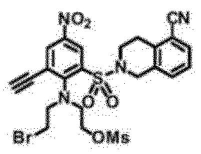
1085

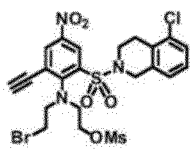
1086

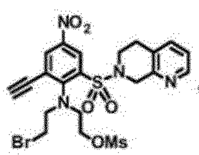
1087

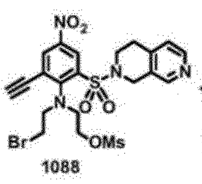
1088

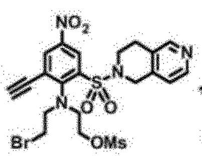
1089

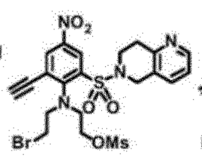
1090

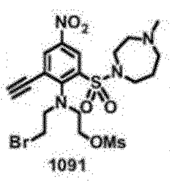
1091

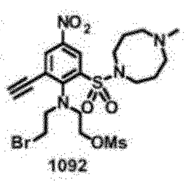
1092

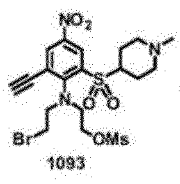
1093

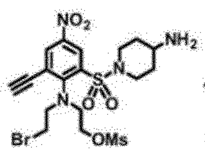
1094

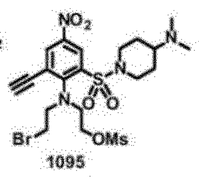
1095

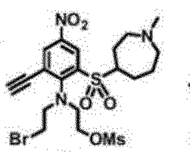
1096

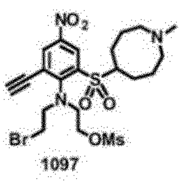
1097

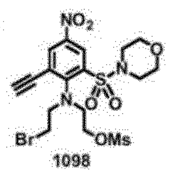
1098

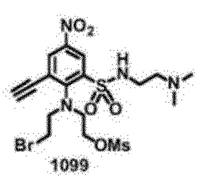
1099

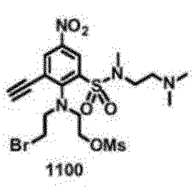
1100

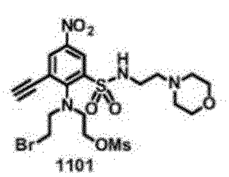
1101

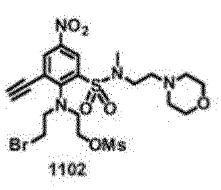
1102

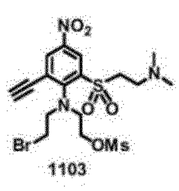
1103

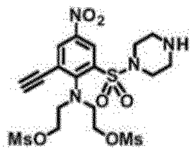
1108
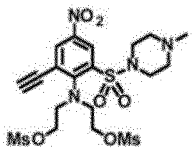
1109
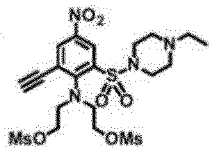
1110
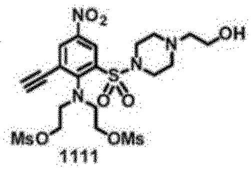
1111
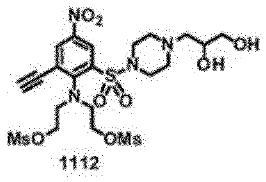
1112
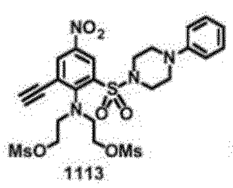
1113
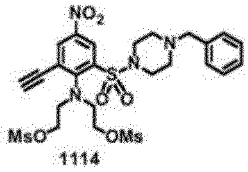
1114
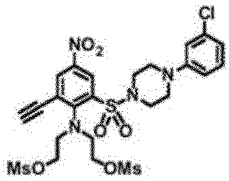
1115
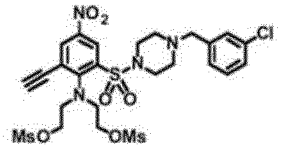
1116
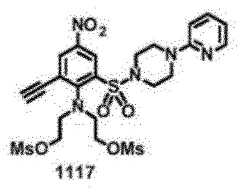
1117
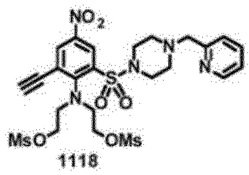
1118
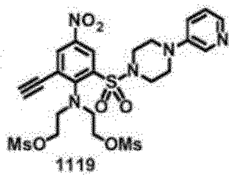
1119
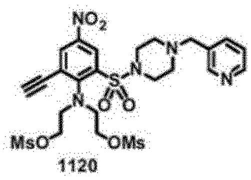
1120
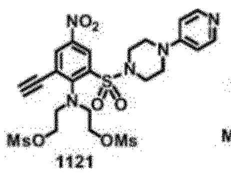
1121
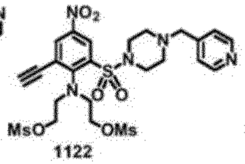
1122
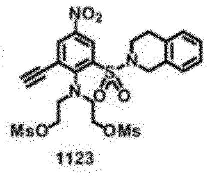
1123
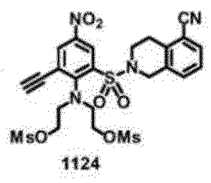
1124
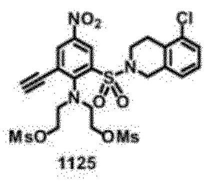
1125
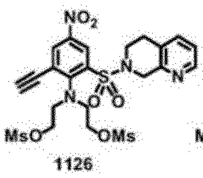
1126
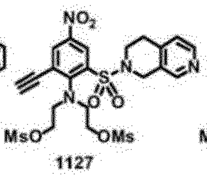
1127
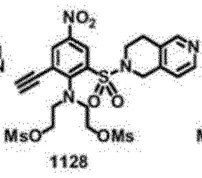
1128
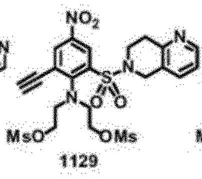
1129
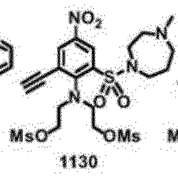
1130
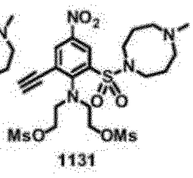
1131
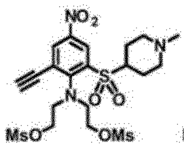
1132
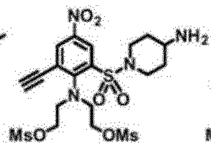
1133
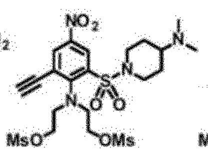
1134
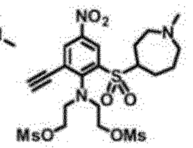
1135
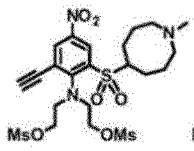
1136
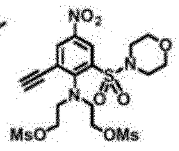
1137
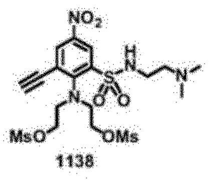
1138
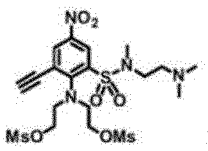
1139
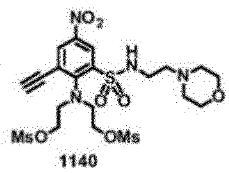
1140
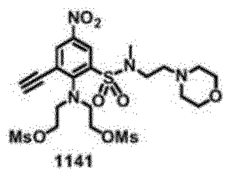
1141
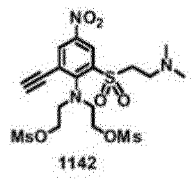
1142
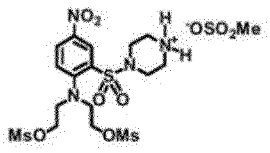
640.Ms
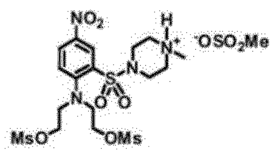
641.Ms
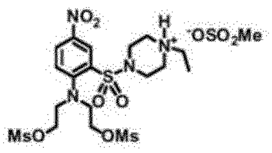
642.Ms
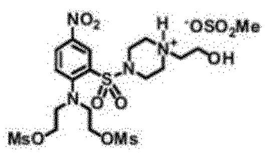
643.Ms

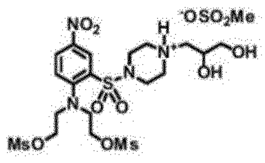
644.Ms

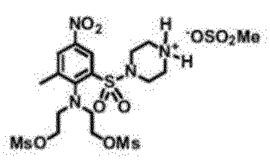
757.Ms

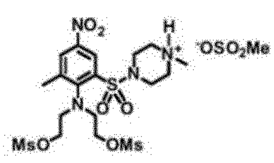
758.Ms

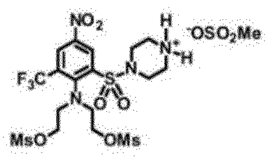
991.Ms

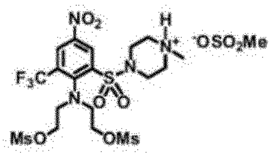
992.Ms

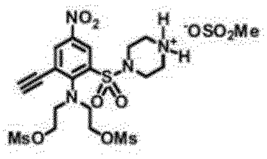
1108.Ms

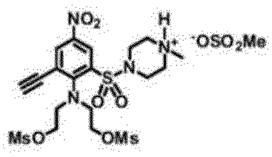
1109.Ms

Specific definitions and meanings of the groups, and the preparation methods and spectral data of the compounds have been described in Patent Application PCT/NZ2019/050030 with Publication No. WO2019190331A1 (corresponding to Chinese Patent Application No. 2019800234236 with Publication No. CN111918864A), which is incorporated herein by reference in its entirety.

The compounds of structural Formula (3), similar to AST-3424 or AST, are prodrugs of nitrogen mustard analogues

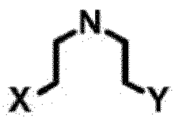

, and can be activated by the AKR1C3 enzyme to form nitrogen mustard analogues

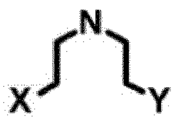

(a DNA alkylating agent) to exhibit its anti-cancer efficacy:

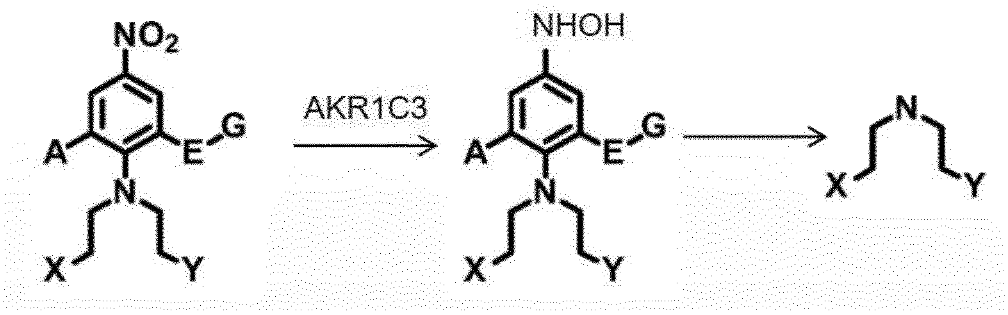


.

A treatment method which uses a drug monotherapy containing an AKR1C3-activated DNA alkylating agent prodrug compound or in combination with other therapeutic drugs for treating cancer and tumor patients having KRAS mutations, wherein the compound is selected from structural Formula (4) and salts, esters, solvates, and isotopic isomers thereof:

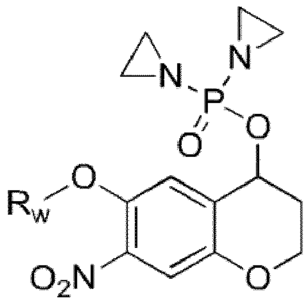

(4).

**Pharmaceutical use** of an AKR1C3-activated DNA alkylating agent prodrug compound, wherein the compound is used in the manufacture of a drug monotherapy or in combination with other therapeutic drugs for treating cancer and tumor patients having KRAS mutations, wherein the compound is selected from the structural Formula (4) and salts, esters, solvates, and isotopic isomers thereof:

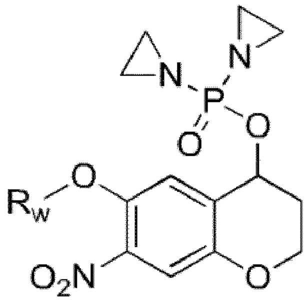

(4),

wherein the definition of Rw has been described in the claims of Patent Application PCT/CN2020/120281 with Publication No. WO2021068952A1. Specifically, the groups are defined as follows:

Rw is

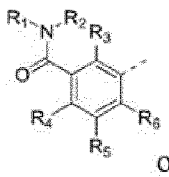

or

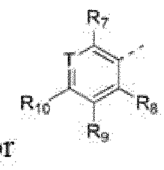

$R_1$ is H, $C_{1-6}$ alkyl, $C_{3-6}$ cycloalkyl, 4-6 membered heterocycloalkyl, 5-6 membered heteroaryl or phenyl, wherein the $C_{1-6}$ alkyl, $C_{3-6}$ cycloalkyl, 4-6 membered heterocycloalkyl, 5-6 membered heteroaryl and phenyl are optionally substituted with 1, 2 or 3 $R^a$;
each $R^a$ is independently H, F, Cl, Br, I, -CN, -OH, $C_{1-3}$ alkoxy or $C_{1-3}$ alkyl;
$R_2$ is H or $C_{1-6}$ alkyl;
or $R_1$ and $R_2$, together with the N atom to which they are attached, to form a 4-6 membered heterocycloalkyl, wherein the 4-6 membered heterocycloalkyl is optionally substituted with 1, 2 or 3 $R^b$;
each $R^b$ is independently H, F, Cl, Br, I, -CN, -OH, $-NH_2$, $-OCH_3$, $-OCH_2CH_3$, $-CH_3$ or $-CH_2CH_3$;
$R_3$ is H, F, Cl, Br, I, -OH, $-NH_2$, $C_{1-3}$ alkoxy or $C_{1-3}$ alkyl;
or $R_2$ and $R_3$ are attached together to make the structural unit

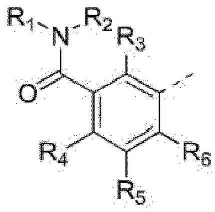

to be

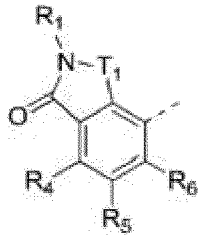,

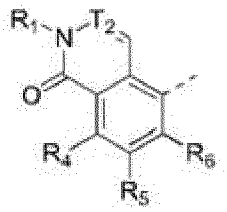

or

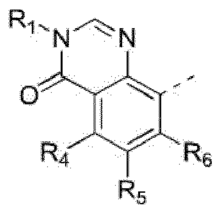.

$T_1$ is $-(CR^cR^d)_m-$ or $-(CR^cR^d)_n-O-$;
m is 1, 2 or 3;
n is 1 or 2;
$T_2$ is N or CH;
$R^c$ and $R^d$ are each independently H, F, $C_{1-3}$ alkyl or $C_{1-3}$ alkoxy;
$R_4$, $R_5$ and $R_6$ are each independently H, F, Cl, Br, I, $C_{1-3}$ alkyl or $C_{1-3}$ alkoxy;
T is N or CH;
$R_7$ and $R_8$ are each independently H, F, Cl, Br or I;
$R_9$ and $R_{10}$ are each independently H, F, Cl, Br, I, -CN or
the 4-6 membered heterocycloalkyl and 5-6 membered heteroaryl each contain 1, 2, 3 or 4 heteroatoms independently selected from N, -O- and -S-.

Specifically, the compound of Formula (4) is selected from the group consisting of:

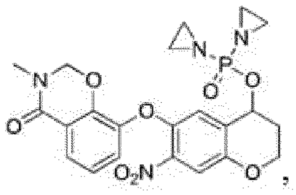,
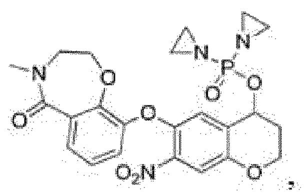,
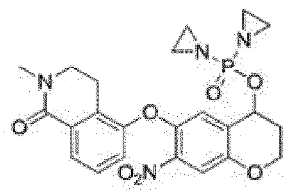,

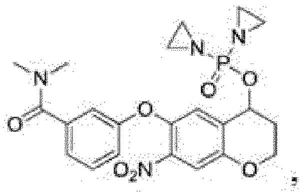,
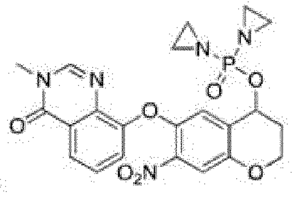,
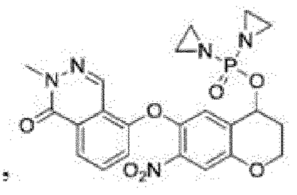,

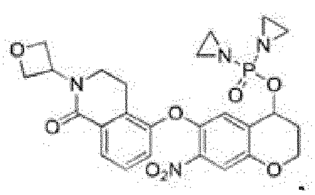,
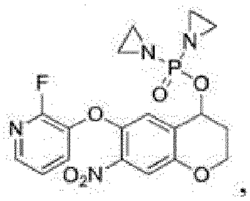,
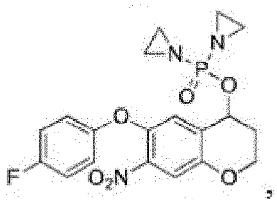,

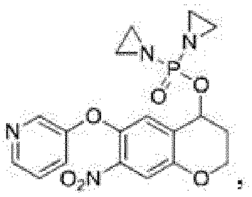, 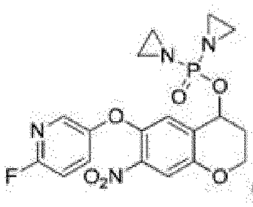 or 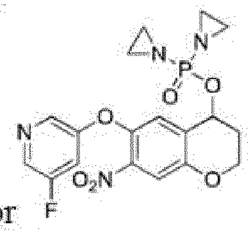

The definitions and meanings of the groups, and the preparation methods and spectral data of the compounds have been described in Patent Application PCT/CN2020/120281 with Publication No. WO2021068952A1, which is incorporated herein by reference in its entirety.

Evidently, the compounds of structural Formula (3), similar to AST-3424 or AST, are prodrugs of AST-2660, and can be activated by the AKR1C3 enzyme to form AST-2660 (a DNA alkylating agent) to exhibit its anti-cancer efficacy:

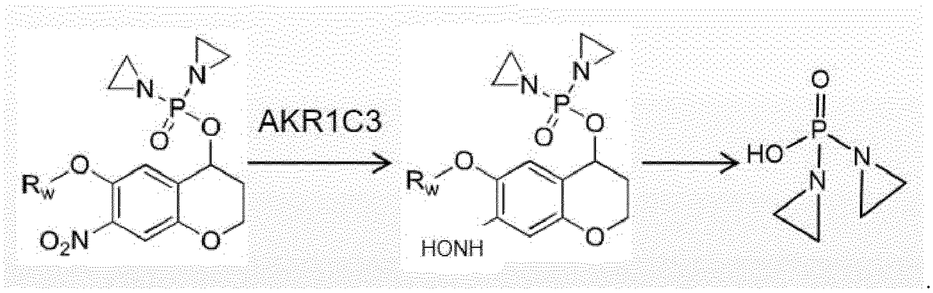

.

A treatment method which uses a drug monotherapy containing an AKR1C3-activated DNA alkylating agent prodrug compound or in combination with other therapeutic drugs for treating cancer and tumor patients having KRAS mutations, wherein the compound is selected from structural Formula (5) and salts, esters, solvates, and isotopic isomers thereof:

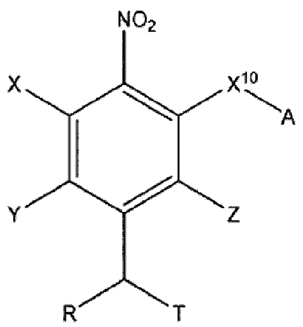
(5).

**Pharmaceutical use** of an AKR1C3-activated DNA alkylating agent prodrug compound, wherein the compound is used in the manufacture of a drug monotherapy or in combination with other therapeutic drugs for treating cancer and tumor patients having KRAS mutations, wherein the compound is selected from the structural Formula (5) and salts, esters, solvates, and isotopic isomers thereof:

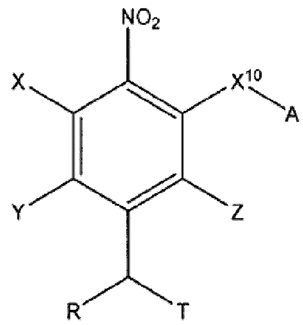

(5),

wherein the definitions of X, Y, Z, R, T, A and $X^{10}$ have been described in the claims of Patent Application PCT/US2016/062114 with Publication No. WO2017087428A1 (corresponding to Chinese Patent Application No. 2016800200132 with Publication No. CN108136214A).

Specifically, the groups are defined as follows:

$X^{10}$is O, S, SO or $SO_2$;
A is $C_6$-$C_{10}$ aryl, 5-15 memberred heteroaryl, or $-N=CR^1R^2$;
each $R_1$ and $R_2$ independently is hydrogen, $C_1$-$C_6$ alkyl, $C_3$-$C_8$ cycloalkyl, $C_6$-$C_{10}$ aryl, 4-15 membered heterocycle, ether, $-CONR^{13}R^{14}$, or $-NR^{13}COR^{14}$;
each X, Y, and Z independently is hydrogen, CN, halogeno, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, ether, $-CONR^{13}R^{14}$, or-$NR^{13}COR^{14}$;
R is hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, ether, $-CONR^{13}R^{14}$, or $-NR^{13}COR^{14}$;
each $R^{13}$ and $R^{14}$ independently is hydrogen, $C_1$-$C_6$ alkyl, $C_3$-$C_8$ cycloalkyl, $C_6$-$C_{10}$ aryl, 4-15 membered heterocycle, or ether;
T comprises a phosphoramidate alkylating agent comprising one or more $Z^5$-$X^5$-$Y^3$ moieties bonded to an $-O-P(Z^1)$ moiety, where $Z^5$ is a heteroatom such as nitrogen, sulfur or oxygen, $X^5$ is substituted or unsubstituted ethylene, $Y^5$ is halogeno or another leaving group, or $Z^5$-$X^5$-$Y^5$ together form an aziridinyl ($NCH_2CH_2$) moiety, and $Z^1$ is O or S; and
wherein the alkyl, alkenyl, alkynyl, cycloalkyl, aryl, heterocycle, heteroaryl, and ether groups are substituted or unsubstituted.

Specifically, the compound of Formula (5) is selected from the group consisting of:

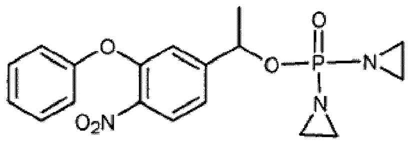
,
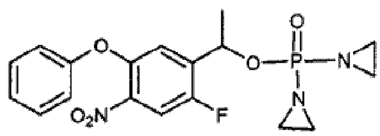
,

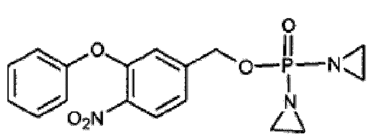
,
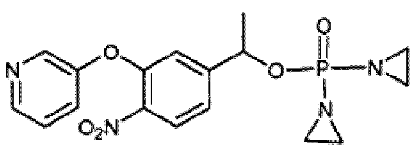
,

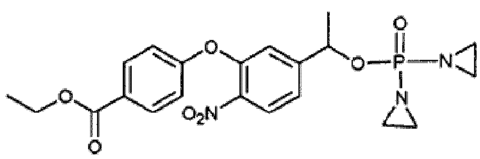
,
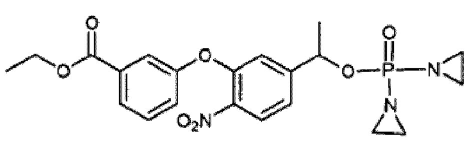
,

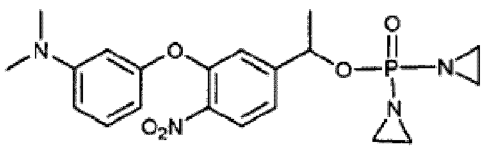 , 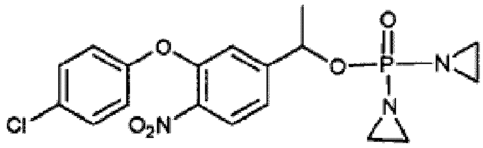 ,

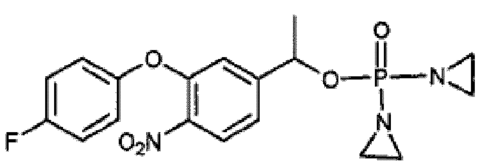 , 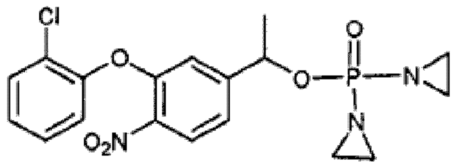 ,

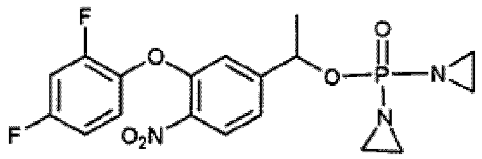 , 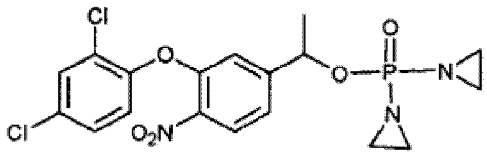 ,

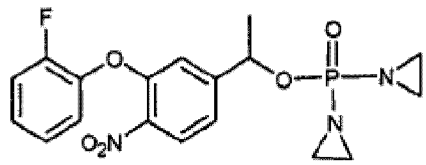 , 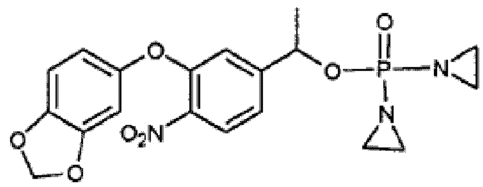 ,

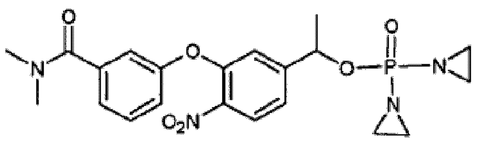 , 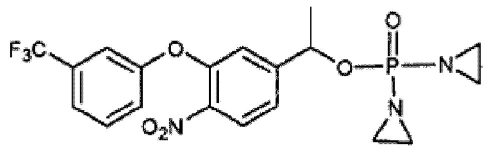 ,

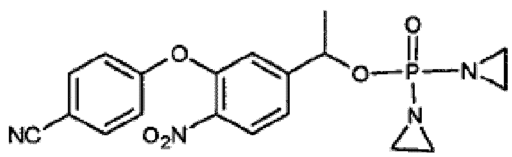 , 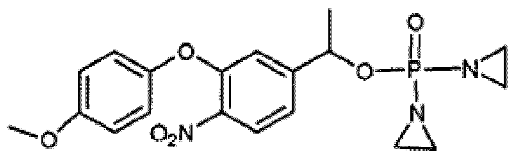 ,

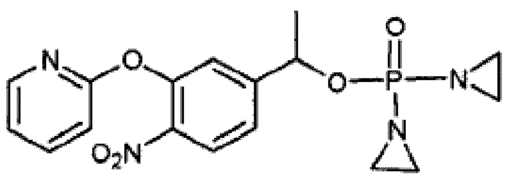 , 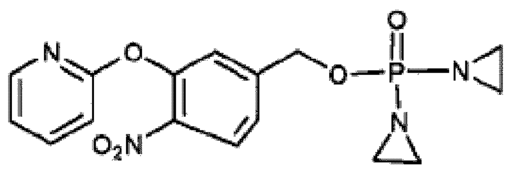 ,

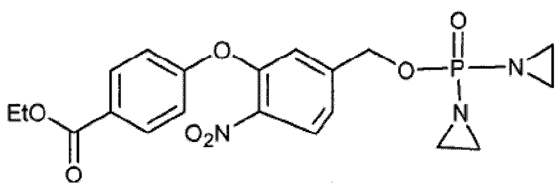 , 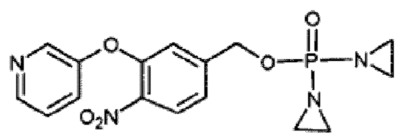 ,

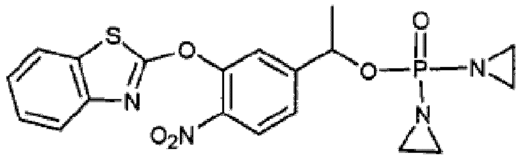,
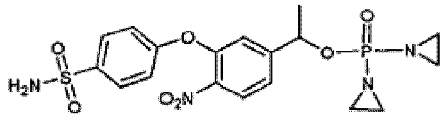,

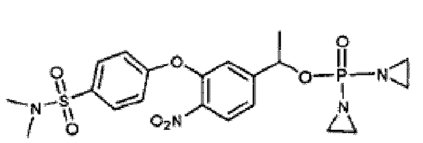,
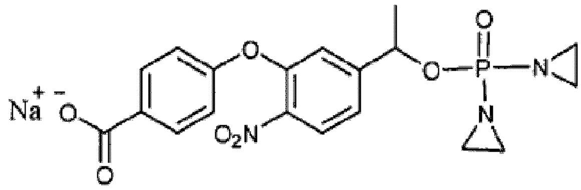,

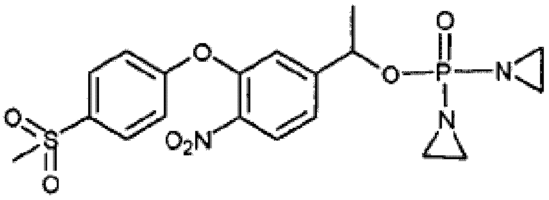,
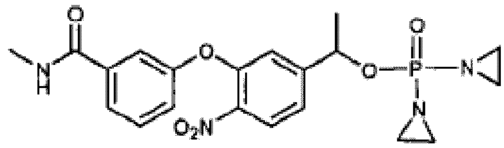,

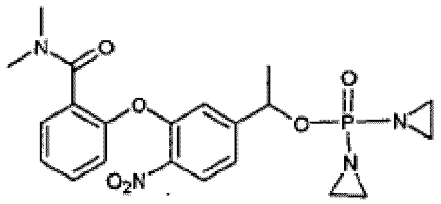,
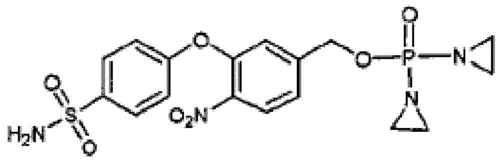,

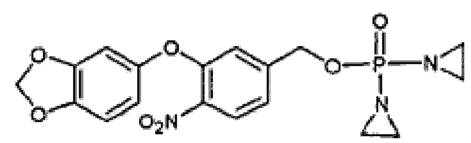,
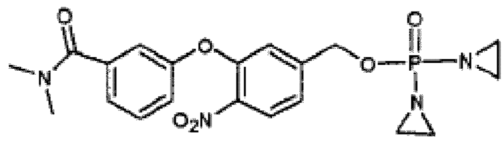,

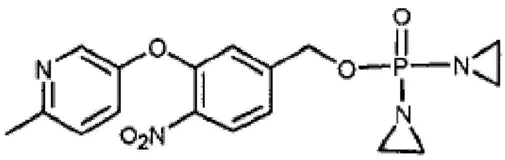,
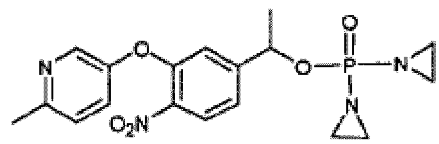,

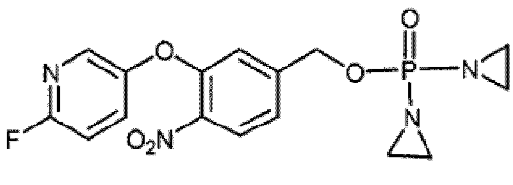,
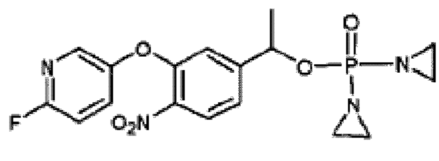,

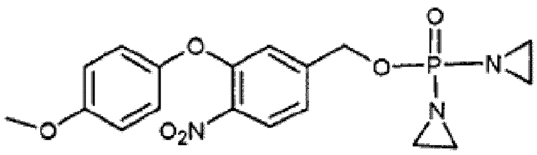,
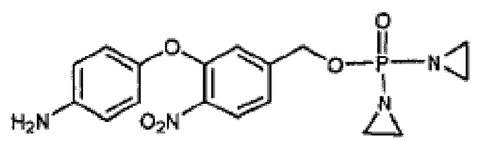,

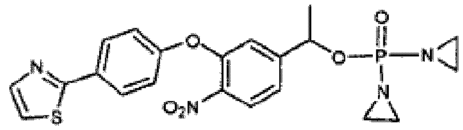,
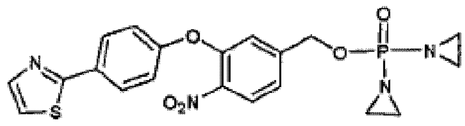,

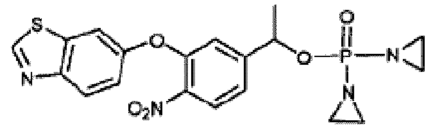,
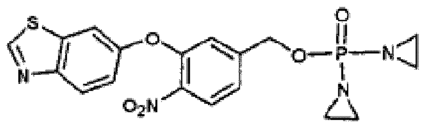,

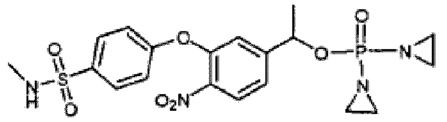,
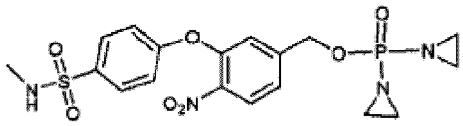,

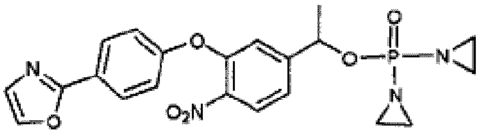,
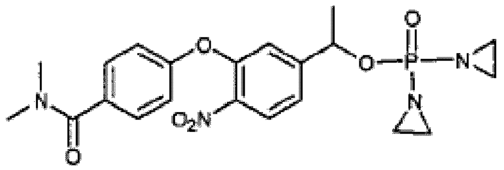,

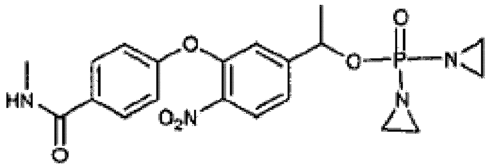,
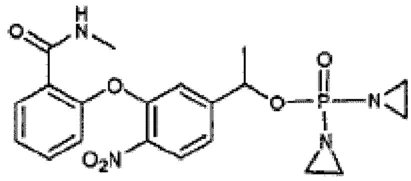,

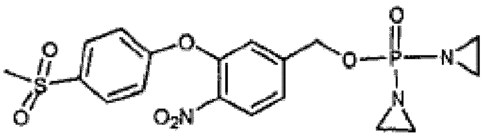,
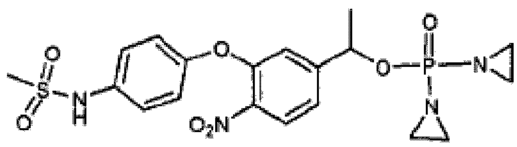,

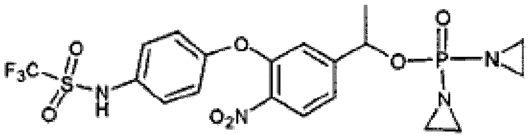,
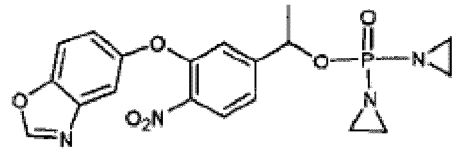,

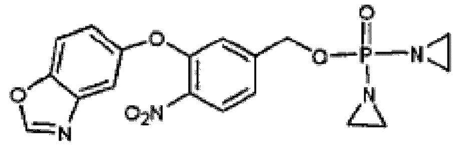,
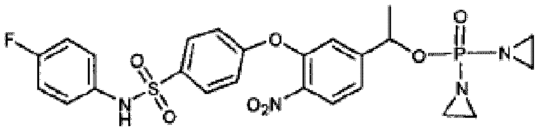,

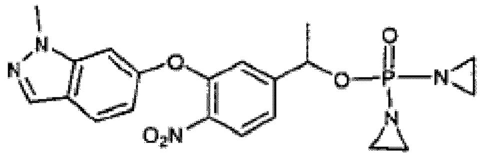
,
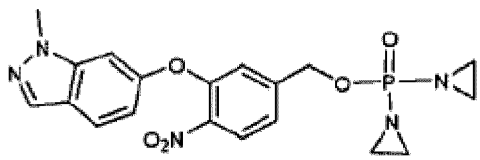
,
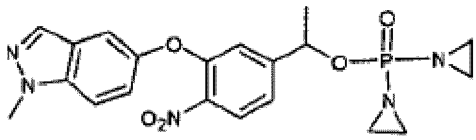
,
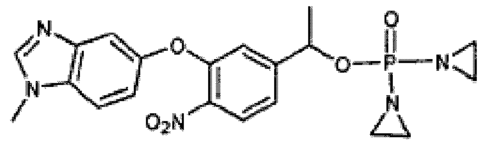
,
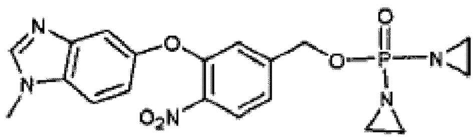
,
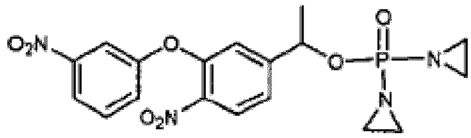
,
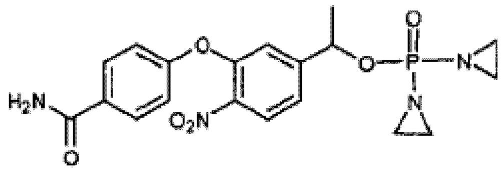
,
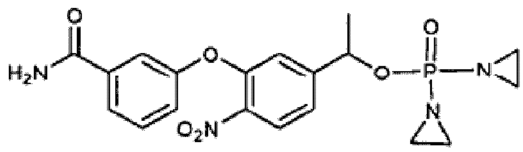
,
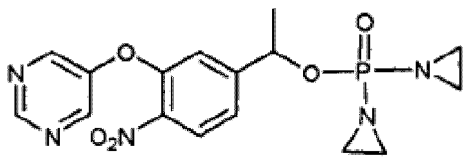
,
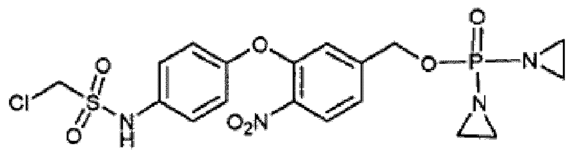
,
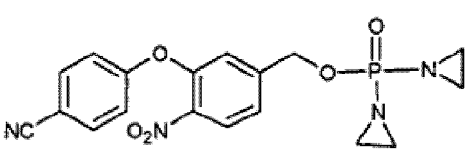
,
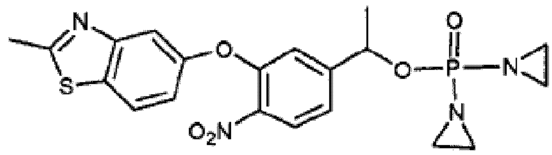
,
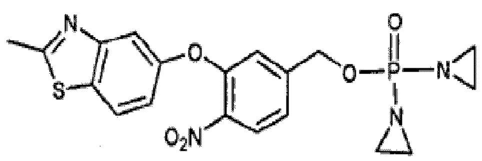
,
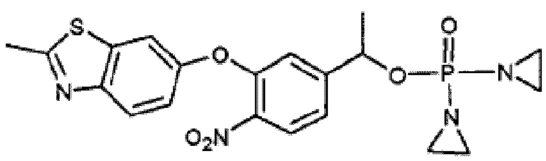
,
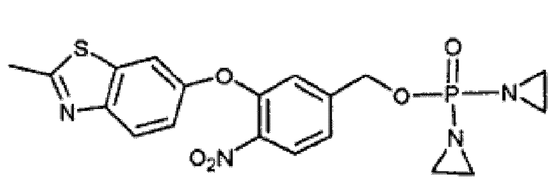
,
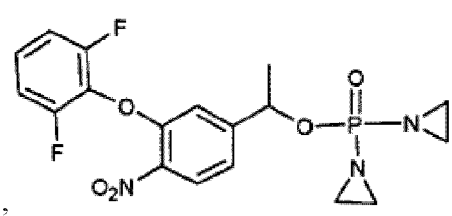
,

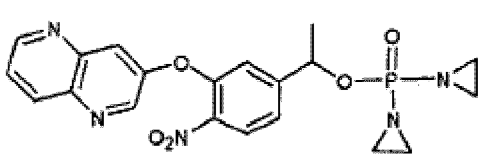,
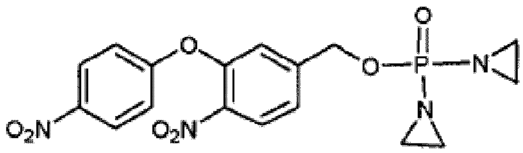,

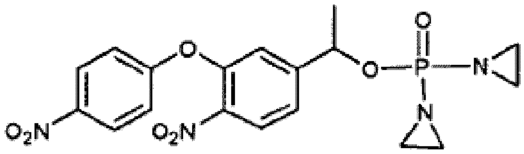,
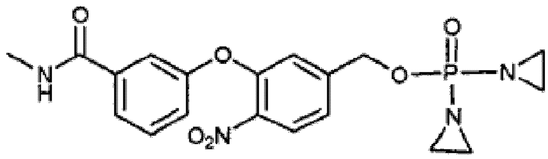,

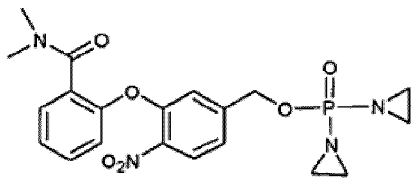,
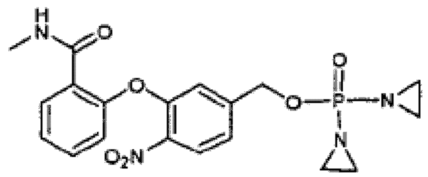,

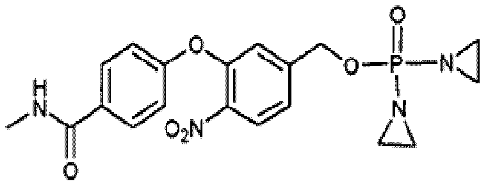,
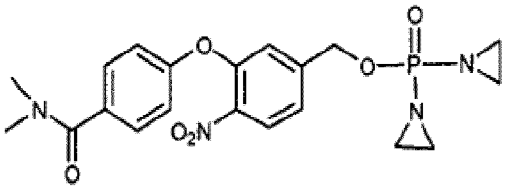,

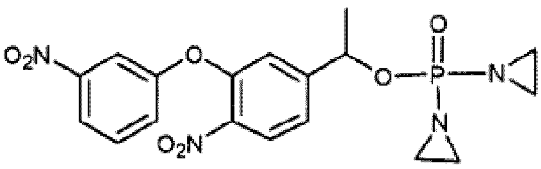,
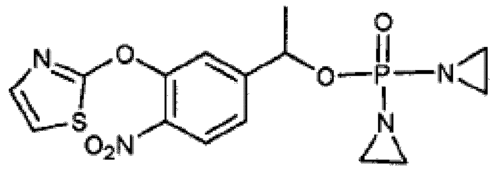,

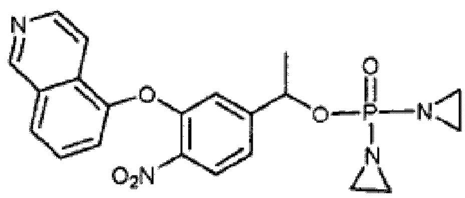,
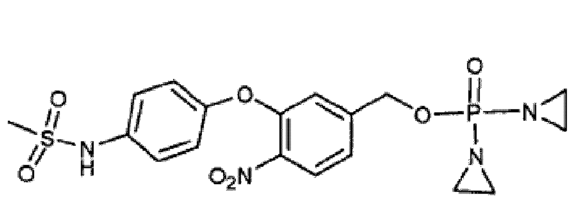,

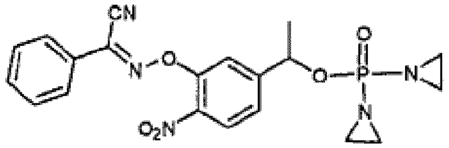,
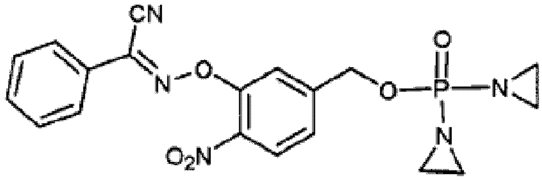,

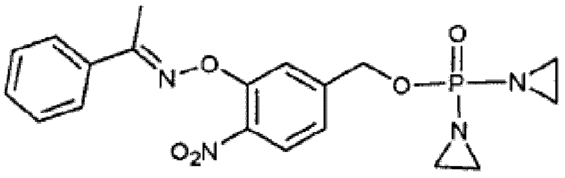,
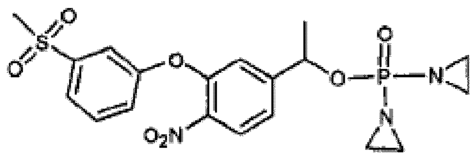,

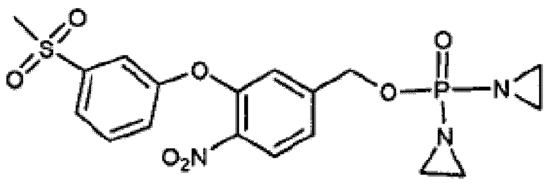,
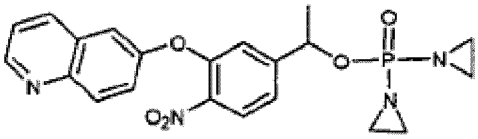,

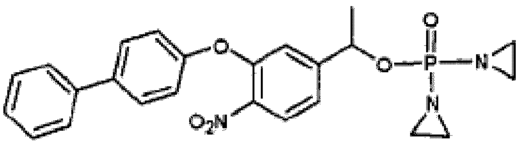,
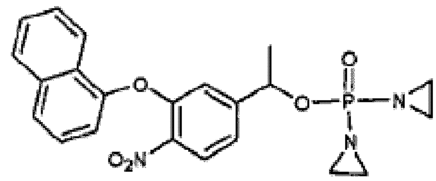,

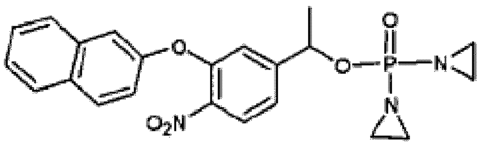,
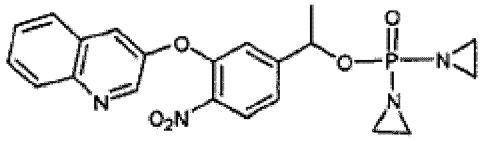,

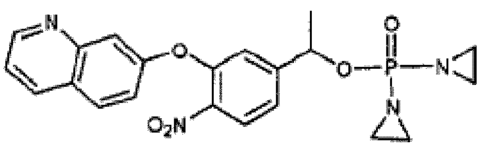,
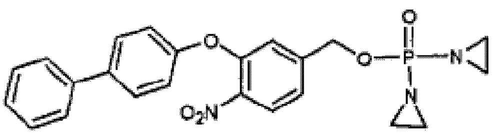,

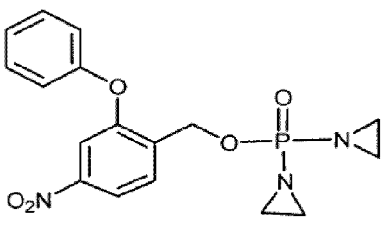,
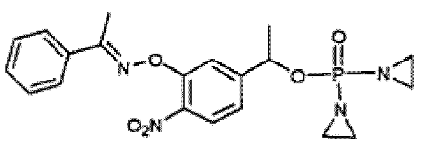,

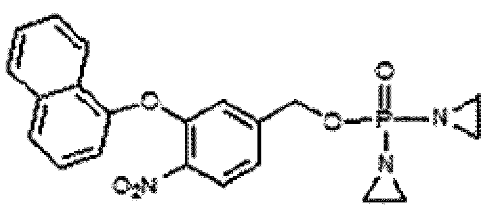,
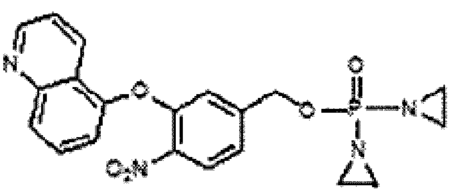,

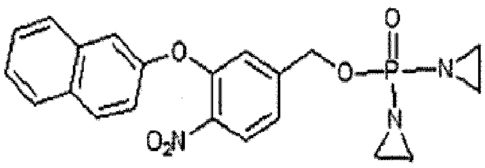,
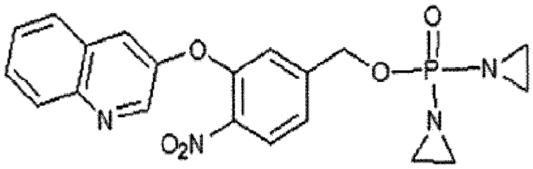,
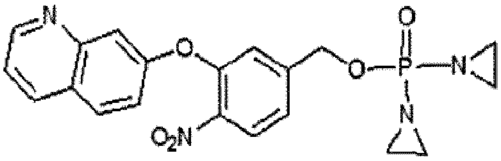,
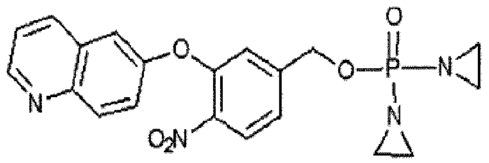,
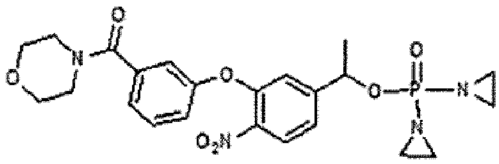,
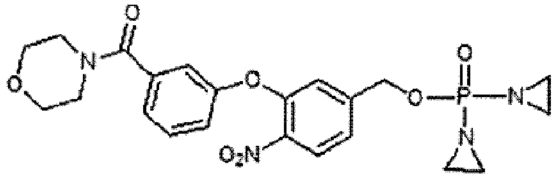,
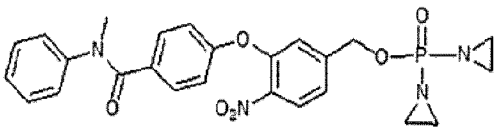,
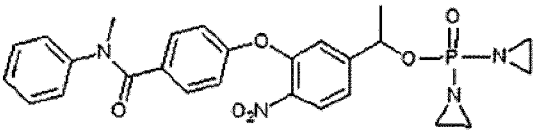,
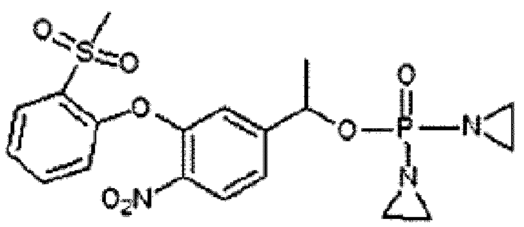,
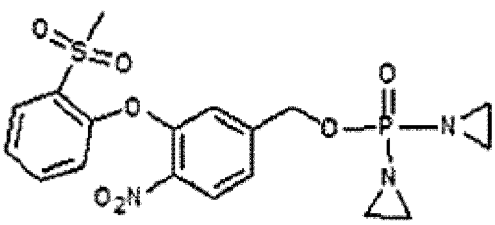,
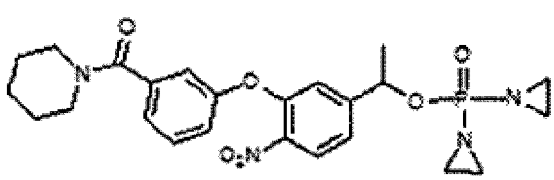,
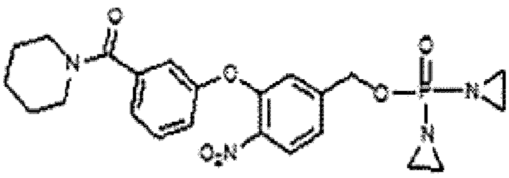,
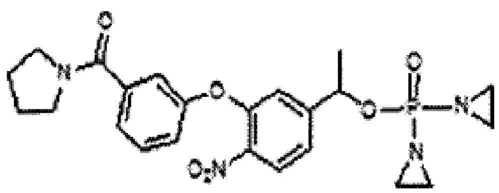,
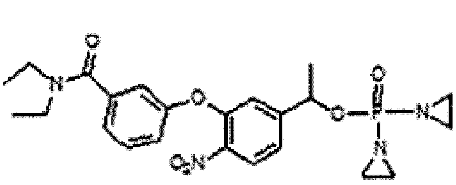,

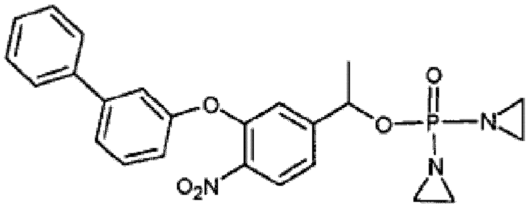 , 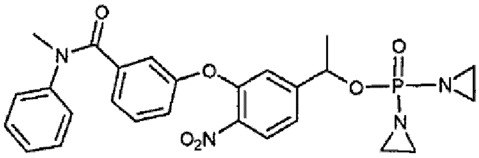 ,

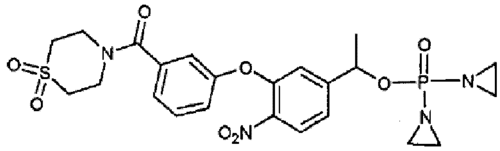 , 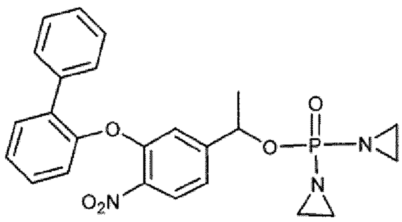 ,

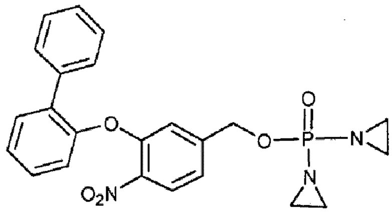 , 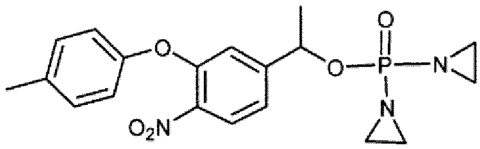 ,

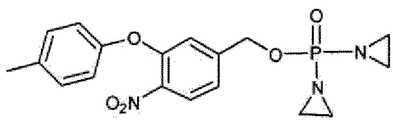 , 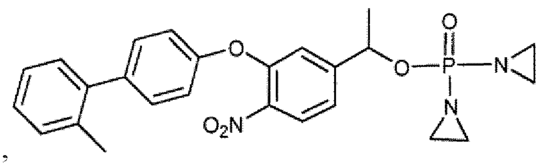 ,

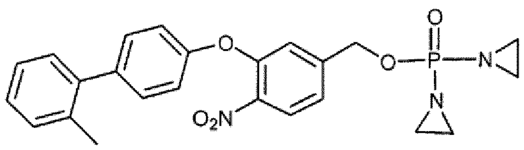 , 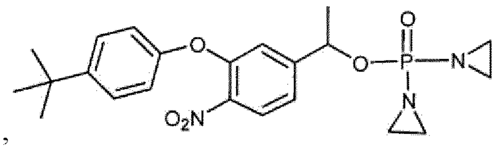 ,

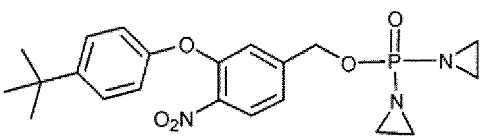 , 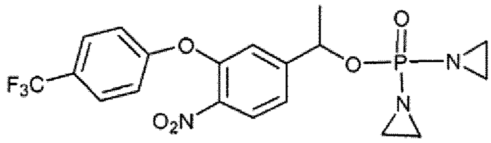 ,

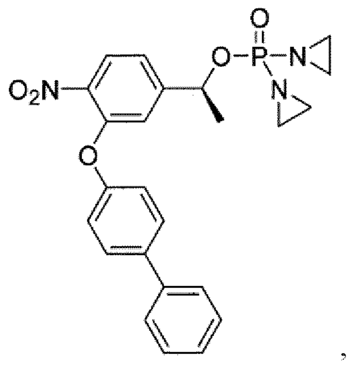 , 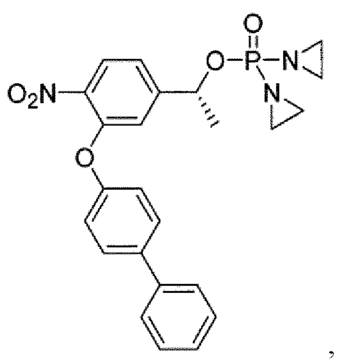 ,

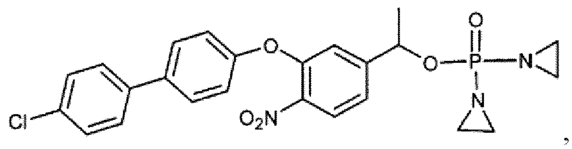 , 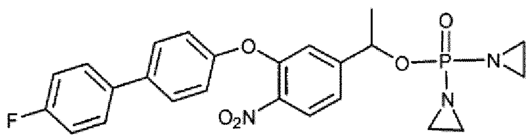 ,

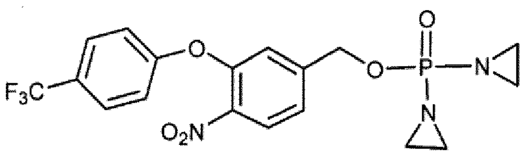 and 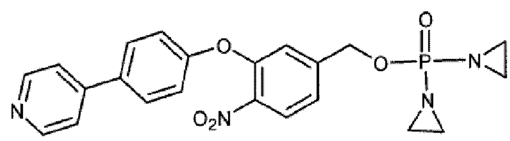 .

Specific definitions and meanings of the groups, and the preparation methods and spectral data of the compounds have been described in Patent Application PCT/US2016/062114 with Publication No. WO2017087428A1 (corresponding to Chinese Patent Application No. 2016800200132 with Publication No. CN108136214A), which is incorporated herein by reference in its entirety.

Evidently, the compounds of structural Formula (5), similar to AST-3424 or AST, are prodrugs of a phosphoramidate alkylating agent, and can be activated by the AKR1C3 enzyme to form T (a phosphoramidate alkylating agent, AST-2660 is a phosphoramidate alkylating agent) to exert its anti-cancer efficacy:

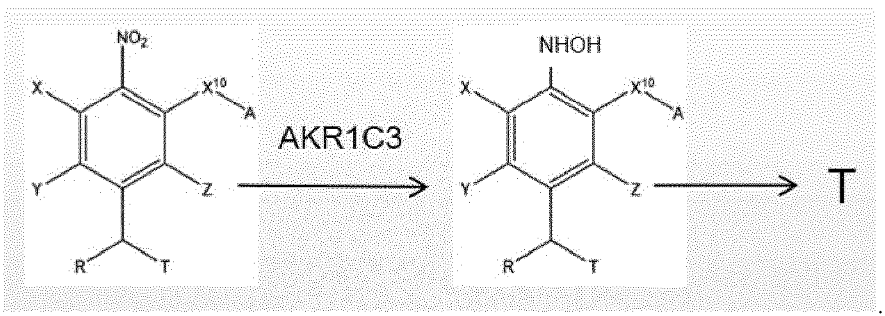

.

A treatment method which uses a drug monotherapy containing an AKR1C3-activated DNA alkylating agent prodrug compound or in combination with other therapeutic drugs for treating cancer and tumor patients having KRAS mutations, wherein the compound is selected from structural Formula (6) and salts, esters, solvates, and isotopic isomers thereof:

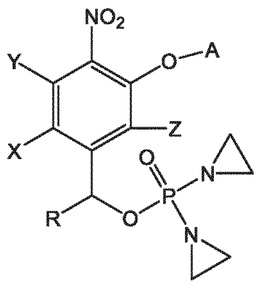

(6).

**Pharmaceutical use** of an AKR1C3-activated DNA alkylating agent prodrug compound, wherein the compound is used in the manufacture of a drug monotherapy or in combination with other therapeutic drugs for treating cancer and tumor patients having KRAS mutations, wherein the compound is selected from the structural Formula (6) and salts, esters, solvates, and isotopic isomers thereof:

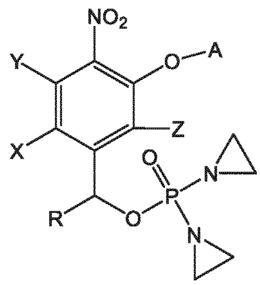

(6)

wherein,

A is substituted or unsubstituted $C_6$-$C_{10}$ aryl, biaryl or substituted biaryl, 5-15 membered heteroaryl, or -N=CR$^1$R$^2$; wherein the substituents are selected from the group consisting of halogeno, -CN, -$NO_2$, -O-($CH_2$)-O-, -$CO_2$H and salt thereof, -OR$^{100}$, -$CO_2$R$^{100}$, -CONR$^{101}$R$^{102}$, -NR$^{101}$R$^{102}$, -NR$^{100}$$SO_2$R$^{100}$, -$SO_2$R$^{100}$, -$SO_2$NR$^{101}$R$^{102}$, $C_1$-$C_6$ alkyl, and $C_3$-$C_{10}$ heterocyclyl;

wherein R$^{100}$, R$^{101}$ and R$^{102}$ are each independently hydrogen, $C_1$-$C_8$ alkyl, or $C_6$-$C_{12}$ aryl; or R$^{101}$ and R$^{102}$ together with the nitrogen atom to which they are attached to form a 5-7 membered heterocycle;

wherein the alkyl group and the aryl group are each substituted by 1-3 halogeno groups or 1-3 $C_1$-$C_6$ alkyl groups;

$R_1$ and $R_2$ are each independently phenyl or methyl;

X, Y and Z are each independently hydrogen or halogeno; and

R is hydrogen or $C_1$-$C_6$ alkyl or halogen-substituted alkyl.

Evidently, the "compound" also includes the compound itself as well as solvate, salt, ester or isotopic isomer thereof.

"Cx-Cy" or "Cx-y" before a group refers to a range of the number of carbon atoms that are present in that group. For example, $C_1$-$C_6$ alkyl refers to an alkyl group having at least 1 and up to 6 carbon atoms.

"Alkyl" refers to monovalent saturated aliphatic hydrocarbyl groups having from 1 to 10 carbon atoms and, in some embodiments, from 1 to 6 carbon atoms. "Cx-y alkyl" refers to alkyl groups having from x to y carbon atoms. This term includes (by way of example) linear and branched hydrocarbyl groups such as methyl ($CH_3$-), ethyl ($CH_3CH_2$-), n-propyl ($CH_3CH_2CH_2$-), isopropyl (($CH_3$)$_2$CH-), n-butyl ($CH_3CH_2CH_2CH_2$-), isobutyl(($CH_3$)$_2$CHCH$_2$-), sec-butyl (($CH_3$)($CH_3CH_2$)CH-), t-butyl (($CH_3$)$_3$C-), n-pentyl ($CH_3CH_2CH_2CH_2CH_2$-), and neopentyl (($CH_3$)$_3$CCH$_2$-).

"Aryl" refers to an aromatic group having from 6 to 14 carbon atoms and no ring heteroatoms and having a single ring (e.g., phenyl) or multiple condensed (fused) rings (e.g., naphthyl or anthryl). For multiple ring systems, including fused, bridged, and spiro ring systems having aromatic and non-aromatic rings that have no ring heteroatoms, the term "Aryl" or "Ar" applies when the point of attachment is at an aromatic carbon atom (e.g., 5,6,7,8 tetrahydronaphthalene-2-yl is an aryl group as its point of attachment is at the 2-position of the aromatic phenyl ring). "Arylene" refers to a divalent aryl radical having the appropriate hydrogen content.

"Cycloalkyl" refers to a saturated or partially saturated cyclic group having from 3 to 14 carbon atoms and no ring heteroatoms and having a single ring or multiple rings including fused, bridged, and spiro ring systems. For multiple ring systems having aromatic and non- aromatic rings that have no ring heteroatoms, the term "cycloalkyl" applies when

the point of attachment is at a non-aromatic carbon atom (e.g., 5,6,7,8,-tetrahydronaphthalene-5-yl). The term "cycloalkyl" includes cycloalkenyl groups. Examples of cycloalkyl groups include, for instance, adamantyl, cyclopropyl, cyclobutyl, cyclopentyl, cyclooctyl, and cyclohexenyl. "Cycloalkylene" refers to a divalent cycloalkyl radical having the appropriate hydrogen content.

"Halogeno" refers to one or more of fluoro, chloro, bromo, and iodo.

"Heteroaryl" refers to an aromatic group having from 1 to 14 carbon atoms and 1 to 6 heteroatoms selected from the group consisting of oxygen, nitrogen, and sulfur and includes single ring (e.g. imidazolyl-2-yl and imidazole-5-yl) and multiple ring systems (e.g. imidazopyridyl, benzotriazolyl, benzimidazol-2-yl and benzimidazol-6-yl). For multiple ring systems, including fused, bridged, and spiro ring systems having aromatic and non-aromatic rings, the term "heteroaryl" applies if there is at least one ring heteroatom, and the point of attachment is at an atom of an aromatic ring (e.g., 1,2,3,4-tetrahydroquinolin-6-yl and 5,6,7,8- tetrahydroquinolin-3-yl). In some embodiments, the nitrogen and/or the sulfur ring atom(s) of the heteroaryl group are optionally oxidized to provide N-oxide ($N \rightarrow O$), sulfinyl, or sulfonyl moieties. The term heteroaryl includes, but is not limited to, acridinyl, azocinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, benzothienyl, benzimidazolinyl, carbazolyl, NH-carbazolyl, carbolinyl, chromanyl, chromenyl, cinnolinyl, dithiazinyl, furanyl, furazanyl, imidazolidinyl, imidazolinyl, imidazopyridyl, imidazolyl, indazolyl, indolenyl, indolinyl, indolizinyl, indolyl, isobenzofuranyl, isochromanyl, isoindazolyl, isoindolinyl, isoindolyl, isoquinolinyl, isoquinolyl, isothiazolyl, isoxazolyl, naphthyridinyl, octahydroisoquinolinyl, oxadiazolyl, oxazolidinyl, oxazolyl, pyrimidinyl, phenanthridinyl, phenanthrolinyl, phenazinyl, phenothiazinyl, phenoxathiinyl, phenoxazinyl, phthalazinyl, piperazinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazole, pyridinyl, pyridyl, pyrimidinyl, pyrrolyl, quinazolinyl, quinolinyl, quinoxalinyl, quinuclidinyl, tetrahydroisoquinolinyl, tetrahydroquinolinyl, tetrazolyl, thiadiazinyl, thiadiazolyl, thianthrenyl, thiazolyl, thienyl, thienothiazolyl, thienooxazolyl, thienoimidazolyl, thiophenyl, triazinyl and xanthenyl. "Heteroarylene" refers to a divalent heteroaryl radical having the appropriate hydrogen content.

"Heterocyclic" or "heterocycle" or "heterocycloalkyl" or "heterocyclyl" refers to a saturated or partially saturated cyclic group having from 1 to 14 carbon atoms and from 1 to 6 heteroatoms selected from the group consisting of nitrogen, sulfur, or oxygen and includes single ring and multiple ring systems including fused, bridged, and spiro ring systems. For multiple ring systems having aromatic and/or non-aromatic rings, the terms "heterocycle", "heterocycle", "heterocycloalkyl" or "heterocyclyl" apply when there is at least one ring heteroatom, and the point of attachment is at an atom of a non-aromatic ring (e.g. 1,2,3,4-tetrahydroquinoline-3-yl, 5,6,7,8-tetrahydroquinoline-6-yl, and decahydroquinolin-6-yl). In some embodiment, the heterocyclic groups herein are 3-15 membered, 4-14 membered, 5-13 membered, 7-12, or 5-7 membered heterocycles. In some other embodiment, the heterocycles contain 4 heteroatoms. In some other embodiment, the heterocycles contain 3 heteroatoms. In another embodiment, the heterocycles contain up to 2 heteroatoms. In some embodiments, the nitrogen and/or sulfur atom(s) of the heterocyclic group are optionally oxidized to provide the N-oxide, sulfinyl, sulfonyl moieties. Heterocyclyl includes, but is not limited to, tetrahydropyranyl, piperidinyl, N-methylpiperidin-3-yl, piperazinyl, N-methylpyrrolidin-3-yl, 3-pyrrolidinyl, 2-pyrrolidon-1-yl, morpholinyl, and pyrrolidinyl. A prefix indicating the number of carbon atoms (e.g., $C_{3-10}$) refers to the total number of carbon atoms in the portion of the heterocyclyl group exclusive of the number of heteroatoms. A divalent heterocyclic radical will have the appropriately adjusted hydrogen content.

"Biaryl" refers to a structure in which two aromatic rings are linked by a C-C single bond, such as biphenyl, bipyridine, and the like.

The term "optionally substituted" refers to a substituted or unsubstituted group. The group may be substituted with one or more substituents, such as e.g., 1, 2, 3, 4 or 5 substituents. Preferably, the substituents are selected from the group consisting of oxo, halogeno, -CN, $NO_2$, $-N_2+$, $-CO_2R^{100}$, $-OR^{100}$, $-SR^{100}$, $-SOR^{100}$, $-SO_2R^{100}$, $-NR^{100}SO_2R^{100}$, $-NR^{101}R^{102}$, $-CONR^{101}R^{102}$, $-SO_2NR^{101}R1^{02}$, $C_1$-$C_6$ alkyl, $C_1$-$C_6$ alkoxy, $-CR^{100} = C(R^{100})_2$, $-CCR^{100}$, $C_3$-$C_{10}$ cycloalkyl, $C_3$-$C_{10}$ heterocyclyl, $C_6$-$C_{12}$ aryl and $C_2$-$C_{12}$ heteroaryl, or a divalent substituent such as $-O-(CH_2)-O-$, $-O-(CH_2)_2-O-$, and, 1-4 methyl substituted version thereof, wherein each $R^{100}$, $R^{101}$, and $R^{102}$ independently is hydrogen or $C_1$-$C_8$ alkyl; $C_3$-$C_{12}$ cycloalkyl; $C_3$-$C_{10}$ heterocyclyl; $C_6$-$C_{12}$ aryl; or $C_2$-$C_{12}$ heteroaryl; or $R^{100}$ and $R^{102}$ together with the nitrogen atom to which they are attached to form a 5-7 membered heterocycle; wherein each alkyl, cycloalkyl, heterocyclyl, aryl, or heteroaryl is optionally substituted with 1-3 halogeno, 1-3 $C_1$-$C_6$ alkyl, 1-3 $C_1$-$C_6$ haloalkyl or 1-3 $C_1$-$C_6$ alkoxy groups. Preferably, the substituents are selected from the group consisting of chloro, fluoro, $-OCH_3$, methyl, ethyl, iso-propyl, cyclopropyl, $-CO_2H$ and salts and $C_1$-$C_6$ alkyl esters thereof, $CONMe_2$, CONHMe, $CONH_2$, $-SO_2Me$, $-SO_2NH_2$, $-SO_2NMe_2$, $-SO_2NHMe$, $-NHSO_2Me$, $-NHSO_2CF_3$,$-NHSO_2CH_2Cl$, $-NH_2$, $-OCF_3$, $-CF_3$ and $-OCHF_2$.

Specifically, the compound of Formula (6) is selected from the group consisting of:

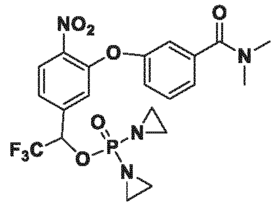,
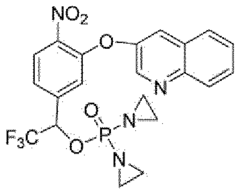,
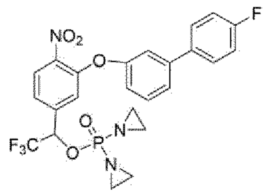,

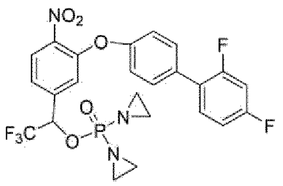,
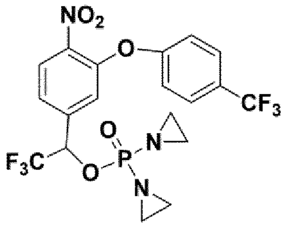,
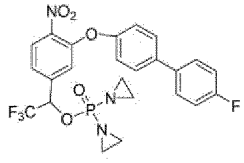,

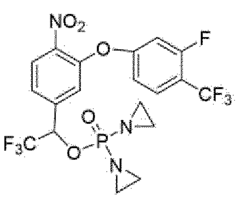
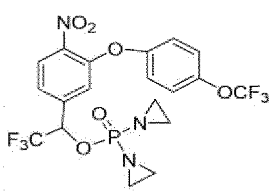
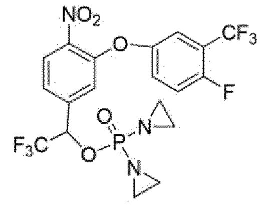

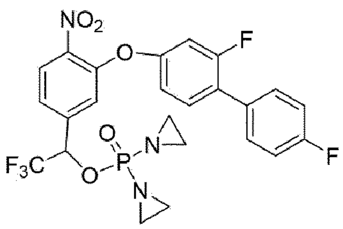
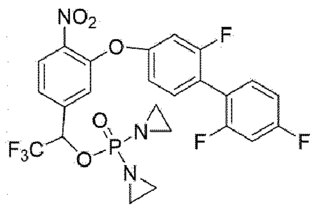
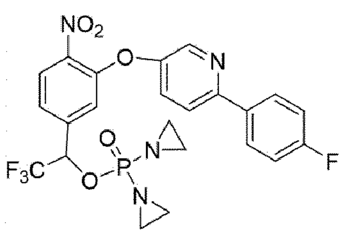

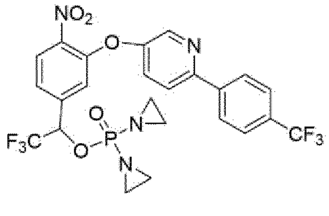
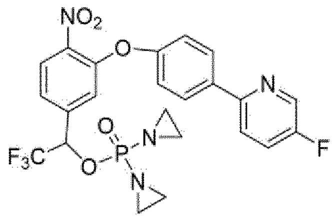
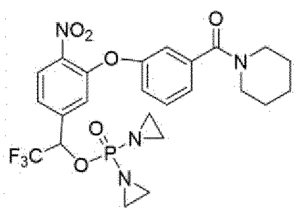

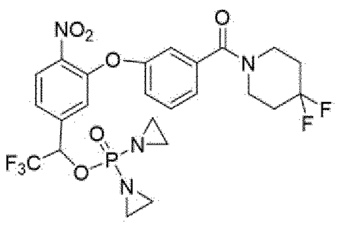
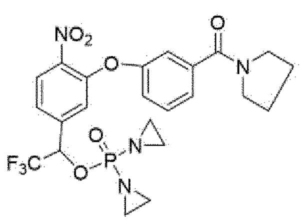
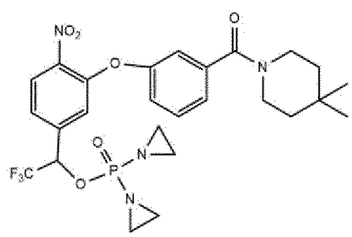

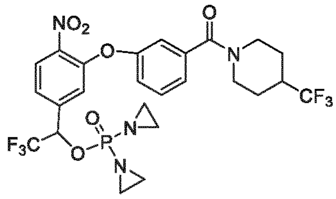
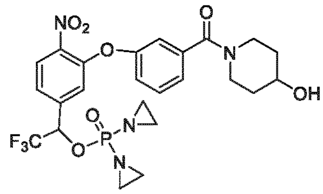
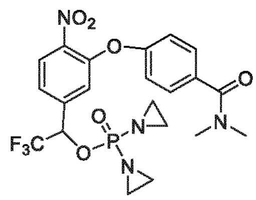

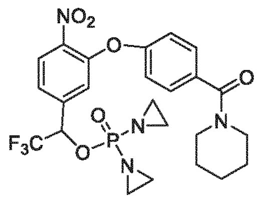
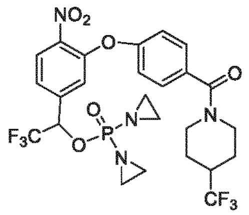
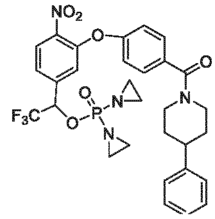

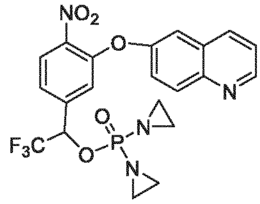
,
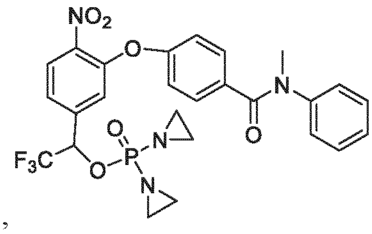
,
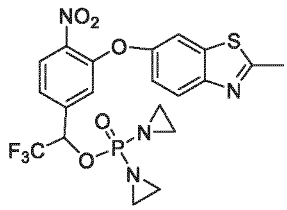
,

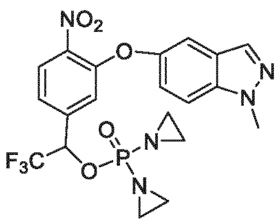
,
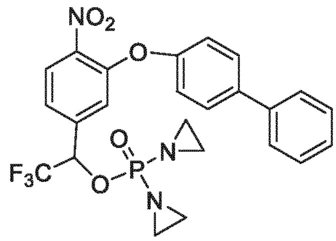
,
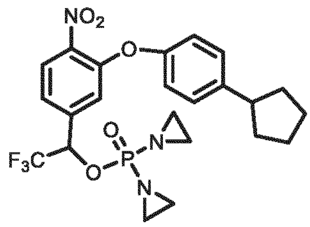
,

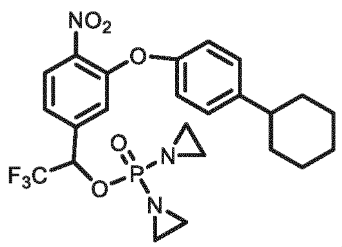
,
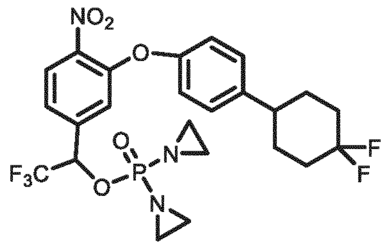
,

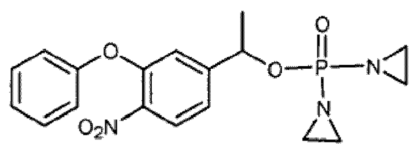
,
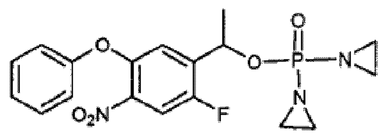
,

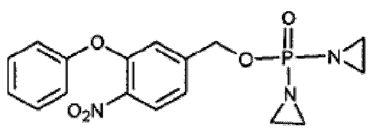
,
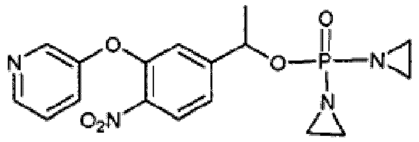
,

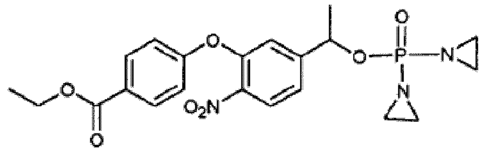
,
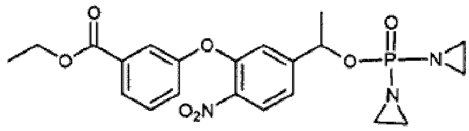
,

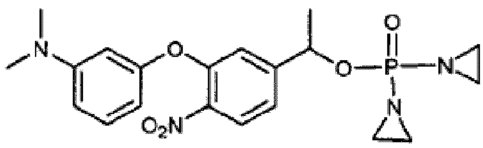
,
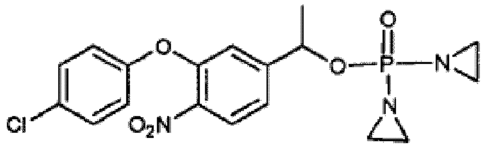
,

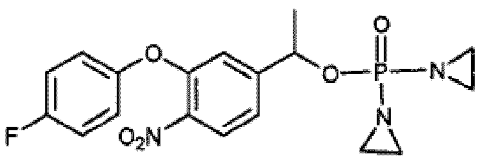
,
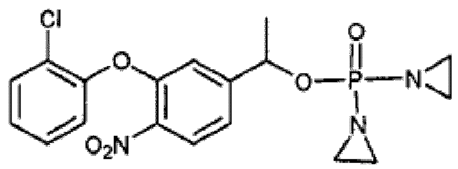
,

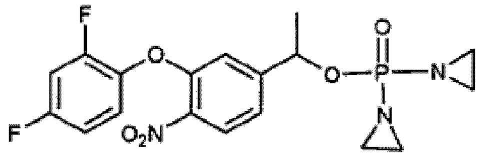
,
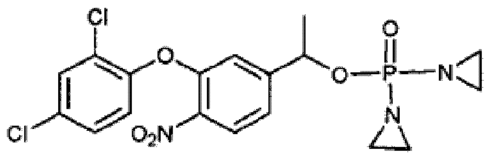
,

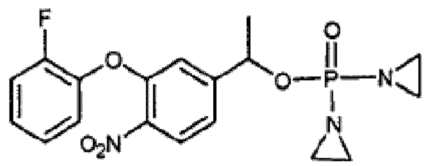
,
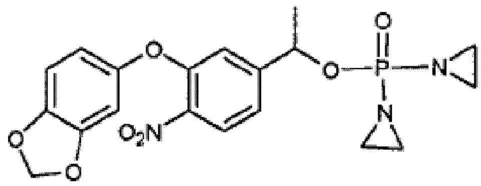
,

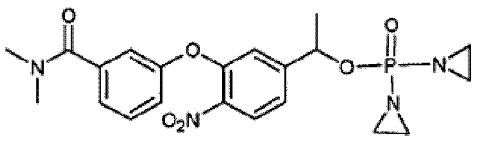
,
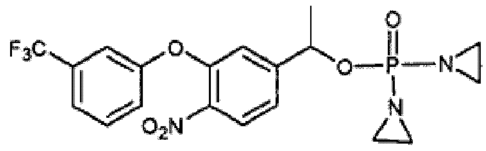
,

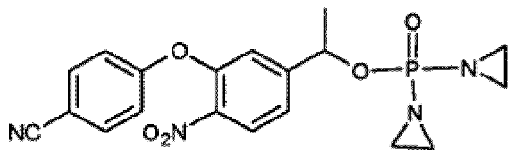
,
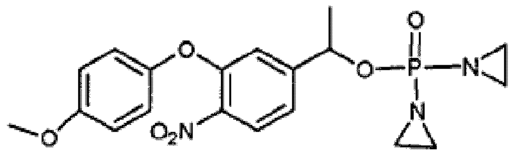
,

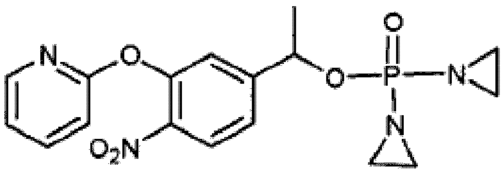, 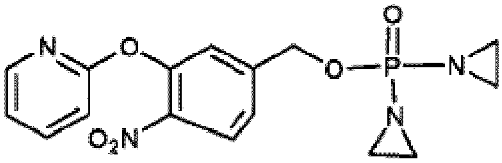,

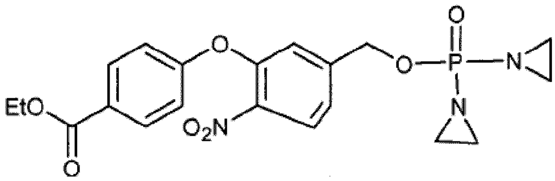, 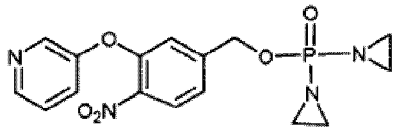,

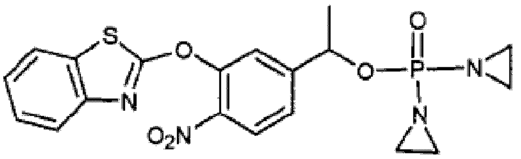, 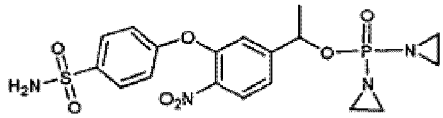,

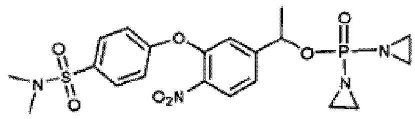, 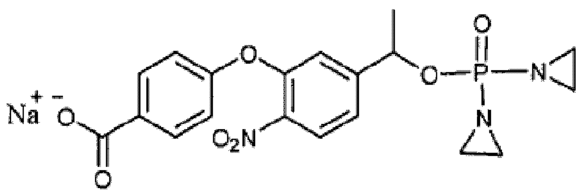,

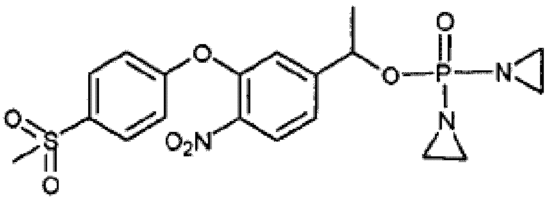, 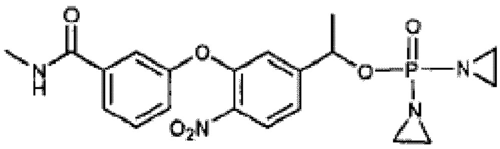,

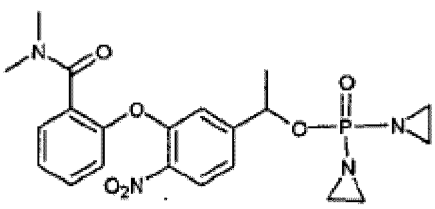, 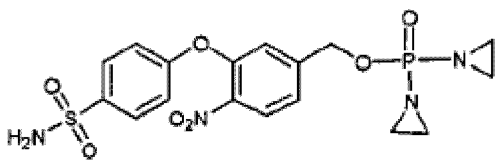,

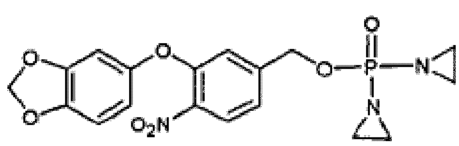, 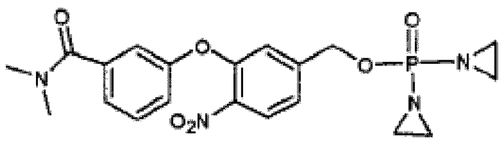,

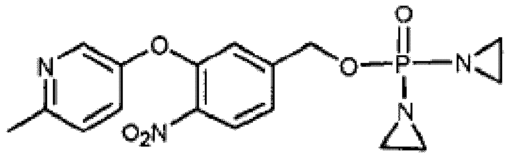 , 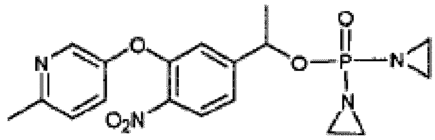 ,

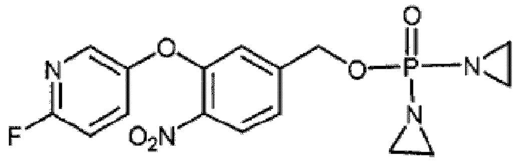 , 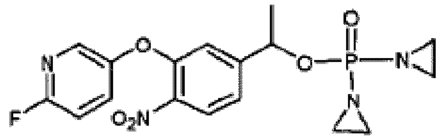 ,

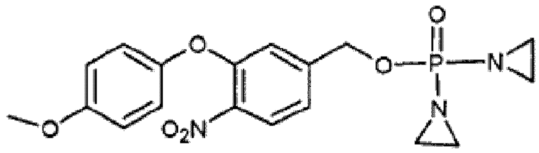 , 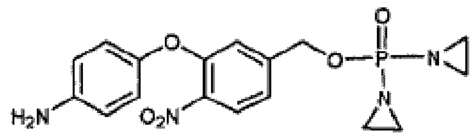 ,

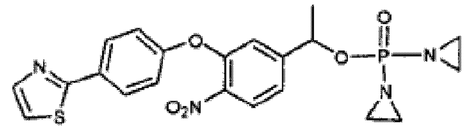 , 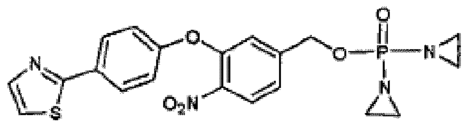 ,

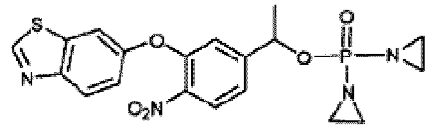 , 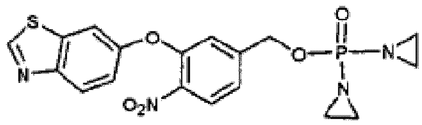 ,

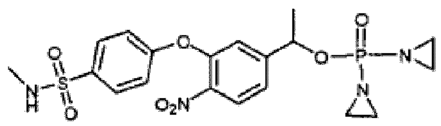 , 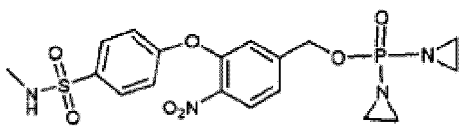 ,

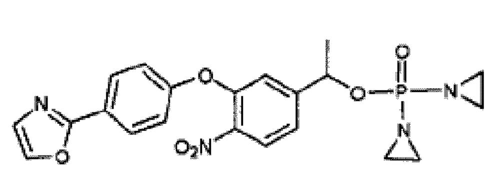 , 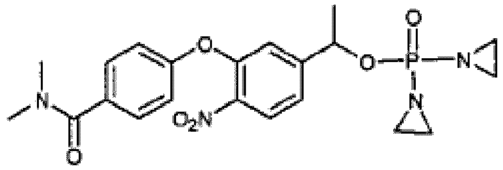 ,

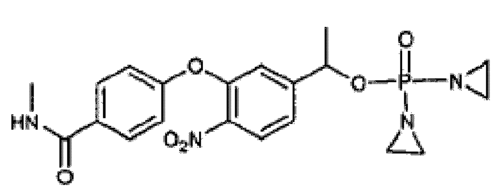 , 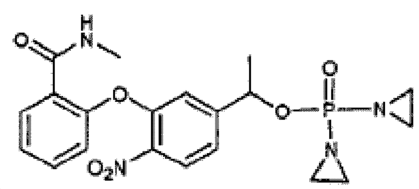 ,

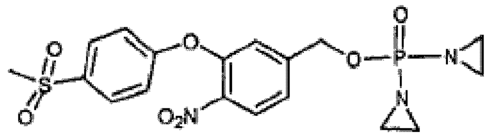,
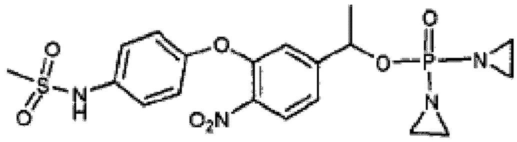,

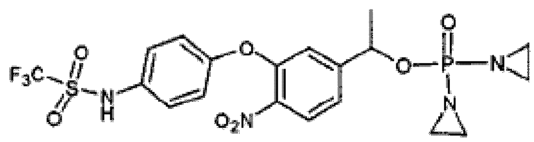,
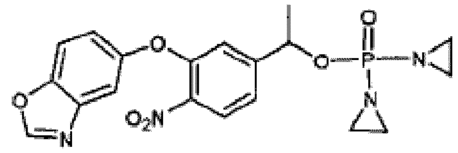,

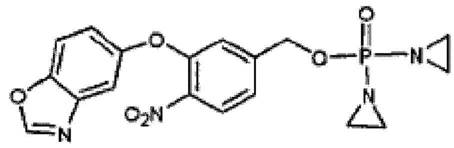,
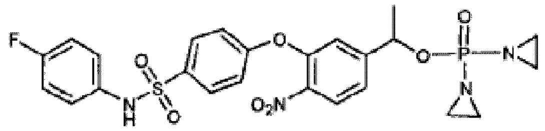,

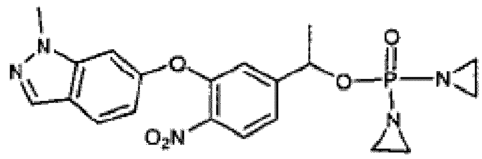,
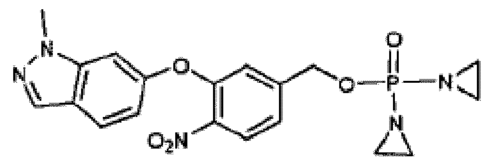,

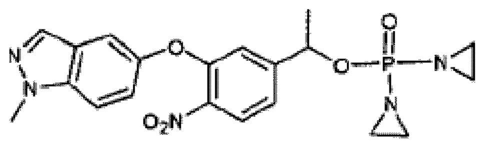,
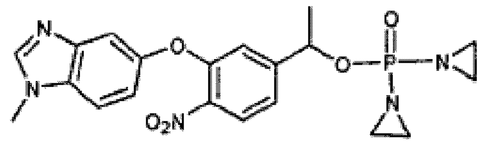,

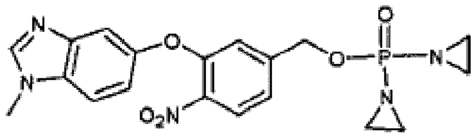,
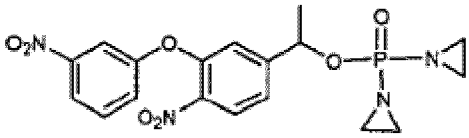,

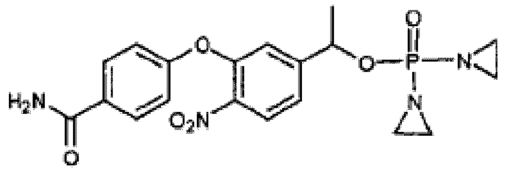,
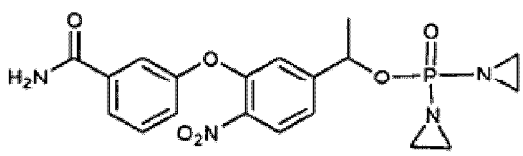,

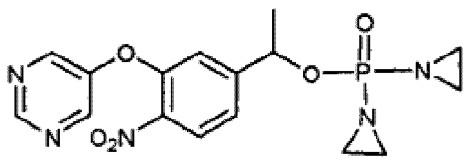,
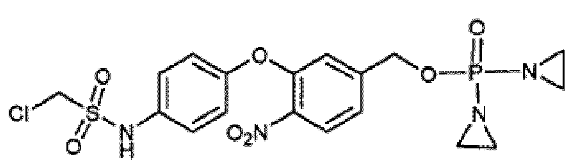,

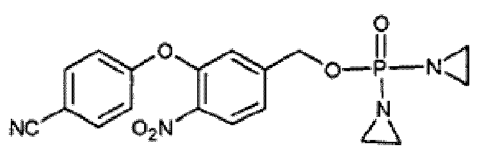,
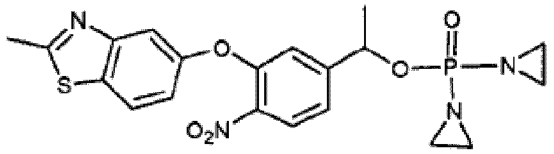,

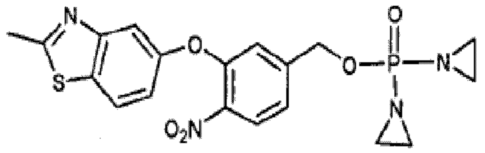,
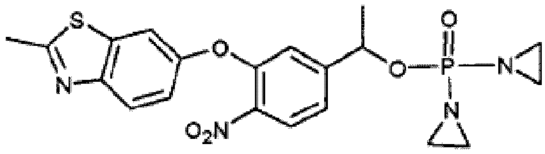,

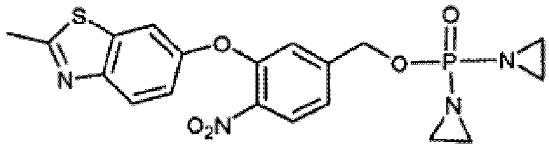,
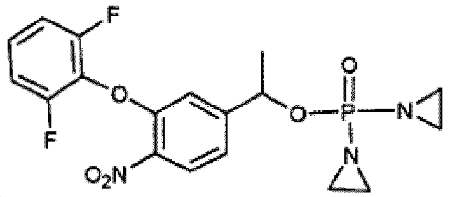,

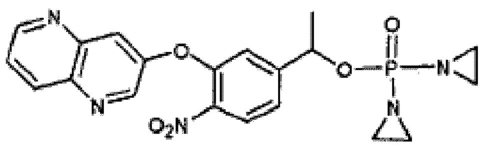,
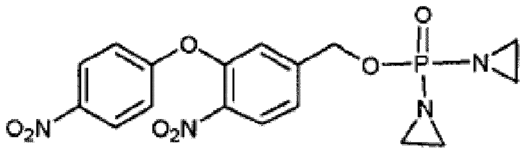,

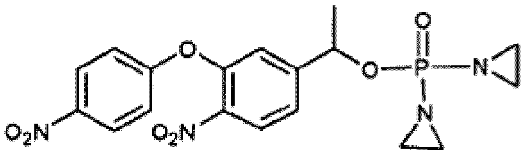,
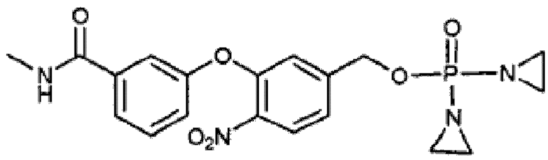,

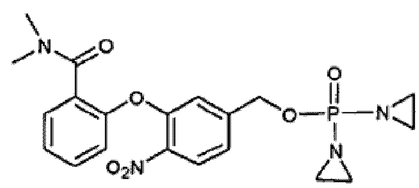,
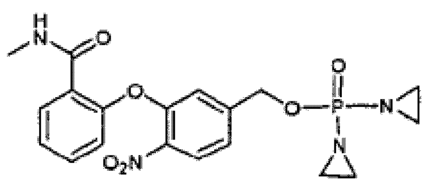,

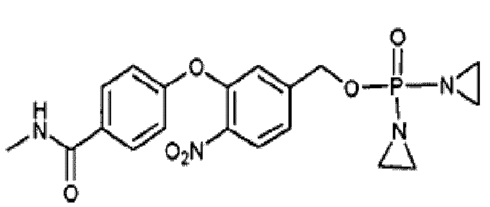,
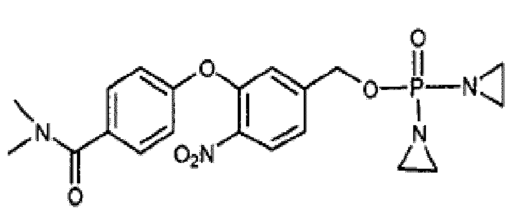,

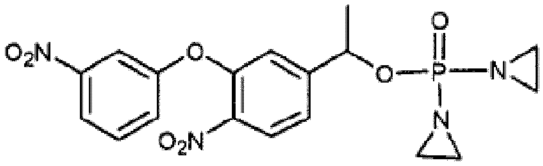,
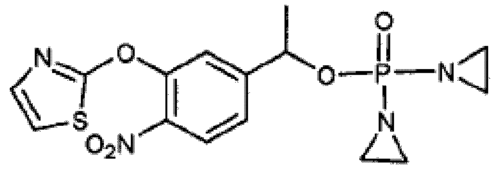,

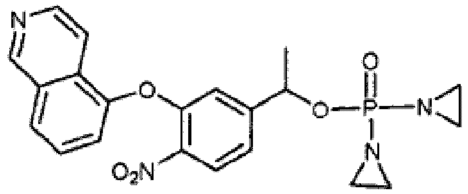,
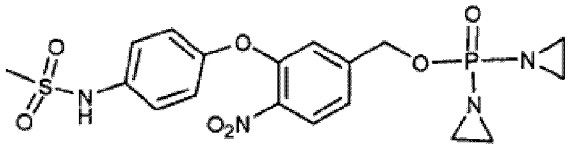,

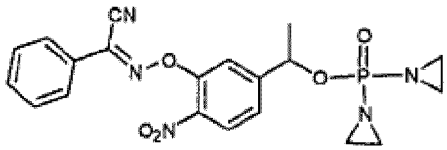,
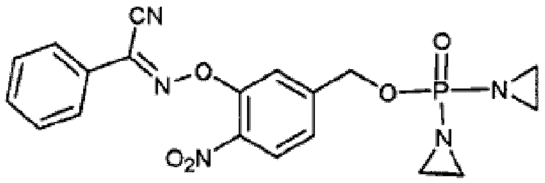,

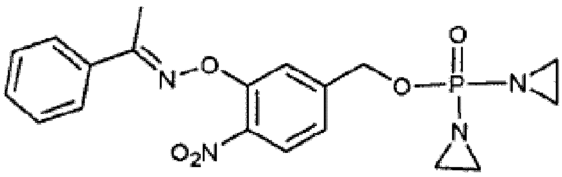,
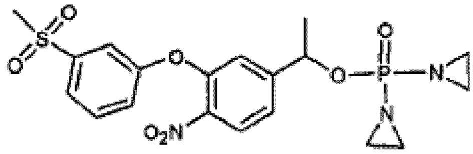,

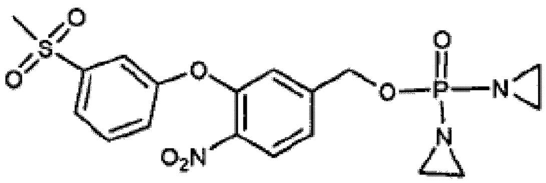,
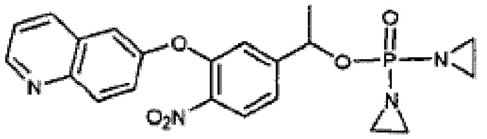,

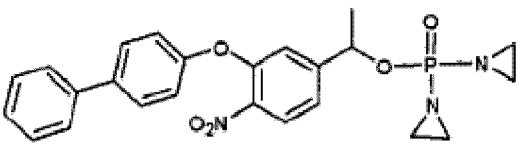,
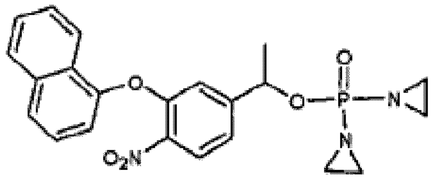,

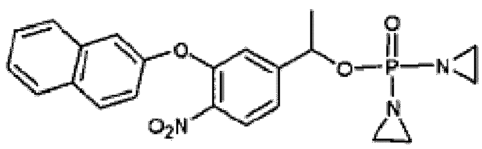,
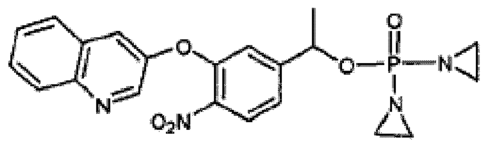,

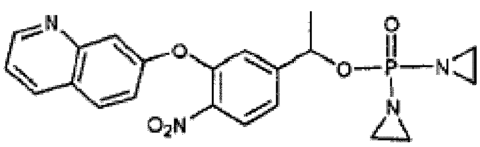,
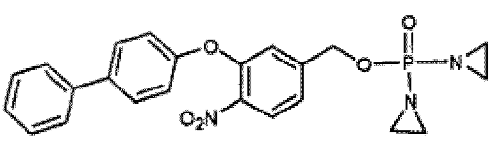,

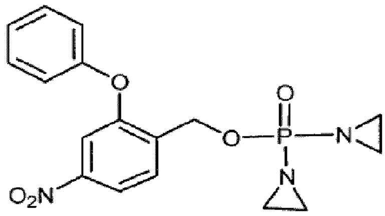 , 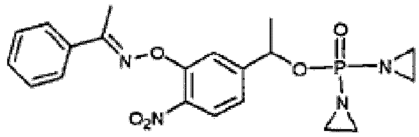 ,

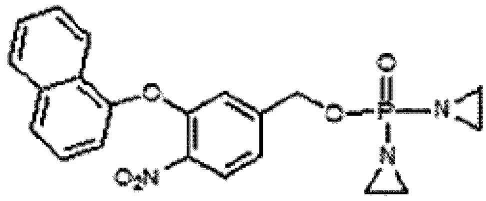 , 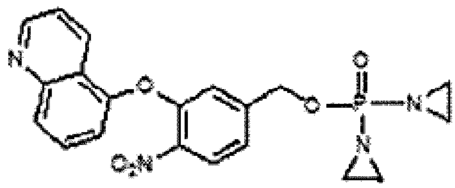 ,

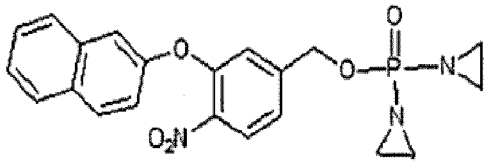 , 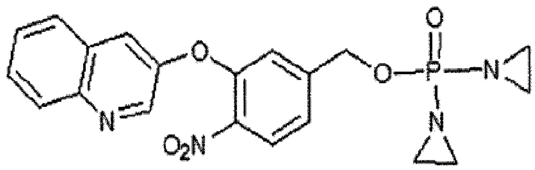 ,

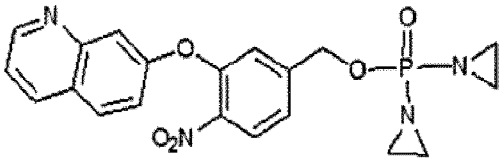 , 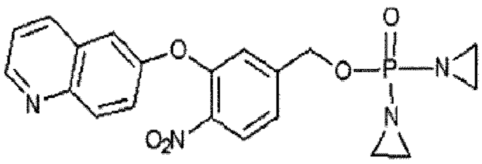 ,

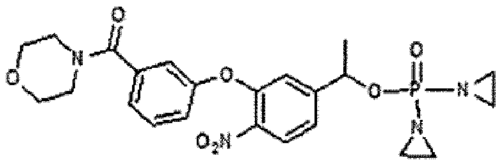 , 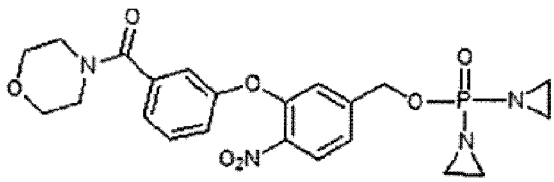 ,

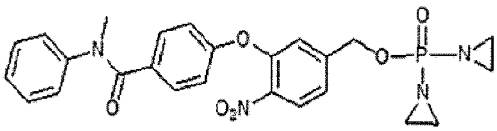 , 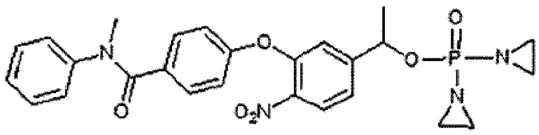 ,

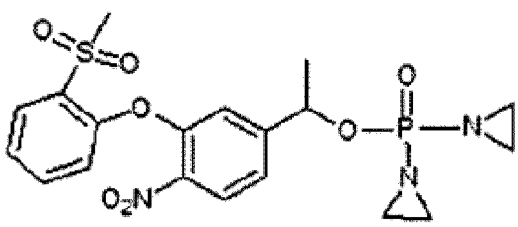 , 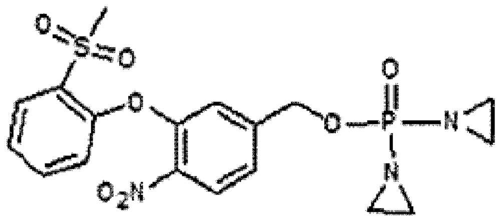 ,

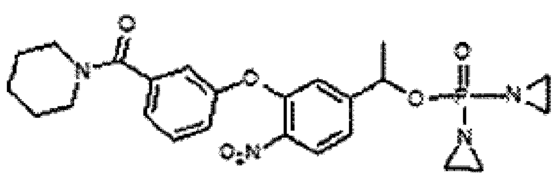,
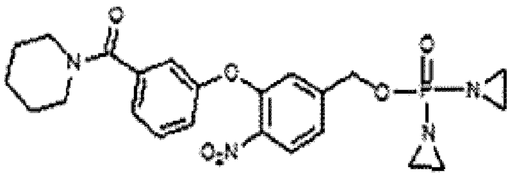,

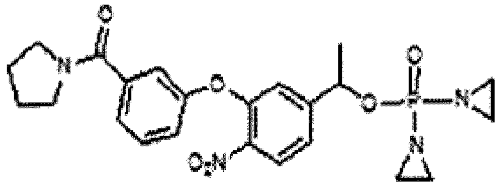,
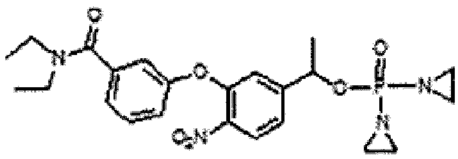,

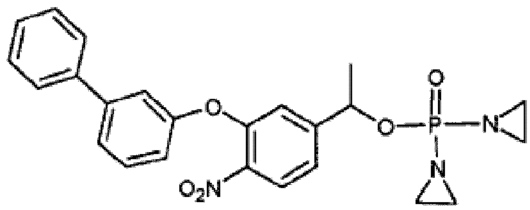,
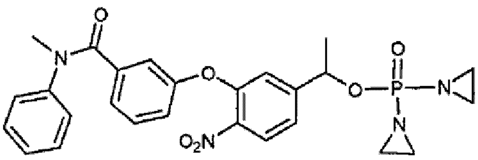,

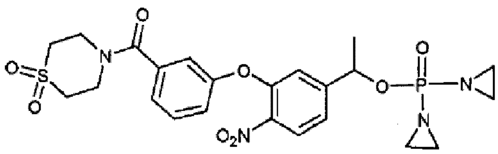,
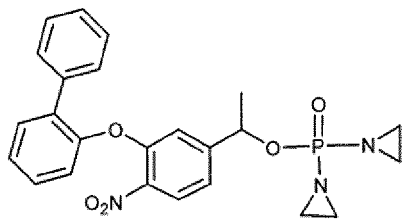,

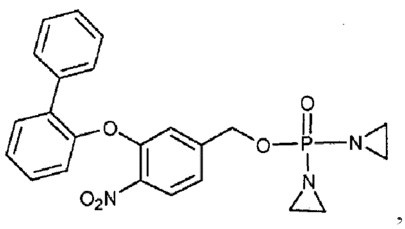,
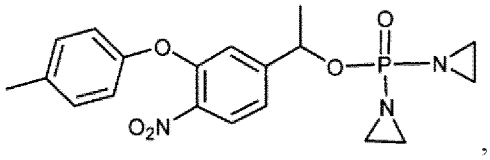,

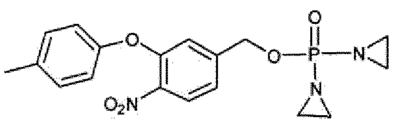,
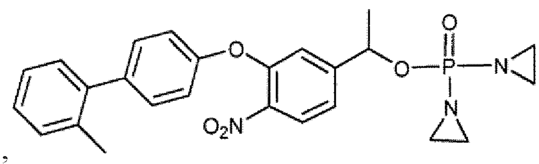,

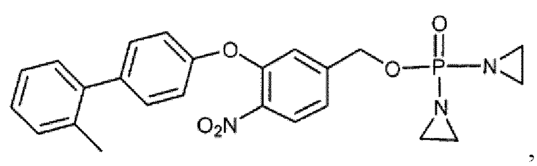,
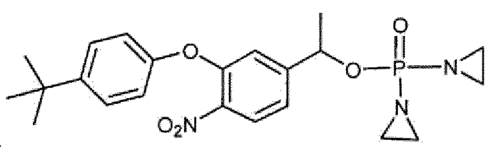,

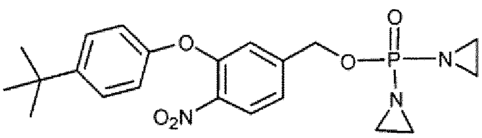,
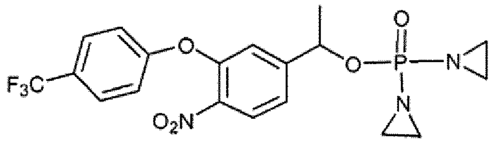,

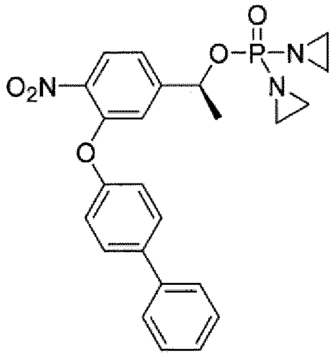
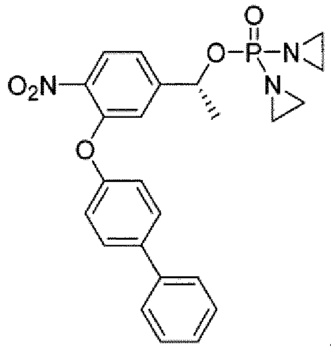
, , ,

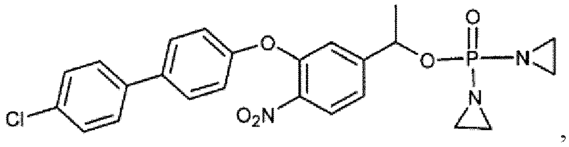,
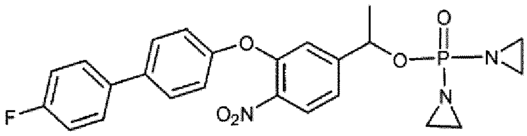,

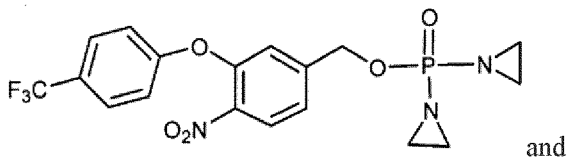
and
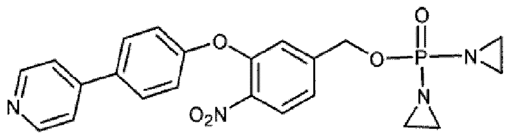.

Specific definitions and meanings of the groups, and the preparation methods and spectral data of the compounds have been described in PCT/US2016/021581 with Publication No. WO2016145092A1 (corresponding to Chinese Patent Application No. 2016800150788 with Publication No. CN107530556A); PCT/US2016/062114 with Publication No. WO2017087428A1 (corresponding to Chinese Patent Application No. 2016800446081 with Publication No. CN108290911A); and PCT/CN2020/089692 with Publication No. WO2020228685A1, which are incorporated herein by reference in its entirety.

Evidently, the compounds of Formula (6), similar to AST-3424 or AST, are prodrugs of AST-2660, and can be activated by the AKR1C3 enzyme to form AST-2660 to exert its anticancer efficacy:

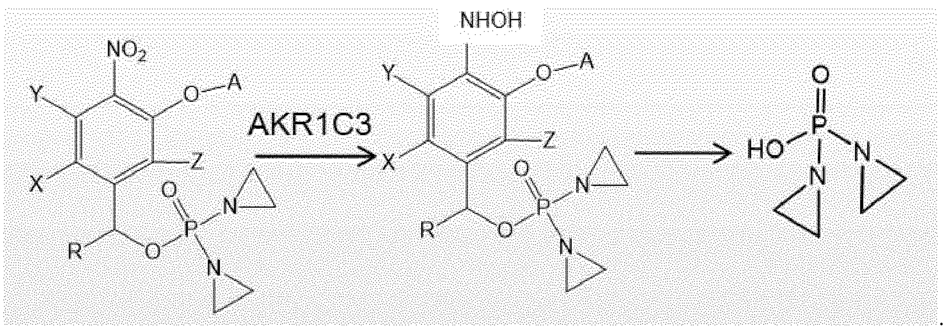

.

The preparation methods and spectral data of AST-3424 (OBI-3424),

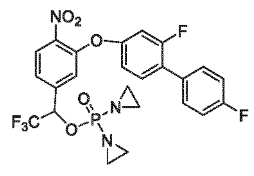

(hereinafter referred to as AST) and

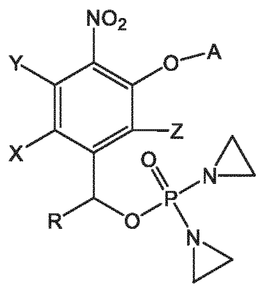

have been disclosed in the following Patent Applications: PCT/US2016/021581 with Publication No. WO2016145092A1 (corresponding to Chinese Patent Application No. 2016800150788 with Publication No. CN107530556A); PCT/US2016/062114 with Publication No. WO2017087428A1 (corresponding to Chinese Patent Application No. 2016800446081 with Publication No. CN108290911A); and PCT/CN2020/089692 with Publication No. WO2020228685A1; the relevant preparation concentrate injection, and the relevant prescription, preparation method and clinical compatibility and administration method have been described in detail and disclosed by the relevant patents: WO2021008520A1, WO2021043275A1, which are incorporated herein by reference in its entirety.

Monotherapy refers to a single drug therapy. Combination refers to a combined drug therapy. Single drug therapy refers to the use of only one anticancer drug in a course of treatment. Combination therapy refers to the simultaneous or sequential use of two or more anticancer drugs in a course of treatment.

Generally speaking, combination therapy needs to explore different administration dosages and administration cycles according to the characteristics of the disease and the types of drugs to be used in combination, and only according to the above situation, the combination therapy plan obtained from the exploration may achieve better therapeutic effect as compared with single drug therapy.

The administration dosages and administration cycles of drug of both monotherapy and combination therapy regimens need to be explored through clinical trials with reference to the dosage and administration regimens of AST-3424 and analogues thereof and other drugs as described above.

The administration dosages of the monotherapy can be determined with reference to the animal experimental dosages of WO2019062919A1 as well as WO2016145092A1 and WO2017087428A1.

Furthermore, the KRAS mutation is selected from the group consisting of KRAS-G12D mutation, KRAS-G12V mutation and KRAS-G12C mutation.

Mutations in either or both of the genes corresponding to KRAS can be detected and diagnosed with commercially available (companion) diagnostic kits, such as those approved in China:

Amoy Dx, State instrument registration 20153401126, Human KRAS gene mutation detection kit (fluorescent PCR method)

Tellgen, State instrument registration 20163401341, Human K-RAS gene 7 mutations detection kit (PCR fluorescence method).

Evidently, NGS sequencing (YS 450 gene NGS large panel) can also be used to determine the specific KRAS mutation subtype.

More preferably, the KRAS mutation is selected from the KRAS-G12D mutation.

The TMB (Tumor Mutation Load (burden)) level of the described gene mutation is medium.

Since the TMB (Tumor Mutation Load (burden)) is differently high or low between different tumor types: it is generally considered that: a TMB of more than 20 mutations/Mb (Mb represents bases per million) is high; a TMB of less than 10 mutations/Mb is low, and a TMB in the middle is medium. At the World Conference on Lung Cancer in 2017, Squibb Inc. had announced the results of a clinical trial named CheckMate-032. This was a phase II clinical trial that

enrolled 401 patients with advanced lung cancer who had failed first-line therapy and were treated with a PD-1 inhibitor alone or in combination with Ipilimumab. The patients were divided into three categories of patients with high TMB, medium TMB, and low TMB according to their TMB levels, then among those who received the combination therapy, the efficiency of the three groups was 62%, 20%, and 23%, respectively, and the efficiency of the group with high TMB was three times higher than the remaining two groups; and the median overall survival of the three groups was: 22.0 months, 3.6 months, and 3.4 months, respectively, with 22.0 months versus 3.4 months, a 6-fold difference! This trial demonstrated that for different cancer treatment drugs, different TMB levels have a significant impact on the efficacy of the drugs.

Further, the prodrug compound is preferably selected from the group consisting of:

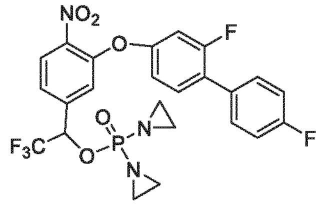 , 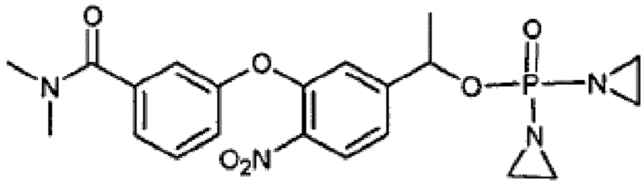 ,

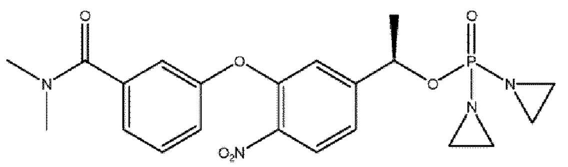 , 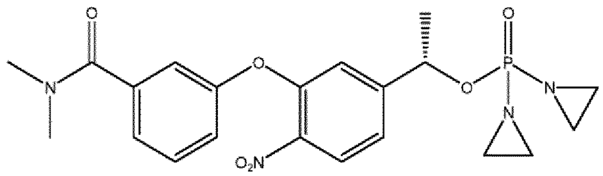 ,

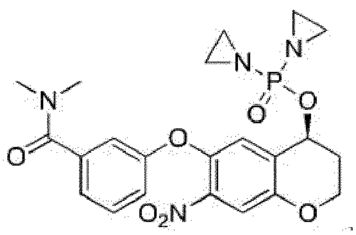 , 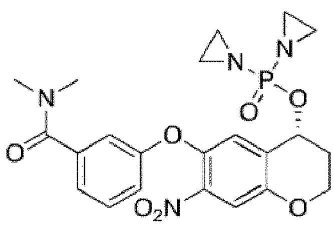 , 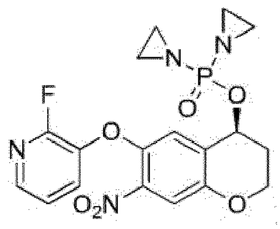 ,

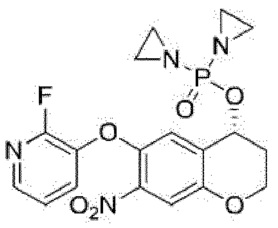 .

The above-mentioned cancer is selected from the group consisting of ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

Other therapeutic drugs are selected from the group consisting of KRAS inhibitors and immunotherapy drugs (immune checkpoint inhibitors).

KRAS inhibitors are substances that inhibit KRAS enzyme activity. For details, please refer to the review document Goebel, Lisa & Müller, Matthias & Goody, Roger & Rauh, Daniel. (2020). KRasG12C inhibitors in clinical trials: A short historical perspective. RSC Medicinal Chemistry. 11. 10.1039/D0MD00096E.

KRAS inhibitors that have entered clinical development or have already been marketed include Sotorasib (AMG510) developed by Amgen in the United States, adagrasib (MRTX849) developed by Mirati Therapeutics, GDC6036 developed by Roche, LY3499446 developed by Lilly, JNJ74699157 (ARS3248) jointly developed by Araxes and Janssen, D-1553 developed by InventisBio Co., Ltd, JAB-3312 developed by Jacobio Pharma, JAB-3068 developed by Jacobio Pharma, GH35 developed by Gen House, and BPI-421286 developed by Betta Pharma, BI17016963 developed by Boehringer-Ingelheim, mRNA-5671 developed by Moderna, and AZD-4785 developed in collaboration with AstraZeneca

and Ionis.

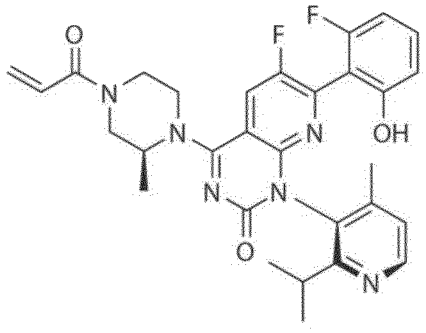
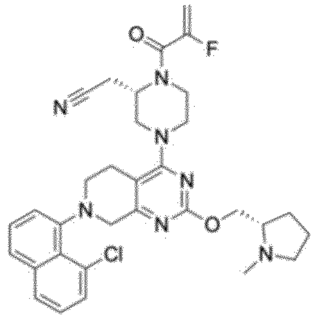

Sotorasib (AMG510) adagrasib (MRTX849)

Wherein, KRAS inhibitor is selected from the group consisting of Sotorasib (AMG510), adagrasib (MRTX849), GDC6036, LY3499446, JNJ74699157 (ARS3248), D-1553;

Immune cheeckpoint molecules are regulatory molecules in the immune system that play an inhibitory role and are essential for maintaining self-tolerance, preventing autoimmune reactions, and minimizing tissue damage by controlling the timing and intensity of the immune response. Immune checkpoint molecules are expressed on immune cells, and will inhibit the function of immune cell, thereby preventing the body from generating an effective anti-tumor immune response, and causing immune escape of tumor. Tumor-related immune checkpoint molecules include PD1, PD-L1, CTLA4, Tim3, and LAG3, etc. Currently, PD1, PD-L1, and CTLA4 are more frequently studied. Immune checkpoint inhibitors are some monoclonal antibody drugs developed for corresponding immune checkpoints. Their main function is to block the interaction between tumor cells expressing immune checkpoints and immune cells, thereby blocking the inhibitory effect of tumor cells on immune cells. Immunotherapy drugs are selected from the group consisting of PD-1 monoclonal antibodies and PD-L1 monoclonal antibodies.

The cancer and tumor are preferably selected from the group consisting of gastric cancer, pancreatic cancer and lung cancer.

In addition to containing corresponding prodrug compounds, the above-mentioned drugs should also be supplemented with pharmaceutically acceptable auxiliaries or excipients based on the specific characteristics of the drug, medicament or formulation. The medicament can be any dosage form for clinical administration, such as tablets, suppositories, dispersible tablets, enteric-coated tablets, chewable tablets, orally disintegrating tablets, capsules, sugar coated agents, granules, dry powders, oral solutions, a small needle for injection, lyophilized powder for injection, or infusion solutions. According to the specific dosage form and the mode of administration, the pharmaceutically acceptable auxiliaries or excipients in the medicament may include one or more of the following: diluent, solubilizer, disintegrant, suspension, lubricant, adhesive, filler, flavoring agent, sweetener, antioxidant, surfactant, preservative, wrapping agent and pigment, etc.

## BRIEF DESCRIPTION OF THE DRAWINGS

Fig. 1 is growth curves of tumor volume in each group of mice in the HuPrime® gastric cancer GA6201 model;

Fig. 2 is relative tumor growth inhibition rate curves in each group of mice in the HuPrime® gastric cancer GA6201 model;

Fig. 3 is body weight curves in each group of mice in the HuPrime® gastric cancer GA6201 model;

Fig. 4 is percentage change curves of body weight in each group of mice in the HuPrime® gastric cancer GA6201 model;

Fig. 5 is growth curves of tumor volume in each group of mice in the HuPrime® pancreatic cancer PA1222 model;

Fig. 6 is relative tumor growth inhibition rate curves in each group of mice in the HuPrime® pancreatic cancer PA1222 model;

Fig. 7 is body weight curves in each group of mice in the HuPrime® pancreatic cancer PA1222 model;

Fig. 8 is percentage change curves of body weight in each group of mice in the HuPrime® pancreatic cancer PA1222 model;

Fig. 9 is growth curves of tumor volume in each group of mice in the HuPrime® lung cancer LU11693 model;

Fig. 10 is relative tumor growth inhibition rate curves in each group of mice in the HuPrime® lung cancer LU11693 model;

Fig. 11 is body weight curves in each group of mice in the HuPrime® lung cancer LU11693 model;

Fig. 12 is percentage change curves of body weight in each group of mice in the HuPrime® lung cancer LU11693 model;

Fig. 13 is growth curves of tumor volume in each group of mice in the human-derived pancreatic cancer HPAF-II subcutaneous xenograft model;

Fig. 14 is percentage change curves of body weight in each group of mice in the human-derived pancreatic cancer HPAF-II subcutaneous xenograft model;

Fig. 15 is growth curves of tumor volume in each group of mice in the HuPrime® lung cancer LU5161 subcutaneous model;

Fig. 16 is percentage change curves of body weight in each group of mice in the HuPrime® lung cancer LU5161 subcutaneous model;

Figure 17 is growth curves of tumor volume in each group of mice in the HuPrime® intestinal cancer CR3820 subcutaneous model;

Fig. 18 is percentage change curves of body weight in each group of mice in the HuPrime® intestinal cancer CR3820 subcutaneous model;

Fig. 19 is growth curves of tumor volume in each group of mice in the HuPrime® pancreatic cancer PA2637 subcutaneous model;

Fig. 20 is percentage change curves of body weight in each group of mice in the HuPrime® pancreatic cancer PA2637 subcutaneous model;

Fig. 21 is growth curves of tumor volume in each group of mice in the HuPrime® lung cancer LU11873 subcutaneous model;

Fig. 22 is percentage change curves of body weight in each group of mice in the HuPrime® lung cancer LU11873 subcutaneous model;

Fig. 23 is growth curves of tumor volume in each group of mice in the HuPrime® pancreatic cancer PA1383 subcutaneous model;

Fig. 24 is percentage change curves of body weight in each group of mice in the HuPrime® pancreatic cancer PA1383 subcutaneous model;

Fig. 25 is the photographs of the IHC staining results of the GA6201, LU11693, PA1222 models and the control group.

## Detailed Description of the Invention

The present invention will be described below with reference to specific embodiments. Those skilled in the art will understand that these examples are only used to illustrate the present invention and do not limit the scope of the present invention in any way.

"Administering" or "administration of" a drug to a patient (and the grammatical equivalents of this phrase) refers to direct administration, which may be administered to a patient by a medical professional or may be self-administered, and/or indirect administration, which may be the act of prescribing a drug. For example, a physician who instructs a patient to self-administer a drug and/or provides a patient with a prescription for a drug is administering the drug to the patient.

"Cancer" refers to leukemias, lymphomas, carcinomas, and other malignant tumors (including solid tumors) with potentially unrestrained growth that can expand locally by invasion and systemically by metastasis. Examples of cancers include, but are not limited to, cancer of the adrenal gland, bone, brain, breast, bronchi, colon and/or rectum, gallbladder, head and neck, kidney, larynx, liver, lung, nervous tissue, pancreas, prostate, parathyroid, skin, stomach and thyroid. Certain other examples of cancers include acute and chronic lymphocytic and granulocytic neoplasms, adenocarcinoma, adenoma, basal cell carcinoma, cervical dysplasia carcinoma and in situ carcinoma, Ewing's sarcoma, epidermoid carcinoma, giant cell carcinoma, glioblastoma multiforme, hairy-cell tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, medullary epithelial carcinoma, metastatic skin cancer, mucosal neuroma, myeloma, mycosis fungoides, neuroblastoma, osteosarcomas, osteogenic and other sarcomas, ovarian tumors, pheochromocytoma, polycythemia vera, primary brain tumor, small cell lung cancer, squamous cell carcinoma of both ulcerative and papillary types, hyperplasia, seminoma, soft tissue sarcoma, retinoblastoma, rhabdomyosarcoma, renal cell tumors, topical skin lesion, reticulum cell sarcoma and Wilm's tumor.

"Patient" and "individual" are used interchangeably and refer to a mammal in need of treatment for cancer. Typically, the patient is a human. Typically, the patient is a human diagnosed with cancer. In certain embodiments, a "patient" or "individual" may refer to a non-human mammal, such as a non-human primate, a dog, cat, rabbit, pig, mouse, or rats used for screening, characterizing, and evaluating drugs and therapies.

"Solid tumors" refers to solid tumors including, but not limited to, metastatic tumors in bone, brain, liver, lung, lymph node, pancreas, prostate, skin, and soft tissue (sarcoma).

"Therapeutically effective amount" of a drug refers to an amount of a drug that, when administered to a patient with cancer, will have the intended therapeutic effect (e.g., alleviation, amelioration, palliation, or elimination of one or more clinical manifestations of cancer in the patient). A therapeutic effect does not necessarily occur by administration of one dose, and may occur only after administration of a series of doses. Thus, a therapeutically effective amount may be administered in one or more administrations.

"Treating", "treatment of" or "therapy of" a condition or a patient refers to taking steps to obtain a beneficial or desired result (including clinical results). For purposes of the present invention, beneficial or desired clinical results include, but are not limited to, alleviation or amelioration of one or more symptoms of cancer; diminishment of extent of disease; delay or slowing of disease progression; amelioration, remission, or stabilization of disease status; or other beneficial results. In some cases, treatment of cancer can result in partial response or stable disease.

"Tumor cells" refers to tumor cells of any appropriate species, e.g., mammalian such as murine, canine, feline, equine or human.

"Patient" and "individual" are used interchangeably and refer to a mammal in need of treatment for cancer. Typically, the patient is a human. Typically, the patient is a human diagnosed with cancer. In certain embodiments, a "patient" or "individual" may refer to a non-human mammal, such as a non-human primate, a dog, cat, rabbit, pig, mouse, or rats used for screening, characterizing, and evaluating drugs and therapies.

"Treatment" or "treatment of a patient" is to administer, use or administer a therapeutic effective amount of a drug to a patient in relation to the present invention.

"Administering" or "administration of" or "use of" a drug to a patient refers to direct administration, which may be administered to a patient by a medical professional or may be self-administered, and/or indirect administration, which may be the act of prescribing a drug. For example, a physician who instructs a patient to self-administer a drug and/or provides a patient with a prescription for a drug is administering the drug to the patient.

"Treating", "treatment of" or "therapy of" a condition or a patient refers to taking steps to obtain a beneficial or desired result (including clinical results). For purposes of the present invention, beneficial or desired clinical results include, but are not limited to, alleviation or amelioration of one or more symptoms of cancer; diminishment of extent of disease; delay or slowing of disease progression; amelioration, remission, or stabilization of disease status; or other beneficial results. In some cases, treatment of cancer can result in partial response or stable disease.

The experimental methods in the following examples are conventional methods unless specified otherwise. The medicinal raw materials, reagent materials, etc. used in the following examples are all commercially available

products unless specified otherwise.

The above description of embodiments of the present invention does not limit the present invention. Those skilled in the art can make various modifications and changes according to the present invention, and any modification and change within the spirit of the present invention shall be covered in the scope of the claims appended to the present invention.

Specific experiments of the present invention are provided below.

**1. Pharmacodynamics evaluation of the test substances AST, AST-3424 and Ifosfamide in the HuPrime® gastric cancer GA6201 subcutaneous xenograft model**

The HuPrime® gastric cancer GA6201 PDX model was a model of KRAS pathogenic mutation having G12D amino acid mutation (KRAS-G12D). BALB/c nude mice were subcutaneously inoculated with HuPrime® model GA6201 tumor blocks so as to establish a subcutaneous transplantation tumor model of human gastric cancer. The test was divided into the test drug Ifosfamide 60 mg/kg group, the test drug AST-3424 5 mg/kg group, AST 2.5 mg/kg and 5 mg/kg groups, and physiological saline (pH 7.0-7.6) vehicle control group, with a total of 5 groups and 5 mice in each group. Among them, physiological saline (pH 7.0-7.6) vehicle control group, the test drug AST-3424 5 mg/kg group, AST 2.5 mg/kg group and 5 mg/kg group were administered by tail vein injection once a week for a total of three weeks, and observed for four weeks. The Ifosfamide 60 mg/kg group was administered through intraperitoneal injection continuously for five days a week and discontinued for two days for a total of two weeks, and observed for five weeks. The therapeutic effect was evaluated based on the relative tumor growth inhibition rate (TGI (%)), and the safety was evaluated based on the body weight changes and the death situation of the animals.

The specific administration regimen for each group was shown in Table 1 below.

Table 1: Experimental design of anti-tumor effects of test drugs at different doses in the HuPrime® gastric cancer GA6201 tumor model

| Group No. | The numberof Animals | Administration group | Dosage (mg/kg) | Administration method | Planned administration period | Actual administration period |
|---|---|---|---|---|---|---|
| 1 | 5 | Physiological saline, pH 7.0-7.6 | 0 | tail vein | Q7D×3 | Q7D×3 |
| 2 | 5 | Ifosfamide | 60 | intraperitoneal | QD×5/week×2 wks | QD×5/week×2 wks |
| 3 | 5 | AST-3424 | 5 | tail vein | Q7D×3 | Q7D×3 |
| 4 | 5 | AST | 5 | tail vein | Q7D×3 | Q7D×3 |
| 5 | 5 | AST | 2.5 | tail vein | Q7D×3 | Q7D×3 |

The tumor volumes of mice in different groups were measured on different days and the mean values were obtained. The results were shown in Table 2 below.

Table 2: Changes in tumor volume in each group of mice over treatment time in the HuPrime® gastric cancer GA6201 model

| Time | **Tumor Volume (mm³)** ($\bar{x}$±S) | | | | |
|---|---|---|---|---|---|
| | Group 1 Physiological saline, pH 7.0-7.6 | Group 2 Ifosfamide 60 mg/kg | Group 3 AST-3424 5 mg/kg | Group 4 AST 5 mg/kg | Group 5 AST 2.5 mg/kg |
| 0 day after initial administration | 94.22±8.5 | 94.15±6.68 | 94.19±12.46 | 94.36±14.61 | 94.61±11.1 |
| 3 days after initial administration | 123.26±13.74 | 122.22±12.32 | 114.98±21.76 | 118.42±20.83 | 120.19±15.57 |
| 7 days after initial administration | 161.08±22.55 | 129.39±13.47 | 84.33±17.54 | 101.91±19.72 | 118.33±27.54 |

(continued)

| Time | Tumor Volume (mm³) ($\bar{x}$±S) | | | | |
|---|---|---|---|---|---|
| | Group 1 Physiological saline, pH 7.0-7.6 | Group 2 Ifosfamide 60 mg/kg | Group 3 AST-3424 5 mg/kg | Group 4 AST 5 mg/kg | Group 5 AST 2.5 mg/kg |
| 10 days after initial administration | 182.31±28.05 | 175±22.09 | 77.04±15.37 | 97.17±25.14 | 118.65±31.45 |
| 14 days after initial administration | 242.26±40.75 | 190.74±29.81 | 63.59±10.46 | 74.2±16.99 | 96.08±26.3 |
| 17 days after initial administration | 297.28±47.21 | 189.23±29.28 | 52.08±9.25 | 59.77±15.76 | 76.14±18.07 |
| 21 days after initial administration | 398.37±87.32 | 217.96±41.06 | 4581±13.3 | 46.21±13.48 | 71.72±9.08 |
| 24 days after initial administration | 479.52±119.81 | 225.66±44.63 | 40.55±10.4 | 44.03±11.57 | 57.94±10.4 |
| 28 days after initial administration | 579.9±154.47 | 247.04±50.8 | 30.28±3.78 | 38.5±11 | 45.51±1.64 |
| 31 days after initial administration | 648.12±177.44 | 265.76±52.69 | 23.24±7.23 | 35.92±10.45 | 43.44±4.51 |
| 35 days after initial administration | 831.15±234.39 | 324.51±73.75 | 18.55±5.77 | 22.7±11.73 | 32.04±4.52 |
| 38 days after initial administration | 905.75±252.4 | 371.02±75.96 | 12.65±7.91 | 1997±1018 | 28.69±8.2 |
| 42 days after initial administration | 1026.01±294.05 | 403.47±72.29 | 13.42±8.22 | 5.05±5.05 | 23.49+6.18 |
| 45 days after initial administration | 1130.43±319.13 | 484.83±98.33 | 10.82±7.05 | 11.14±7.04 | 25±6.37 |
| 49 days after initial administration | 1283.11±366.66 | 578.6±99.79 | 8.15±5.03 | 9.96±7.12 | 17.72±4.6 |

Fig. 2 showing the tumor growth of each treatment group and control group was made according to the data in Table 2.

The drug efficacy evaluation and analysis data was made according to the data in Table 2. The specific data were shown in Table 3.

Table 3: Drug efficacy analysis table of each group in the HuPrime® gastric cancer GA6201 model

| Experiment group | Day 3 after the end of administration (i.e., Day 38, 2/27/2020) | | | | |
|---|---|---|---|---|---|
| | Tumor volume ($\bar{x}\pm S$) | Relative tumor volume ($\bar{x}\pm S$) | TGI (%) | T/C (%) | P Value (comparing with the control group) |
| Group 1 Physiological saline, pH 7.0-7.6 | 905.75+252.4 | 9.34±2.42 | - | - | - |
| Group 2 Ifosfamide 60 mg/kg | 371.02±75.96 | 3.85±0.63 | 58.83 | 41.17 | 0.857 |
| Group 3 AST-3424 5 mg/kg | 12.65±7.91 | 0.11±0.07 | 98.79 | 1.21 | 0.00000265 |
| Group 4 AST 5 mg/kg | 1997±10.18 | 0.19±0.08 | 97.96 | 2.04 | 0.0000182 |
| Group 5 AST 2.5 mg/kg | 28.69±8.2 | 0.29±0.08 | 96.85 | 3.15 | 0.000135 |

The relative tumor proliferation rate (T/C%) in Table 3 was the percentage of relative tumor volume or tumor weight between the treatment group and the control group at a certain time point. The calculation formula was as follows:

T/C%=TRTV/CRTV×100% (TRTV: the mean RTV of the treatment group; CRTV: the mean RTV of the vehicle control group; RTV=Vt/V0, V0 was the tumor volume of the animal when grouped, Vt was the tumor volume of the animal after treatment);

The relative tumor growth inhibition rate (TGI (%)) was calculated as follows: TGI% = (1-T/C) × 100%. (T and C were the mean relative tumor volumes (RTV) of the treatment group and the control group at a specific time point, respectively).

Table 4: Relative tumor growth inhibition rate of each group of tumors in the HuPrime® gastric cancer GA6201 model

| Group | 0 | 3 | 7 | 10 | 14 | 17 | 21 | 24 | 28 | 31 | 35 | 38 |
|---|---|---|---|---|---|---|---|---|---|---|---|---|
| Group 01 | | | | | | | | | | | | |
| Group 02 | 0.08% | 0.85% | 19.67% | 4.01% | 21.27% | 36.35% | 45.29% | 52.94% | 57.40% | 59.00% | 60.96% | 59.04% |
| Group 03 | 0.04% | 6.72% | 47.65% | 57.74% | 73.75% | 82.48% | 88.50% | 91.54% | 94.78% | 96.41% | 97.77% | 98.60% |
| Group 04 | -0.15% | 3.93% | 36.73% | 46.70% | 69.37% | 79.89% | 88.40% | 90.82% | 93.36% | 94.46% | 97.27% | 97.80% |
| Group 05 | -0.42% | 2.50% | 26.54% | 34.92% | 60.34% | 74.39% | 82.00% | 87.92% | 92.15% | 93.30% | 96.15% | 96.83% |
| Group 06 | -0.02% | 1.19% | 23.52% | 15.82% | 21.09% | 26.03% | 38.10% | 43.33% | 51.57% | 58.24% | 63.99% | 69.40% |

The above Table 4 was made into a curve graph to obtain Fig. 2.

The body weights of mice in different groups were measured on different days and the mean values were obtained. The results were shown in Table 5 below.

Table 5: Body weights of mice on different inoculation days in the HuPrime® gastric cancer GA6201 model

| Group | 0 | 1 | 2 | 3 | 4 | 7 | 8 | 9 | 10 | 11 | 14 | 15 | 16 | 17 | 18 | 21 | 24 | 28 | 31 | 35 | 38 |
|---|---|---|---|---|---|---|---|---|---|---|---|---|---|---|---|---|---|---|---|---|---|
| Group 01 | 25.3 | | | 25.4 | | 25.3 | | | 24.4 | | 25.4 | | | 24.6 | | 23.8 | 24.3 | 23.7 | 24.0 | 24.4 | 25.1 |
| Group 02 | 25.8 | 25.5 | 25.0 | 25.5 | 24.4 | 24.4 | 24.5 | 24.1 | 24.4 | 24.4 | 24.3 | | | 25.1 | | 26.3 | 26.1 | 25.5 | 25.2 | 26.0 | 26.2 |
| Group 03 | 24.8 | | | 25.1 | | 25.1 | | | 24.5 | | 25.4 | | | 25.0 | | 25.5 | 25.6 | 25.3 | 25.4 | 25.7 | 26.0 |
| Group 04 | 25.0 | | | 24.4 | | 24.8 | | | 24.5 | | 25.2 | | | 24.9 | | 25.4 | 25.1 | 24.6 | 25.2 | 25.6 | 25.7 |
| Group 05 | 25.3 | | | 25.1 | | 25.0 | | | 25.0 | | 25.2 | | | 24.9 | | 25.4 | 25.8 | 25.0 | 25.4 | 25.6 | 25.4 |

The above table was made into a curve graph to obtain Fig. 3, which was body weight curve in each group of mice in the HuPrime® gastric cancer GA6201 model.

Similarly, the data in Table 5 was processed to obtain the following Table 6.

Table 6: Percentage changes in body weight of mice on different inoculation days in the HuPrime® gastric cancer GA6201 model (% Group Mean Change=mean((T-T0)/T0)*100, T represented current value, T0 represented initial value)

| Group | 0 | 1 | 2 | 3 | 4 | 7 | 8 | 9 | 10 | 11 | 14 | 15 | 16 | 17 | 18 | 21 | 24 | 28 | 31 | 35 | 38 |
|---|---|---|---|---|---|---|---|---|---|---|---|---|---|---|---|---|---|---|---|---|---|
| Group 01 | 0.00% | | | 0.72% | | -0.09% | | | -3.34% | | 0.31% | | | -2.58% | | -4.69% | -2.40% | -4.98% | -3.68% | -2.33% | 0.69% |
| Group 02 | 0.00% | -1.02% | -2.93% | -1.20% | -5.32% | -5.50% | -5.07% | -6.48% | -5.33% | -5.32% | -5.80% | | | -2.46% | | 1.96% | 1.12% | -0.90% | -2.41% | 0.86% | 1.53% |
| Group 03 | 0.00% | | | 1.21% | | 1.46% | | | -1.11% | | 2.52% | | | 0.87% | | 3.03% | 3.31% | 2.27% | 2.52% | 3.58% | 4.87% |
| Group 04 | 0.00% | | | -2.44% | | -0.77% | | | -2.14% | | 0.65% | | | -0.46% | | 1.57% | 0.53% | -1.72% | 0.85% | 2.51% | 2.73% |
| Group 05 | 0.00% | | | -0.70% | | -1.21% | | | -1.22% | | -0.28% | | | -1.77% | | 0.50% | 1.97% | -1.37% | 0.38% | 1.23% | 0.43% |

Group01, Group02, Group03, Group04, and Group05 in the above tables 4/5/6 were the above-mentioned Group 1, Group 2, Group 3, Group 4, Group 5, and Group 6. 0/1/2/3/4/7/8/9/10/11/14/15/16/17/18/21/24/28/31/35/38 was the number of days after inoculation.

The above table was made into a curve graph to obtain Fig. 4.

It can be known from the analysis of experimental data that in the respect of therapeutic effect:
The test drug AST-3424 at the dose of 5 mg/kg, and the test drug AST at the doses of 2.5 mg/kg and 5 mg/kg, had a significant inhibitory effect on tumor growth of the HuPrime® gastric cancer GA6201, with statistically significant difference compared with the control group.

The test drug Ifosfamide at the dose of 60 mg/kg, had a certain inhibitory effect on tumor growth of HuPrime® gastric cancer GA6201, but no statistically significant difference compared with the control group.

Analysis of the experimental data showed that Ifosfamide, AST-3424, and AST were well tolerated by tumor-bearing mice at the tested doses.

**2. Pharmacodynamics and safety evaluation of the test substances AST and Gemcitabine in the HuPrime® pancreatic cancer PA1222 subcutaneous xenograft model**

The HuPrime® pancreatic cancer PA1222 PDX model was a model of KRAS pathogenic mutation having G12D amino acid mutation (KRAS-G12D). BALB/c nude mice were subcutaneously inoculated with the HuPrime® model PA1222 tumor blocks so as to establish a subcutaneous transplantation tumor model of human pancreatic cancer. The test was divided into the test drug Gemcitabine 120 mg/kg group, the test drug AST 10 mg/kg group and 7.5% absolute ethanol + 7.5% polyoxyethylene (35) castor oil + 85% glucose injection D5W (pH 7.4) vehicle control group, with a total of 3 groups and 5 mice in each group. Among them, the 7.5% absolute ethanol + 7.5% polyoxyethylene (35) castor oil + 85% glucose injection D5W (pH 7.4) vehicle control group and the test drug AST 10 mg/kg group were administered by tail vein injection once a week for three consecutive weeks. The test drug Gemcitabine 120 mg/kg group was administered through intraperitoneal injection once a week for three consecutive weeks. The therapeutic efficacy was evaluated based on the relative tumor growth inhibition rate (TGI (%)), and the safety was evaluated based on the body weight changes and the death situation of the animals.

The specific administration regimen for each group was shown in Table 7 below.

Table 7: Experimental design of anti-tumor effects of test drugs at different doses in the HuPrime® pancreatic cancer PA1222 PDX tumor model

| Group No. | The number of Animals | Administration group | Dosage (mg/kg) | Administration method | Administration period |
|---|---|---|---|---|---|
| 1 | 5 | 7.5% absolute ethanol + 7.5% polyoxyethylene (35) castor oil + 85% glucose injection D5W (pH 7.4) | - | i.v. | QW×3 |
| 2 | 5 | Gemcitabine | 120 | i.p. | QW×3 |
| 3 | 5 | AST | 10 | i.v. | QW×3 |
| Note: 1. Dosing volume was 10 μl/g.<br>2. QW×3; administered once a week for three weeks | | | | | |

The tumor volumes of mice in different groups were measured on different days and the mean values were

obtained. The results were shown in Table 8 below.

Table 8: Changes in tumor volume in each group of mice over treatment time in the HuPrime® pancreatic cancer PA1222 model

| | **Tumor volume(mm$^3$)** ($\bar{x}$ ±S) | | |
|---|---|---|---|
| **Time** | Group 1 7.5% absolute ethanol + 7.5% polyoxyethylene (35) castor oil + 85% glucose injection D5W (pH 7.4), 0 mg/kg, QW×3, i.v. | Group 2 Gemcitabine, 120 mg/kg, QW×3, i.p. | Group 3 AST, 10 mg/kg, QW×3, i.v. |
| 0 day after initial administration | 112.01±7.53 | 111.91±7.78 | 111.50±8.63 |
| 3 days after initial administration | 139.75±1:12.85 | 13617±12.64 | 117.62±6.91 |
| 7 days after initial administration | 177.43±24.82 | 153.75±10.18 | 113.91±10.61 |
| 10 days after initial administration | 199.20±31.63 | 157.55±9.68 | 107.17±11.77 |
| 14 days after initial administration | 249.21±43.56 | 147.44±16.31 | 66.08±4.54 |
| 17 days after initial administration | 306.79±53.54 | 136.71±13.80 | 42.94±2.17 |
| 21 days after initial administration | 339.57±63.97 | 138.01±14.81 | 23.22+2.75 |
| 24 days after initial administration | 362.58±69.17 | 153.69±22.96 | 21.20±2.38 |
| 28 days after initial administration | 395.45±76.89 | 166.11+24.40 | 12.51±5.25 |

The tumor growth of each treatment group and control group was shown in Table 8 and Fig. 5, and the drug efficacy evaluation was shown in Table 9.

Table 9: Drug efficacy analysis table of each group in the HuPrime® gastric cancer GA6201 model

| **Experiment group** | **Day 28 after first administration (i.e., Day 28, 12/29/2020)** | | | | |
|---|---|---|---|---|---|
| | **Tumor volume** ($\bar{x}$±S) | **Relative tumor volume** - ($\bar{x}$±S) | **TGI (%)** | **T/C (%)** | **P Value (compared with the control group)** |
| Group 1 7.5% absolute ethanol + 7.5% polyoxyethylene (35) castor oil + 85% glucose injection D5W (pH7.4), 0mg/kg, QW×3, i.v. | 395.45±76.89 | 3.49±0.62 | - | - | - |

(continued)

| Experiment group | Day 28 after first administration (i.e., Day 28, 12/29/2020) | | | | |
|---|---|---|---|---|---|
| | Tumor volume ($\bar{x}\pm S$) | Relative tumor volume - ($\bar{x}\pm S$) | TGI (%) | T/C (%) | P Value (compared with the control group) |
| Group 2 Gemcitabine, 120 mg/kg, QW×3, i.p. | 166.11+24.40 | 1.49±0.19 | 57.17% | 42.83% | 0.000778 |
| Group 3 AST, 10 mg/kg, QW×3, i.v. | 12.51±5.25 | 0.10±0.04 | 97.14% | 2.86% | 0.000000914 |

The relative tumor proliferation rate (T/C%) in Table 9 was the percentage of relative tumor volume or tumor weight between the treatment group and the control group at a certain time point. The calculation formula was as follows:

T/C%=TRTV/CRTV× 100% (TRTV: the mean RTV of the treatment group; CRTV: the mean RTV of the vehicle control group; RTV=Vt/V0, V0 was the tumor volume of the animal when grouped, Vt was the tumor volume of the animal after treatment);

The relative tumor growth inhibition rate (TGI (%)) was calculated as follows: TGI% = (1-T/C) × 100%. (T and C were the mean relative tumor volumes (RTV) of the treatment group and the control group at a specific time point, respectively).

Table 10: Relative tumor growth inhibition rate of each group of tumors in the HuPrime® pancreatic cancer PA1222 model

| Group | Dates/Study Days | | | | | | | | |
|---|---|---|---|---|---|---|---|---|---|
| | 12/1/2020 | 12/4/2020 | 12/8/2020 | 12/11/2020 | 12/15/2020 | 12/18/2020 | 12/22/2020 | 12/25/2020 | 12/29/2020 |
| | 0 | 3 | 7 | 10 | 14 | 17 | 21 | 24 | 28 |
| Group 01 | | | | | | | | | |
| Group 02 | 0.09% | 2.56% | 13.35% | 20.91% | 40.84% | 55.44% | 59.36% | 57.61% | 57.99% |
| Group 03 | 0.45% | 15.84% | 35.80% | 46.20% | 73.48% | 86.00% | 93.16% | 94.15% | 96.84% |

The above table was made into a curve graph to obtain Fig. 6.

The body weights of mice in different groups were measured on different days and the mean values were obtained. The results were shown in Table 11 below.

Table 11: Body weight of mice on different inoculation days in the HuPrime® pancreatic cancer PA1222 model

| Group | 0 | 3 | 7 | 10 | 14 | 17 | 21 | 24 | 28 |
|---|---|---|---|---|---|---|---|---|---|
| Group 01 | 24.0 | 24.1 | 24.6 | 24.6 | 24.4 | 24.3 | 25.1 | 24.8 | 25.0 |
| Group 02 | 24.1 | 23.9 | 24.4 | 24.3 | 24.6 | 24.2 | 25.1 | 25.1 | 25.4 |
| Group 03 | 24.0 | 23.5 | 24.0 | 23.6 | 23.6 | 23.6 | 24.0 | 24.0 | 24.1 |

The above table was made into a curve graph to obtain the Fig. 7, which was the body weight curve in each group of mice in the HuPrime® pancreatic cancer PA1222 model.

Similarly, the data in Table 11 was processed to obtain the following Table 12.

Table 12: Percentage change in body weight of mice on different inoculation days in the HuPrime® pancreatic cancer PA1222 model (% Group mean Change=mean((T-T0)/T0)*100, T represented current value, T0 represented initial value)

| Group | 0 | 3 | 7 | 10 | 14 | 17 | 21 | 24 | 28 |
|---|---|---|---|---|---|---|---|---|---|
| Group 01 | 0.00% | 0.67% | 2.49% | 2.75% | 1.91% | 1.52% | 4.70% | 3.60% | 4.52% |
| Group 02 | 0.00% | -0.63% | 1.18% | 0.82% | 2.12% | 0.38% | 4.19% | 4.36% | 5.38% |
| Group 03 | 0.00% | -1.99% | 0.18% | -1.50% | -1.50% | -1.51% | 0.24% | 0.09% | 0.66% |

The above table was made into a curve graph to obtain Fig. 8.

It can be known from the analysis of experimental data that in the respect of therapeutic effect:
The test drug Gemcitabine at the dose of 120 mg/kg (Group 2) had a certain inhibitory effect on tumor growth of HuPrime® pancreatic cancer PA1222, with statistically significant difference compared with the control group. The test drug AST at the dose of 10 mg/kg (Group 3) had a significant inhibitory effect on tumor growth of HuPrime® pancreatic cancer PA1222, with statistically significant difference compared with the control group, and two mice in this group had tumors cured, wherein the cure rate both was 40%. The tumor inhibitory effect of the test drug AST 10mg/kg (Group 3) was significantly better than that of the test drug Gemcitabine (120 mg/kg, Group 2) (p=0.000778).

Analysis of experimental data showed that the mice in the test drug Gemcitabine (120 mg/kg, Group 2) treatment group, AST 10 mg/kg (Group 3) treatment group and control group (Group 1) did not have any obvious weight loss and were well tolerated during the treatment period.

**3. Pharmacodynamics and safety evaluation of the test substances AST and Cisplatin in the HuPrime® lung cancer LU11693 subcutaneous xenograft model**

The HuPrime® lung cancer LU11693 PDX model was a model of KRAS pathogenic mutation having G12C amino acid mutation. BALB/c nude mice were subcutaneously inoculated with HuPrime® model LU11693 tumor blocks so as to establish a subcutaneous transplantation tumor model of human lung cancer. The test was divided into the test drug Cisplatin 4 mg/kg group, the test drug AST 10 mg/kg group and the 7.5% absolute ethanol + 7.5% polyoxyethylene (35) castor oil + 85% glucose injection D5W (pH 7.4) vehicle control group, with a total of 3 groups and 6 mice in each group. The mice in each group were administered by tail vein injection once a week for three consecutive weeks. The therapeutic efficacy was evaluated based on the relative tumor growth inhibition rate (TGI (%)), and the safety was evaluated based on the body weight changes and the death situation of the animals.

The specific administration regimen for each group was shown in Table 13 below.

Table 13: Experimental design of anti-tumor effects of test drugs at different doses in the HuPrime® lung cancer LU11693 PDX tumor model

| Group No. | The number of Animals | Administration group | Dosage (mg/kg) | Administration method | Administration period |
|---|---|---|---|---|---|
| 1 | 6 | 7.5% absolute ethanol + 7.5% polyoxyethylene (35) castor oil + 85% glucose injection D5W (pH 7.4) | 0 | Tail vein | QW×3 |
| 2 | 6 | Cisplatin | 4 | Tail vein | QW×3 |
| 3 | 6 | AST | 10 | Tail vein | QW×3 |
| Note: 1. Dosing volume was 10 μl/g<br>2. QW×3; administered once a week for three weeks | | | | | |

The tumor volumes of mice in different groups were measured on different days and the mean values were obtained. The results were shown in Table 14 below.

Table 14: Changes in tumor volume in each group of mice over treatment time in the HuPrime® lung cancer LU11693 model

| Time | Tumor volume($mm^3$) ($\bar{x}$ ±S) | | |
|---|---|---|---|
| | Group 1 7.5% absolute ethanol + 7.5% polyoxyethylene (35) castor oil + 85% glucose injection D5W (pH 7.4) | Group 2 Cisplatin 4 mg/kg | Group 3 AST 10 mg/kg |
| 0 day after initial administration | 100.73±6.73 | 100.12±6.13 | 100.52±7.48 |
| 4 days after initial administration | 148.49±20.70 | 134.73+12.15 | 136.39±17.11 |
| 7 days after initial administration | 176.25±19.10 | 156.35±14.10 | 150.25±21.20 |
| 11 days after initial administration | 220.66±33.68 | 176.12±15.18 | 164.92±23.46 |
| 14 days after initial administration | 259.63+48.15 | 196.54±20.83 | 175.66+24.12 |
| 18 days after initial administration | 298.53±50.33 | 244.94±21.34 | 216.17±27.04 |
| 21 days after initial administration | 364.53±61.94 | 267.58+22.10 | 221.04±25.74 |
| 25 days after initial administration | 432.25±71.05 | 309.77±23.28 | 220.53±26.00 |
| 28 days after initial administration | 485.88±78.14 | 365.88±29.58 | 221.93±25.52 |

The tumor growth of each treatment group and control group was shown in Table 14 and Fig. 9, and the drug efficacy evaluation was shown in Table 15.

Table 15: Drug efficacy analysis table of each group in the HuPrime® lung cancer LU11693 model

| Experiment group | Day 28 after first administration (i.e., Day 28, 1/28/2(21) | | | | |
|---|---|---|---|---|---|
| | Tumor volume ($\bar{x}$±S) | Relative tumor volume ($\bar{x}$±S) | TGI (%) | T/C (%) | P Value (compared with the control group) |
| Group 1 7.5% absolute ethanol + 7.5% polyoxyethylene (35) castor oil + 85% glucose injection D5W (pH 7.4) | 485.88±78.14 | 4.79±0.58 | - | - | - |
| Group 2 Cisplatin 4 mg/kg | 365.88±29.58 | 3.64±0.14 | 23.98 | 76.02 | 0.0152 |
| Group 3 AST 10 mg/kg | 221.93±25.52 | 2.17±0.11 | 54.64 | 45.36 | 0.00000337 |

The relative tumor proliferation rate (T/C%) in Table 15 was the percentage of relative tumor volume or tumor weight between the treatment group and the control group at a certain time point. The calculation formula was as follows:

T/C%=TRTV/CRTV× 100% (TRTV: the mean RTV of the treatment group; CRTV: the mean RTV of the vehicle control group; RTV=Vt/V0, V0 was the tumor volume of the animal when grouped, Vt was the tumor volume of the animal after treatment);

The relative tumor growth inhibition rate (TGI (%)) was calculated as follows: TGI% = (1-T/C) × 100%. (T and C

were the mean relative tumor volumes (RTV) of the treatment group and the control group at a specific time point, respectively).

Table 16: Relative tumor growth inhibition rate of each group of tumors in the HuPrime® lung cancer LU11693 model

| Group | Dates/Study Days | | | | | | | | |
|---|---|---|---|---|---|---|---|---|---|
| | 12/31/2020 | 1/4/2021 | 1/7/2021 | 1/11/2021 | 1/14/2021 | 1/18/2021 | 1/21/2021 | 1/25/2021 | 1/28/2021 |
| | 0 | 4 | 7 | 11 | 14 | 18 | 21 | 25 | 28 |
| Group 01 | | | | | | | | | |
| Group 02 | 0.61% | 9.27% | 11.29% | 20.19% | 24.30% | 17.95% | 26.60% | 28.34% | 24.70% |
| Group 03 | 0.21% | 8.15% | 14.75% | 25.26% | 32.34% | 27.59% | 39.36% | 48.98% | 54.32% |

The above table was made into a curve graph to obtain Fig. 10.

The body weights of mice in different groups were measured on different days and the mean values were obtained. The results were shown in Table 17 below.

Table 17: Body weight of mice on different inoculation days in the HuPrime® lung cancer LU11693 model

| Group | 0 | 4 | 7 | 11 | 14 | 18 | 21 | 25 | 28 |
|---|---|---|---|---|---|---|---|---|---|
| Group 01 | 24.5 | 24.6 | 24.5 | 24.5 | 24.9 | 24.9 | 24.6 | 24.3 | 24.4 |
| Group 02 | 24.3 | 23.6 | 23.9 | 22.8 | 24.0 | 22.4 | 23.0 | 23.4 | 23.6 |
| Group 03 | 24.3 | 24.3 | 23.9 | 23.3 | 23.6 | 23.3 | 23.0 | 22.4 | 22.8 |

The above table was made into a curve graph to obtain Fig. 11, which was the body weight curve in each group of mice in the HuPrime® lung cancer LU11693 model.

Similarly, the data in Table 17 was processed to obtain the following Table 18.

Table 18: Percentage change in body weight of mice on different inoculation days in the HuPrime® lung cancer LU11693 model (% Group Mean Change=mean((T-T0)/T0)*100, T represented current value, T0 represented initial value)

| Group | 0 | 4 | 7 | 11 | 14 | 18 | 21 | 25 | 28 |
|---|---|---|---|---|---|---|---|---|---|
| Group 01 | 0.00% | 0.53% | 0.07% | 0.20% | 1.54% | 1.81% | 0.63% | -0.54% | -0.18% |
| Group 02 | 0.00% | -2.95% | -1.68% | -6.23% | -1.32% | -8.04% | -5.33% | -3.86% | -2.74% |
| Group 03 | 0.00% | 0.15% | -1.86% | -4.24% | -3.09% | -4.15% | -5.47% | -7.78% | -6.38% |

The above table was made into a curve graph to obtain Fig. 12.

It can be known from the analysis of experimental data that in the respect of therapeutic effect:
The test drug Cisplatin (4 mg/kg) treatment group showed a certain tumor inhibitory effect on Day 28 after first administration, with a statistically significant difference compared with the control group (p=0.0152), and the relative tumor growth inhibition rate TGI (%) was 23.98%.

The test drug AST (10 mg/kg) treatment group showed a certain tumor inhibitory effect on Day 28 after first administration, with a statistically significant difference compared with the control group (p<0.001), and the relative tumor growth inhibition rate TGI (%) was 54.64%, while the TGI was less than 60% with no obvious tumor inhibitory effect.

Analysis of the experimental data showed that some mice in the test drug AST (10 mg/kg) and Cisplatin (4 mg/kg) treatment groups displayed severe weight loss, which might be related to the potential toxicity of high-dose drugs.

From the above three sets of experimental data, we can draw the following conclusions:

1. AST-3424 and AST both had significant drug efficacy in the KRAS pathogenic mutation models having G12D amino acid mutation: gastric cancer GA6021 and pancreatic cancer PA1222 PDX model, with TGI% greater than 90%;
2. The tumor inhibitory effect of AST in the KRAS pathogenic mutation model having G12C amino acid mutation: lung cancer LU11693 was not obvious, with TGI% less than 60%;
3. Both AST-3424 and AST had relatively good tolerance in each model.

The inventor further found through research that in the KRAS pathogenic mutation model having G12D amino acid mutation, AST-3424 and AST had generally significant therapeutic effects on a variety of cancer indications. In the KRAS pathogenic mutation model having G12C amino acid mutation, the therapeutic effect of AST was not only related to the cancer type, but also related to other factors.

**4. Anti-tumor effect and safety evaluation of the test substances AST, AST-3424 and Ifosfamide in human-derived pancreatic cancer HPAF-II subcutaneous xenograft model**

The human-derived pancreatic cancer HPAF-II subcutaneous xenograft model was a CDX model harboring the KRAS G12D pathogenic mutation.

Balb/c nude female mice were subcutaneously inoculated with human-derived pancreatic cancer HPAF-II cells so as to establish a subcutaneous transplantation model of human-derived pancreatic cancer. The test was divided into the following groups: the test drug treatment group Ifosfamide 60 mg/kg monotherapy group (Group 2) was administered intraperitoneally once a day for 5 consecutive days, rested for 2 days, and then administered once a day for 5 consecutive days again; AST 4 mg/kg monotherapy group (Group 3) was administered through the tail vein once a day for 5 consecutive days, rested for 2 days, then rested for 2 weeks, and then administered once a day for 5 consecutive days again; AST 8 mg/kg monotherapy group (Group 4) was administered through the tail vein once a week for a total of 3 weeks; AST-3424 1 mg/kg monotherapy group (Group 5) was administered through the tail vein once a day for 5 consecutive days, rested for 2 days, then rested for 2 weeks, and then administered once a day for 5 consecutive days again; and glucose injection (pH 7.7-8.0) vehicle control group (Group 1) was administered through the tail vein once a day for 5 consecutive days, rested for 2 days, then rested for 2 weeks, and then administered once a day for 5 consecutive days again. There were 5 groups in total and 6 mice in each group in this study. The administration routes, dosages and regimens of the experimental design were shown in Table 19.

Table 19: Administration routes, dosages and regimens in human-derived pancreatic cancer HPAF-II animal model

| Group No. | The number of Animals | Administration group | Dosage (mg/kg) | Administration method | Administration period |
|---|---|---|---|---|---|
| 1 | 6 | glucose injection (pH 7.7-8.0) | - | *i.v.* | QDx5, 2 days off, 2 weeks off, QD×5 |
| 2 | 6 | Ifosfamide | 60 | *i.p.* | QD×5 week×2 weeks |
| 3 | 6 | AST | 4 | *i.v.* | QD×5, 2 days off, 2 weeks off, QD×5 |
| 4 | 6 | AST | 8 | *i.v.* | QW×3 |
| 5 | 6 | AST-3424 | 1 | *i.v.* | QD×5, 2 days off, 2 weeks off, QD×5 |

In the present application, with respect to the abbreviations related to administration route, i.v. represented tail vein injection, *i.p.* represented intraperitoneal injection; with respect to the abbreviations related to the dosing cycle, QW represented once a week, QD represented once a day, "QD*5, 2 days off, 2 weeks off, QD*5" represented administering once a day for 5 consecutive days, resting for 2 days, then resting for 2 weeks, and then administering once a day for 5 consecutive days again.

The tumor growth of each treatment group and control group was recorded on different days of the test, as shown in Table 20. The corresponding growth curve of tumor volume in each group of mice was shown in Fig. 13. The therapeutic effect was evaluated based on the relative tumor proliferation rate and relative tumor growth inhibition rate. The drug efficacy analysis of each group was shown in Table 21. The body weight changes of the treatment group and the control group after administration were recorded, and the safety of each group in the human-derived pancreatic cancer HPAF-II subcutaneous xenograft model was studied. The results of body weight changes of mice were shown in Table 22. The curve graph of the percentage change of body weight in each treatment group over time was shown in Fig. 14.

Table 20: Changes in tumor volume in each group of mice over treatment time in the human-derived pancreatic cancer HPAF-II subcutaneous model ($mm^3$)

| Group No. | Days after inoculation | | | | | | | | | |
|---|---|---|---|---|---|---|---|---|---|---|
| | 0 | 3 | 7 | 10 | 14 | 17 | 21 | 24 | 28 | 31 |
| Group 1 | 148.71 | 298.82 | 427.94 | 521.32 | 669.76 | 844.44 | 1064.44 | 1321.14 | 1787.35 | 2212.64 |
| Group 2 | 148.64 | 313.53 | 436.26 | 588.61 | 801.41 | 1041.60 | 1347.90 | 1617.30 | 195958 | 2678.00 |
| Group 3 | 148.81 | 286.38 | 287.09 | 223.40 | 199.45 | 189.54 | 274.92 | 373.75 | 422.27 | 457.66 |
| Group 4 | 148.63 | 320.80 | 364.88 | 306.82 | 262.16 | 241.53 | 200.54 | 188.10 | 163.95 | 170.65 |
| Group 5 | 149.44 | 344.77 | 382.62 | 285.47 | 243.00 | 263.64 | 344.86 | 45708 | 596.76 | 685.85 |

Table 21: Drug efficacy analysis table of each group in the human-derived pancreatic cancer HPAF-II subcutaneous model

| **Experiment group** | **Day 31 after grouping** | | | | |
|---|---|---|---|---|---|
| | **Tumor volume** ($\bar{x}\pm$S) | **Relative tumor volume** ($\bar{x}\pm$S) | **TGI (%)** | **T/C (%)** | **P Value (comparing with the control group)** |
| **Group 1** Glucose injection (pH 7.7-8.0), 10 μL/g, i.v., QD×5, 2 days off, 2 weeks off, QD×5 | 2212.64±232.88 | 15.17±1.64 | - | - | - |
| **Group 2** Ifosfamide, 60 mg/kg,10 μL/g, i.p., QD×5/week×2weeks | 2678.00±517.46 | 17.38±2.69 | -14.56 | 114.56 | >0.05 |
| **Group 3** AST, 4 mg/kg,10 μL/g, i.v., QD×5, 2 days off, 2 weeks off, QD×5 | 457.66×69.87 | 3.10±0.40 | 79.55 | 20.45 | <0.001*** |
| **Group 4** AST, 8 mg/kg,10 μL/g, i.v., QW×3 | 170.65±56.64 | 1.12±0.32 | 92.64 | 7.36 | <0.001*** |
| **Group 5** AST-3424, 1 mg/kg,10 μL/g, i.v., QD×5, 2 days off, 2 weeks off, QD×5 | 685.85±130.78 | 4.63±0.79 | 69.46 | 30.54 | <0.001*** |

Table 22: Changes in body weight of mice in the human-derived pancreatic cancer HPAF-II subcutaneous model

| Group No. | 0 | 1 2 | | 3 | 4 | 7 | 8 | 9 | 10 | 11 | 14 | 17 | 21 | 22 | 23 | 24 | 25 | 28 | 31 |
|---|---|---|---|---|---|---|---|---|---|---|---|---|---|---|---|---|---|---|---|
| Group 1 | 23.9 | 23.6 | 23.7 | 24.0 | 23.8 | 24.2 | - | - | 25.4 | - | 25.1 | 25.4 | 26.3 | 26.4 | 26.3 | 26.2 | - | 26.3 | 26.8 |
| Group 2 | 23.3 | 23.1 | 23.2 | 23.5 | 23.2 | 23.3 | 23.2 | 23.8 | 23.1 | 23.6 | 23.6 | 24.8 | 25.9 | - | - | 25.6 | - | 25.4 | 25.9 |
| Group 3 | 23.4 | 23.7 | 23.6 | 23.8 | 23.4 | 23.9 | - | - | 24.8 | - | 24.5 | 24.7 | 25.0 | 25.1 | 25.3 | 25.2 | 25.6 | 25.3 | 25.8 |
| Group 4 | 23.5 | - | - | 24.0 | - | 24.2 | - | - | 24.5 | - | 24.2 | 25.0 | 25.1 | - | - | 25.3 | - | 25.6 | 25.5 |
| Group 5 | 22.8 | 22.7 | 22.8 | 23.1 | 22.9 | 23.3 | - | - | 24.2 | - | 23.9 | 24.1 | 24.5 | 24.6 | 24.8 | 24.6 | 24.9 | 24.9 | 25.6 |

The mean tumor volume of mice in the vehicle control group was 2212.64 $mm^3$ on Day 31 after the initial administration. The mean tumor volume of the test drug Ifosfamide treatment group (Group 2) at the dose of 60 mg/kg on Day 31 was 2678.00 $mm^3$, and the relative tumor growth inhibition rate TGI (%) was -14.65%, with no statistically significant difference compared with the control group (p>0.05).

The mean tumor volumes of the test drug AST treatment groups (Groups 3 and 4) at the dose of 4 mg/kg (QD×5, 2 days off, 2 weeks off, QD×5) and at the dose of 8 mg/kg (QW×3), and AST-3424 group (Group 5) at the dose of 1 mg/kg (QD×5, 2 days off, 2 weeks off, QD×5) on Day 31 were 457.66, 170.65 and 685.85 $mm^3$, respectively, with statistically significant difference compared with the control group (p<0.05), and the relative tumor growth inhibition rates TGI (%) were 79.55%, 92.64% and 69.46%, respectively.

The above experimental results showed that AST-3424 and AST in the model having KRAS G12D pathogenic mutation, AST at the dose of 8 mg/kg (QW×3), at the dose of 4 mg/kg (QD×5, 2 days off, 2 weeks off, QD× 5), and AST-3424 at the dose of 1 mg/kg (QD × 5, 2 days off, 2 weeks off, QD × 5) had a significant anti-tumor effect on the human-derived pancreatic cancer HPAF-II subcutaneous model. Moreover, the mice in each test drug treatment group did not lose weight and were well tolerated during the treatment period.

**5. Anti-tumor effect and safety evaluation of the test substances AST, AST-3424 and Ifosfamide monotherapy in the HuPrime® lung cancer LU5161 subcutaneous model**

The HuPrime® lung cancer LU5161 subcutaneous model was a PDX model harboring the KRAS G12D pathogenic mutation.

Balb/nude female mice were subcutaneously inoculated with HuPrime® lung cancer LU5161 tumor blocks so as to establish a subcutaneous transplantation tumor model of human lung cancer. The test was divided into the following groups: the test drug Ifosfamide 60 mg/kg monotherapy group (Group 2) was administered once a day for 5 consecutive days, rested for 2 days, and then administered once a day for 5 consecutive days again; AST 4 mg/kg monotherapy group (Group 3) and AST 8 mg/kg monotherapy group (Group 4) were administered once a week for a total of 3 weeks; AST 4 mg/kg monotherapy group (Group 5) and AST-3424 1 mg/kg monotherapy group (Group 6) was administered once a day for 5 consecutive days, rested for 2 days, then rested for 2 weeks, and then administered once a day for 5 consecutive days again; and glucose injection (pH 7.7-8.0) vehicle control group (Group 1) was administered once a day for 5 consecutive days, rested for 2 days, then rested for 2 weeks, and then administered once a day for 5 consecutive days again. There were 6 groups in total and 6 mice in each group in this study. Among them, the test drug Ifosfamide was administered intraperitoneally, and the vehicle control group, AST, and AST-3424 were all administered by tail vein injection. The administration routes, dosages and regimens of the experimental design were shown in Table 23.

Table 23: Administration routes, dosages and regimens in the HuPrime® lung cancer LU5161 subcutaneous model

| Group No. | The number of Animals | Administration group | Dosage (mg/kg) | Administration method | Administration period |
|---|---|---|---|---|---|
| 1 | 6 | Glucose injection (pH 7.7-8.0) | - | *i.v.* | QD×5, 2 days off, 2 weeks off, QD×5 |
| 2 | 6 | Ifosfamide | 60 | *i.p.* | QDx5/week×2 weeks |
| 3 | 6 | AST | 4 | *i.v.* | QW×3 |
| 4 | 6 | AST | 8 | *i.v.* | QW×3 |
| 5 | 6 | AST | 4 | *i.v.* | QD×5, 2 days off, 2 weeks off, QD×5 |
| 6 | 6 | AST-3424 | 1 | *i.v.* | QD×5, 2 days off, 2 weeks off, QD×5 |

The tumor growth of each treatment group and control group was recorded on different days of the test, as shown in Table 24. The corresponding growth curve of tumor volume in each group of mice was shown in Fig. 15. The therapeutic effect was evaluated based on the relative tumor proliferation rate and relative tumor growth inhibition rate. The drug efficacy analysis of each group was shown in Table 25. The body weight changes of the treatment group and the control group after administration was recorded, and the safety of each group in the HuPrime® lung cancer LU5161 subcutaneous xenograft model was studied. The results of body weight changes of mice were shown in Table 26. The curve graph of the percentage changes of body weight in each treatment group over time was shown in Fig. 16.

Table 24: Changes in tumor volume in each group of mice over treatment time in the HuPrime® lung cancer LU5161 subcutaneous model ($mm^3$)

| | Days after inoculation | | | | | | | | | |
|---|---|---|---|---|---|---|---|---|---|---|
| Group No. | 0 | 4 | 7 | 11 | 14 | 18 | 21 | 25 | 28 | 31 |
| 1 | 132.27 | 185.36 | 393.39 | 678.36 | 839.01 | 1106.30 | 1423.45 | 1777.59 | 2238.97 | 2045.61 |
| 2 | 132.21 | 182.89 | 322.31 | 462.34 | 577.36 | 696.39 | 957.80 | 1284.49 | 1636.39 | 1887.60 |
| 3 | 132.11 | 144.28 | 209.41 | 206.30 | 161.45 | 136.83 | 103.27 | 88.21 | 71.72 | 58.51 |
| 4 | 133.19 | 124.17 | 179.20 | 165.66 | 122.07 | 95.27 | 86.00 | 75.80 | 68.18 | 49.88 |
| 5 | 133.15 | 111.35 | 110.86 | 86.51 | 66.85 | 62.20 | 53.86 | 47.74 | 37.14 | 27.78 |
| 6 | 133.17 | 145.58 | 155.12 | 199.04 | 171.71 | 134.63 | 134.55 | 103.78 | 94.42 | 85.23 |

Table 25: Drug efficacy analysis table of each group in the HuPrime® lung cancer LU5161 subcutaneous model

| Experiment group | Day 28 | | | | | |
|---|---|---|---|---|---|---|
| | **Tumor volume** ($\bar{x}\pm S$) | **Relative tumor volume** ($\bar{x}\pm S$) | **TGI (%)** | **T/C (%)** | **P Value (compared with the control group)** | **Tumor clearance rate Day 31** |
| **Group 1** Glucose injection (pH 7.7-8.0), 10 μL/g, *i.v.,* QD×5, 2 days off, 2 weeks off, QD×5 | 2238.97±391.43 | 17.05±3.10 | - | - | - | 0/6 |
| **Group 2** Ifosfamide, 60 mg/kg, 10 μL/g,*i.p.,* QD×5/week×2weeks | 1636.39+234.40 | 12.38±1.21 | 27.39 | 72.61 | >0.05 | 0/6 |
| **Group 3** AST, 4 mg/kg, 10 μL/g, *i.v.*, QWx3 | 71.72±21.03 | 0.53±0.16 | 96.92 | 3.08 | <0.001*** | 1/6 |
| **Group 4** AST, 8 mg/kg, 10 μL/g, *i.v.*, QW×3 | 68.18+18.97 | 0.50±0.11 | 97.05 | 2.95 | <0.001*** | 1/6 |
| **Group 5** AST, 4 mg/kg, 10 μL/g, *i.v.*, QD×5, 2 days off, 2 weeks off, QD×5 | 3714±6.33 | 0.28×0.04 | 98.38 | 1.62 | <0.001*** | 1/6 |
| **Group 6** AST-3424, 1 mg/kg, 10 μL/g, *i.v.*, QDx5, 2 days off, 2 weeks off, QD×5 | 94.42+23.39 | 0.75±0.21 | 95.62 | 4.38 | <0.001*** | 1/6 |

Table 26: Percentage change in body weight of mice in the HuPrime® lung cancer LU5161 subcutaneous model

| Group No. | Days after administration | | | | | | | | | | | | | | |
|---|---|---|---|---|---|---|---|---|---|---|---|---|---|---|---|
| | 0 | 1 | 2 | 3 | 4 | 7 | 11 | 14 | 18 | 21 | 22 | 23 | 24 | 25 | 28 | 31 |
| 1 | 0.00% | 0.24% | -0.03% | -0.88% | -2.09% | 0.11% | 7.31% | -3.03% | 8.10% | -1.66% | 10.74% | 3.54% | 0.09% | 4.36% | 2.14% | -3.04% |
| 2 | 0.00% | 4.81% | 0.76% | -1.96% | -3.53% | -3.86% | -1.15% | -7.43% | 3.92% | -1.71% | - | - | - | 1.76% | 1.34% | -5.07% |
| 3 | 0.00% | - | - | - | -3.51% | -2.32% | 4.33% | -4.89% | 8.46% | 2.57% | - | - | - | 5.72% | 5.99% | 4.57% |
| 4 | 0.00% | - | - | - | -3.88% | -1.58% | 2.80% | -3.82% | 3.09% | 2.73% | - | - | - | 4.54% | 5.95% | 3.58% |
| 5 | 0.00% | 1.92% | 1.12% | -1.35% | -1.45% | 2.90% | 3.96% | 3.59% | 10.22% | 4.17% | 9.52% | 5.89% | 5.29% | 7.03% | 7.88% | 4.83% |
| 6 | 0.00% | 0.49% | -3.79% | -3.57% | -3.51% | -0.88% | -1.37% | 1.02% | 12.01% | 3.21% | 13.16% | 4.38% | 1.05% | 1.17% | 7.51% | 3.83% |

The mean tumor volume of mice in the vehicle control group was 2238.97 $mm^3$ on Day 28 after the initial administration. The mean tumor volume of the test drug Ifosfamide treatment group (Group 2) at the dose of 60 mg/kg on Day 28 was 1636.39 $mm^3$, and the relative tumor growth inhibition rate TGI (%) was 27.39%, with no statistically significant difference compared with the control group (p>0.05).

The mean tumor volumes of the test drug AST treatment groups at the dose of 4 mg/kg (QW×3, Group 3), at the dose of 8 mg/kg (QW×3,Group 4), at the dose of 4 mg/kg (QD×5, 2 days off, 2 weeks off, QD×5, Group 5), and AST-3424 group (Group 6) at the dose of 1 mg/kg (QD×5, 2 days off, 2 weeks off, QD×5) on Day 28 were 71.72 $mm^3$, 68.18 $mm^3$, 37.14 $mm^3$ and 94.42 $mm^3$, respectively, with a statistically significant difference compared with the control group (p<0.001), and the relative tumor growth inhibition rates TGI (%) were 96.92%, 97.05%, 98.38% and 95.62%, respectively. Each one mouse in all treatment groups of test drugs AST and AST-3424 had tumors cleared, with a clearance rate of 16.7%.

The above experimental results showed that AST-3424 and AST in the model having KRAS G12D pathogenic mutation, AST treatment groups (Group 3, Group 4 and Group 5) at the dose of 4 mg/kg (QW×3), at the dose of 8 mg/kg (QW×3), at the dose of 4 mg/kg (QD×5, 2 days off, 2 weeks off, QD × 5), and AST-3424 group (Group 6) at the dose of 1 mg/kg (QD × 5, 2 days off, 2 weeks off, QD × 5) had a significant anti-tumor effect on the HuPrime® lung cancer LU5161 subcutaneous model under the tested dosage and dosing frequency of this study. Ifosfamide administration group had no tumor inhibitory effect. The mice in each test drug treatment group were well tolerated during the treatment period.

### 6. Anti-tumor effect and safety evaluation of the test substances AST and Ifosfamide monotherapy in the HuPrime® intestinal cancer CR3820 subcutaneous model

The HuPrime® intestinal cancer CR3820 subcutaneous model was a PDX model harboring the KRAS G12D pathogenic mutation.

NOD.SCID female mice were subcutaneously inoculated with HuPrime® intestinal cancer CR3820 tumor blocks so as to establish a subcutaneous transplantation tumor model of human intestinal cancer. The test was divided into the following groups: the test drug Ifosfamide 60 mg/kg monotherapy group (QD×5/week×2 weeks, Group 2) was administered intraperitoneally once a day for 5 consecutive days, rested for 2 days, and then administrated once a day for 5 consecutive days again; AST 8 mg/kg monotherapy group (QW×3, Group 3) was administered through the tail vein once a week for a total of 3 weeks; AST 4 mg/kg monotherapy group (QD × 5, 2 days off, 2 weeks off, QD × 5, Group 4) and the vehicle control group glucose injection (pH 7.7-8.0, Group 1) both were administered through the tail vein, and both had the same dosing cycle: administering once a day for 5 consecutive days, resting for 2 days, then resting for 2 weeks, and then administering once a day for 5 consecutive days again. There are 4 groups in total and 6 mice in each group in this experiment. The administration routes, dosages and regimens of the experimental design were shown in Table 27.

Table 27: Administration routes, dosages and regimens in the HuPrime® intestinal cancer CR3820 subcutaneous model

| Group No. | The number of Animals | Administration group | Dosage (mg/kg) | Administration method | Administration period |
|---|---|---|---|---|---|
| 1 | 6 | Glucose injection (pH 7.7-8.0) | - | *i.v.* | QD×5, 2 days off; 2 weeks off, QD×5 |
| 2 | 6 | Ifosfamide | 60 | *i.p.* | QD×5/week×2 weeks |
| 3 | 6 | AST | 8 | *i.v.* | QW×3 |
| 4 | 6 | AST | 4 | *i.v.* | QD×5, 2 days off; 2 weeks off, QD×5 |

The tumor growth of each treatment group and control group was recorded on different days of the test, as shown in Table 28. The corresponding growth curve of tumor volume in each group of mice was shown in Fig. 17. The therapeutic effect was evaluated based on the relative tumor proliferation rate and relative tumor growth inhibition rate. The drug efficacy analysis of each group was shown in Table 29. The body weight changes of the treatment group and the control group after administration were recorded, and the safety of each group in the HuPrime® intestinal cancer CR3820 subcutaneous xenograft model was studied. The results of body weight changes of the mice were shown in Table 30. Correspondingly, the curve graph of the percentage changes of body weight in each treatment group over time was shown in Fig. 18.

Table 28: Changes in tumor volume in each group of mice over treatment time in the HuPrime® intestinal cancer CR3820 subcutaneous model ($mm^3$)

| Group | Days after initial administration | | | | | | | | | |
|---|---|---|---|---|---|---|---|---|---|---|
| | 0 | 3 | 7 | 10 | 14 | 17 | 21 | 24 | 28 | 31 |
| 01 | 137.43 | 171.88 | 387.71 | 591.57 | 923.47 | 1171.58 | 1624.45 | 1792.37 | 1865.61 | 2203.47 |
| 02 | 137.64 | 183.78 | 362.76 | 495.03 | 640.83 | 758.49 | 1099.76 | 1199.09 | 1596.60 | 1753.12 |
| 03 | 137.23 | 172.55 | 209.91 | 197.67 | 176.74 | 148.74 | 121.71 | 110.59 | 85.22 | 88.44 |
| 04 | 137.49 | 148.90 | 177.26 | 147.06 | 137.48 | 117.53 | 152.25 | 146.58 | 73.81 | 75.21 |

Table 29: Drug efficacy analysis table of each group in the HuPrime® intestinal cancer CR3820 subcutaneous model

| Experiment group | Day 24 | | | | |
|---|---|---|---|---|---|
| | **Tumor volume -** ($x\pm$S) | **Relative tumor volume-**($x\pm$S) | **TGI (%)** | **T/C (%)** | **P Value (compared with the control group)** |
| **Group 1** Glucose injection (pH 7.7-8.0), 10 μL/g, *i.v.*, QD×5; 2 days off; 2weeks off; QD×5 | 1792.37±347.13 | 13.58±7.27 | - | - | - |
| **Group 2** Ifosfamide, 60 mg/kg, 10 μL//g, *i.p.,* QD×5/week×2weeks | 1199.09+228.15 | 8.46±2.85 | 37.73 | 62.27 | >0.05 |
| **Group 3** AST, 8 mg/kg, 10 μL/g, i.v., QW×3 | 110.59±13.60 | 0.80±0.24 | 94.08 | 5.92 | <0.001*** |
| **Group 4** AST, 4 mg/kg, 10 μL/g, *i.v.*, QD×5; 2 days off; 2weeks off; QD×5 | 146.58±44.99 | 1.03±0.71 | 92.44 | 7.56 | <0.001*** |

Table 30: Changes in body weight of mice in the HuPrime® intestinal cancer CR3820 subcutaneous model

| Group | Days after initial administration | | | | | | | | | | | | | | | |
|---|---|---|---|---|---|---|---|---|---|---|---|---|---|---|---|---|
| | 0 | 1 | 2 | 3 | 4 | 7 | 10 | 14 | 17 | 21 | 22 | 23 | 24 | 25 | 28 | 31 |
| 01 | 24.5 | 23.8 | 24.5 | 24.0 | 23.6 | 24.3 | 24.1 | 24.4 | 24.6 | 25.0 | 24.1 | 24.0 | 23.4 | 23.1 | 22.2 | 22.5 |
| 02 | 25.0 | 24.5 | 25.1 | 25.2 | 24.1 | 24.1 | 23.7 | 24.1 | 24.7 | 25.5 | - | - | 25.2 | - | 25.1 | 25.4 |
| 03 | 25.5 | - | - | 25.2 | - | 25.9 | 25.5 | 25.7 | 26.4 | 26.5 | - | - | 26.4 | - | 26.0 | 26.4 |
| 04 | 24.8 | 25.1 | 25.3 | 25.3 | 24.9 | 25.3 | 25.6 | 24.9 | 25.5 | 26.3 | 25.8 | 25.1 | 25.8 | 25.9 | 25.2 | 25.9 |

The mean tumor volume of mice in the vehicle control group was 1792.37 $mm^3$ on Day 24 after the initial administration. The mean tumor volume of the test drug Ifosfamide treatment group (QD×5/week×2 weeks, Group 2) at the dose of 60 mg/kg on Day 24 was 1199.09 $mm^3$, and the relative tumor growth inhibition rate TGI (%) was 37.73%, with no statistically significant difference compared with the control group ($p>0.05$).

The mean tumor volumes of the test drug AST treatment groups at the dose of 8 mg/kg (QW × 3, Group 3), at the dose of 4 mg/kg (QD × 5, 2 days off, 2 weeks off, QD × 5, Group 4) on Day 24 were 110.59 $mm^3$ and 146.58 $mm^3$, and the relative tumor growth inhibition rates TGI (%) were 94.08% and 92.44%, respectively, with statistically significant difference compared with the control group ($p<0.05$).

The above experimental results showed that AST in the model having KRAS G12D pathogenic mutation, the test drug AST 8 mg/kg (QW×3, Group 3), 4 mg/kg (QD×5, 2 days off, 2 weeks off, QD× 5, Group 4) had a statistically significant anti-tumor effect on the HuPrime® intestinal cancer CR3820 subcutaneous model at the tested dosage and dosing frequency of this experiment. The test drugs Ifosfamide and AST were well tolerated at the tested doses in this study.

**7. Anti-tumor effect and safety evaluation of the test substances AST, AST-3424 and Ifosfamide monotherapy in the HuPrime® pancreatic cancer PA2637 subcutaneous model**

The HuPrime® pancreatic cancer PA2637 subcutaneous model was a PDX model harboring the KRAS G12D pathogenic mutation.

NOD.SCID female mice were subcutaneously inoculated with the HuPrime® pancreatic cancer PA2637 tumor blocks so as to establish a subcutaneous transplantation tumor model of human pancreatic cancer. The test was divided into the following groups: the test drug Ifosfamide 60 mg/kg monotherapy treatment group (QD×5/week×2 weeks, Group 2) was administered intraperitoneally once a day for 5 consecutive days, rested for 2 days, and then administered

once a day for 5 consecutive days again; AST 8 mg/kg monotherapy group (QW×3, Group 3) was administered through the tail vein once a week for a total of 3 weeks; AST 4 mg/kg monotherapy group (QD×5, 2 days off, 2 weeks off, QD×5, Group 4), AST-3424 1 mg/kg monotherapy group (QD×5, 2 days off, 2 weeks off, QD×5, Group 5) and the vehicle control group glucose injection (pH 7.7-8.0, Group 1) both were administered through the tail vein, and both had the same dosing cycle: administering once a day for 5 consecutive days, resting for 2 days, then resting for 2 weeks, and then administering once a day for 5 consecutive days again. There were 5 groups in total and 6 mice in each group in this experiment. The administration routes, dosages and regimens of the experimental design were shown in Table 31.

Table 31: Administration routes, dosages and regimens of the HuPrime® pancreatic cancer PA2637 subcutaneous model

| Group No. | The number of Animals | Administration group | Dosage (mg/kg) | Administration method | Administration period |
|---|---|---|---|---|---|
| 1 | 6 | Glucose injection (pH 7.7-8.0) | - | *i.v.* | QD×5, 2 days off, 2 weeks off, QD×5 |
| 2 | 6 | Ifosfamide | 60 | *i.p.* | QD×5/week×2 weeks |
| 3 | 6 | AST | 8 | *i.v.* | QW×3 |
| 4 | 6 | AST | 4 | *i.v.* | QD×5, 2 days off, 2 weeks off, QD×5 |
| 5 | 6 | AST-3424 | 1 | *i.v.* | QD×5, 2 days off, 2 weeks off, QD×5 |

The tumor growth of each treatment group and control group was recorded on different days of the test, as shown in Table 32. The corresponding growth curve of tumor volume in each group of mice was shown in Fig. 19. The therapeutic effect was evaluated based on the relative tumor proliferation rate and relative tumor growth inhibition rate. The drug efficacy analysis of each group was shown in Table 33. The body weight changes of the treatment group and the control group after administration were recorded, and the safety of each group in the HuPrime® pancreatic cancer PA2637 subcutaneous xenograft model was studied. The results of body weight changes of the mice were shown in Table 34. Correspondingly, the curve graph of the percentage changes of body weight in each treatment group over time was shown in Fig. 20.

Table 32: Changes in tumor volume in each group of mice over treatment time in the HuPrime® pancreatic cancer PA2637 subcutaneous model ($mm^3$)

| Group | Days after initial administration | | | | | | | | | | |
|---|---|---|---|---|---|---|---|---|---|---|---|
| | 0 | 3 | 7 | 10 | 14 | 17 | 21 | 24 | 28 | 31 | 35 |
| Group 01 | 130.37 | 186.35 | 246.28 | 308.00 | 402.92 | 472.45 | 531.56 | 606.41 | 700.31 | 820.04 | 977.46 |
| Group 02 | 130.24 | 211.69 | 295.87 | 307.84 | 377.51 | 437.58 | 491.24 | 582.72 | 684.36 | 771.40 | 938.33 |
| Group 03 | 130.35 | 206.38 | 221.16 | 235.81 | 190.59 | 178.00 | 138.24 | 125.43 | 115.08 | 103.87 | 123.47 |
| Group 04 | 130.55 | 184.14 | 235.94 | 210.81 | 223.54 | 204.85 | 175.78 | 170.26 | 160.17 | 125.16 | 141.48 |
| Group 05 | 130.32 | 184.00 | 204.51 | 190.00 | 167.36 | 185.97 | 188.97 | 201.89 | 230.64 | 169.27 | 186.08 |

Table 33: Drug efficacy analysis table of each group in the HuPrime® pancreatic cancer PA2637 subcutaneous model

| Experiment group | Day 35 | | | | |
|---|---|---|---|---|---|
| | **Tumor volume** - ($x$±S) | **Relative tumor volume** - ($x$±S) | **TGI (%)** | **T/C (%)** | **P Value (compared with the control group)** |
| **Group 1** Glucose injection ((pH 7.7-8.0), 10 μL/g, *i.v.*, QD×5; 2 days off; 2weeks off; QD×5 | 977.46±113.44 | 7.45±0.73 | - | - | - |
| **Group 2** Ifosfamide, 60 mg/kg, 10 μL/g, *i.p.*, QD×5/week×2weeks | 938.33±143.94 | 7.25±1.18 | 2.74 | 97.26 | >0.05 |
| **Group 3** AST, 8 mg/kg, 10 μL/g, *i.v*, QWx3 | 123.47+21.61 | 0.95±0.16 | 87.28 | 12.72 | <0.001*** |
| **Group 4** AST, 4 mg/kg, 10 μL/g, *i.v.*, QDx5; 2 days off; 2weeks off; QD×5 | 141.48±13.32 | 1.08±0.10 | 85.46 | 14.54 | <0.001*** |
| **Group 5** AST-3424, 1 mg/kg, 10 μL/g, *i.v.*, QD×5; 2 days off; 2weeks off; QD×5 | 186.08±29.94 | 1.43±0.23 | 80.78 | 19.22 | <0.001*** |

Table 34: Percentage changes in body weight of mice in the HuPrime® pancreatic cancer PA2637 subcutaneous model

| Group | Days after initial administration | | | | | | | | | | | | | | |
|---|---|---|---|---|---|---|---|---|---|---|---|---|---|---|---|
| | 0 | 1 | 2 | 3 | 4 | 7 | 10 | 14 | 17 | 21 | 22 | 23 | 24 | 28 | 31 | 35 |
| 01 | 0.00% | 0.35% | -0.87% | -1.42% | -1.42% | -0.76% | -1.90% | -0.63% | -0.55% | -0.96% | -2.41% | -2.65% | -3.61% | -2.23% | -1.71% | -2.96% |
| 02 | 0.00% | -0.20% | -0.74% | -1.17% | -2.24% | -1.92% | -4.05% | -1.42% | -3.36% | -0.68% | - | - | -4.03% | -2.16% | -1.99% | -1.15% |
| 03 | 0.00% | - | - | 0.09% | - | 0.45% | -1.49% | -0.09% | -0.89% | -0.12% | - | - | -0.33% | 0.19% | -1.29% | 2.76% |
| 04 | 0.00% | 0.00% | -0.97% | -1.51% | -4.99% | -2.61% | -3.22% | -0.81% | -1.66% | -0.85% | -3.32% | -2.91% | -3.63% | -1.82% | -1.36% | 0.78% |
| 05 | 0.00% | -1.47% | -2.58% | -1.85% | -2.73% | -1.48% | -3.80% | -2.90% | -1.58% | -1.29% | -3.42% | -3.61% | -3.68% | -3.46% | -3.20% | -1.43% |

The mean tumor volume of mice in the vehicle control group was 977.46 $mm^3$ on Day 35 after the initial administration. The mean tumor volume of the test drug Ifosfamide treatment group (Group 2) at the dose of 60 mg/kg on Day 35 was 938.33 $mm^3$, and the relative tumor growth inhibition rate TGI (%) was 2.74%, with no statistically significant difference compared with the control group (p>0.05).

The mean tumor volumes of the test drug AST treatment groups at the dose of 8 mg/kg (QW×3, Group 3), at the dose of 4 mg/kg (QD×5, 2 days off, 2 weeks off, QD×5, Group 4), and the test drug AST-3424 at the dose of 1 mg/kg (QD×5, 2 days off, 2 weeks off, QD×5, Group 5) on Day 35 were 123.47 $mm^3$, 141.48 $mm^3$ and 186.08 $mm^3$, and the relative tumor growth inhibition rates TGI (%) were 87.28%, 85.46% and 80.78%, respectively, with statistically significant difference compared with the control group (p<0.001).

The above experimental results showed that AST and AST-3424 in the model having KRAS G12D pathogenic mutation, the test drug AST 8 mg/kg (QW×3, Group 3), 4 mg/kg (QD×5, 2 days off, 2 weeks off, QD×5, Group 4) and the test drug AST-3424 1 mg/kg (QD×5, 2 days off, 2 weeks off, QD×5, Group 5) had a statistically significant anti-tumor effect on the HuPrime® pancreatic cancer PA2637 subcutaneous model at the tested dosage and dosing frequency of this experiment. During the experiment, the mice in each test drug treatment group were well tolerated during the treatment period.

**8. Anti-tumor effect and safety evaluation of the test substances AST and Ifosfamide monotherapy in the HuPrime® lung cancer LU11873 subcutaneous model**

The HuPrime® lung cancer LU11873 subcutaneous model was a PDX model harboring the KRAS G12C pathogenic mutation.

NOD.SCID female mice were subcutaneously inoculated with the HuPrime® lung cancer LU11873 tumor blocks so as to establish a subcutaneous transplantation tumor model of human lung cancer. The test was divided into the following groups: the test drug Ifosfamide 60 mg/kg monotherapy group (Group 2) was administered once a day for 5 consecutive days, rested for 2 days, and then administered once a day for 5 consecutive days again; AST 4 mg/kg monotherapy group (Group 5) was administered once a day for 5 consecutive days, rested for 2 days, then rested for 2 weeks, and then administered once a day for 5 consecutive days again; and glucose injection (pH 7.7-8.0) vehicle control group (Group 1) was administered once a day for 5 consecutive days, rested for 2 days, then rested for 2 weeks, and then administered once a day for 5 consecutive days again. There were 3 groups in total and 6 mice in each group in this experiment. The test drug Ifosfamide was administered intraperitoneally. The vehicle control group and each AST group were administered by tail vein injection. The administration routes, dosages and regimens of the experimental design were shown in Table 35.

Table 35: Administration routes, doses and regimens in the HuPrime® lung cancer LU11873 subcutaneous model

| Group No. | The number of Animals | Administration group | Dosage (mg/kg) | Administration method | Administration period |
|---|---|---|---|---|---|
| 1 | 6 | Glucose injection (pH 7.7-8.0) | - | *i.v.* | QD×5, 2 days off, 2 weeks off, QD×5 |
| 2 | 6 | Ifosfamide | 60 | *i.p.* | QD×5/week×2 weeks |
| 5 | 6 | AST | 4 | *i.v.* | QD×5, 2 days off, 2 weeks off, QD×5 |

The tumor growth of each treatment group and control group was recorded on different days of the test, as shown in Table 36. The corresponding growth curve of tumor volume in each group of mice was shown in Fig. 21. The therapeutic effect was evaluated based on the relative tumor proliferation rate and relative tumor growth inhibition rate. The drug efficacy analysis of each group was shown in Table 37. The body weight changes of the treatment group and the control group after administration were recorded, and the safety of each group in the HuPrime® lung cancer LU11873 subcutaneous xenograft model was studied. The results of body weight changes of the mice were shown in Table 38. Correspondingly, the curve graph of the percentage changes of body weight in each treatment group over time was shown in Fig. 22.

Table 36: Changes in tumor volume in each group of mice over treatment time in the HuPrime® lung cancer LU11873 subcutaneous model ($mm^3$)

| Group | Days after initial administration | | | | | | | | | |
|---|---|---|---|---|---|---|---|---|---|---|
| | 0 | 3 | 7 | 10 | 14 | 17 | 21 | 24 | 28 | 31 |
| Group 01 | 121.41 | 194.85 | 296.16 | 396.17 | 509.62 | 678.47 | 790.84 | 1125.75 | 1415.78 | 1677.89 |
| Group 02 | 121.82 | 183.55 | 305.91 | 406.78 | 619.15 | 768.80 | 870.60 | 1183.70 | 1354.36 | 1866.37 |
| Group 05 | 121.48 | 135.18 | 188.07 | 197.93 | 214.44 | 240.28 | 286.32 | 344.68 | 371.66 | 406.40 |

Table 37: Drug efficacy analysis table of each group in the HuPrime® lung cancer LU11873 subcutaneous model

| **Experiment group** | **Day 31 after initial administration** | | | | |
|---|---|---|---|---|---|
| | **Tumor volume** - ($x$±S) | **Relative tumor volume** - ($x$±S) | **TGI (%)** | **T/C (%)** | **P Value (compared with the control group)** |
| **Group 1** Glucose injection (pH 7.7-8.0), 10 μL/g, i.v., QD×5, 2 days off, 2 weeks off, QD×5 | 1677.89±262.51 | 13.77±1.89 | - | - | - |
| **Group 2** Ifosfamide,60 mg/kg,10 μL/g, i.p., QD×5/week×2weeks | 1866.37±203.06 | 15.16±1.26 | -10.08 | 110.08 | >0.05 |
| **Group 5** AST, 4 mg/kg, 10 μL/g, i.v., QD×5, 2 days off, 2 weeks off, QD×5 | 406.40±43.43 | 3.42±0.44 | 75.16 | 24.84 | <0.001*** |

Table 38: Percentage change in body weight of mice in the HuPrime® lung cancer LU11873 subcutaneous model

| Group | 0 | 1 | 2 | 3 | 4 | 7 | 10 | 14 | 17 | 21 | 22 | 23 | 24 | 25 | 28 | 31 |
|---|---|---|---|---|---|---|---|---|---|---|---|---|---|---|---|---|
| 01 | 0.00% | -0.82% | -0.37% | 2.00% | 0.51% | 0.24% | 0.43% | -1.45% | -4.10% | -1.68% | 4.47% | -2.36% | -3.01% | 0.05% | 1.13% | 3.37% |
| 02 | 0.00% | -0.95% | -1.01% | -0.90% | -1.74% | -0.52% | 0.39% | 0.34% | 0.38% | 2.33% | - | - | 3.36% | - | 5.36% | 10.46% |
| 05 | 0.00% | -2.18% | -3.41% | -4.15% | -5.47% | -3.20% | -0.29% | -4.42% | -5.73% | -3.45% | -0.11% | -5.76% | -6.17% | -2.27% | -4.48% | 1.21% |

The mean tumor volume of mice in the vehicle control group was 1677.89 $mm^3$ on Day 31 after the initial administration. The mean tumor volume of the test drug Ifosfamide treatment group (Group 2) at the dose of 60 mg/kg on Day 31 was 1866.37 $mm^3$, and the relative tumor growth inhibition TGI (%) was -10.08%, with no statistically significant difference compared with the control group (p>0.05). The mean tumor volume of the test drug AST treatment group (Group 5) at the dose of 4 mg/kg (QD×5, 2 days off, 2 weeks off, QD×5) on Day 31 was 406.40 $mm^3$, with statistically significant difference compared with the control group (p<0.05), and the relative tumor growth inhibition rate TGI (%) was 75.16%.

The above experimental results showed that AST in the model having KRAS G12C pathogenic mutation, the test drug AST treatment group (Group 5) at the dose of 4 mg/kg (QD×5, 2 days off, 2 weeks off, QD×5) had a significant anti-tumor effect on the HuPrime® lung cancer LU11873 subcutaneous model at the tested dosage and dosing frequency of this study. Ifosfamide administration group had no tumor inhibitory effect. During the experiment, the mice in each test drug treatment group did not have any weight loss and were well tolerated during the treatment period.

Specifically, the applicant found that in Example 3, AST in the KRAS pathogenic mutation model lung cancer LU11693 having G12C amino acid mutation had no significant anti-tumor effect, with TGI% of 54.64% at the dose of 10 mg/kg; while in this example, AST in the KRAS pathogenic mutation model lung cancer LU11873 having G12C amino acid mutation had a significant anti-tumor effect, with TGI% of 75.16% at the dose of 4 mg/kg. The difference between the two models was significant, indicating that there might be some sort of difference between these two PDX models. It can be seen from data about the source of the models:

LU11693 originated from a 58-year-old female patient who showed cachexia and mild ulcers clinically;

LU11873 originated from a 51-year-old male patient who showed slight weight loss and mild ulcers clinically.

**9. Antitumor effect and safety evaluation of test substances AST and Ifosfamide monotherapy in the HuPrime® pancreatic cancer PA1383 subcutaneous model**

The HuPrime® pancreatic cancer PA1383 subcutaneous model was a PDX model harboring KRAS G12C pathogenic mutation.

Balb/nude female mice were subcutaneously inoculated with HuPrime® pancreatic cancer PA1383 tumor blocks so as to establish a subcutaneous transplantation tumor model of human pancreatic cancer. The test was divided into the following groups: the test drug Ifosfamide 60 mg/kg monotherapy group (Group 2) was administered once a day for 5 consecutive days, rested for 2 days, and then administered once a day for 5 consecutive days again; AST 8 mg/kg monotherapy group (Group 4) was administered once a week for a total of 3 weeks; AST 4 mg/kg monotherapy group (Group 5) was administered once a day for 5 consecutive days, rested for 2 days, then rested for 2 weeks, and then administered once a day for 5 consecutive days again; and glucose injection (pH 7.7-8.0) vehicle control group (Group 1) was administered once a day for 5 consecutive days, rested for 2 days, then rested for 2 weeks, and then administered once a day for 5 consecutive days again. There were 4 groups in total and 6 mice in each group in this study. Among them, the test drug Ifosfamide was administered intraperitoneally. The vehicle control group and each AST group were administered by tail vein injection. The administration routes, dosages and regimens of the experimental design were shown in Table 39.

Table 39: Administration routes, doses and regimens in the HuPrime® pancreatic cancer PA1383 subcutaneous model

| Group No. | The number of animals | Administration group | Dosage (mg/kg) | Administration method | Administration period |
|---|---|---|---|---|---|
| 1 | 6 | glucose injection (pH 7.7-8.0) | - | *i.v.* | QD × 5, 2 days off, 2 weeks off, QD × 5 |
| 2 | 6 | Ifosfamide | 60 | *i.p.* | QD × 5/week × 2 weeks |
| 4 | 6 | AST | 8 | *i.v.* | QW × 3 |
| 5 | 6 | AST | 4 | *i.v.* | QD × 5, 2 days off, 2 weeks off, QD × 5 |

The tumor growth of each treatment group and control group was recorded on different days of the test, as shown in Table 40. The corresponding growth curve of tumor volume in each group of mice was shown in Fig. 23. The therapeutic effect was evaluated based on the relative tumor proliferation rate and relative tumor growth inhibition rate. The drug efficacy analysis of each group was shown in Table 41. The body weight changes of the treatment group and

the control group after administration were recorded, and the safety of each group in the HuPrime® pancreatic cancer PA1383 subcutaneous xenograft model was studied. The results of body weight changes of the mice were shown in Table 42. Correspondingly, the curve graph of the percentage changes of body weight in each treatment group over time was shown in Fig. 24.

Table 40: Changes in tumor volume in each group of mice over treatment time in the HuPrime® pancreatic cancer PA1383 subcutaneous model ($mm^3$)

| Group | Days after initial administration | | | | | | | | | |
|---|---|---|---|---|---|---|---|---|---|---|
| | 0 | 4 | 7 | 11 | 14 | 18 | 21 | 25 | 28 | 31 |
| 01 | 131.14 | 220.07 | 402.23 | 630.24 | 726.31 | 847.35 | 1077.74 | 1291.64 | 1417.06 | 1536.48 |
| 02 | 131.04 | 215.30 | 391.66 | 570.57 | 643.25 | 727.85 | 859.10 | 986.71 | 1064.57 | 1202.01 |
| 04 | 131.08 | 152.43 | 195.20 | 156.69 | 84.43 | 45.14 | 31.21 | 25.71 | 23.93 | 18.57 |
| 05 | 131.05 | 134.98 | 147.15 | 82.79 | 57.51 | 82.53 | 87.39 | 100.47 | 58.54 | 39.94 |

Table 41: Drug efficacy analysis table of each group in the HuPrime® pancreatic cancer PA1383 subcutaneous model

| Experiment group | Day 31 after initial administration | | | | |
|---|---|---|---|---|---|
| | **Tumor volume -** ($x \pm S$) | **Relative tumor volume** ($x \pm S$) | **TGI (%)** | **T/C (%)** | **P Value (compared with the control group)** |
| **Group 1** glucose injection (pH 7.7-8.0), 10 μL/g, i.v., QD × 5, 2 days off, 2 weeks off, QD × 5 | 1536.48 ± 165.08 | 11.74 ± 1.31 | - | - | - |
| **Group 2** Ifosfamide, 60 mg/kg, 10 μL/g, i.p., QD × 5/week × 2 weeks | 1202.01 ± 85.63 | 9.18 ± 0.54 | 21.84 | 78.16 | > 0.05 |
| **Group 4** AST, 8 mg/kg, 10 μL/g, i.v., QW × 3 | 18.57 ± 7.43 | 0.15 ± 0.06 | 98.72 | 1.28 | < 0.001*** |
| **Group 5** AST, 4 mg/kg, 10 μL/g, i.v., QD × 5, 2 days off, 2 weeks off, QD × 5 | 39.94 ± 20.41 | 0.30 ± 0.15 | 97.46 | 2.54 | < 0.001*** |

Table 42: Percentage change in body weight of mice in the HuPrime® pancreatic cancer PA1383 subcutaneous model

| Group | Days after initial administration | | | | | | | | | | | | | | | |
|---|---|---|---|---|---|---|---|---|---|---|---|---|---|---|---|---|
| | 0 | 1 | 2 | 3 | 4 | 7 | 11 | 14 | 18 | 21 | 22 | 23 | 24 | 25 | 28 | 31 |
| 1 | 0.00% | 0.76% | 0.16% | 0.19% | 0.99% | 2.88% | 5.00% | 2.54% | 5.77% | 4.49% | 10.28% | 8.94% | 8.20% | 5.41% | 4.37% | 3.46% |
| 2 | 0.00% | 1.45% | 0.57% | 1.33% | 2.25% | 1.43% | 0.70% | 2.01% | 3.59% | 9.06% | - | - | - | 9.12% | 4.90% | 4.44% |
| 4 | 0.00% | - | - | - | 0.68% | 5.22% | 5.83% | 7.37% | 5.55% | 4.97% | - | - | - | 6.77% | 6.91% | 7.13% |
| 5 | 0.00% | 2.03% | 2.53% | 0.44% | 0.61% | 2.91% | 5.66% | 6.39% | 7.35% | 6.31% | 9.09% | 6.44% | 5.39% | 5.27% | 8.10% | 8.93% |

The mean tumor volume of mice in the vehicle control group was 1536.48 $mm^3$ on Day 31 after the initial administration. The mean tumor volume of the test drug Ifosfamide treatment group (Group 2) at the dose of 60 mg/kg on Day 31 was 1202.01 $mm^3$, and the relative tumor growth inhibition TGI (%) was 21.84%, with no statistically significant difference compared with the control group (p>0.05).

The mean tumor volumes of the test drug AST treatment groups (Group 4 and Group 5) at the dose of 8 mg/kg (QD×3) and at the dose of 4 mg/kg (QD×5, 2 days off, 2 weeks off, QD×5) on Day 31 were 18.57 $mm^3$ and 39.94 $mm^3$, respectively, with statistically significant difference compared with the control group (p<0.05), and the relative tumor growth inhibition rates TGI (%) were 98.72% and 97.46%, respectively. Two mice in each group had tumor completely cleared, with a clearance rate of 33.3%.

The above experimental results showed that AST in the model having KRAS G12C pathogenic mutation, AST treatment groups (Group 4 and Group 5) at the dose of 8 mg/kg (QD 3) and at the dose of 4 mg/kg (QD×5, 2 days off, 2 weeks off, QDx5) had a significant anti-tumor effect on the HuPrime® pancreatic cancer PA1383 subcutaneous model at the tested dosage and dosing frequency of this study. Ifosfamide administration group had no tumor inhibitory effect. The mice in each test drug treatment group did not have any weight loss and were well tolerated during the treatment period.

**10. Detection of AKR1C3 RNA expression level and enzyme content in tissues**

A. FPKM detection of AKR1C3 RNA expression level

According to the method described in the document (Meng, F., Li, W. F., Jung, D., Wang, C. C., Qi, T., Shia, C. S., Hsu, R. Y, Hsieh, Y C., & Duan, J. (2021). A novel selective AKR1C3-activated prodrug AST-3424/OBI-3424 exhibits broad anti-tumor activity. American journal of cancer research, 11(7), 3645-3659), AKR1C3 RNAexpression levels in tissues of gastric cancer GA6021, pancreatic cancer PA1222, and lung cancer LU11693 as described above were analyzed with RNA-Seq and quantified by Log2 FPKM. The results were as follows:
AKR1C3 LOG2 (FPKM) was detected for GA6201 as 6.78, for LU11693 as 11.14, for PA1222 as 7.39, for HPAF-II as 8.31, for LU5161 as 11.56, for CR3820 as 8.34, for PA2637 as 9.12, for LU11873 as 10.26, and for PA1383 as 9.57 (see Table 43).

According to the above document, AKR1C3 RNA in all the nine tumor tissues was highly expressed.

B. IHC method detection and H-SCORE of AKR1C3 protein content

The AKR1C3 protein contents of the three tissues were determined according to the customary IHC (immunohistochemistry) staining (commercial IHC reagents was used, the first antibody was Rabbit IgG mAb from Abcam, and the second antibody was Bond Polymer Refine Detection from Leica; staining conditions: antigen retrieval 100°C, pH 9.0 EDTA buffer 20 min, dilution ratio: 1:800), and the staining results were performed for H-SCORE:
The immunohistochemical staining intensity was divided into 0 (negative), 1+ (weak staining), 2+ (medium staining), and 3+ (strong staining). The thresholds for weak staining, medium staining, and strong staining were manually set on the scoring instrument, and then color recognition was performed on the staining sample photographs using image processing software. For staining photographs of all the samples, the corresponding staining of a certain cell was scored according to a unified standard by the scoring software: 0/1/2/3. Then the percentage of positive cells with different staining intensities to the total cells in the slice was counted. H-Score was calculated as the score of IHC results for each sample using the following formula. The H-score would be between 0 and 300, and the higher the score, the higher the expression level of the corresponding target of the antibody (AKR1C3 enzyme protein) in the sample. The calculation formula was as follows:

$$\text{H-Score} = (\%\text{ at } 0) \times 0 + (\%\text{ at } 1) \times 1 + (\%\text{ at } 2) \times 2 + (\%\text{ at } 3) \times 3$$

The staining results of GA6201, LU11693, PA1222, and two control groups were shown in Fig, 25, and the scoring results were shown in Table 43 below:

Table 43: IHC and RNA analysis results of nine models and control groups

| **Model ID** | **Cancer type** | **AKR1C3** H-Score | **AKR1C3 $LOG_2$ (FPKM)** |
|---|---|---|---|
| GA6201 | Gastric Cancer | 248.75 | 6.78 |
| LU11693 | Lung Cancer | 269.10 | 11.14 |

(continued)

| Model ID | Cancer type | AKR1C3 H-Score | AKR1C3 LOG$_2$ (FPKM) |
|---|---|---|---|
| PA1222 | Pancreatic Cancer | 204.28 | 7.39 |
| HPAF-II | Pancreatic Cancer | high expression | 8.31 |
| LU5161 | Lung Cancer | 272.78 | 11.56 |
| CR3820 | Intestinal Cancer | 274.88 | 8.34 |
| PA2637 | Pancreatic Cancer | 238.23 | 9.12 |
| LU11873 | Lung Cancer | 236.69 | 10.26 |
| PA1383 | Pancreatic Cancer | 252.92 | 9.57 |
| BL9214 (positive control) | Bladder Cancer | 265.86 | 10.86 |
| LI5129 (negative control) | Liver Cancer | 0.00 | -2.00 |

Specific staining statistical results of models GA6201, LU11693, PA1222, and control groups were shown in Table 44 below:

Table 44: IHC results and H-SCORE of models GA6201, LU11693, PA1222, and control groups

| Model ID | Cancer type | AKR1C3 | | | | |
|---|---|---|---|---|---|---|
| | | 0 | 1 | 2 | 3 | H-Score |
| GA6201 | Gastric Cancer | 1.07 | 8.75 | 30.54 | 59.63 | 248.75 |
| LU11693 | Lung Cancer | 1.70 | 5.51 | 14.79 | 78.00 | 269.10 |
| PA1222 | Pancreatic Cancer | 6.82 | 24.13 | 27.01 | 42.04 | 204.28 |
| BL9214 (positive control) | Bladder Cancer | 1.47 | 7.23 | 15.26 | 76.04 | 265.86 |
| LI5129 (negative control) | Liver Cancer | 100.00 | 0.00 | 0.00 | 0.00 | 0.00 |

In the above staining results, the positive and negative control results within the control range indicated that the H-SCORE results of this IHC staining were reliable.

According to the above results, the corresponding AKR1C3 protein in all the nine tissues was highly expressed.

It can be seen from comprehensive pharmacodynamic experiment results of the above nine models, together with the fact that the tissues used in the nine models were all tumor tissues with high expression of human AKR1C3 that: AST-3424 and AST have generally significant therapeutic effect on cancer with high expression of AKR1C3 and having the KRAS pathogenic mutation of G12D amino acid mutation; AST may have significant therapeutic effect on cancer with high expression of AKR1C3 and having the KRAS pathogenic mutation of G12C amino acid mutation. This means that high expression of AKR1C3 in certain tumors may be associated with KRAS (pathogenic) mutation subtypes, that is, high expression or overexpression of AKR1C3 often coexists with certain subtypes of KRAS (pathogenic) mutation in certain tumors, which leads to higher sensitivity of tumor models with these characteristics to AST-3424 or AST.

According to the document (Meng, F., Li, W. F., Jung, D., Wang, C. C., Qi, T., Shia, C. S., Hsu, R. Y, Hsieh, Y C., & Duan, J. (2021). A novel selective AKR1C3-activated prodrug AST-3424/OBI-3424 exhibits broad anti-tumor activity. American journal of cancer research, 11(7), 3645-3659; Evans, K., Duan, J., Pritchard, T., Jones, C. D., Mc-Dermott, L., Gu, Z., Toscan, C. E., El-Zein, N., Mayoh, C., Erickson, S. W., Guo, Y, Meng, F., Jung, D., Rathi, K. S., Roberts, K. G., Mullighan, C. G., Shia, C. S., Pearce, T., Teicher, B. A., Smith, M. A., ... Lock, R. B. (2019). OBI-3424, a Novel AKR1C3-Activated Prodrug, Exhibits Potent Efficacy against Preclinical Models of T-ALL. Clinical cancer research: an official journal of the American Association for Cancer Research, 25(14), 4493-4503; Yanlan Wang, Yue Liu, Changhua Zhou, Chunnian Wang, Ning Zhang, Donglin Cao, Qing Li & Zhong Wang (2020) An AKR1C3-specific prodrug with potent anti-tumor activities against T-ALL, Leukemia & Lymphoma, 61(7), 1660-1668) and corresponding patent applications:

PCT/US2016/021581 with Publication No. WO2016145092A1 (corresponding to Chinese Patent Application No. 2016800150788 with Publication No. CN107530556A),

PCT/US2016/062114 with Publication No. WO2017087428A1 (corresponding to Chinese Patent Application No. 2016800200132 with Publication No. CN108136214A),

PCT/CN2020/089692 with Publication No. WO2020228685A1;

PCT/NZ2019/050030 with Publication No. WO2019190331A1 (corresponding to Chinese Patent Application No. 2019800234236 with Publication No. CN111918864A);

PCT/CN2020/120281 with Publication No. WO2021068952A1,

These compounds in these patent applications, similar to compounds AST-3424 and AST, are AKR1C3-activated anticancer prodrugs, which are cleaved into the DNA alkylating agent

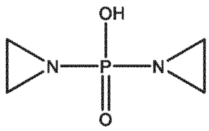

or nitrogen mustard structure after AKR1 C3 activation.

Thus, in combination with the above experimental results of AST-3424 and AST, it can be inferred that AKR1C3-activated DNA alkylating agent prodrugs monotherapy or in combination with other therapeutic drugs have significant therapeutic effect on cancer and tumor patients having KRAS mutations, especially those with KRAS-G12D subtype mutation.

## Claims

1. A treatment method which uses a drug monotherapy containing an AKR1C3-activated DNA alkylating agent prodrug compound or in combination with other therapeutic drugs for treating cancer and tumor patients having KRAS mutations.

2. The treatment method according to claim 1, wherein the compound is selected from structural Formulae 1/2/3/4/5/6 and salts, esters, solvates, isotopic isomers thereof:

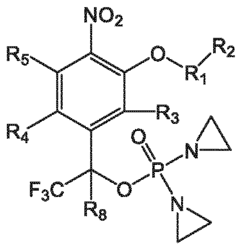
(1)
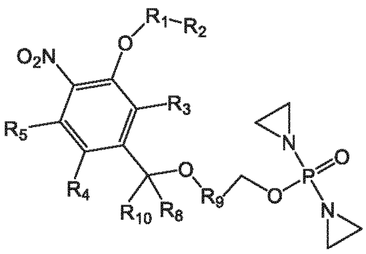
(2),

wherein the definitions of $R_1$, $R_2$, $R_3$, $R_4$, $R_5$, $R_8$, $R_9$, and $R_{10}$ are described in the claims of Patent Application PCT/CN2020/089692 with Publication No. WO2020228685A1;

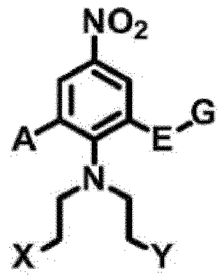

(3),

wherein the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A);

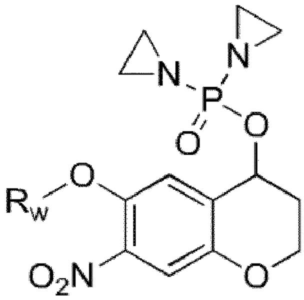

(4),

wherein the definition of $R_w$ is described in the claims of Patent Application PCT/CN2020/120281 with Publication No. WO2021068952A1;

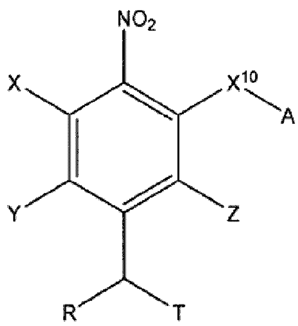

(5),

wherein the definitions of X, Y, Z, R, T, A, and $X^{10}$ are described in the claims of Patent Application PCT/US2016/062114 with Publication No. WO2017087428A1 (corresponding to Chinese Patent Application No. 2016800200132 with Publication No. CN108136214A);

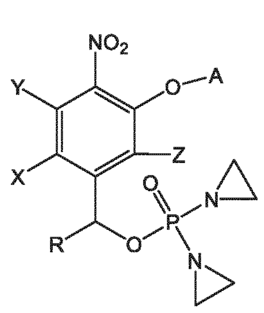

(6),

wherein:

A is a substituted or unsubstituted $C_6$-$C_{10}$ aryl, biaryl or substituted biaryl, 5-15 membered heteroaryl, or -N=$CR^1R^2$, wherein the substituents are selected from the group consisting of halogeno, -CN, -$NO_2$, -O-($CH_2$)-O-, -$CO_2H$ and salts thereof, -$OR^{100}$, -$CO_2R^{100}$, -$CONR^{101}R^{102}$, - $NR^{101}R^{102}$, -$NR^{100}SO_2R^{100}$, -$SO_2R^{100}$, -$SO_2NR^{101}R^{102}$, $C_1$-$C_6$ alkyl, and $C_3$-$C_{10}$ heterocyclyl;
wherein $R^{100}$, $R^{101}$ and $R^{102}$ are each independently hydrogen, Ci-Cs alkyl, or $C_6$-$C_{12}$ aryl; or $R^{101}$ and $R^{102}$ together with the nitrogen atom to which they are attached form a 5-7 membered heterocycle;
wherein the alkyl group and the aryl group are each substituted by 1-3 halogeno groups or 1-3 $C_1$-$C_6$ alkyl groups;
$R^1$ and $R^2$ are each independently phenyl or methyl;
X, Y and Z are each independently hydrogen or halogeno; and
R is hydrogen or $C_1$-$C_6$ alkyl or halogen-substituted alkyl.

3. The treatment method according to claim 1, wherein the cancer is selected from the group consisting of ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

4. The treatment method according to claim 1 or 2, wherein the KRAS mutation is selected from the group consisting ofKRAS-G12D mutation, KRAS-G12V mutation and KRAS-G12C mutation; preferably, the KRAS mutation is selected from the KRAS-G12D mutation.

5. The treatment method according to claim 4, wherein the TMB (Tumor Mutation Load (burden)) level of the mutation is medium.

6. The treatment method according to claim 1 or 2, wherein the other therapeutic drugs are selected from the group consisting of KRAS inhibitors and immunotherapy drugs, wherein the KRAS inhibitors are selected from the group consisting of sotorasib (AMG510), adagrasib (MRTX849), GDC6036, LY3499446, JNJ74699157 (ARS3248) and D-1553, and the immunotherapy drugs are selected from the group consisting of PD-1 monoclonal antibodies and PD-L1 monoclonal antibodies.

7. The treatment method according to claim 2, wherein the compounds of Formulae (1) and (2) are selected from the group consisting of:

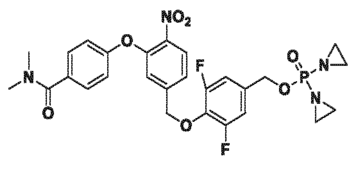 , 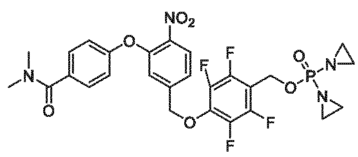 , 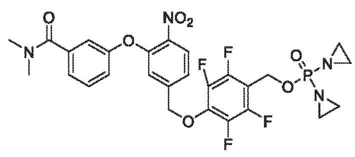

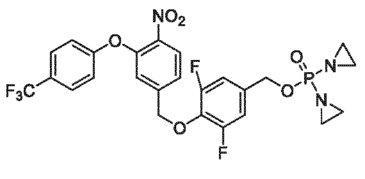 , 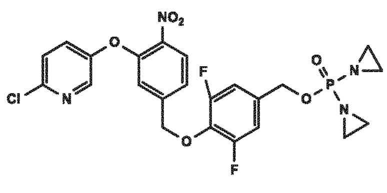 , 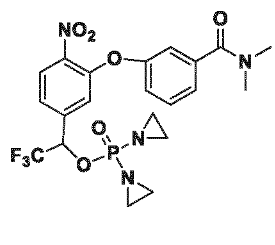

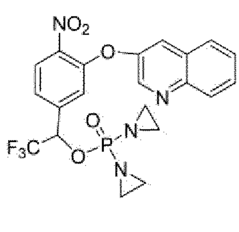 , 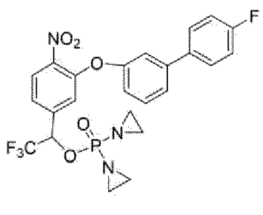 , 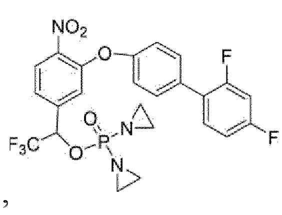

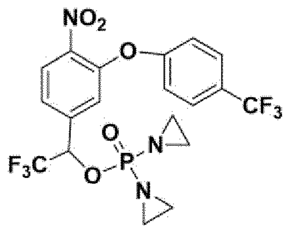
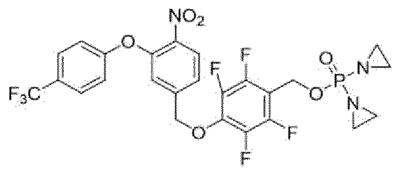
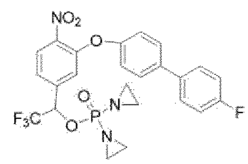

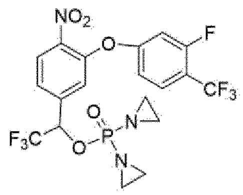
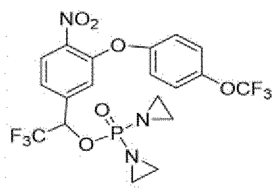
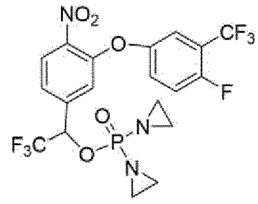

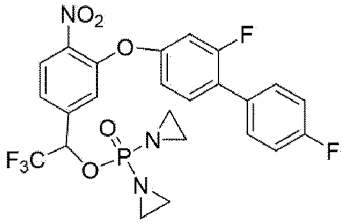
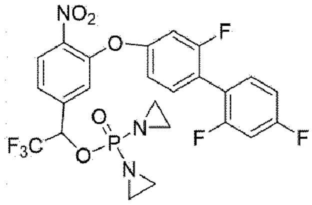
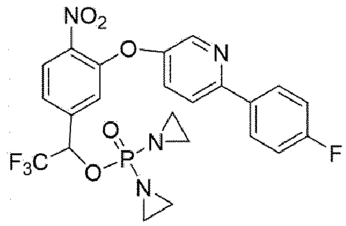

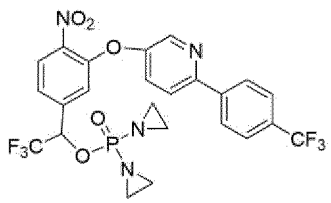
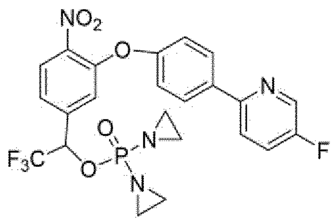
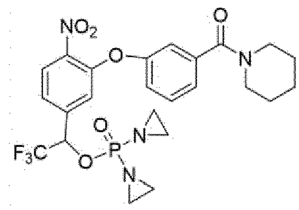

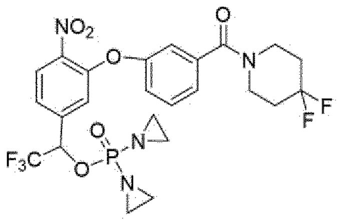
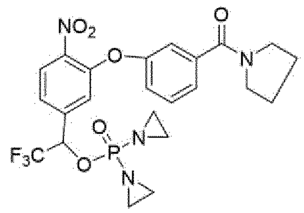
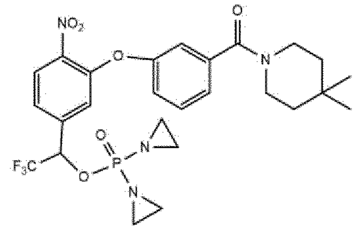

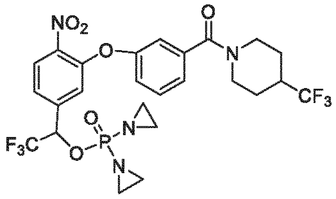
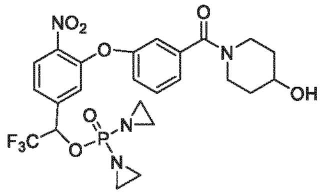
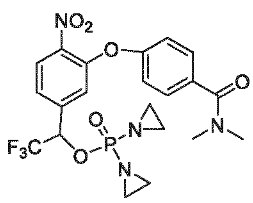

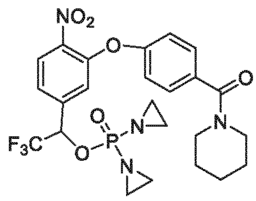
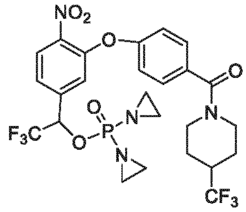
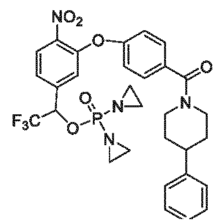

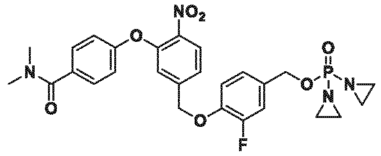,
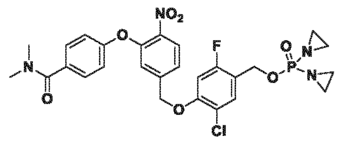,
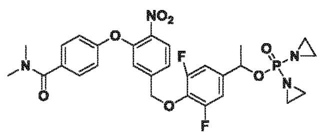,

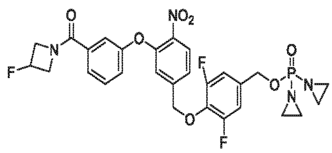,
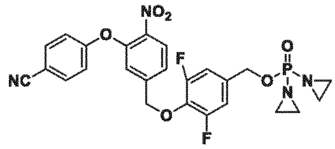,
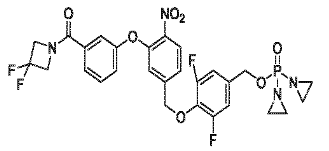,

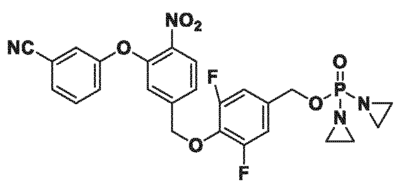,
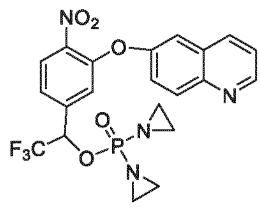,
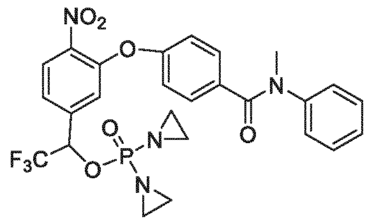,

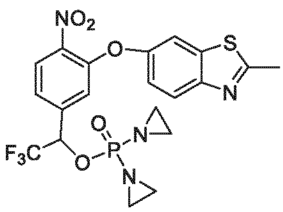,
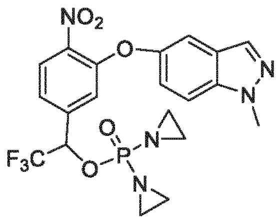,
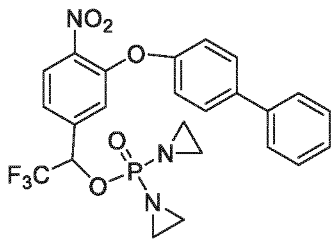

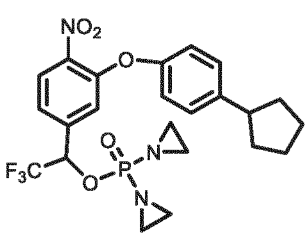,
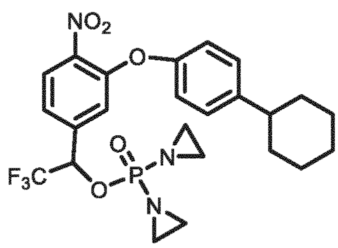,
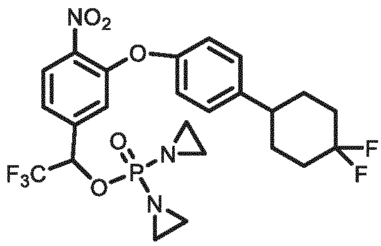;

the compound of Formula (3) is selected from the group consisting of:

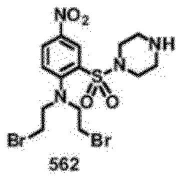
562
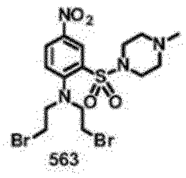
563
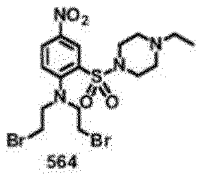
564
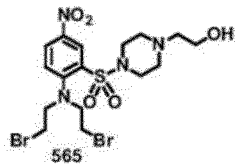
565
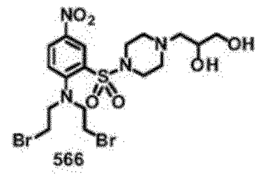
566

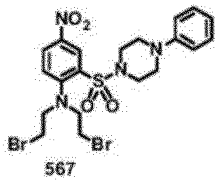
567
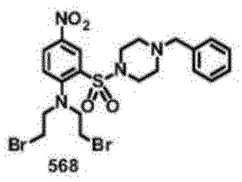
568
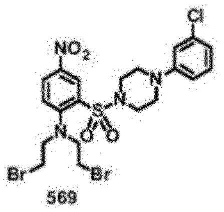
569
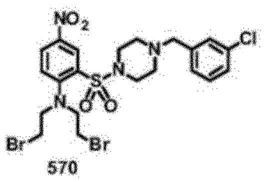
570

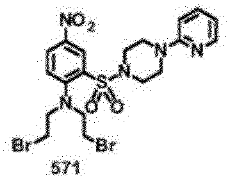
571
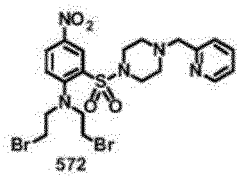
572
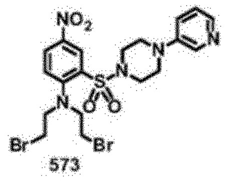
573
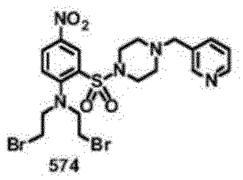
574

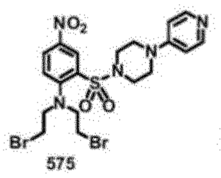
575
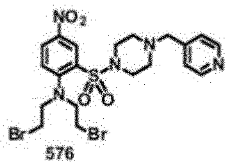
576
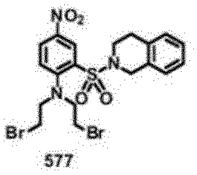
577
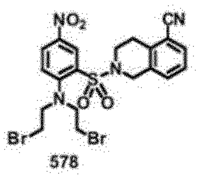
578
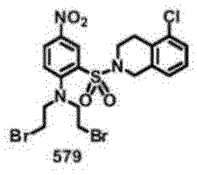
579

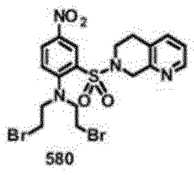
580
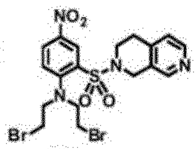
581
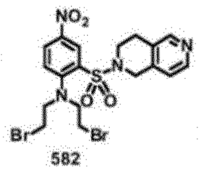
582
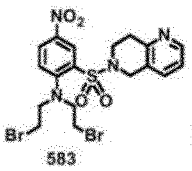
583
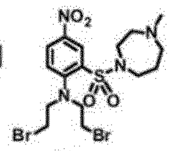
584
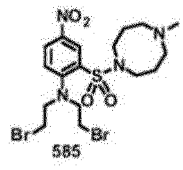
585

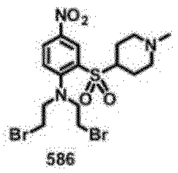
586
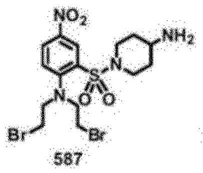
587
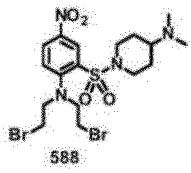
588
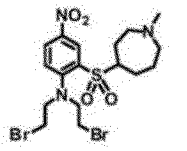
589
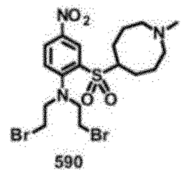
590
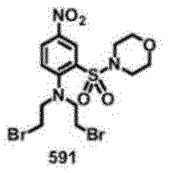
591

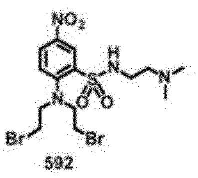
592
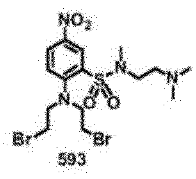
593
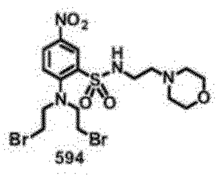
594
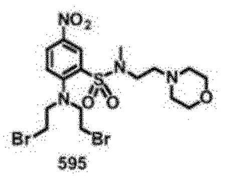
595
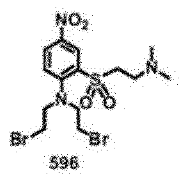
596

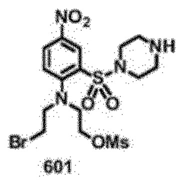
601
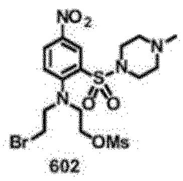
602
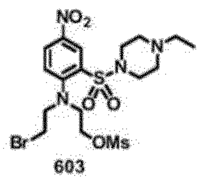
603
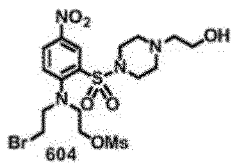
604
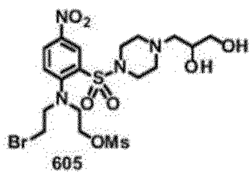
605

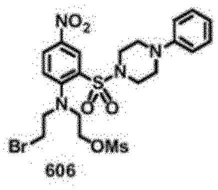
606
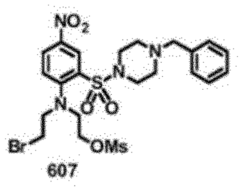
607
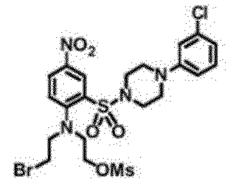
608
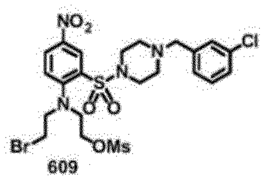
609

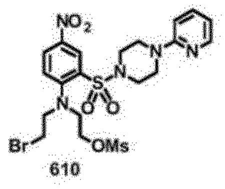
610
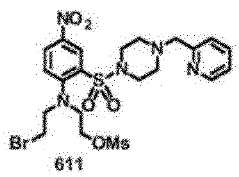
611
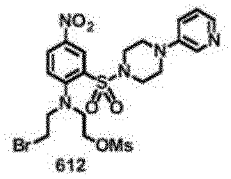
612
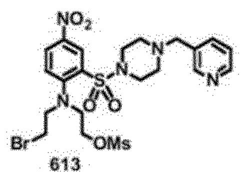
613

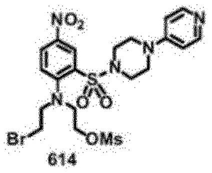
614
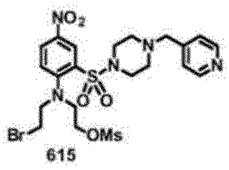
615
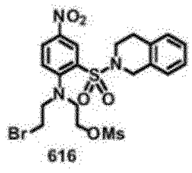
616
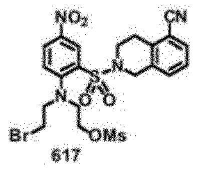
617
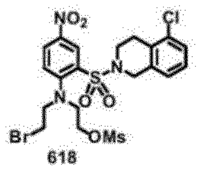
618

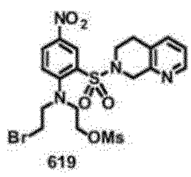
619
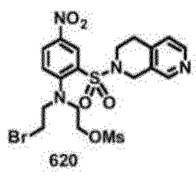
620
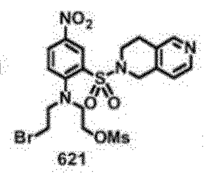
621
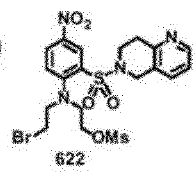
622
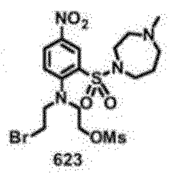
623
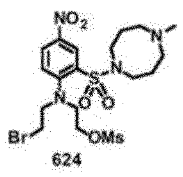
624

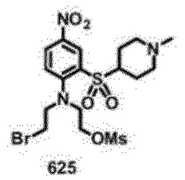
625
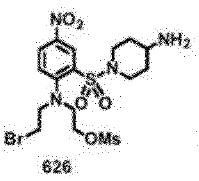
626
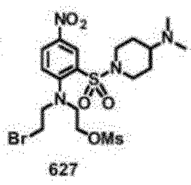
627
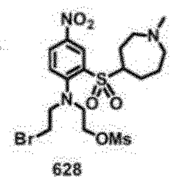
628
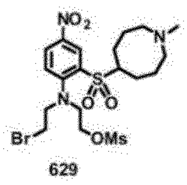
629
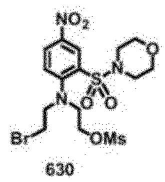
630

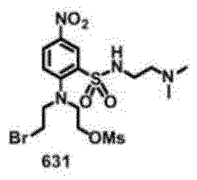
631
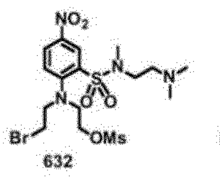
632
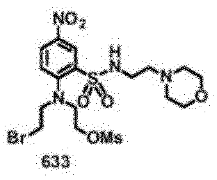
633
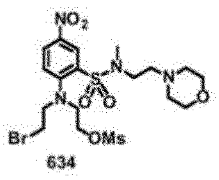
634
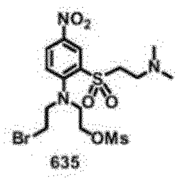
635

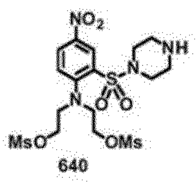
640
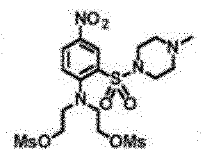
641
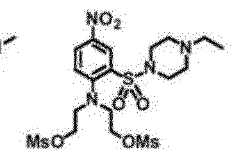
642
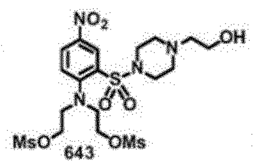
643
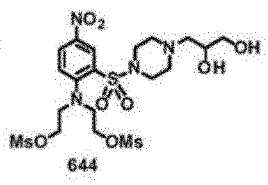
644

Cl

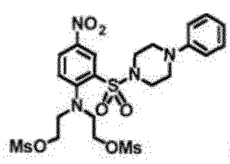
645
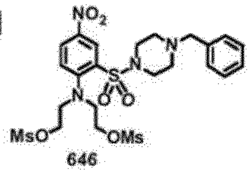
646
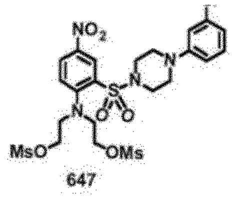
647
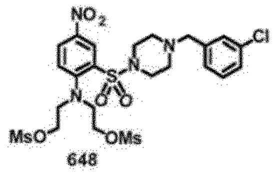
648

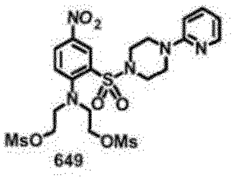
649
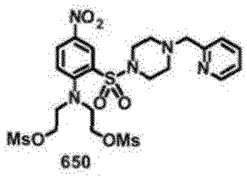
650
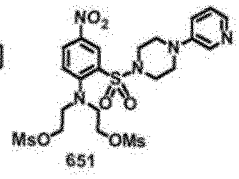
651
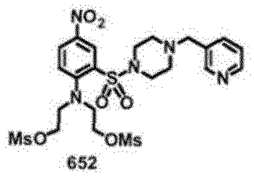
652

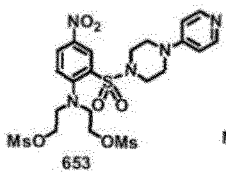
653
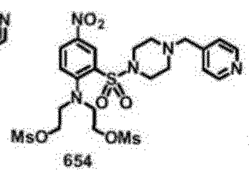
654
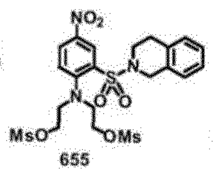
655
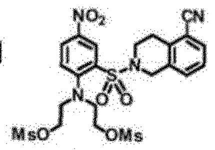
656
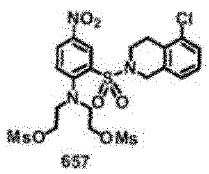
657

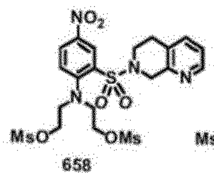
658
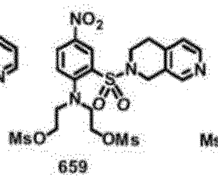
659
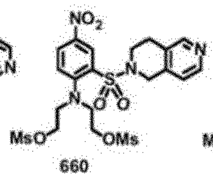
660
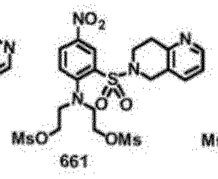
661
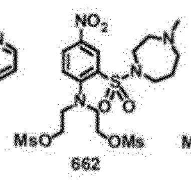
662
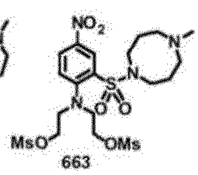
663

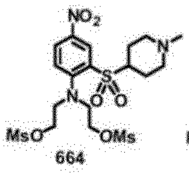
664
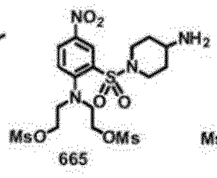
665
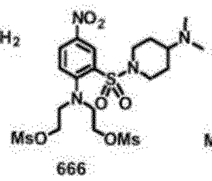
666
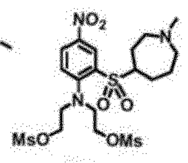
667
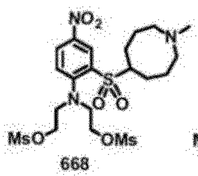
668
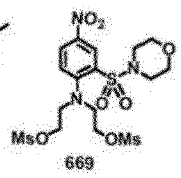
669

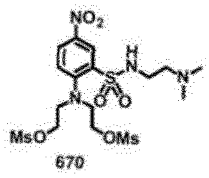
670
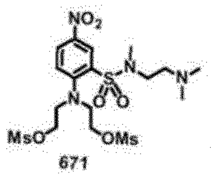
671
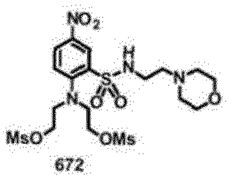
672
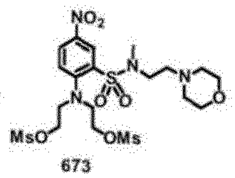
673
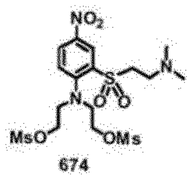
674

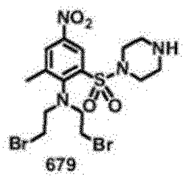
679
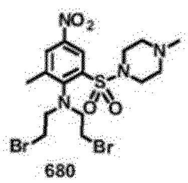
680
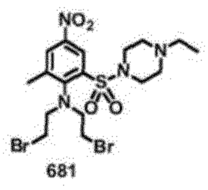
681
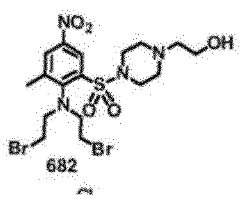
682
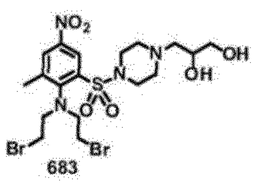
683

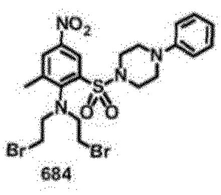
684

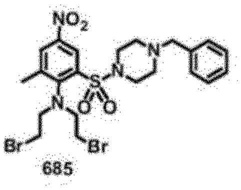
685

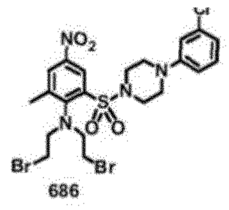
686

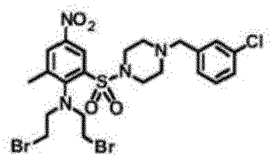
687

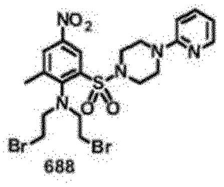
688

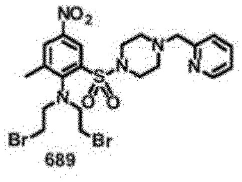
689

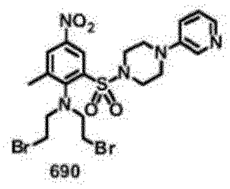
690

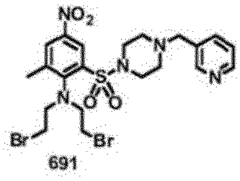
691

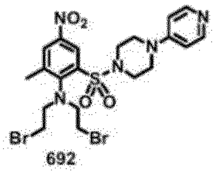
692

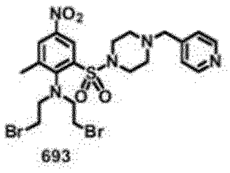
693

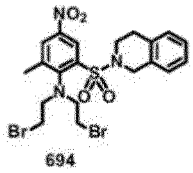
694

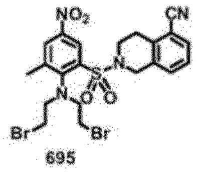
695

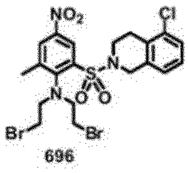
696

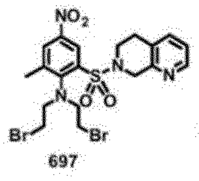
697

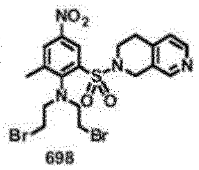
698

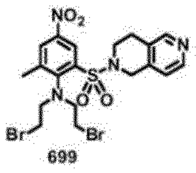
699

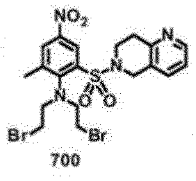
700

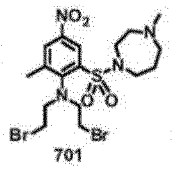
701

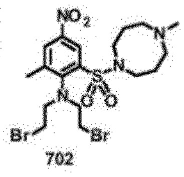
702

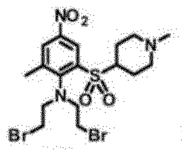
703

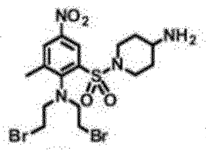
704

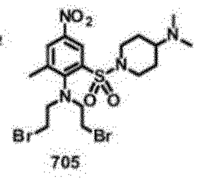
705

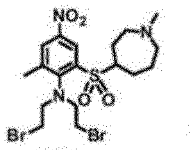
706

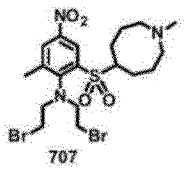
707

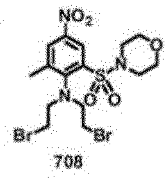
708

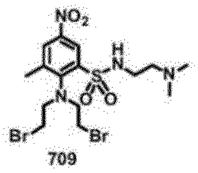
709

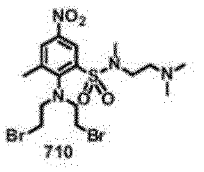
710

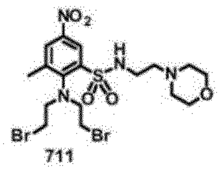
711

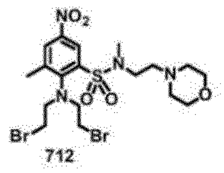
712

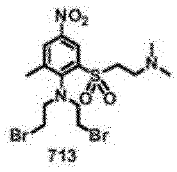
713

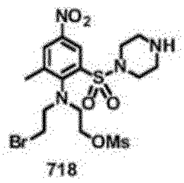
718

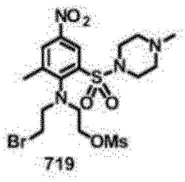
719

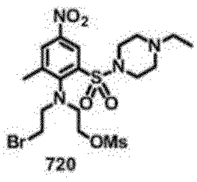
720

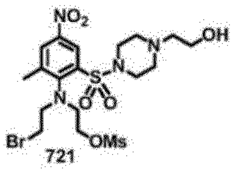
721

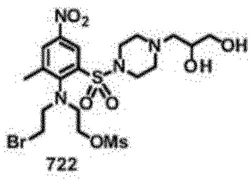
722

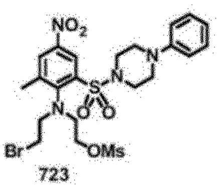
723

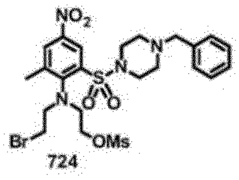
724

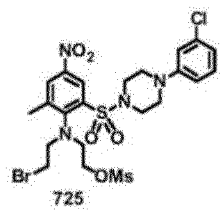
725

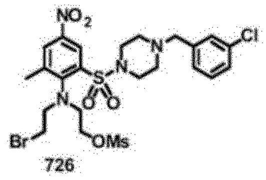
726

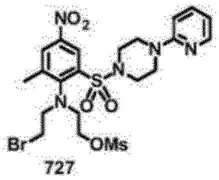
727
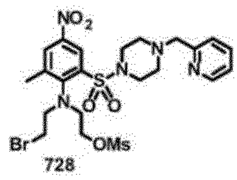
728
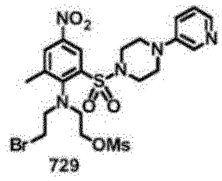
729
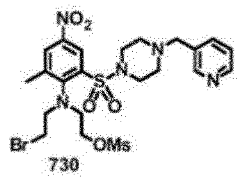
730

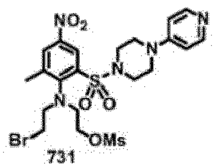
731
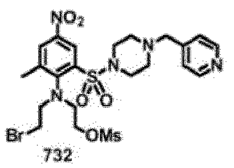
732
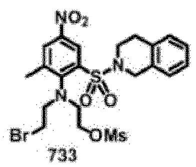
733
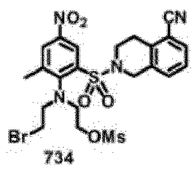
734
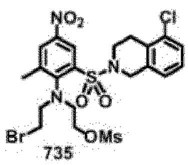
735

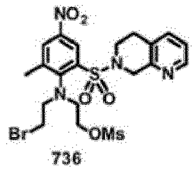
736
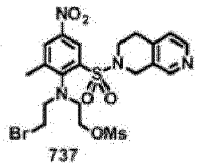
737
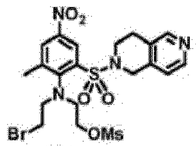
738
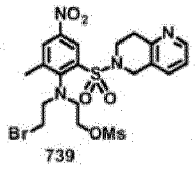
739
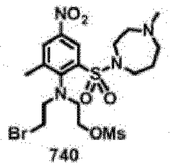
740
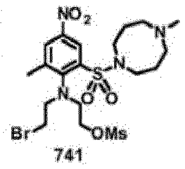
741

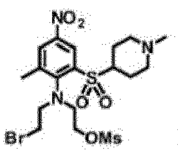
742
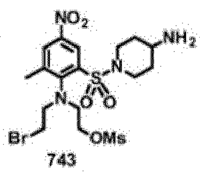
743
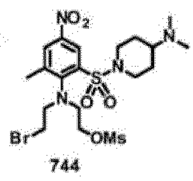
744
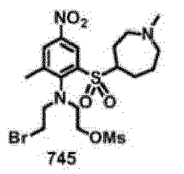
745
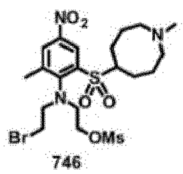
746
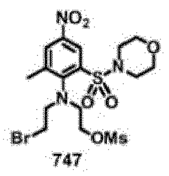
747

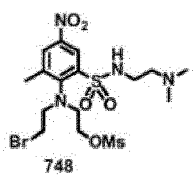
748
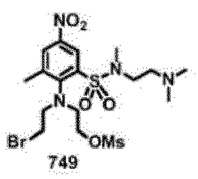
749
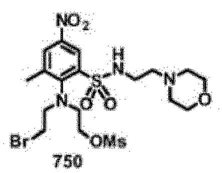
750
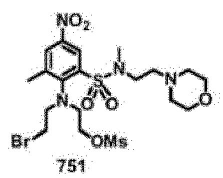
751
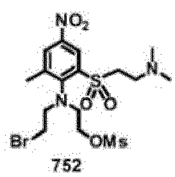
752

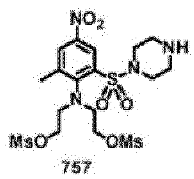
757
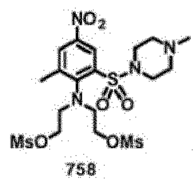
758
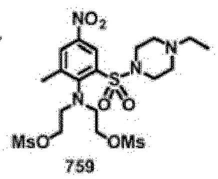
759
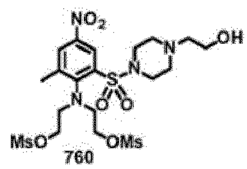
760
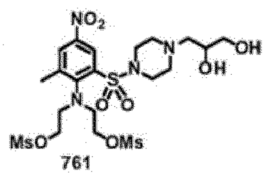
761

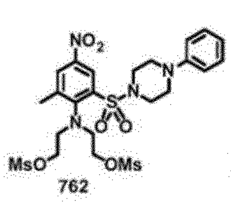
762
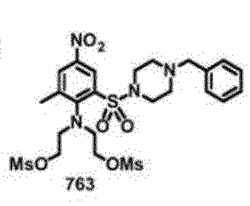
763
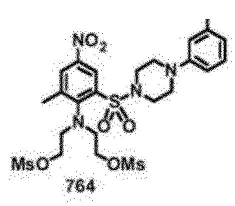
764
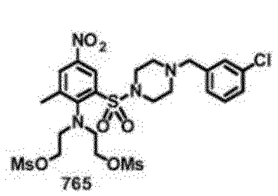
765

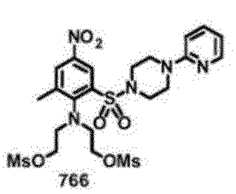
766
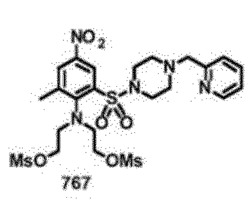
767
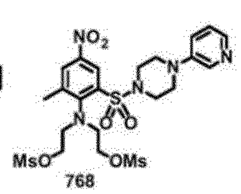
768
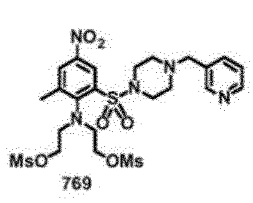
769

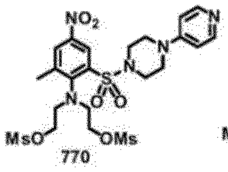
770

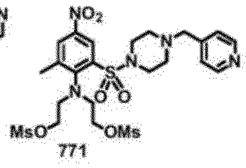
771

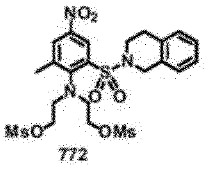
772

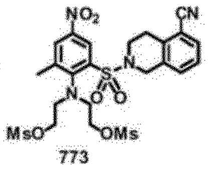
773

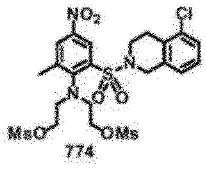
774

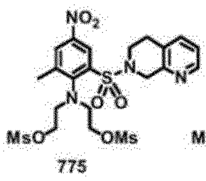
775

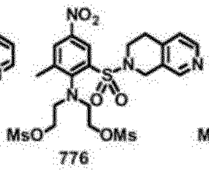
776

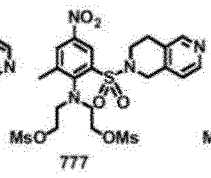
777

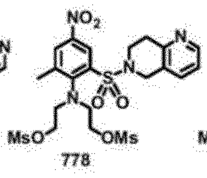
778

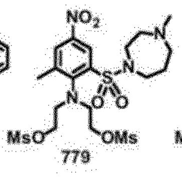
779

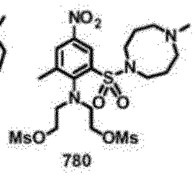
780

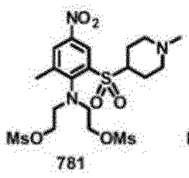
781

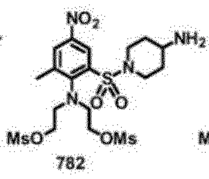
782

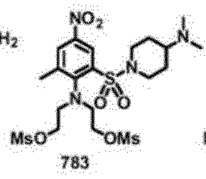
783

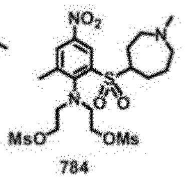
784

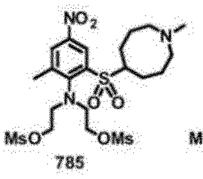
785

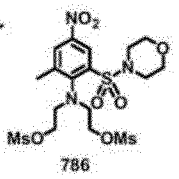
786

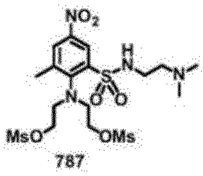
787

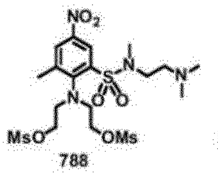
788

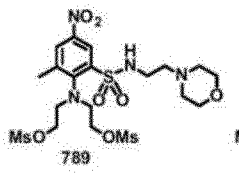
789

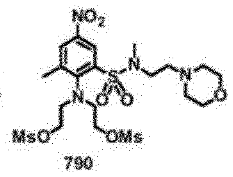
790

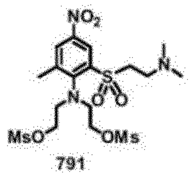
791

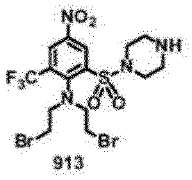
913

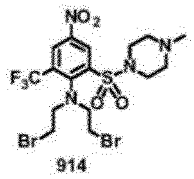
914

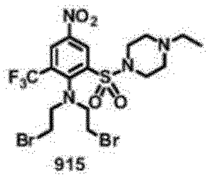
915

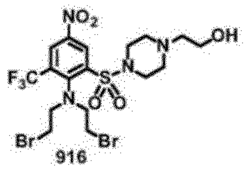
916

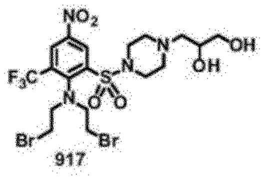
917

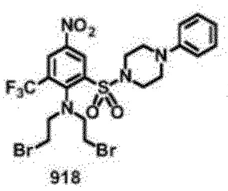
918

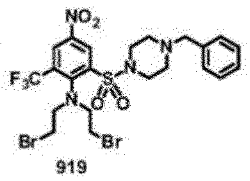
919

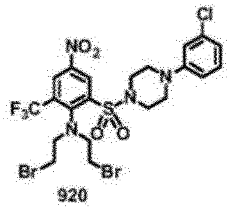
920

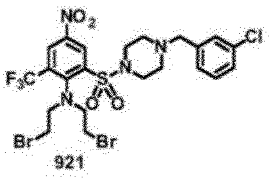
921

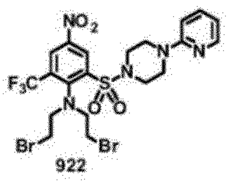
922

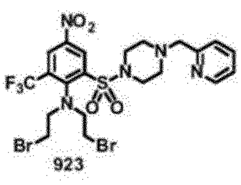
923

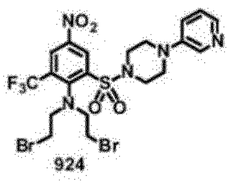
924

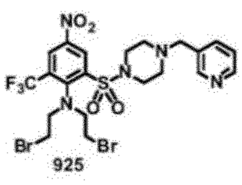
925

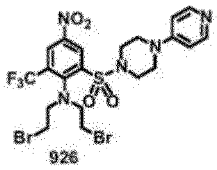
926

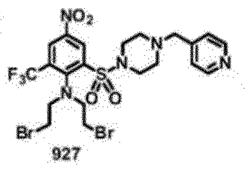
927

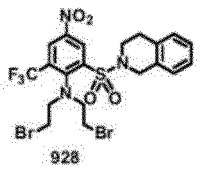
928

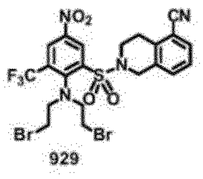
929

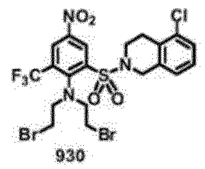
930

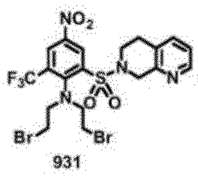 931 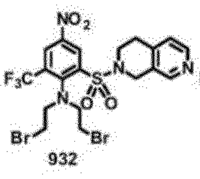 932 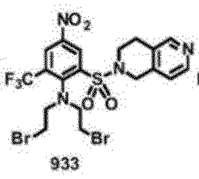 933 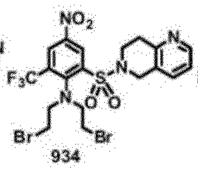 934 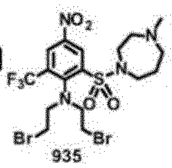 935 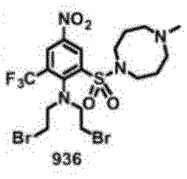 936

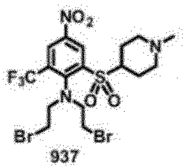 937 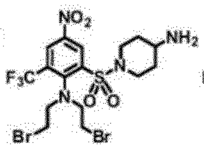 938 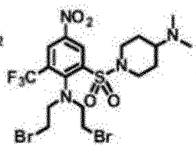 939 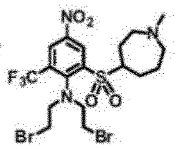 940 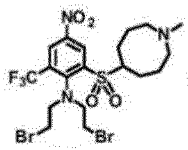 941 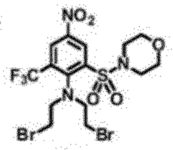 942

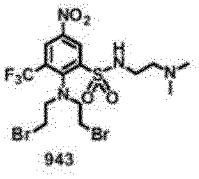 943 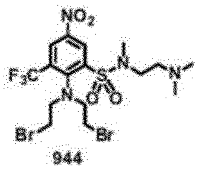 944 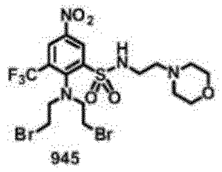 945 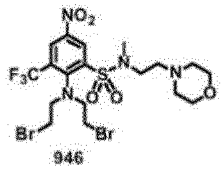 946 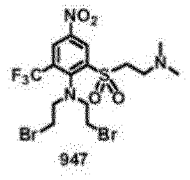 947

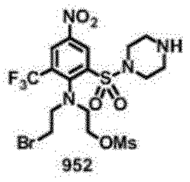 952 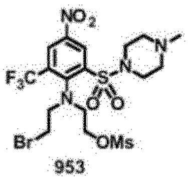 953 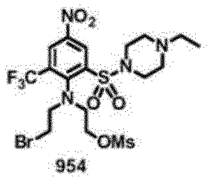 954 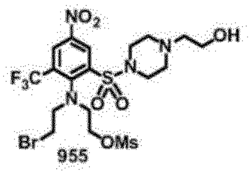 955 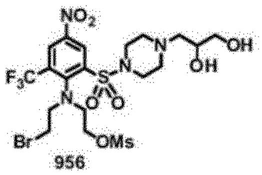 956

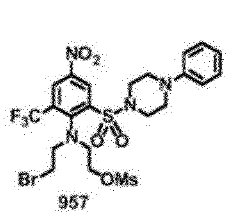 957 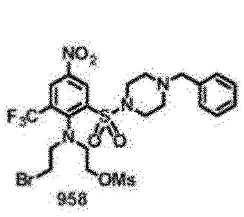 958 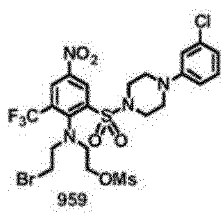 959 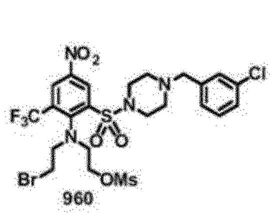 960

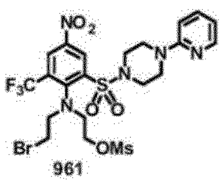 961 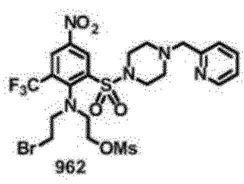 962 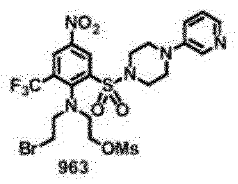 963 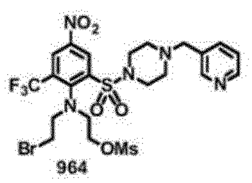 964

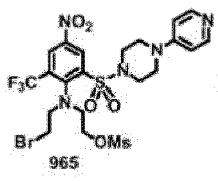 965 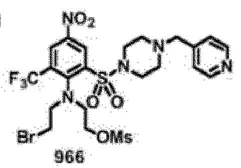 966 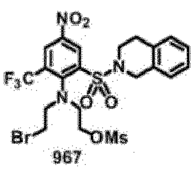 967 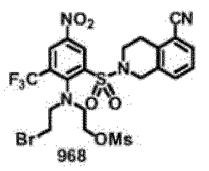 968 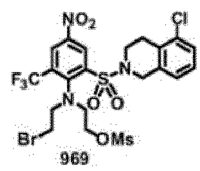 969

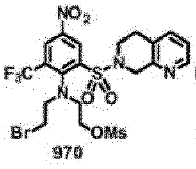 970 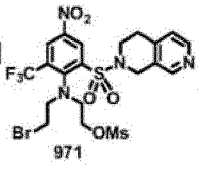 971 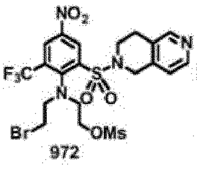 972 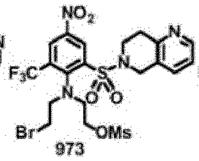 973 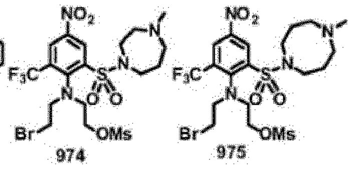 974 975

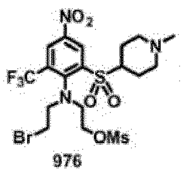
976
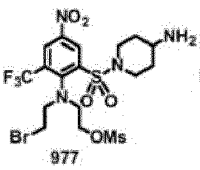
977
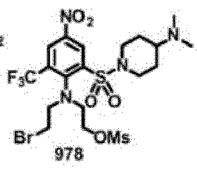
978
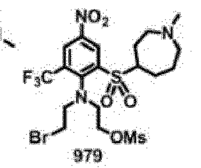
979
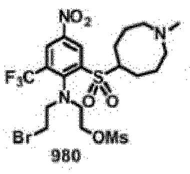
980
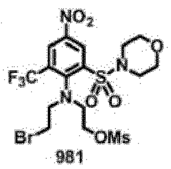
981

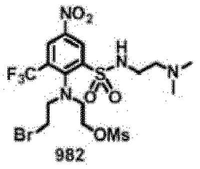
982
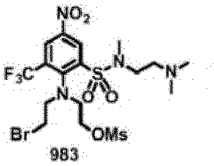
983
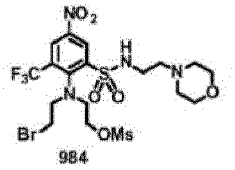
984
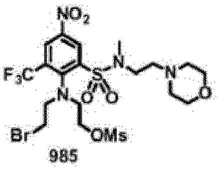
985
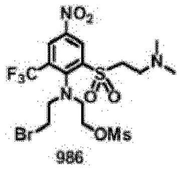
986

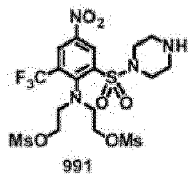
991
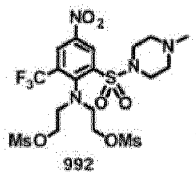
992
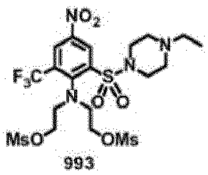
993
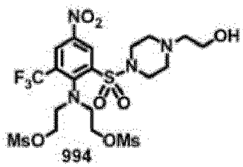
994
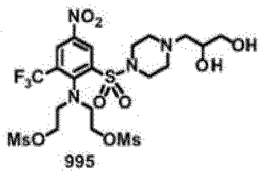
995

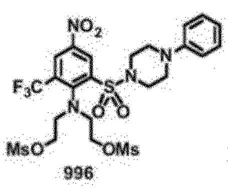
996
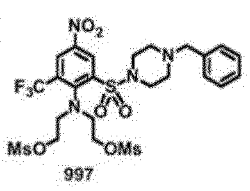
997
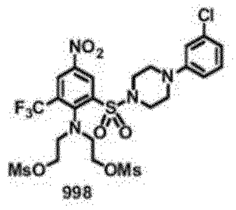
998
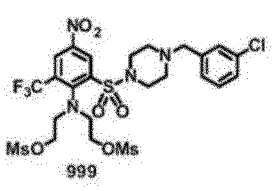
999

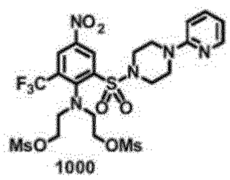
1000
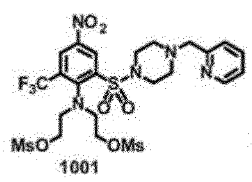
1001
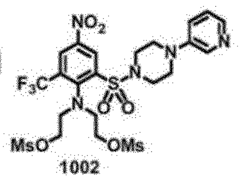
1002
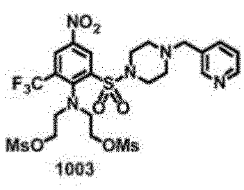
1003

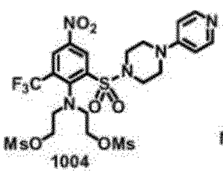
1004
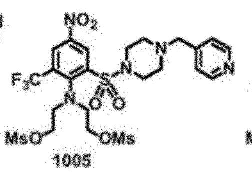
1005
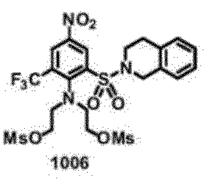
1006
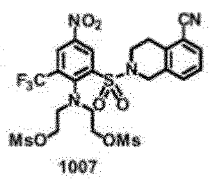
1007
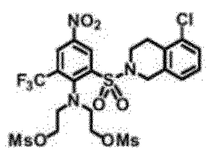
1008

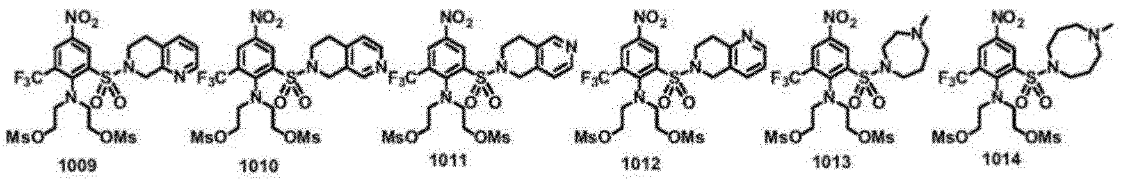
1009
1010
1011
1012
1013
1014

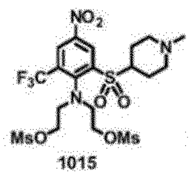
1015
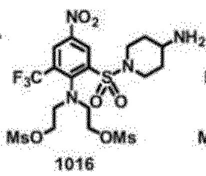
1016
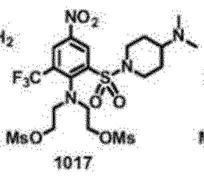
1017
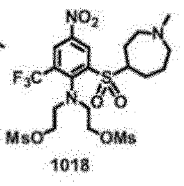
1018
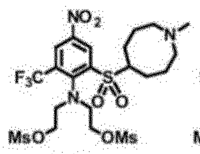
1019
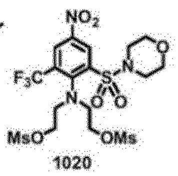
1020

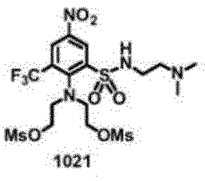
1021
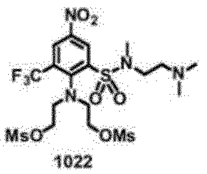
1022
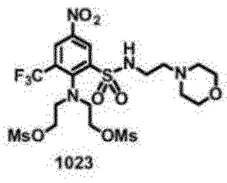
1023
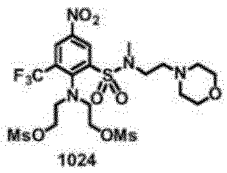
1024
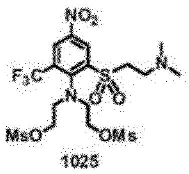
1025

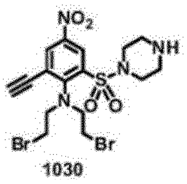
1030
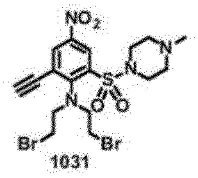
1031
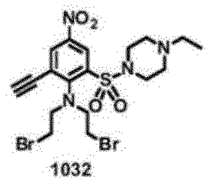
1032
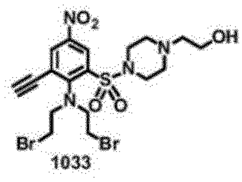
1033
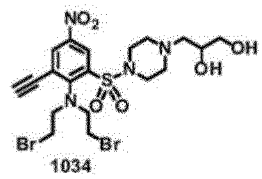
1034

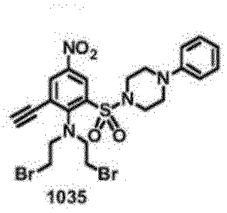
1035
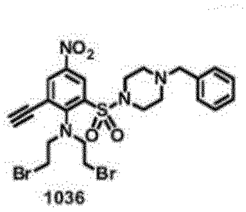
1036
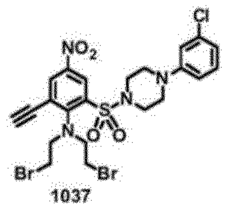
1037
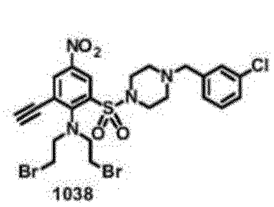
1038

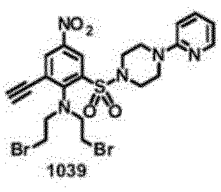
1039
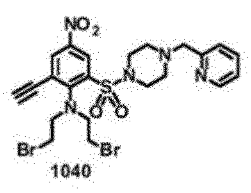
1040
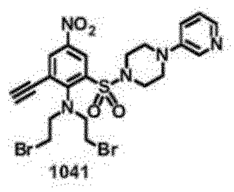
1041
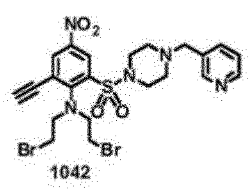
1042

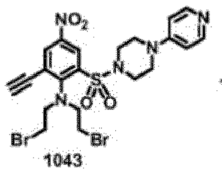
1043
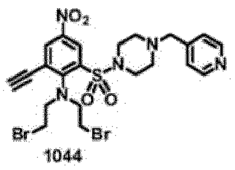
1044
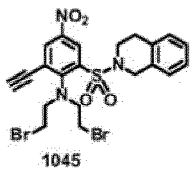
1045
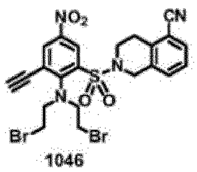
1046
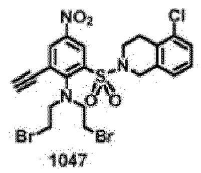
1047

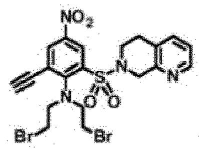
1048
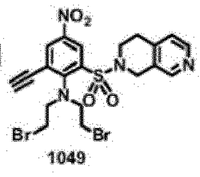
1049
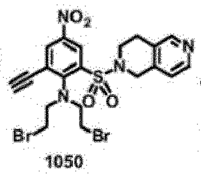
1050
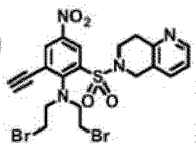
1051
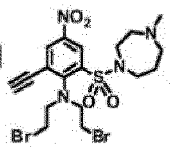
1052
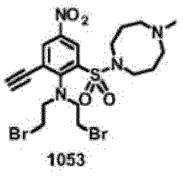
1053

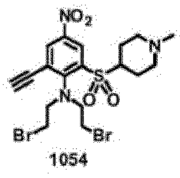
1054
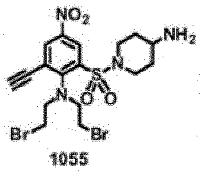
1055
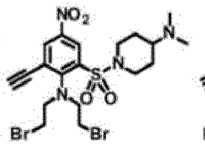
1056
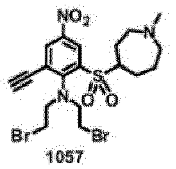
1057
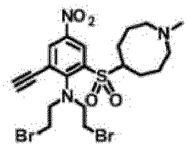
1058
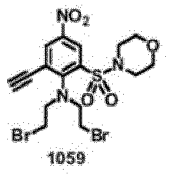
1059

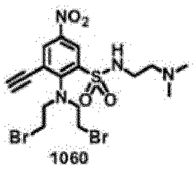
1060
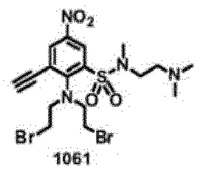
1061
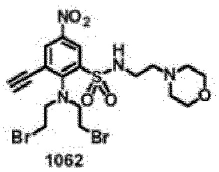
1062
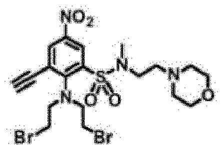
1063
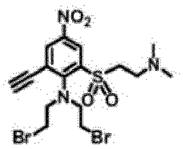
1064

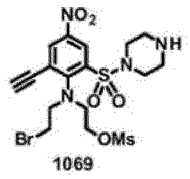
1069
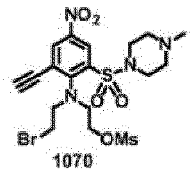
1070
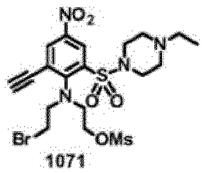
1071
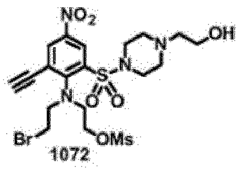
1072
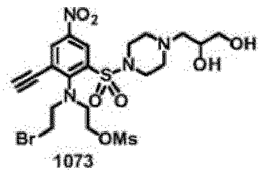
1073

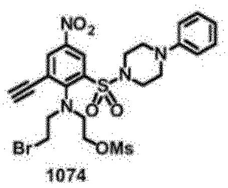
1074
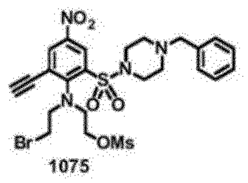
1075
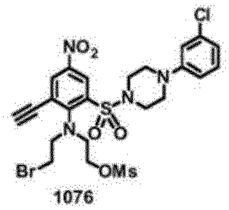
1076
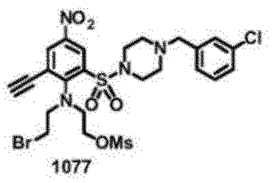
1077

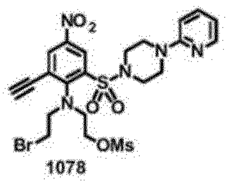
1078
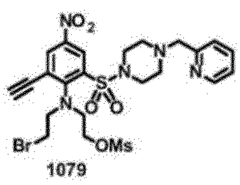
1079
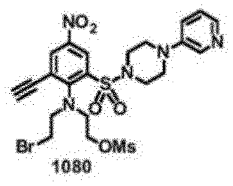
1080
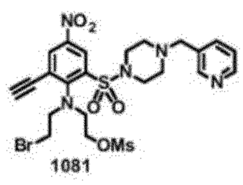
1081

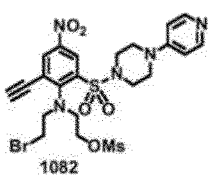
1082
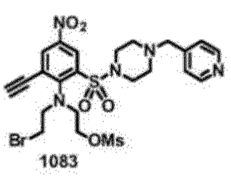
1083
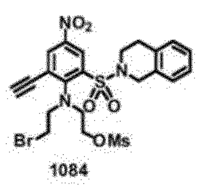
1084
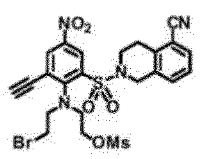
1085
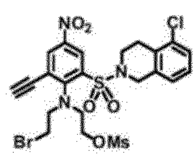
1086

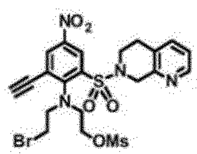
1087
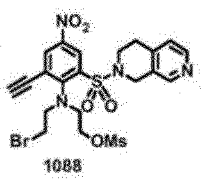
1088
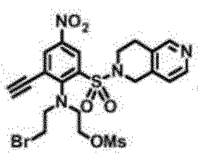
1089
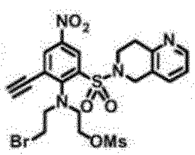
1090
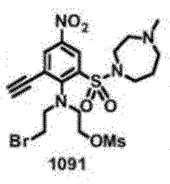
1091
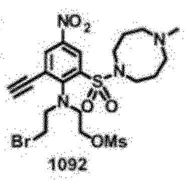
1092

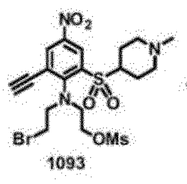
1093
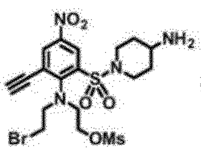
1094
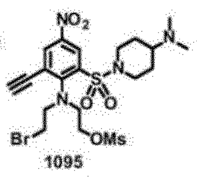
1095
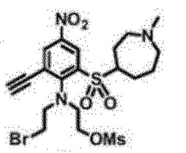
1096
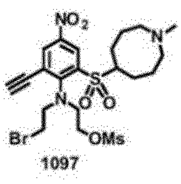
1097
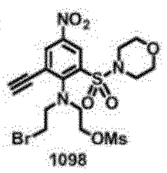
1098

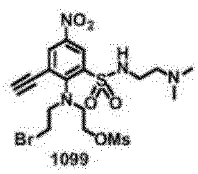
1099
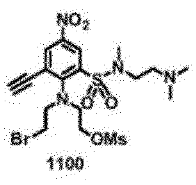
1100
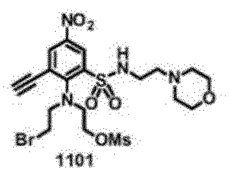
1101
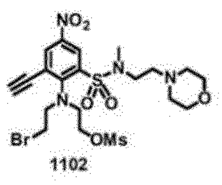
1102
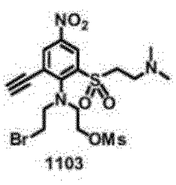
1103

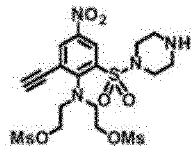
1108
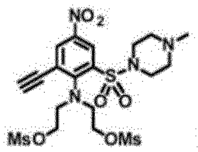
1109
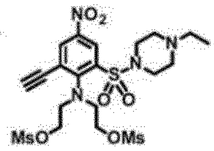
1110
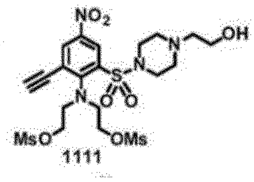
1111
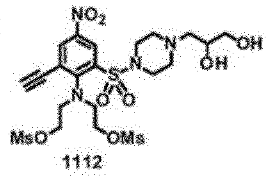
1112

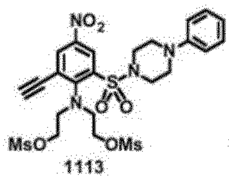
1113

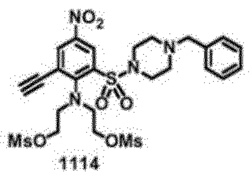
1114

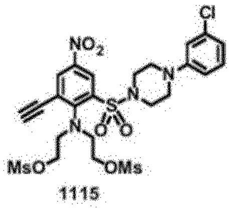
1115

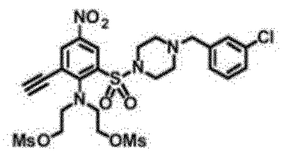
1116

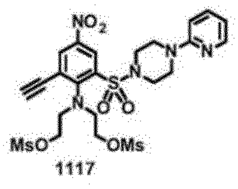
1117

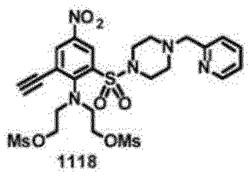
1118

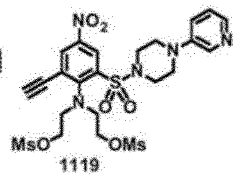
1119

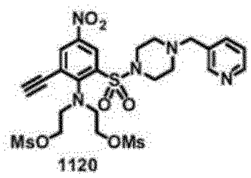
1120

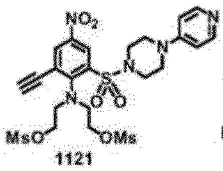
1121

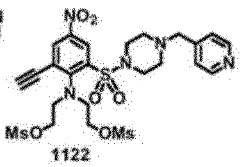
1122

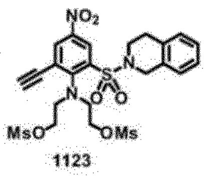
1123

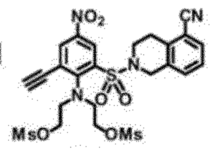
1124

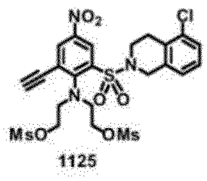
1125

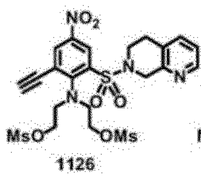
1126

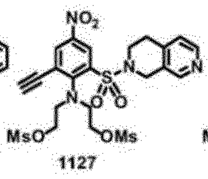
1127

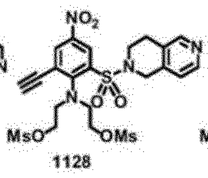
1128

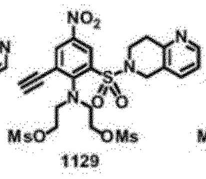
1129

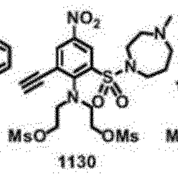
1130

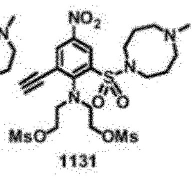
1131

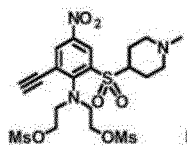
1132

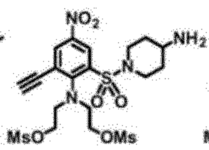
1133

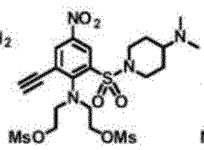
1134

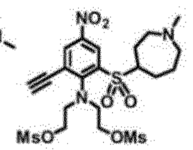
1135

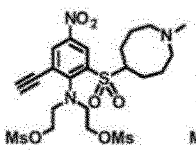
1136

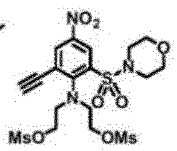
1137

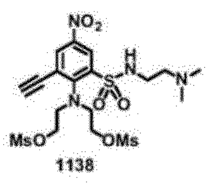
1138

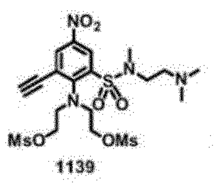
1139

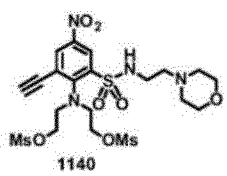
1140

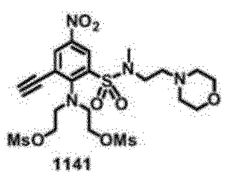
1141

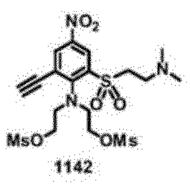
1142

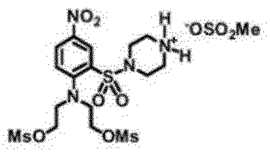
640.Ms

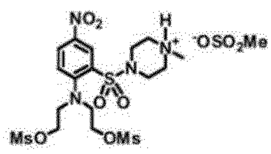
641.Ms

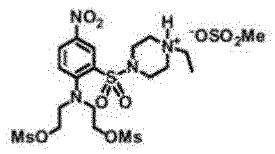
642.Ms

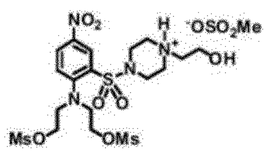
643.Ms

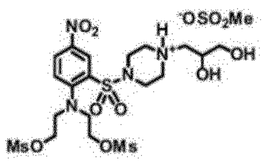
644.Ms

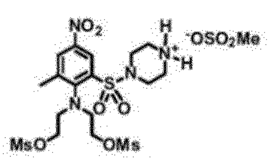
757.Ms

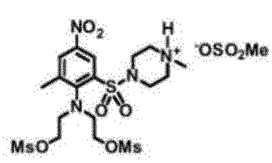
758.Ms

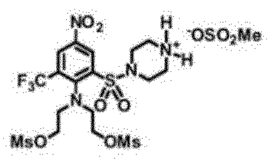
991.Ms

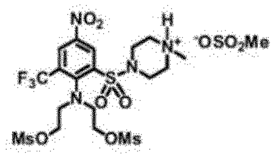

992.Ms

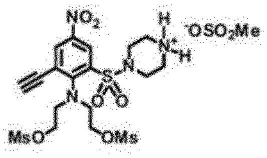

1108.Ms

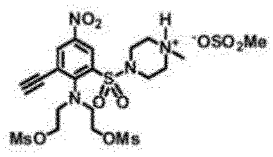

1109.Ms

;

the compound of Formula (4) is selected from the group consisting of:

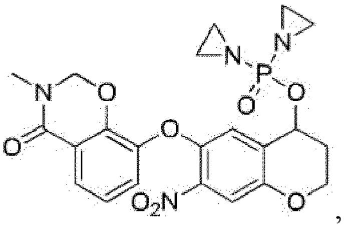
,
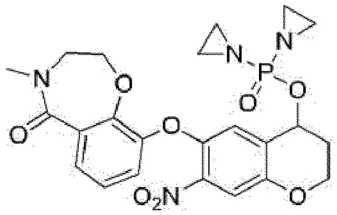
,
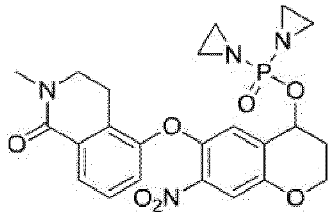
,
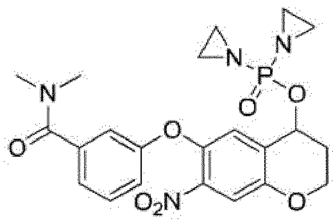
,
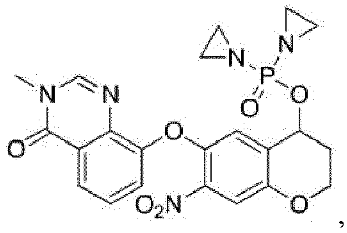
,
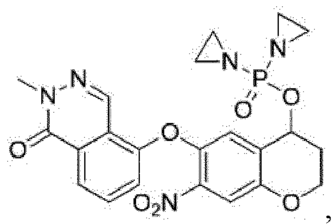
,
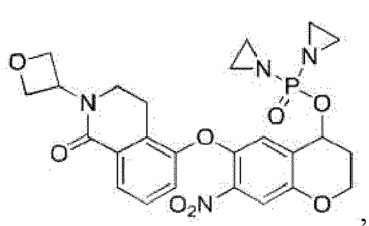
,
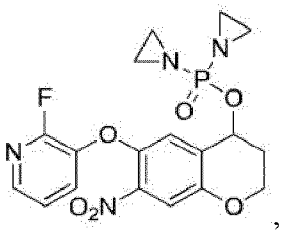
,
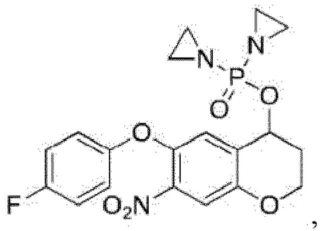
,
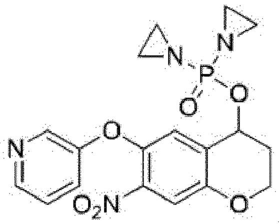
,
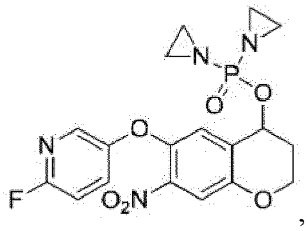
, or
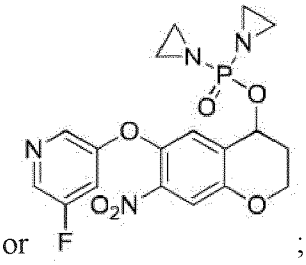
;

the compound of Formula (5) is selected from the group consisting of:

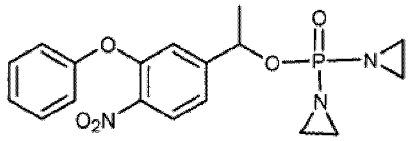
,
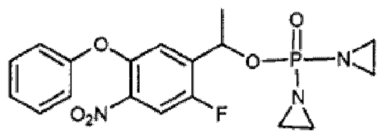
,
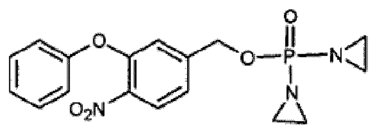
,

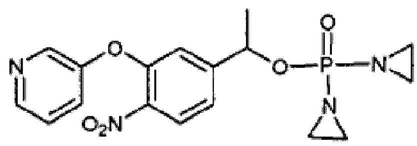,

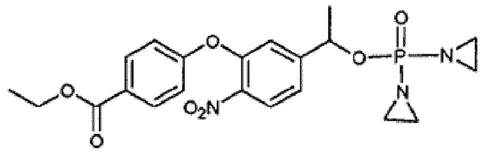, 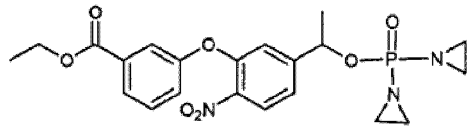,

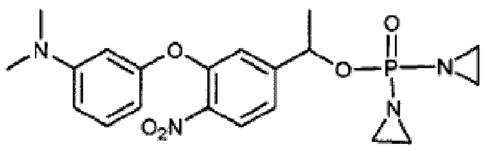, 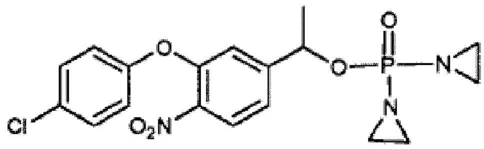,

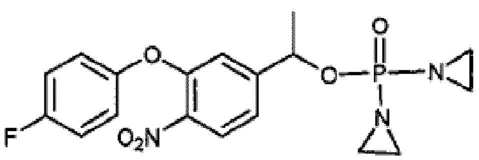, 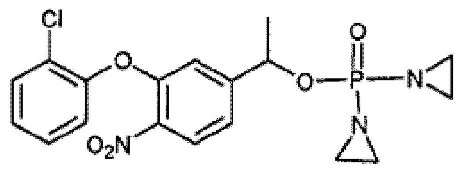,

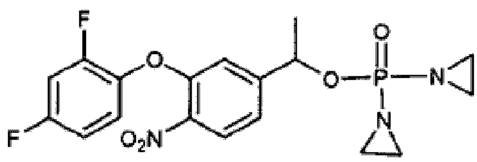, 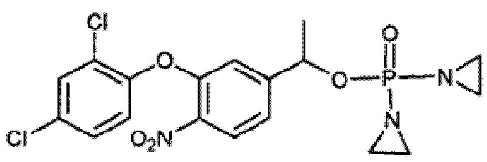,

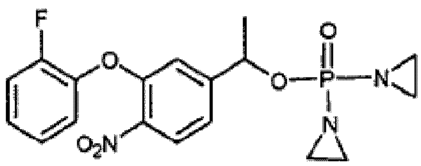, 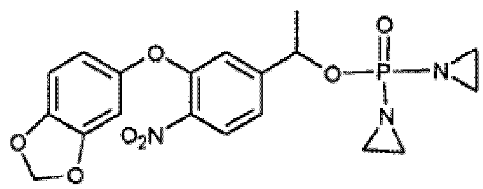,

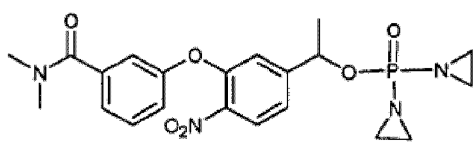, 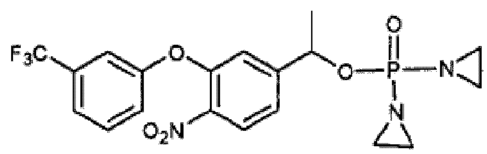,

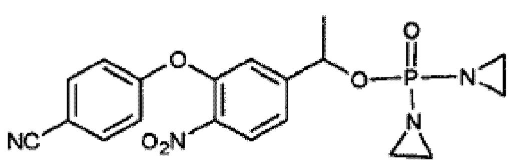, 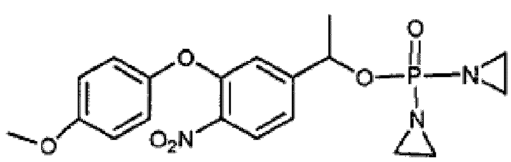,

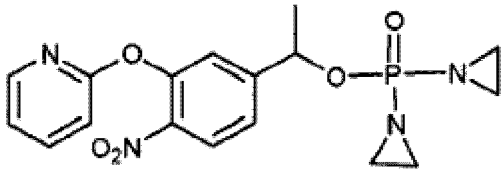
,
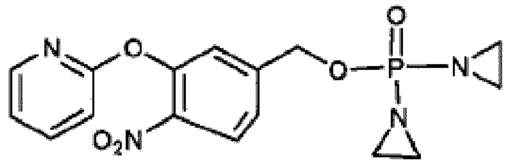
,

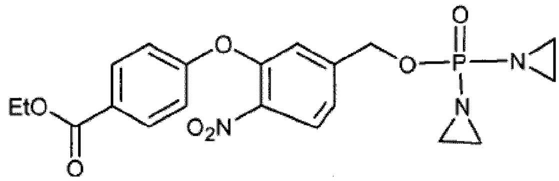
,
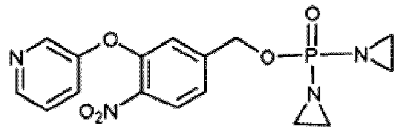
,

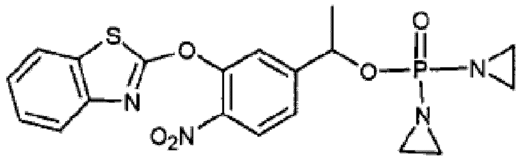
,
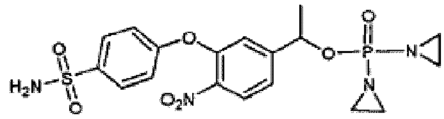
,

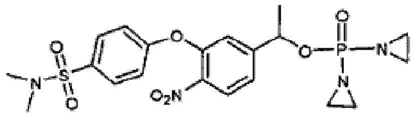
,
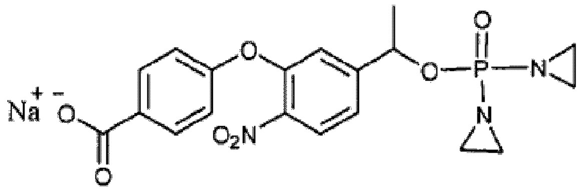
,

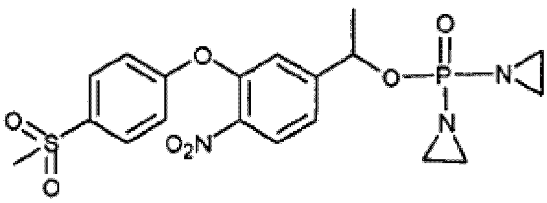
,
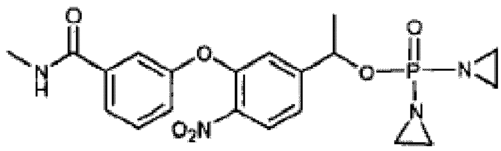
,

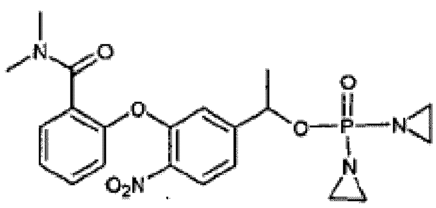
,
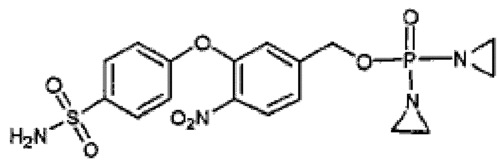
,

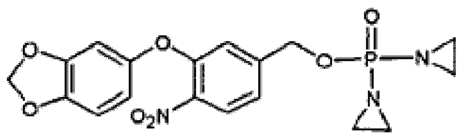
,
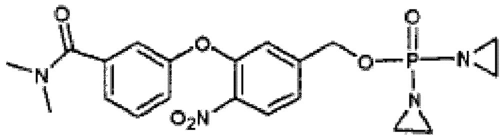
,

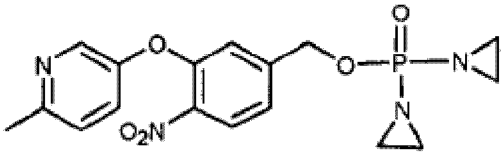,
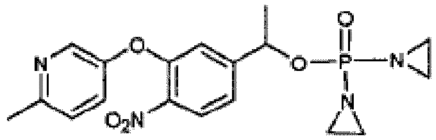,

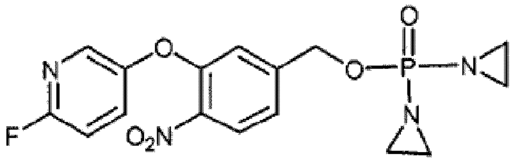,
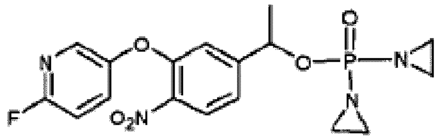,

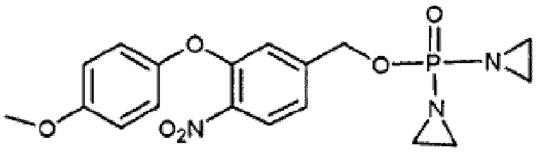,
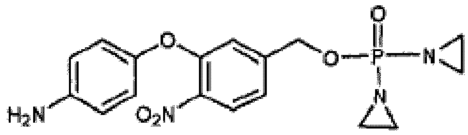,

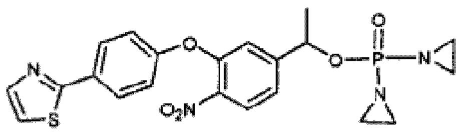,
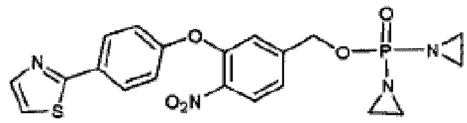,

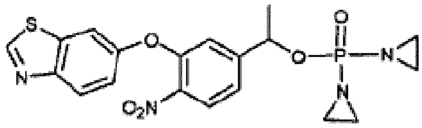,
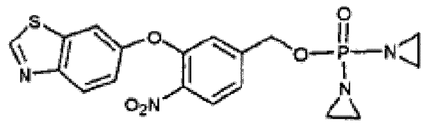,

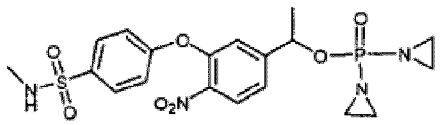,
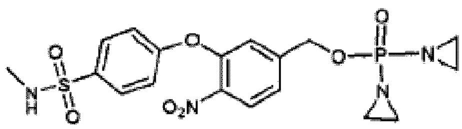,

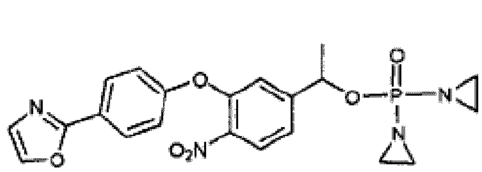,
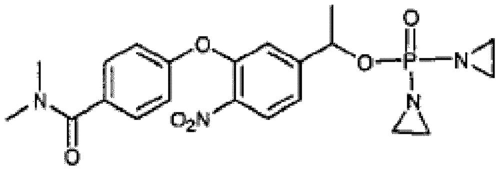,

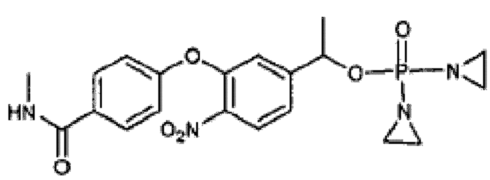,
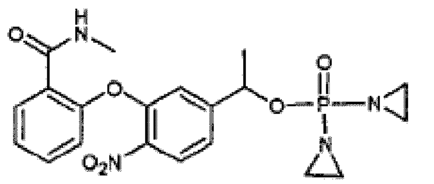,

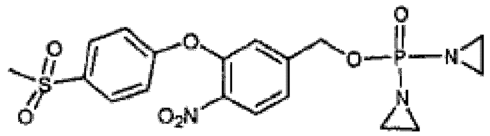
,
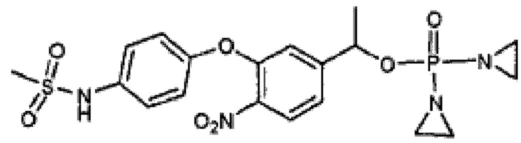
,
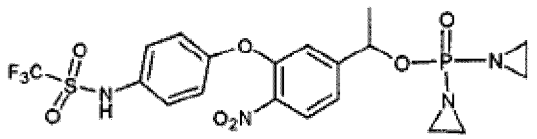
,
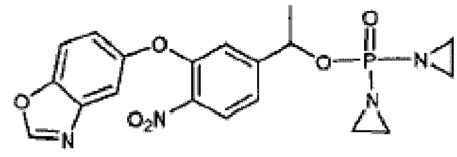
,
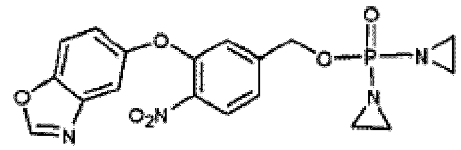
,
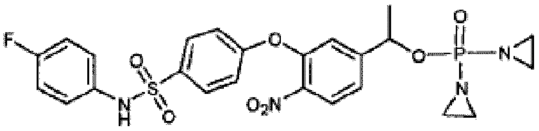
,
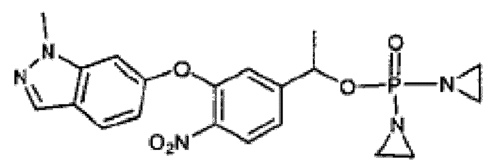
,
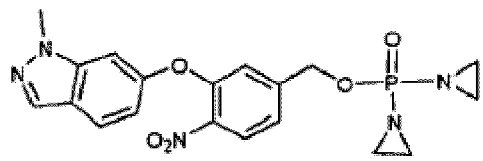
,
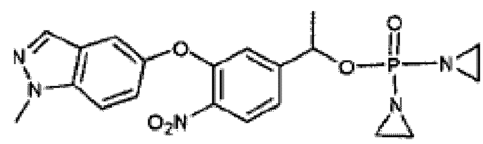
,
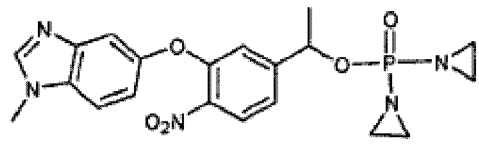
,
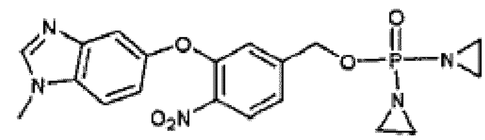
,
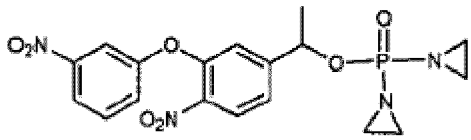
,
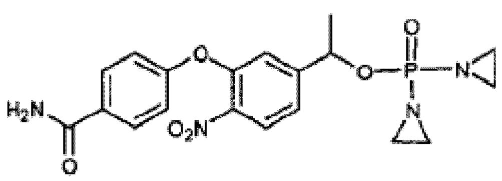
,
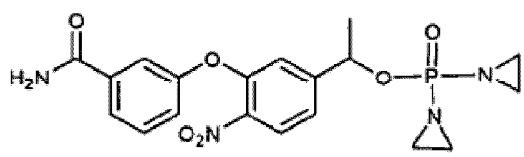
,
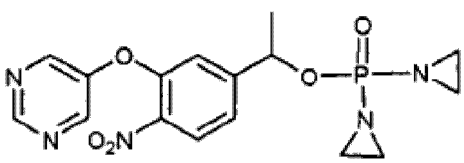
,
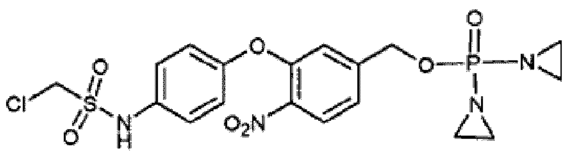
,

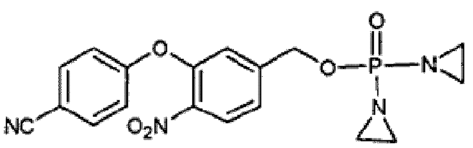
,
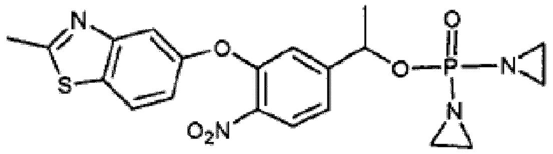
,

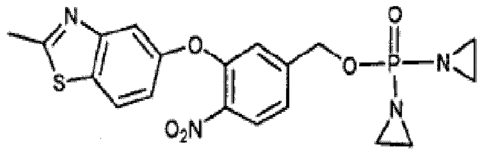
,
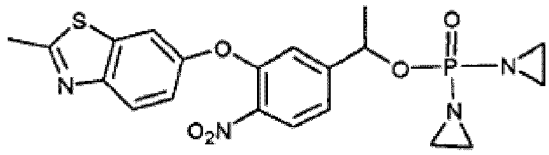
,

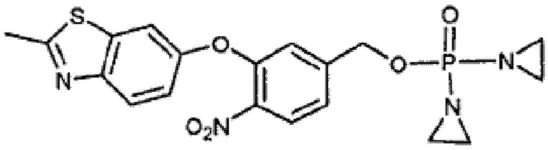
,
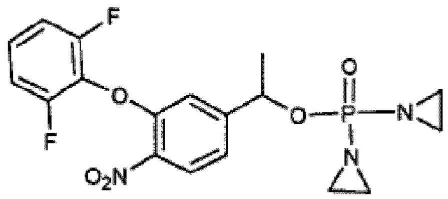
,

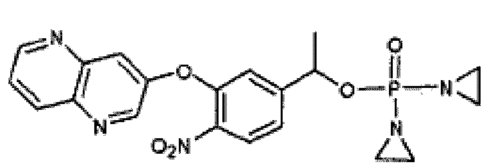
,
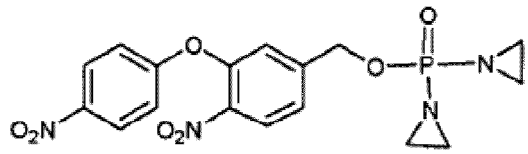
,

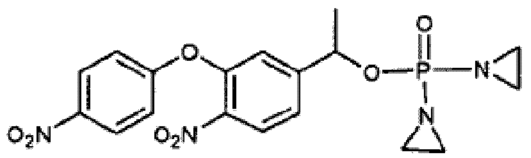
,
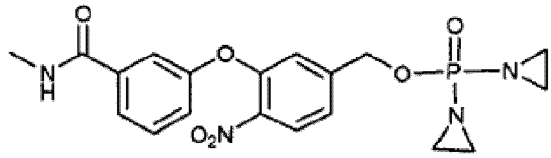
,

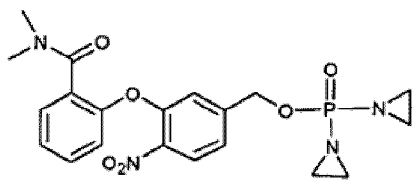
,
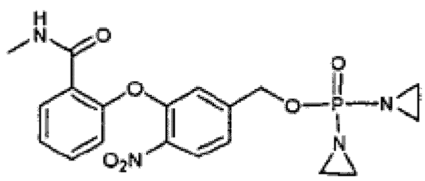
,

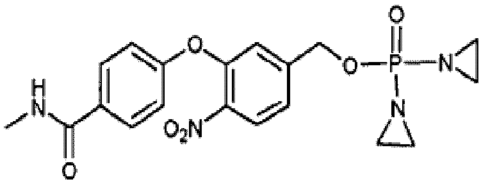
,
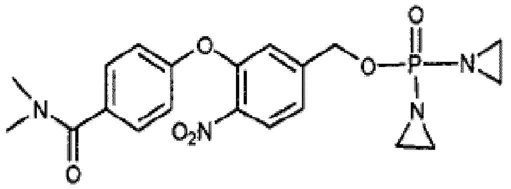
,

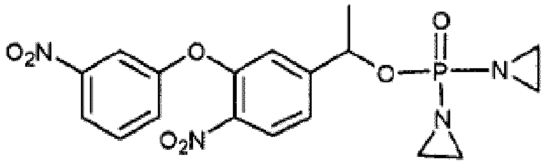
,
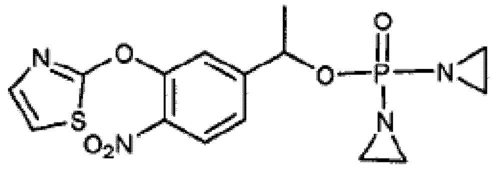
,

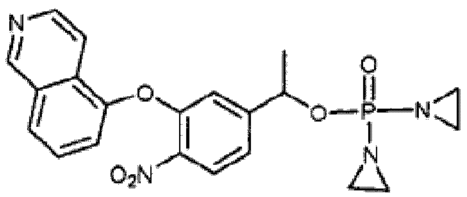
,
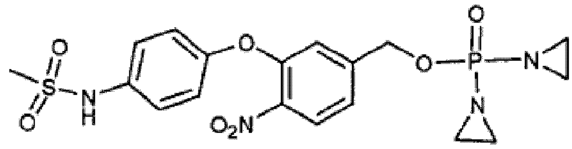
,

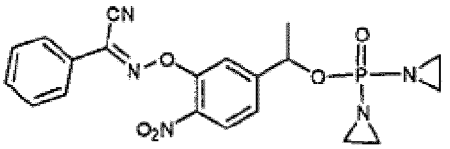
,
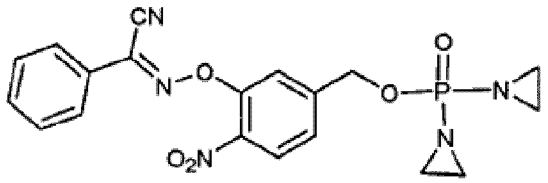
,

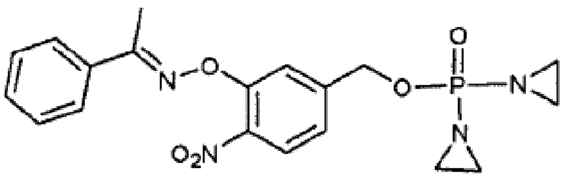
,
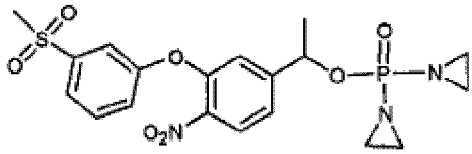
,

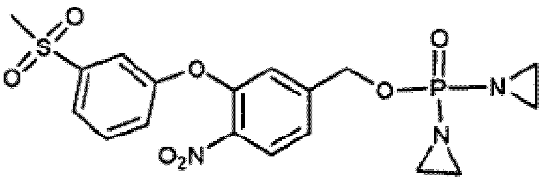
,
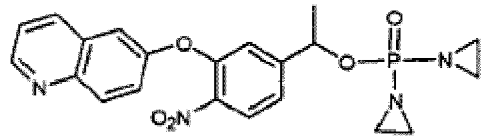
,

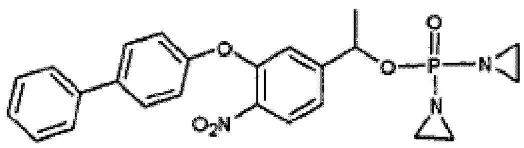
,
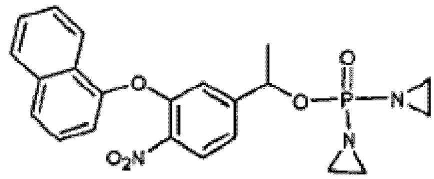
,

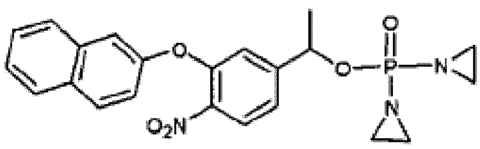
,
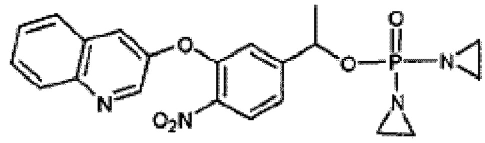
,

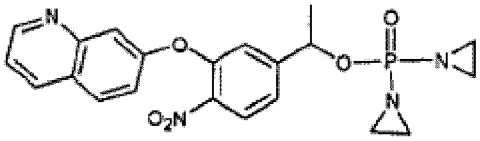
,
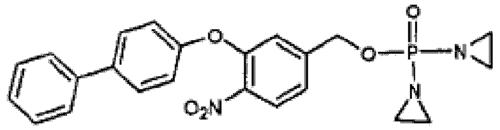
,

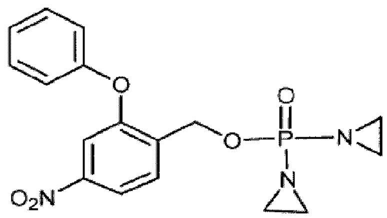
,
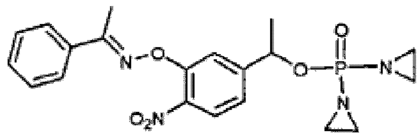
,

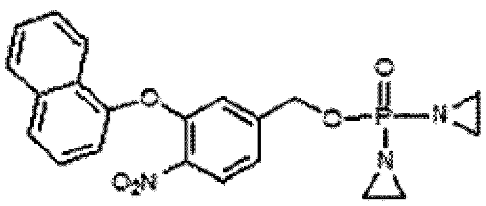
,
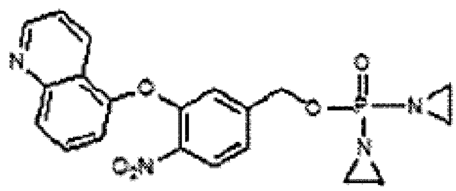
,

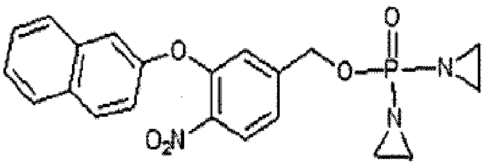
,
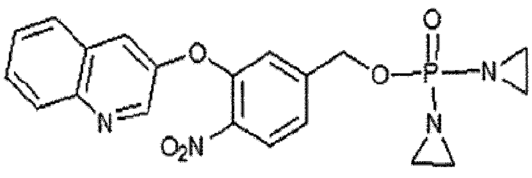
,

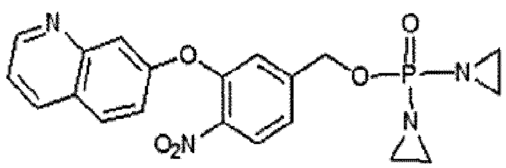
,
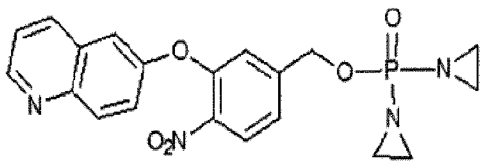
,

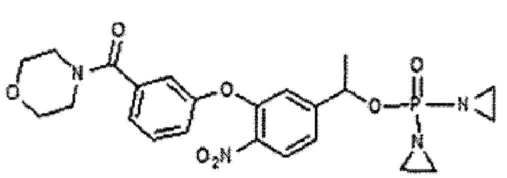
,
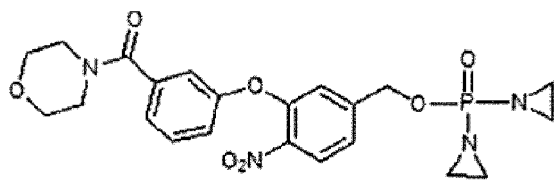
,

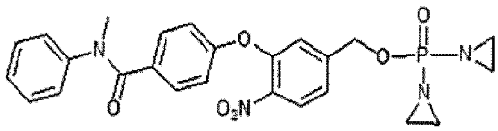
,
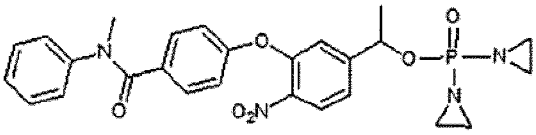
,

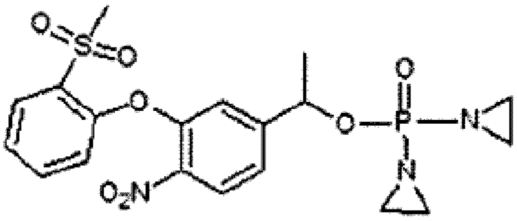
,
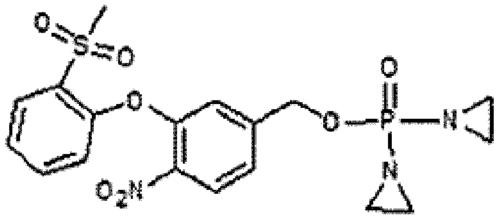
,

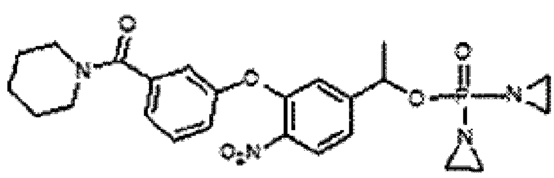,
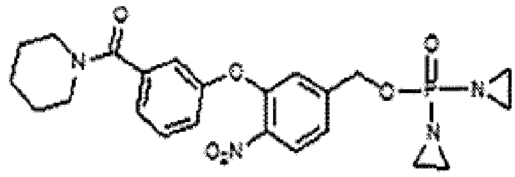,

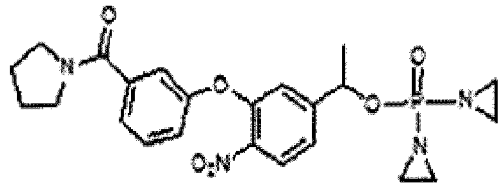,
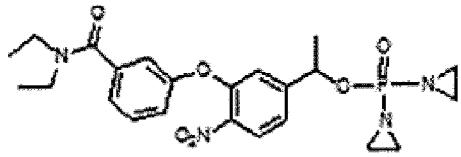,

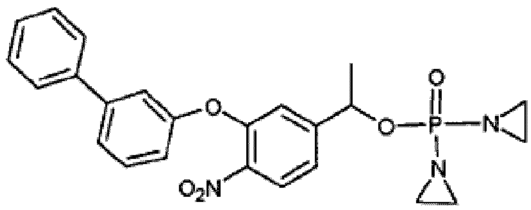,
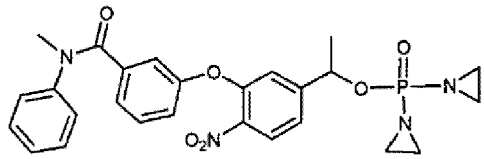,

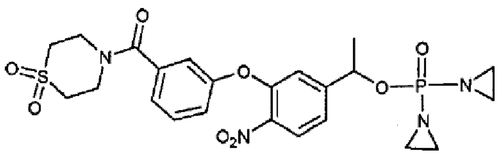,
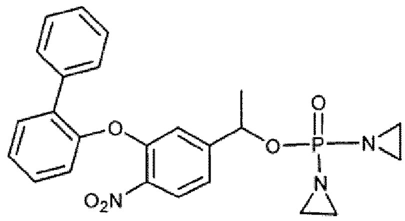,

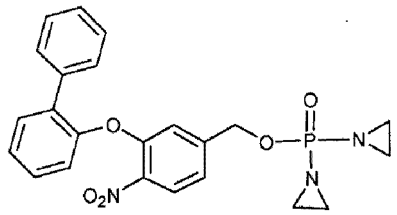,
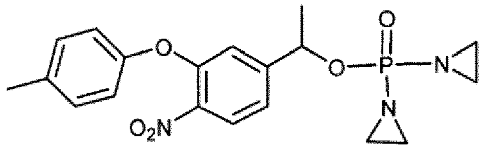,

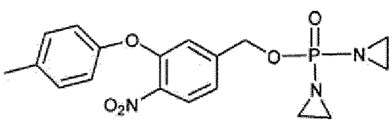,
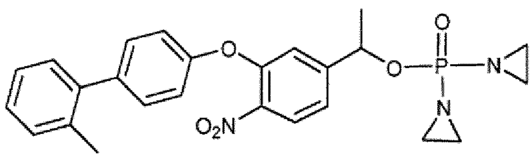,

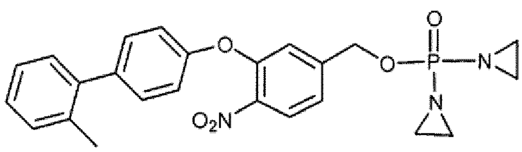,
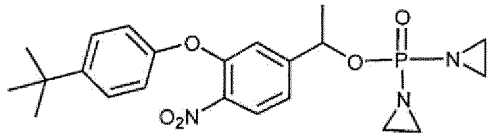,

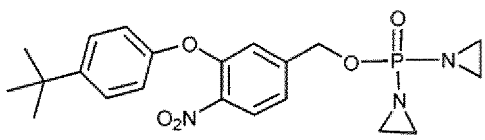, 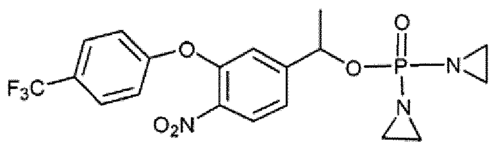,

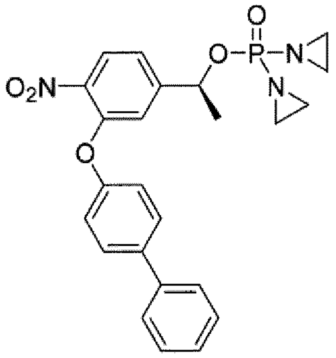, 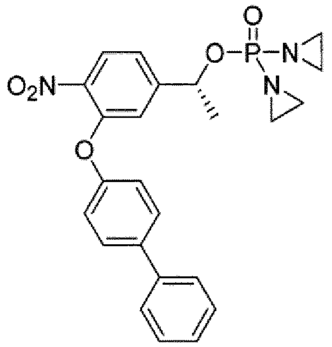,

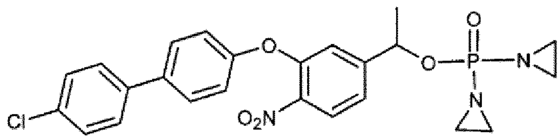, 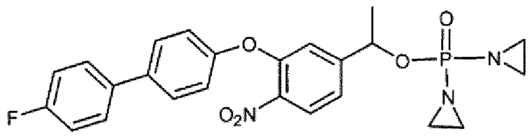,

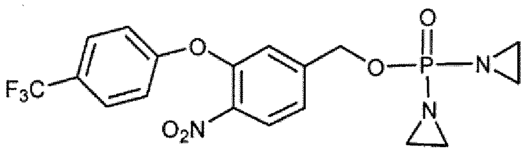 and 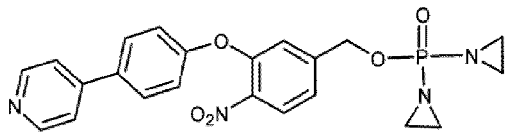;

the compound of Formula (6) is selected from the group consisting of:

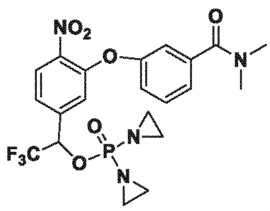, 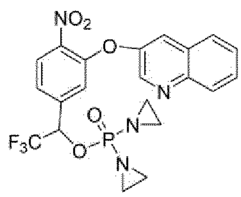, 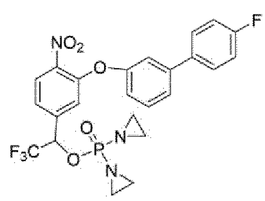,

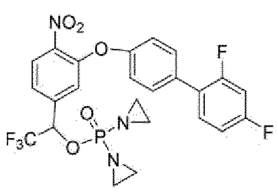, 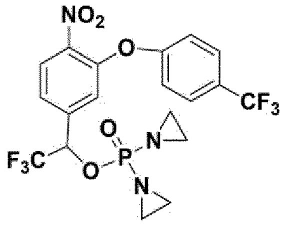, 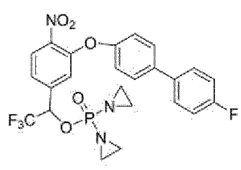,

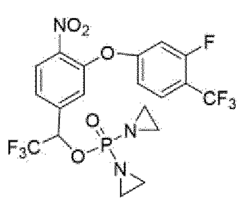
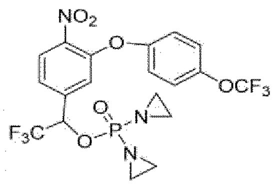
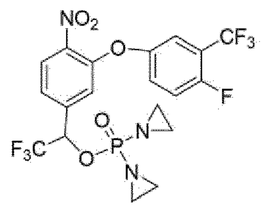

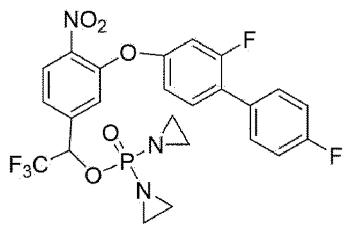
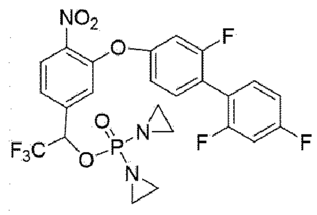
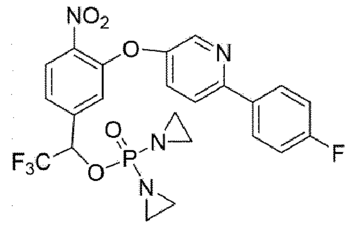

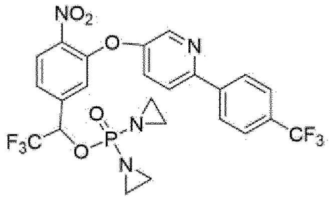
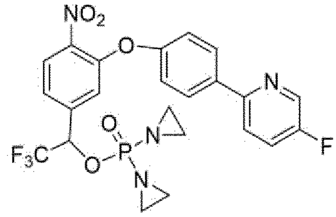
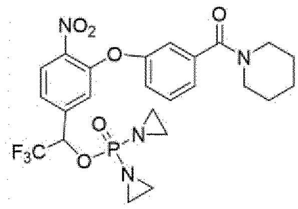

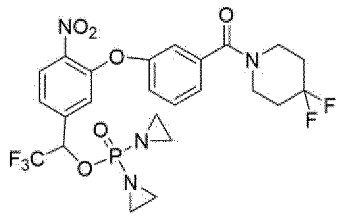
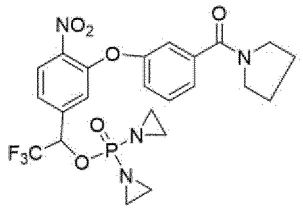
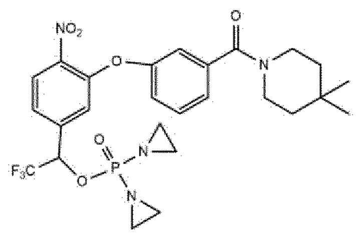

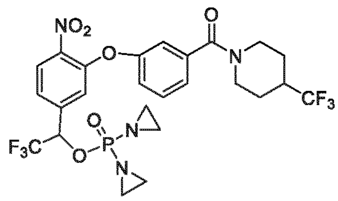
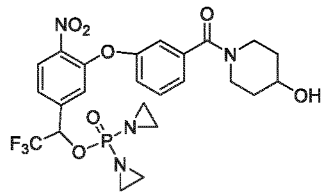
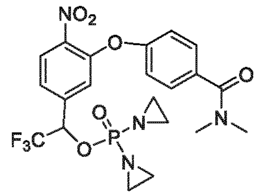

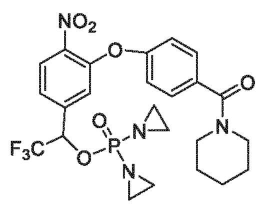
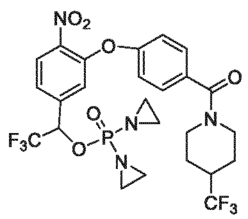
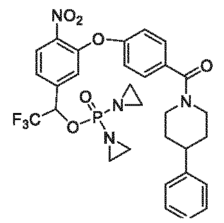

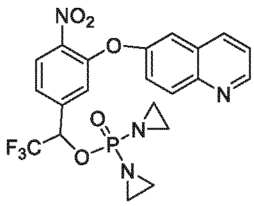,
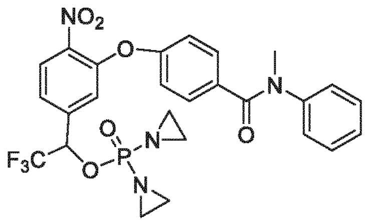,
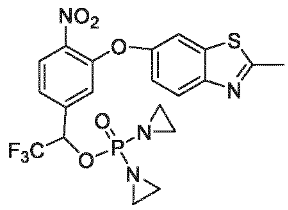,

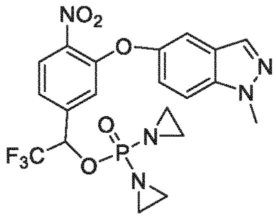,
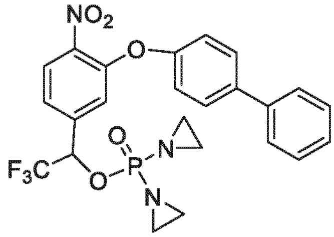,
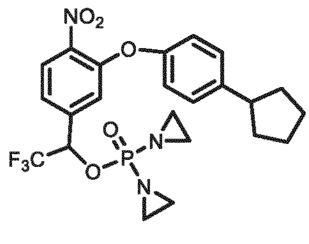,

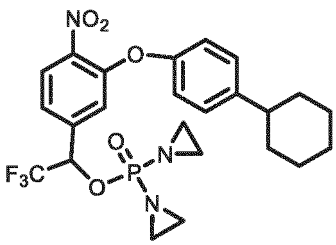,
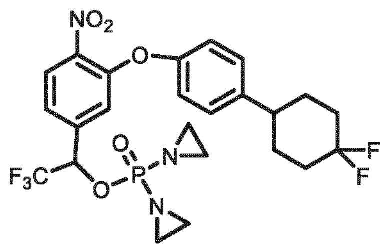

and

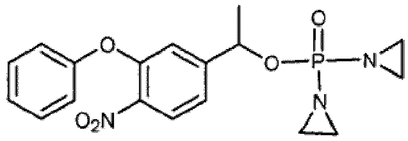,
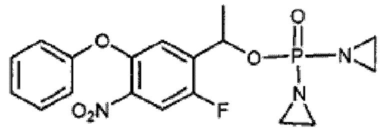,

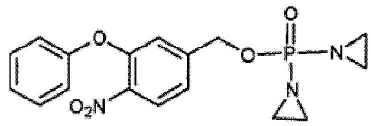,
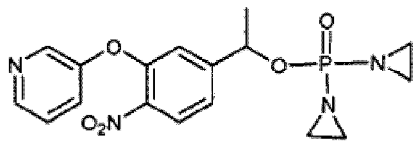,

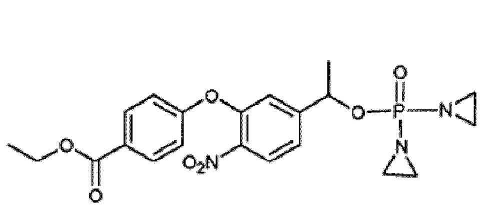,
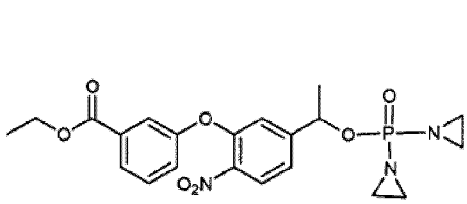,

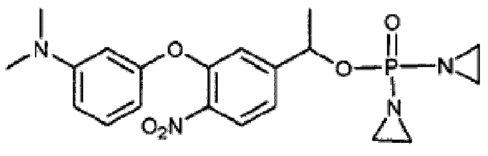,
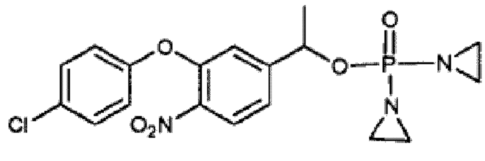,

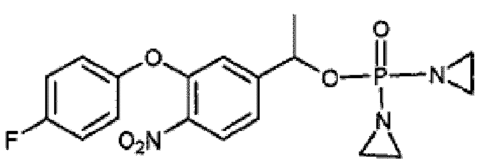
,
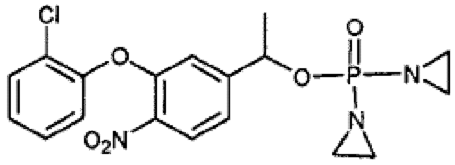
,

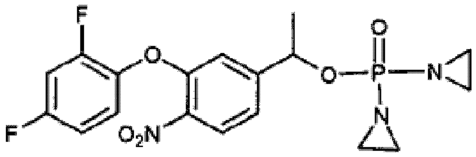
,
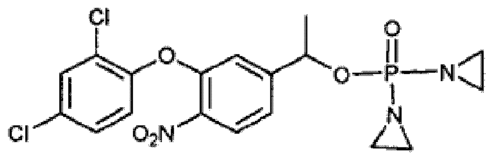
,

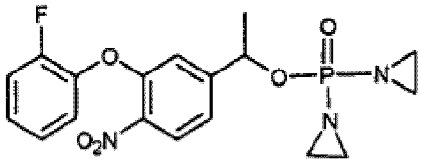
,
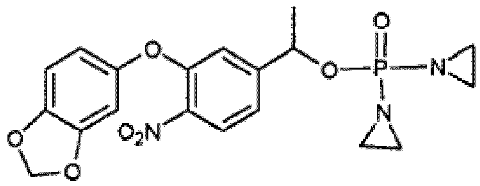
,

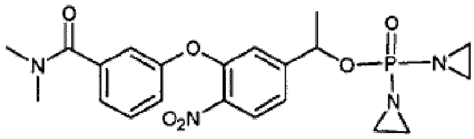
,
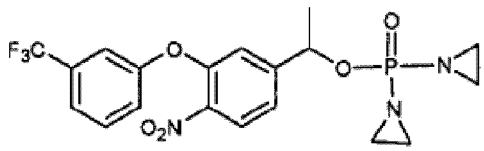
,

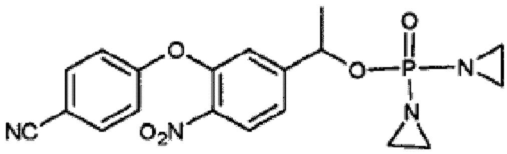
,
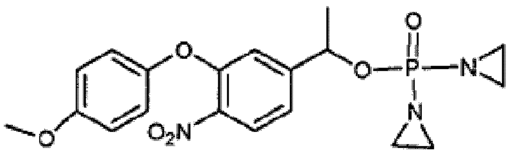
,

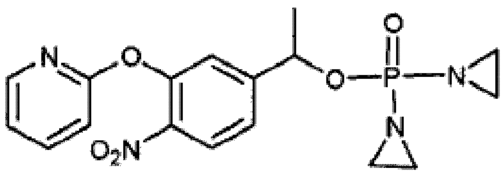
,
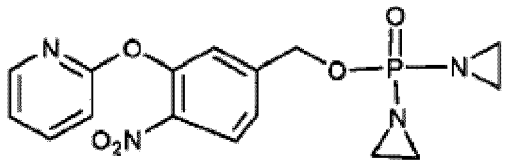
,

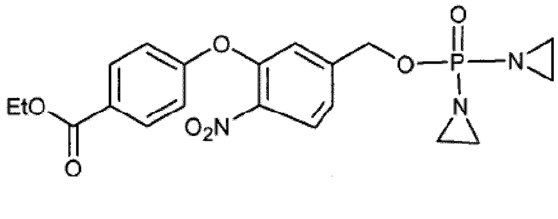
,
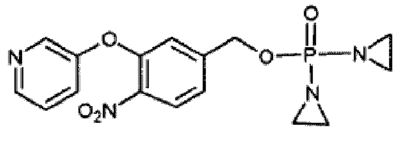
,

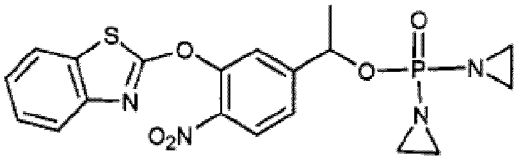
,
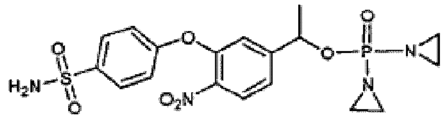
,

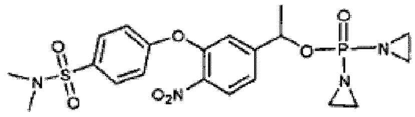,

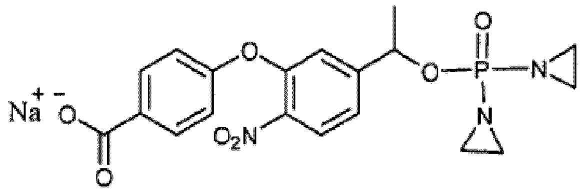,

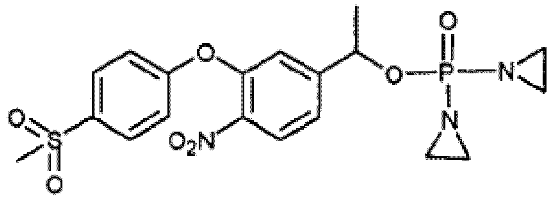,

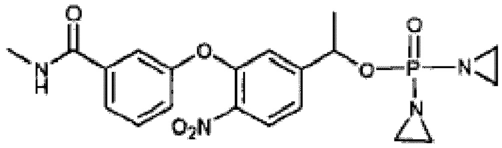,

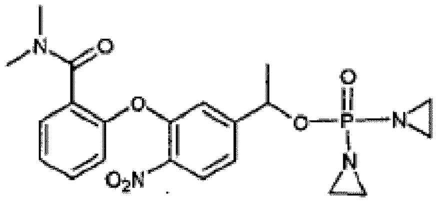,

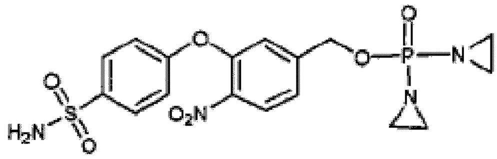,

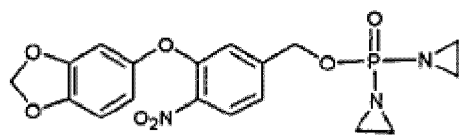,

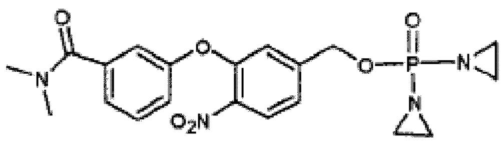,

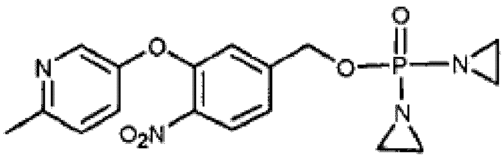,

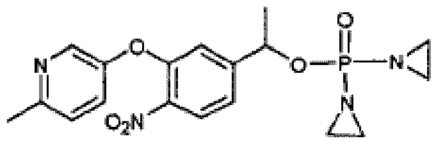,

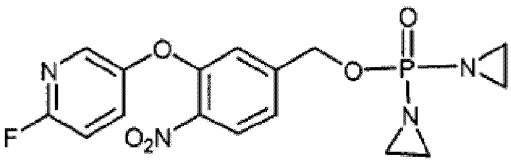,

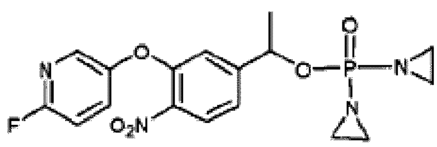,

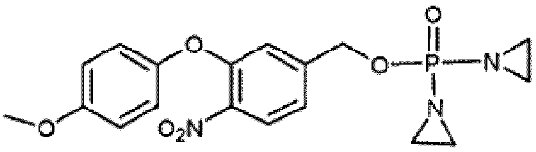,

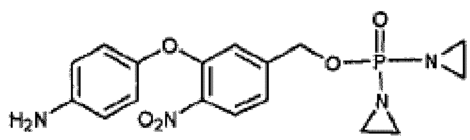,

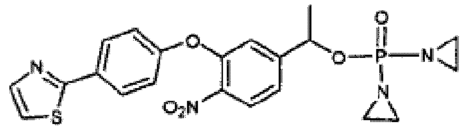,
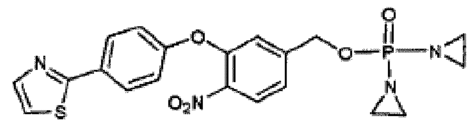,

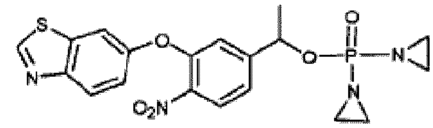,
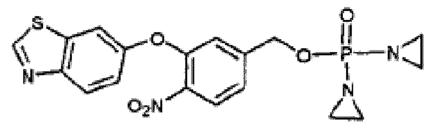,

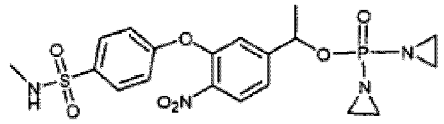,
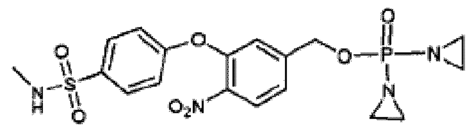,

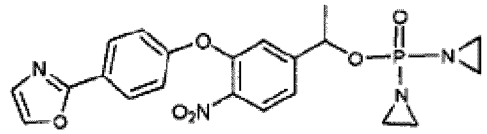,
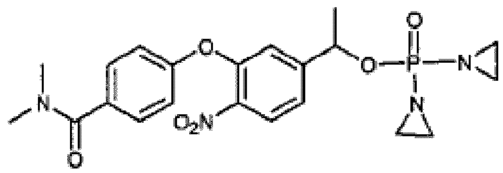,

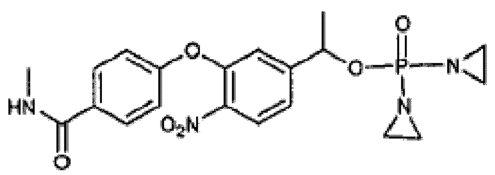,
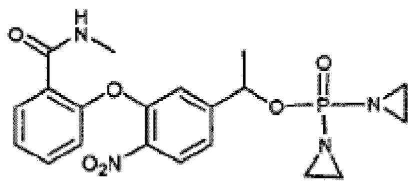,

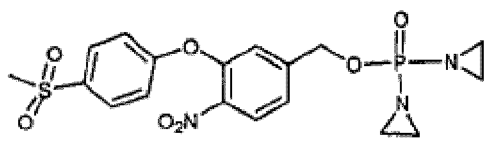,
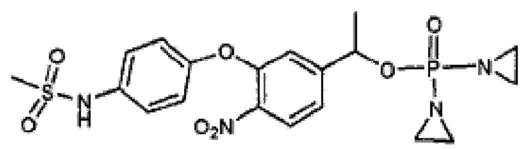,

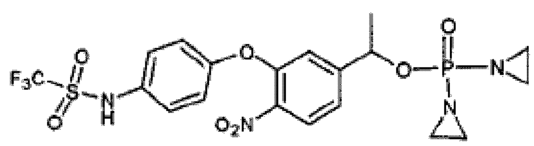,
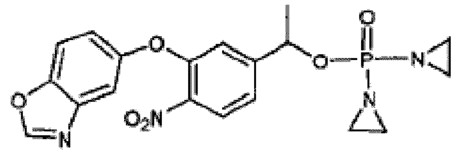,

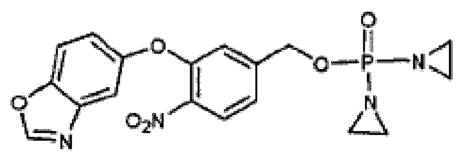,
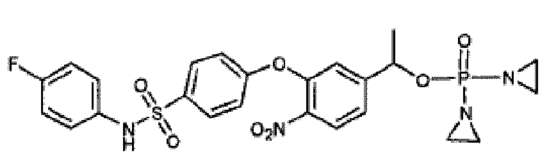,

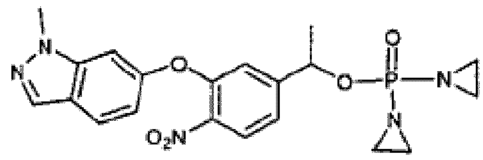
,
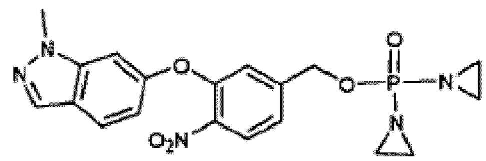
,
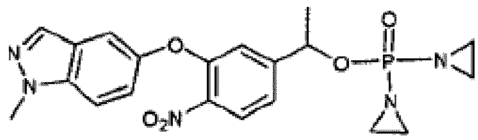
,
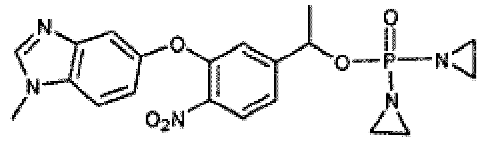
,
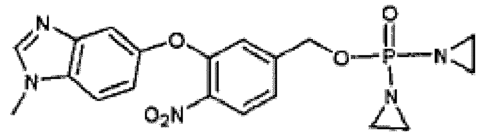
,
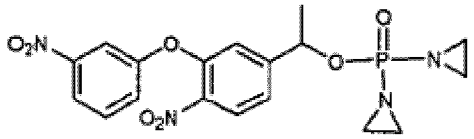
,
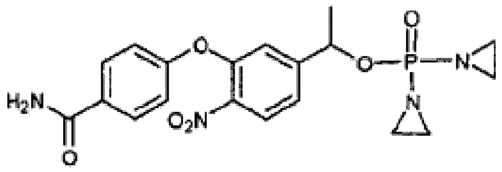
,
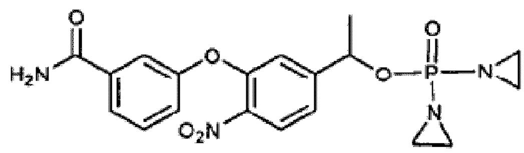
,
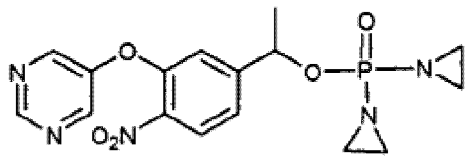
,
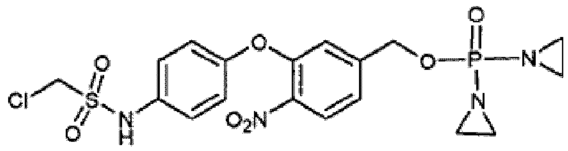
,
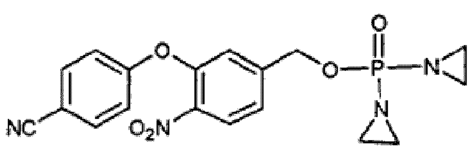
,
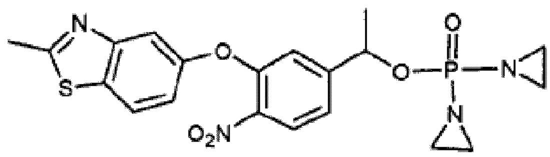
,
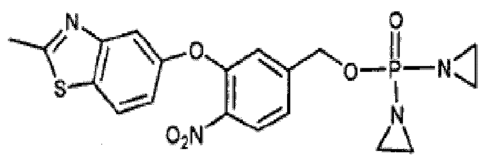
,
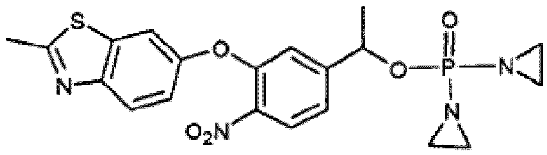
,
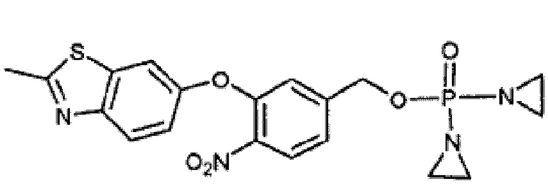
,
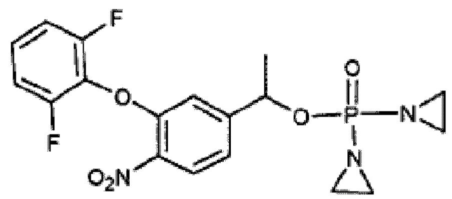
,

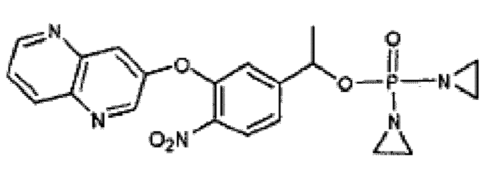,
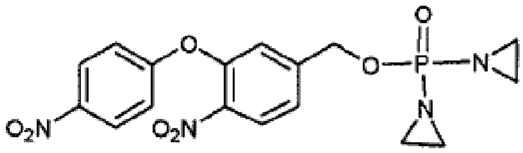,

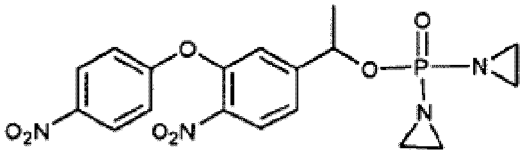,
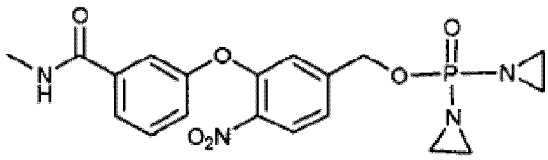,

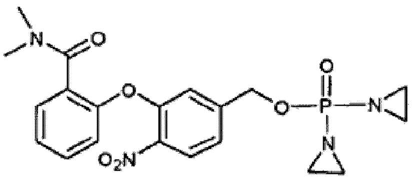,
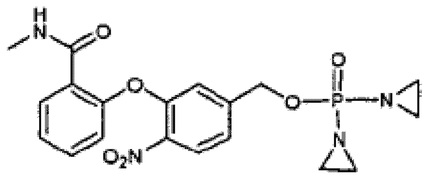,

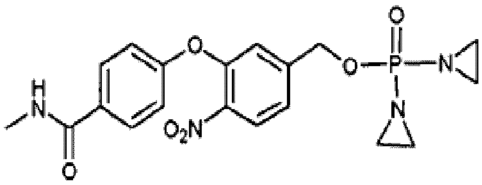,
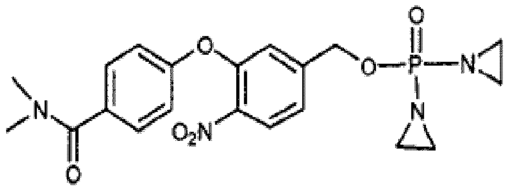,

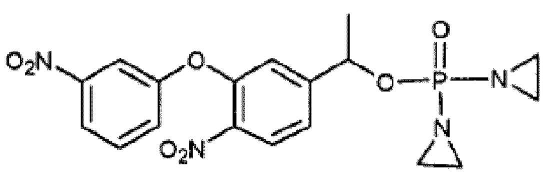,
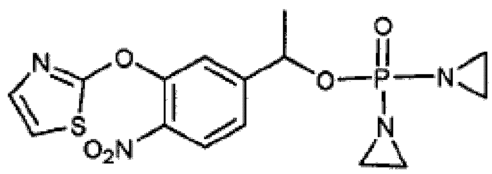,

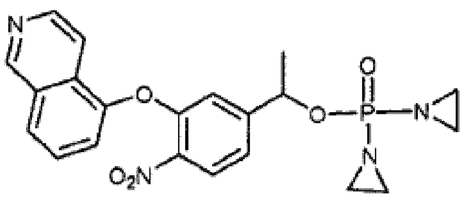,
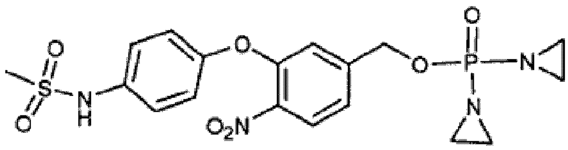,

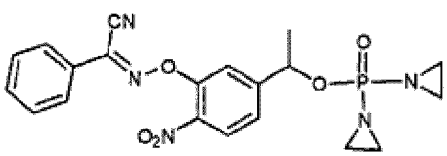,
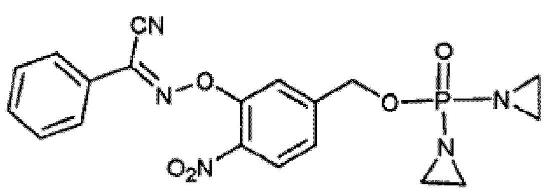,

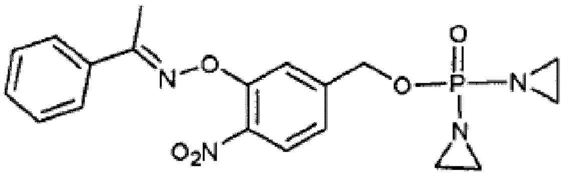
,
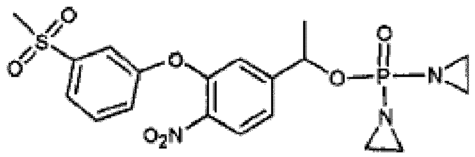
,
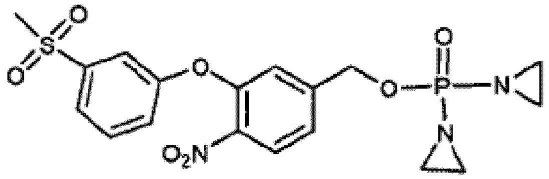
,
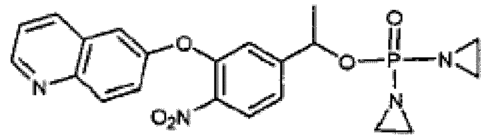
,
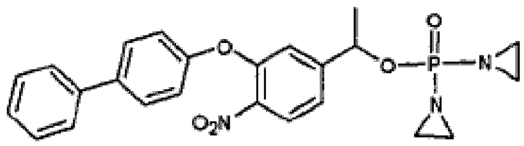
,
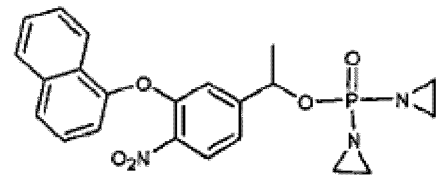
,
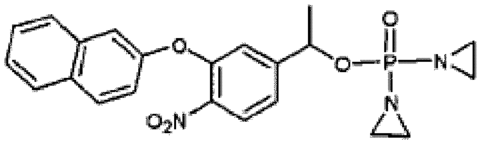
,
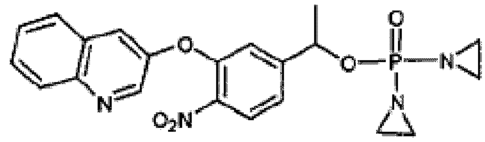
,
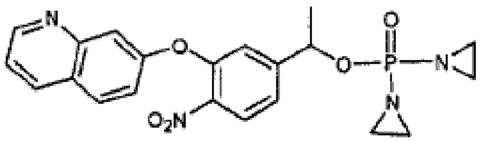
,
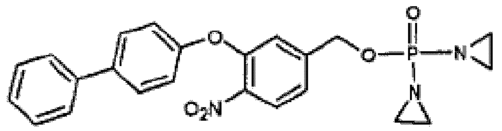
,
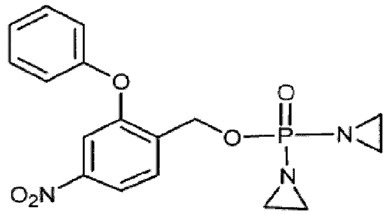
,
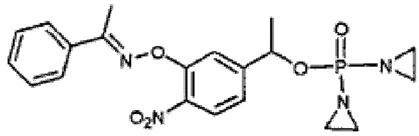
,
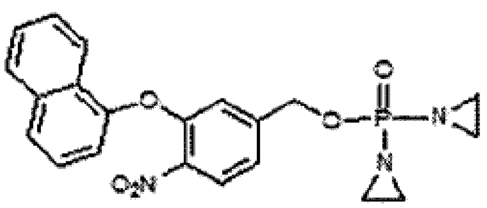
,
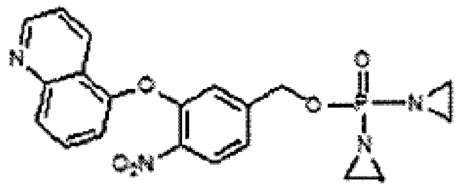
,

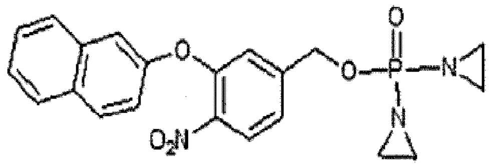,
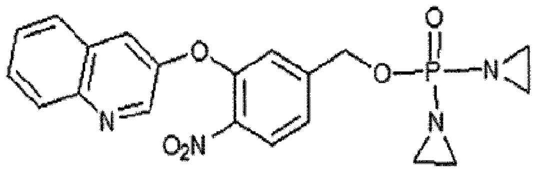,

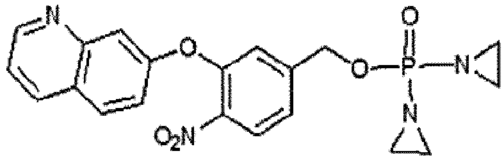,
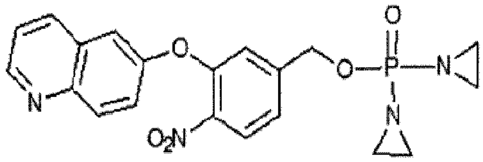,

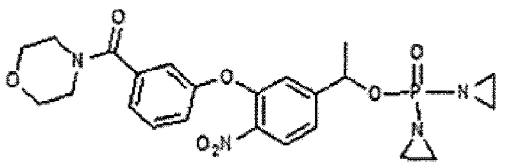,
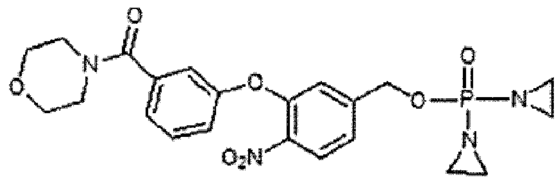,

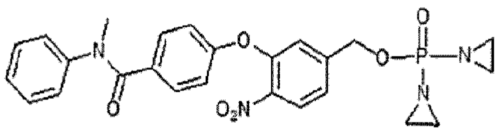,
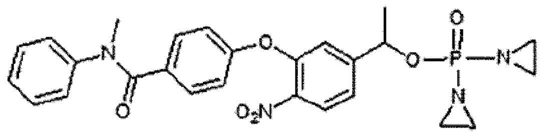,

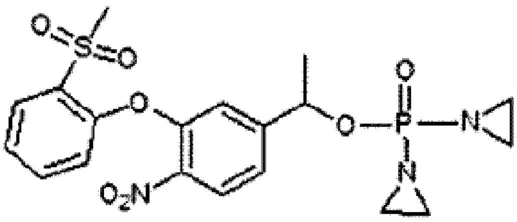,
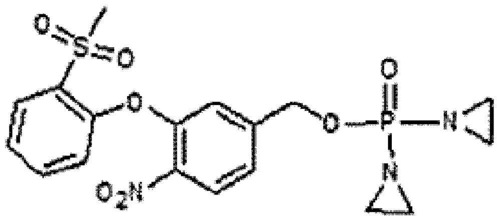,

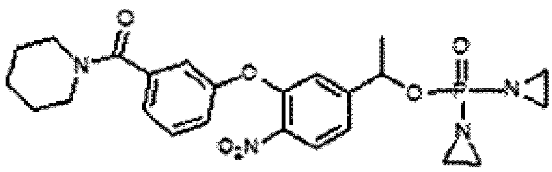,
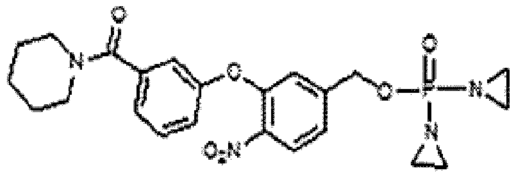,

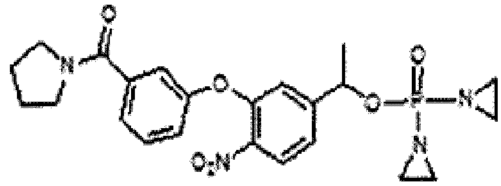,
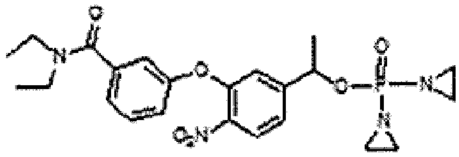,

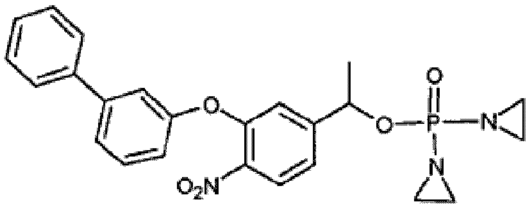
,
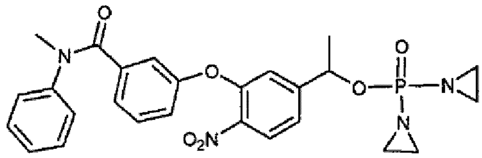
,
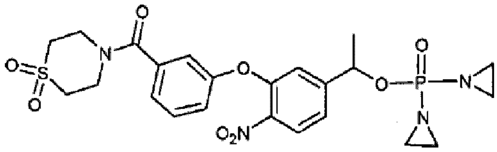
,
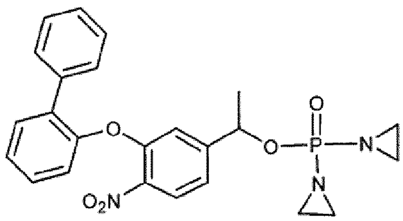
,
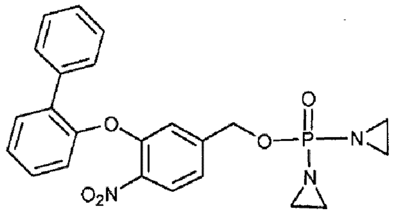
,
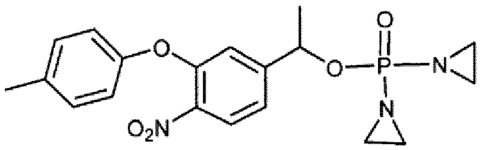
,
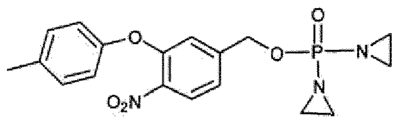
,
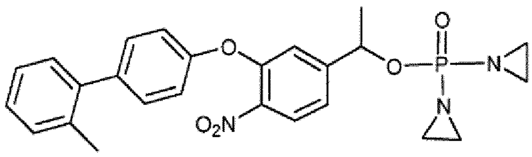
,
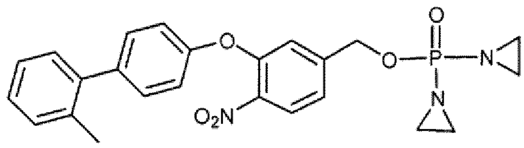
,
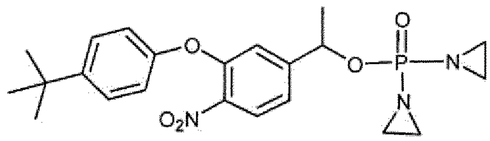
,
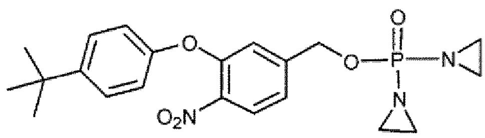
,
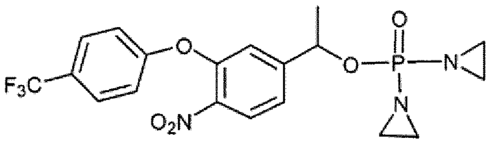
,

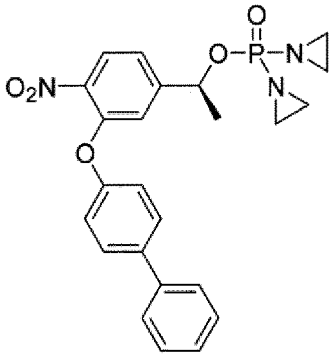,
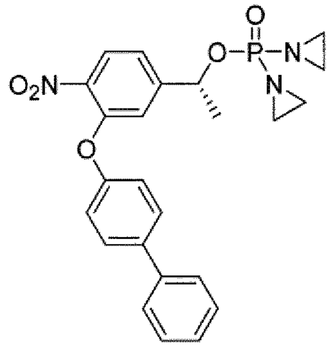,
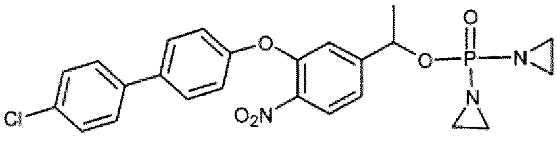,
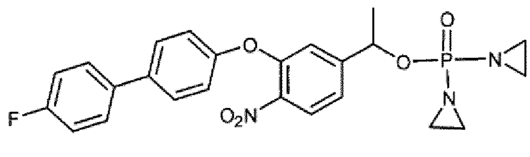,
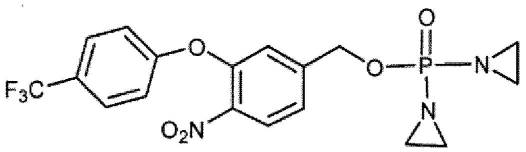 and 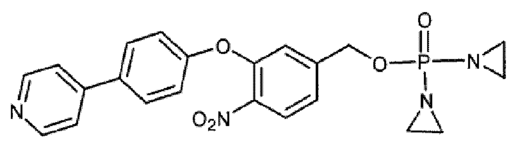.

**8.** Pharmaceutical use of an AKR1C3-activated DNA alkylating agent prodrug compound, wherein the compound is used in the manufacture of a drug monotherapy or in combination with other therapeutic drugs for treating cancer and tumor patients having KRAS mutations.

**9.** The pharmaceutical use according to claim 8, wherein the compound is selected from structural Formulae 1/2/3/4/5/6 and salts, esters, solvates, isotopic isomers thereof:

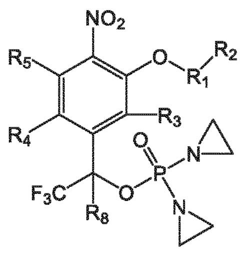 (1) 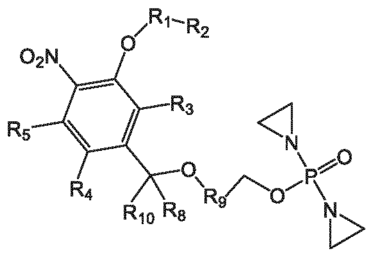 (2),

wherein the definitions of $R_1$, $R_2$, $R_3$, $R_4$, $R_5$, $R_8$, $R_9$, and $R_{10}$ are described in the claims of Patent Application PCT/CN2020/089692 with Publication No. WO2020228685A1;

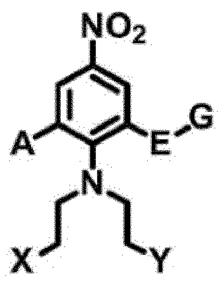 (3),

wherein the definitions of A, E, G, X, and Y are described in the claims of Patent Application PCT/NZ2019/050030 with Publication No. WO2019190331A1 (corresponding to Chinese Patent Application No. 2019800234236

with Publication No. CN111918864A);

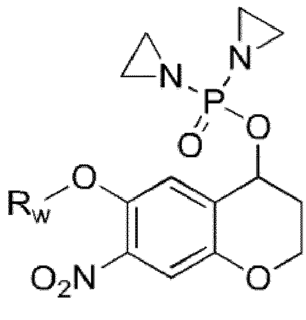

(4),

wherein the definition of $R_w$ is described in the claims of Patent Application PCT/CN2020/120281 with Publication No. WO2021068952A1;

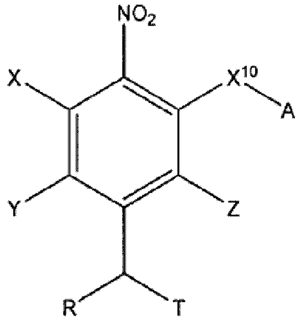

(5),

wherein the definitions of X, Y, Z, R, T, A, and $X^{10}$ are described in the claims of Patent Application PCT/US2016/062114 with Publication No. WO2017087428A1 (corresponding to Chinese Patent Application No. 2016800200132 with Publication No. CN108136214A);

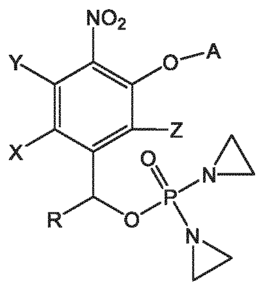

(6),

wherein:

A is a substituted or unsubstituted $C_6$-$C_{10}$ aryl, biaryl or substituted biaryl, 5-15 membered heteroaryl, or -N=$CR^1R^2$, wherein the substituents are selected from the group consisting of halogeno, -CN, -$NO_2$, -O-($CH_2$)-O-, -$CO_2H$ and salts thereof, -$OR^{100}$, -$CO_2R^{100}$, -$CONR^{101}R^{102}$, - $NR^{101}R^{102}$, -$NR^{100}SO_2R^{100}$, -$SO_2R^{100}$, -$SO_2NR^{101}R^{102}$, $C_1$-$C_6$ alkyl, and $C_3$-$C_{10}$ heterocyclyl;
wherein $R^{100}$, $R^{101}$ and $R^{102}$ are each independently hydrogen, $C_1$-$C_8$ alkyl, or $C_6$-$C_{12}$ aryl; or $R^{101}$ and $R^{102}$ together with the nitrogen atom to which they are attached form a 5-7 membered heterocycle;
wherein the alkyl group and the aryl group are each substituted by 1-3 halogeno groups or 1-3 $C_1$-$C_6$ alkyl groups;
$R^1$ and $R^2$ are each independently phenyl or methyl;
X, Y and Z are each independently hydrogen or halogeno; and
R is hydrogen or $C_1$-$C_6$ alkyl or halogen-substituted alkyl.

**10.** The pharmaceutical use according to claim 8, wherein the cancer is selected from the group consisting of ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

**11.** The pharmaceutical use according to claim 8 or 9, wherein the KRAS mutation is selected from the group consisting of KRAS-G12D mutation, KRAS-G12V mutation and KRAS-G12C mutation; preferably, the KRAS mutation is selected from the KRAS-G12D mutation.

**12.** The pharmaceutical use according to claim 11, wherein the TMB (Tumor Mutation Load (burden)) level of the mutation is medium.

**13.** The pharmaceutical use according to claim 8 or 9, wherein the other therapeutic drugs are selected from the group consisting of KRAS inhibitors and immunotherapy drugs, wherein the KRAS inhibitors are selected from the group consisting of sotorasib (AMG510), adagrasib (MRTX849), GDC6036, LY3499446, JNJ74699157 (ARS3248) and D-1553, and the immunotherapy drugs are selected from the group consisting of PD-1 monoclonal antibodies and PD-L1 monoclonal antibodies.

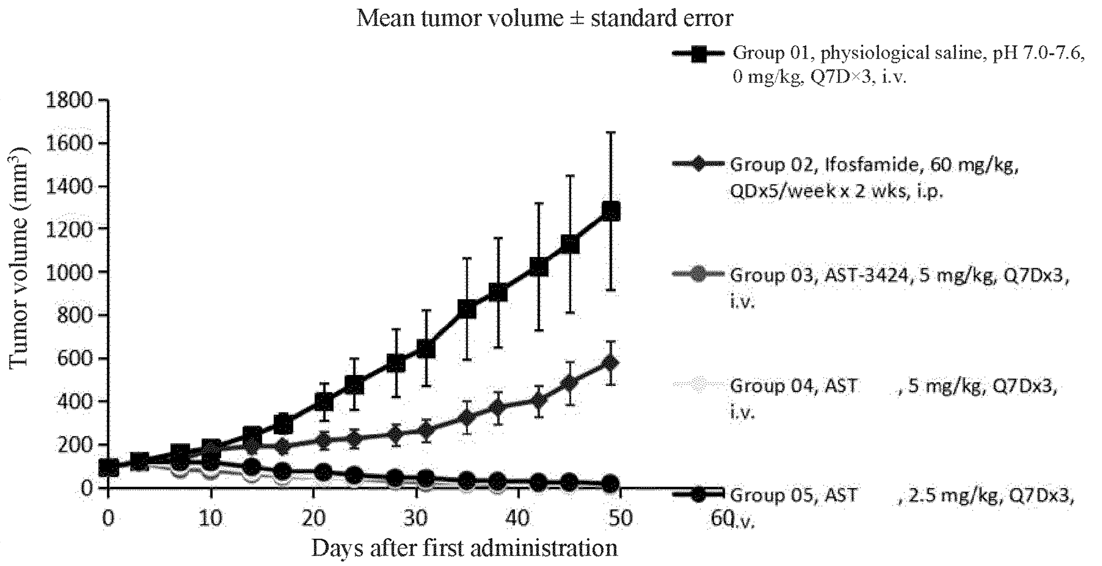


Fig. 1

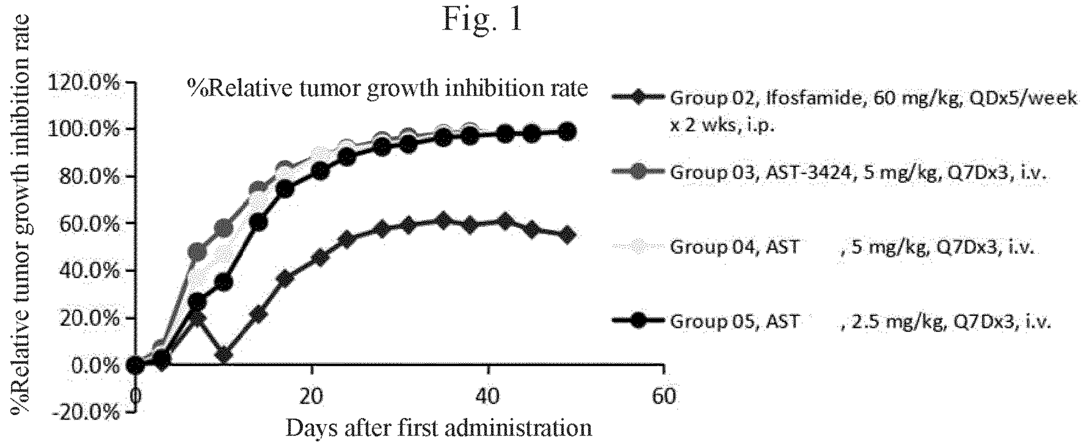


Fig. 2

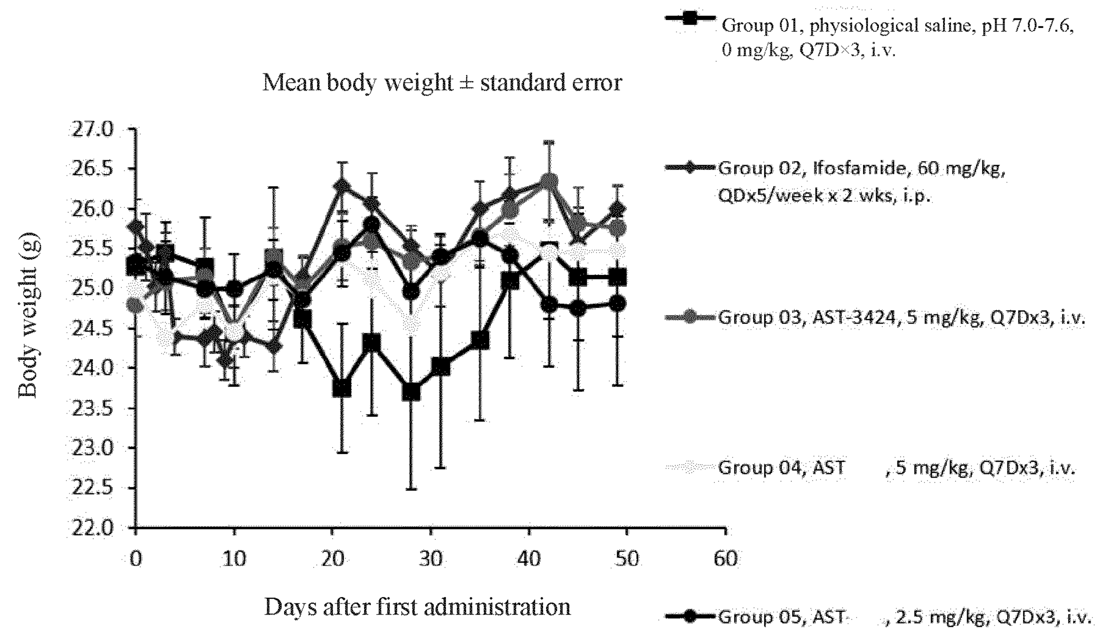


Fig. 3

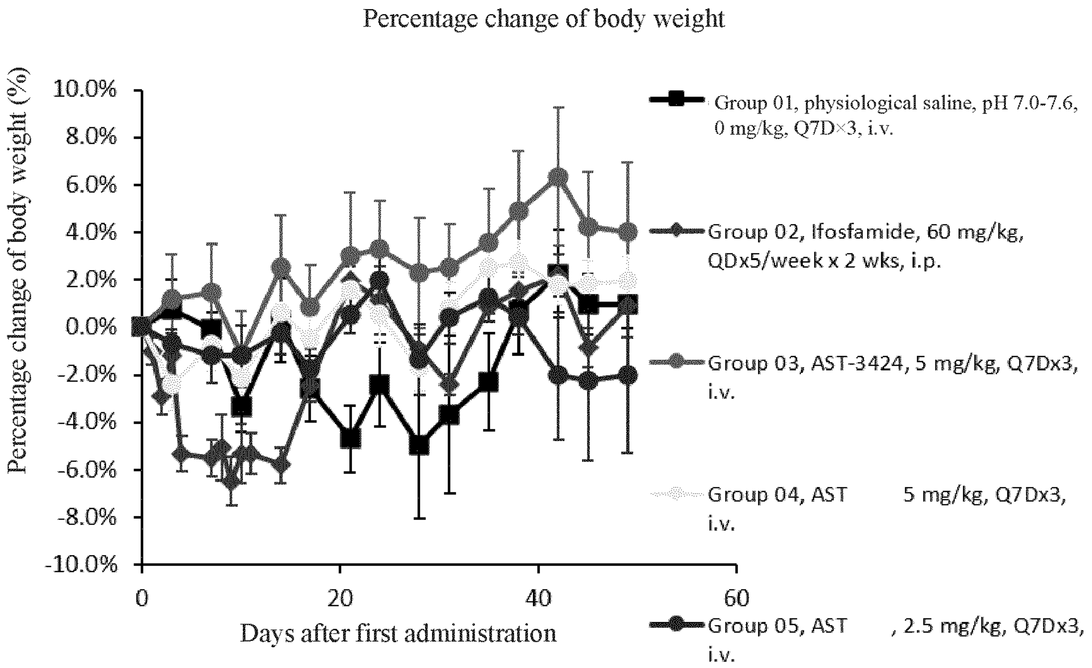


Fig. 4

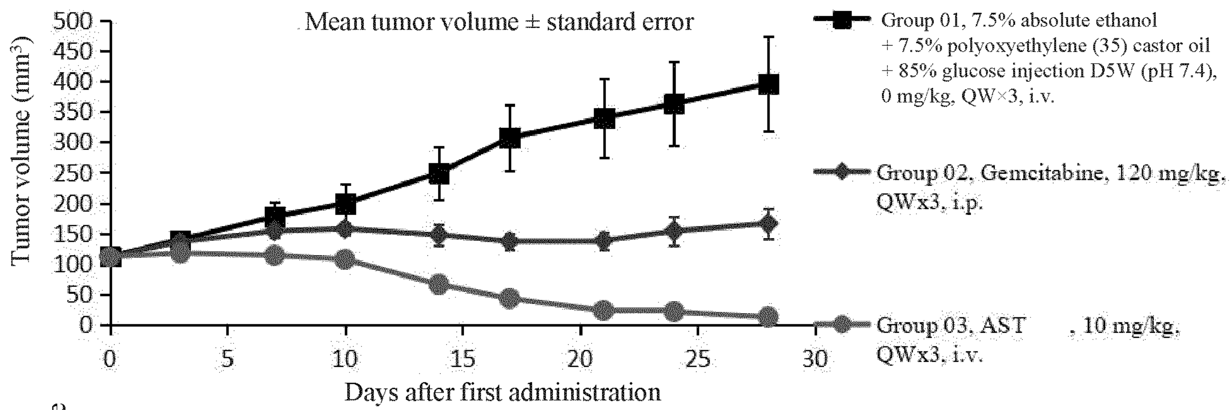


Fig. 5

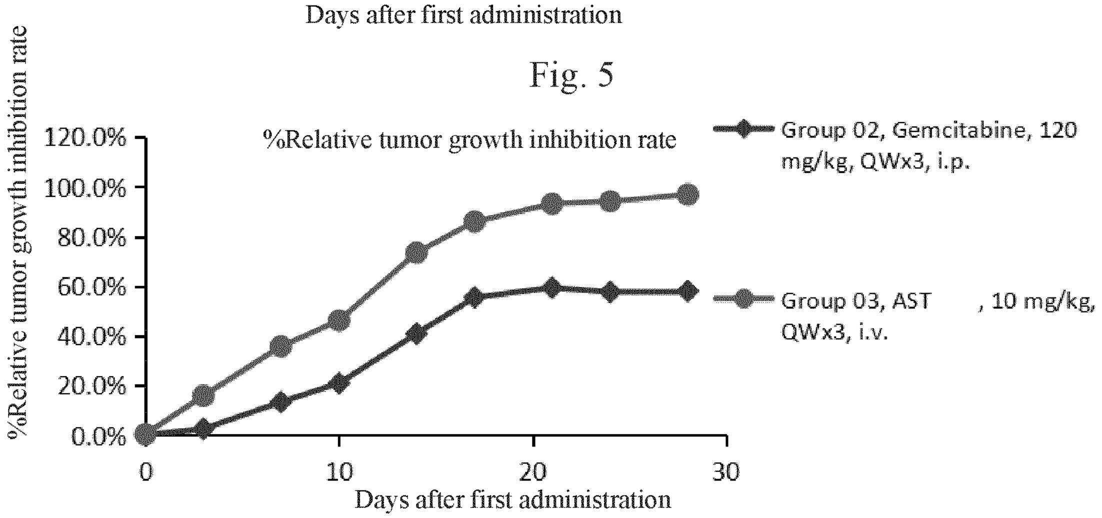


Fig. 6

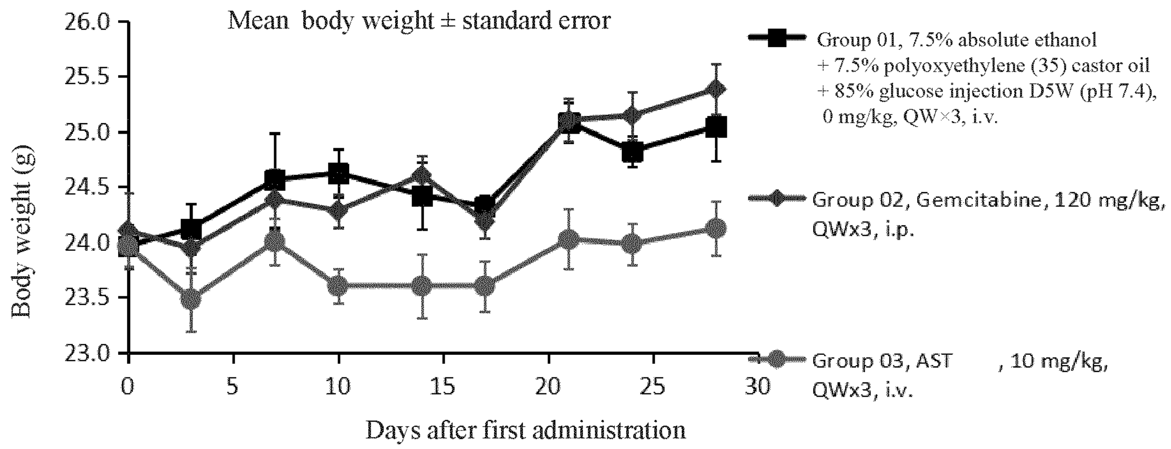


Fig. 7

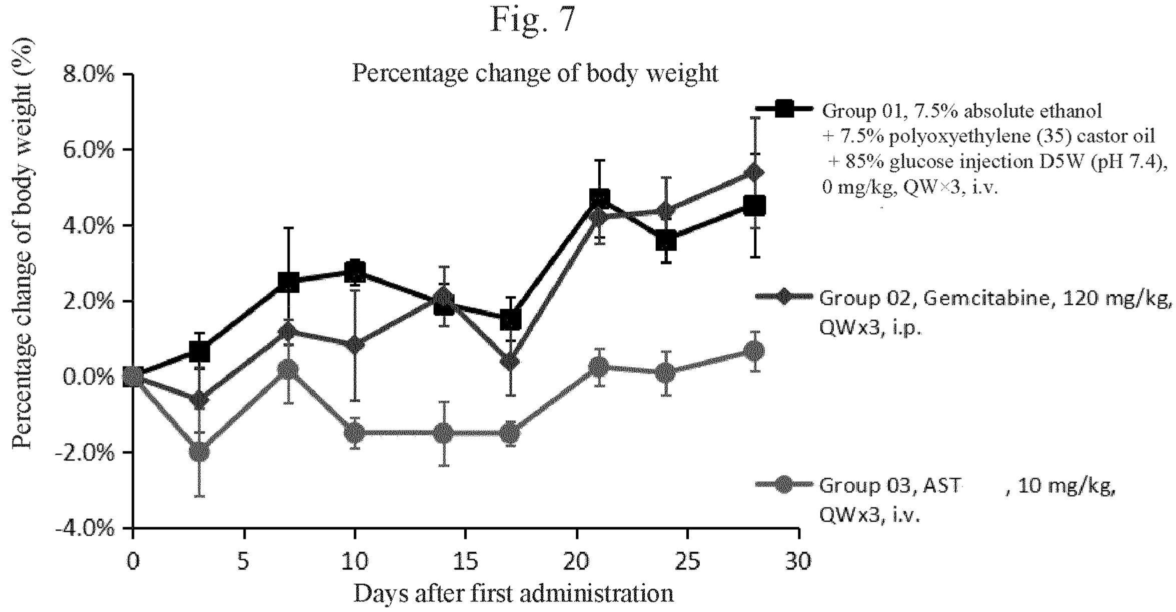


Fig. 8

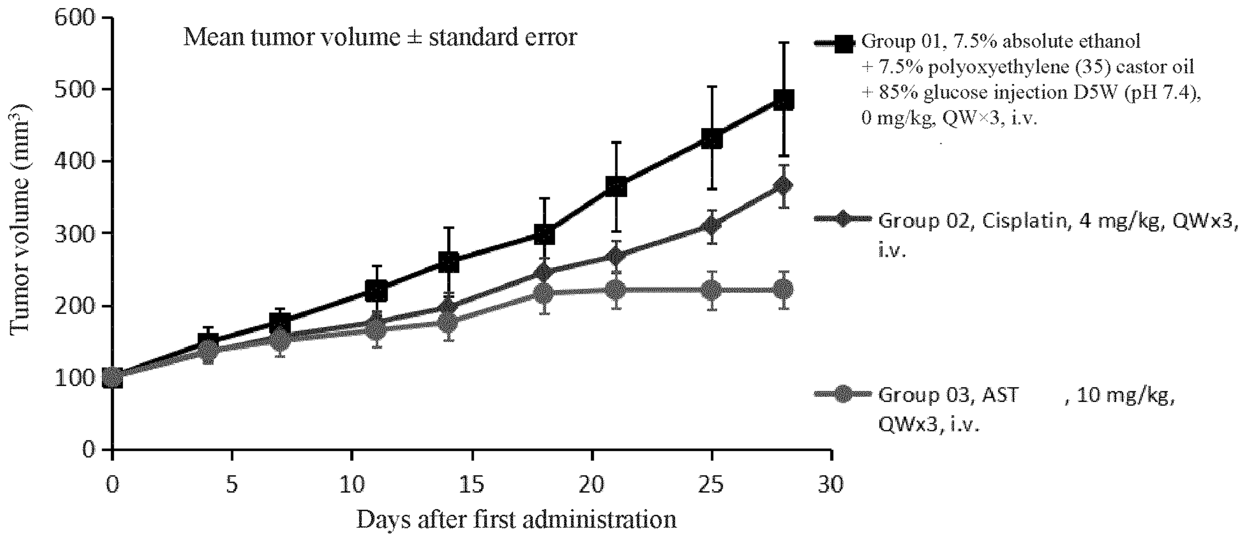


Fig. 9

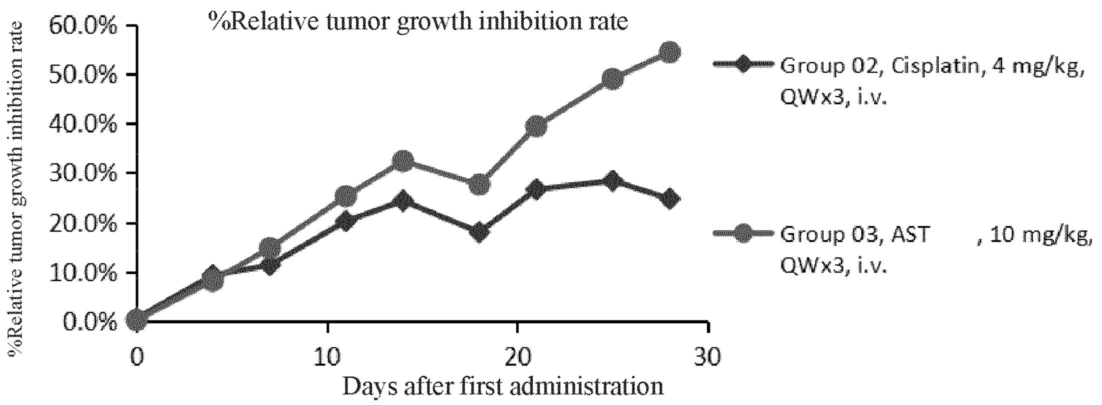


Fig. 10

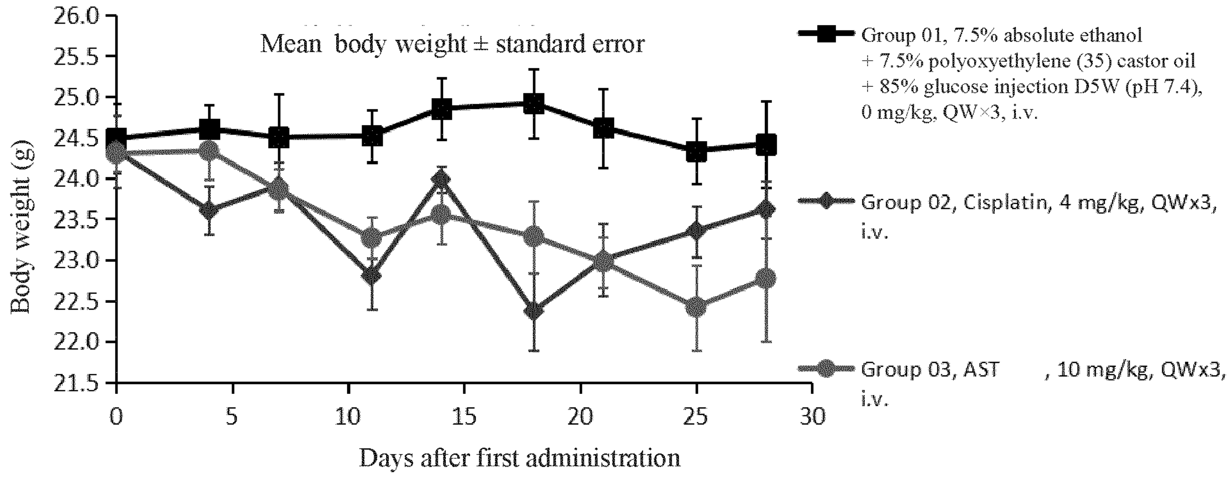


Fig. 11

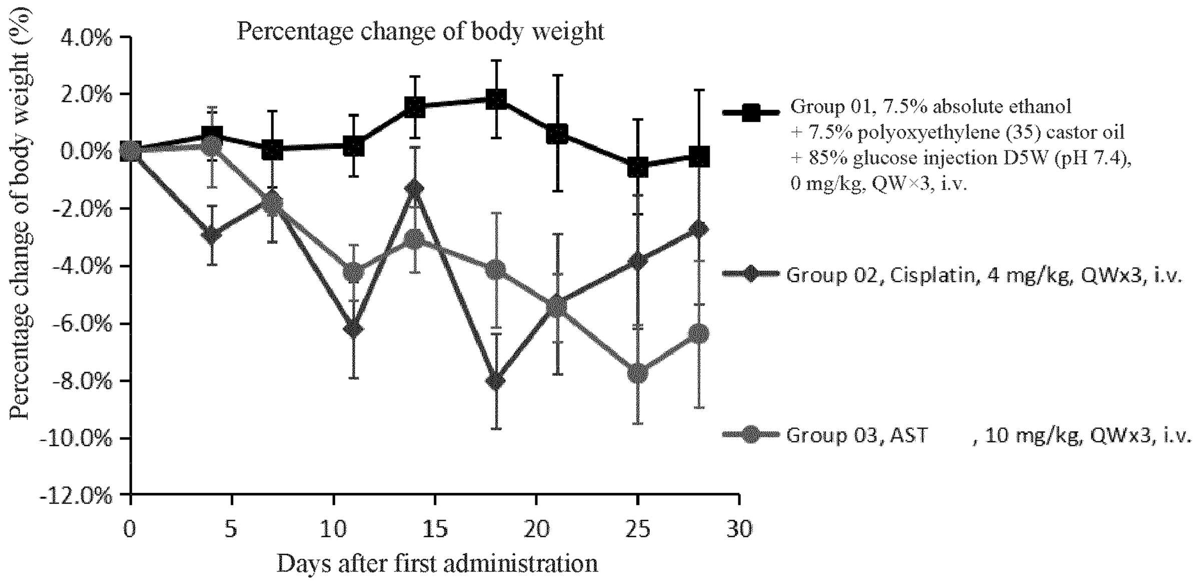


Fig. 12

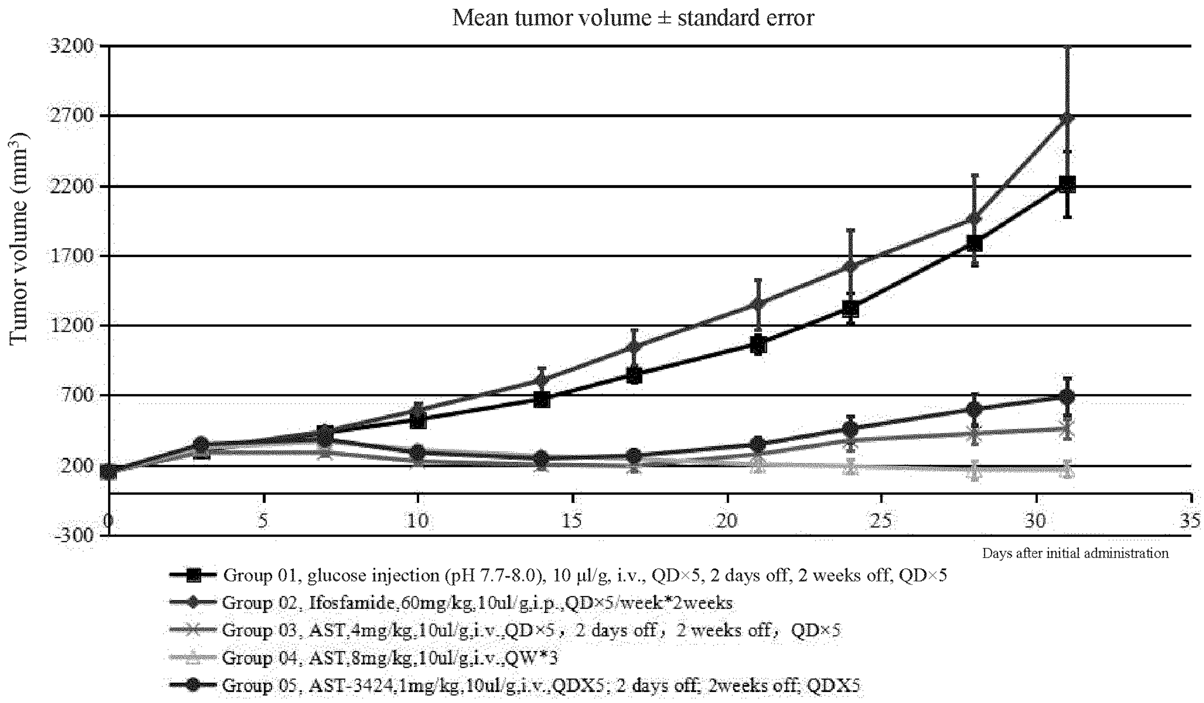
Mean tumor volume ± standard error
Tumor volume (mm3)
3200
2700
2200
1700
1200
700
200
-300
0
5
10
15
20
25
30
35
Days after initial administration
Group 01, glucose injection (pH 7.7-8.0), 10 µl/g, i.v., QD×5, 2 days off, 2 weeks off, QD×5
Group 02, Ifosfamide,60mg/kg,10ul/g,i.p.,QD×5/week*2weeks
Group 03, AST,4mg/kg,10ul/g,i.v.,QD×5, 2 days off, 2 weeks off, QD×5
Group 04, AST,8mg/kg,10ul/g,i.v.,QW*3
Group 05, AST-3424,1mg/kg,10ul/g,i.v.,QDX5; 2 days off; 2weeks off; QDX5

Fig. 13

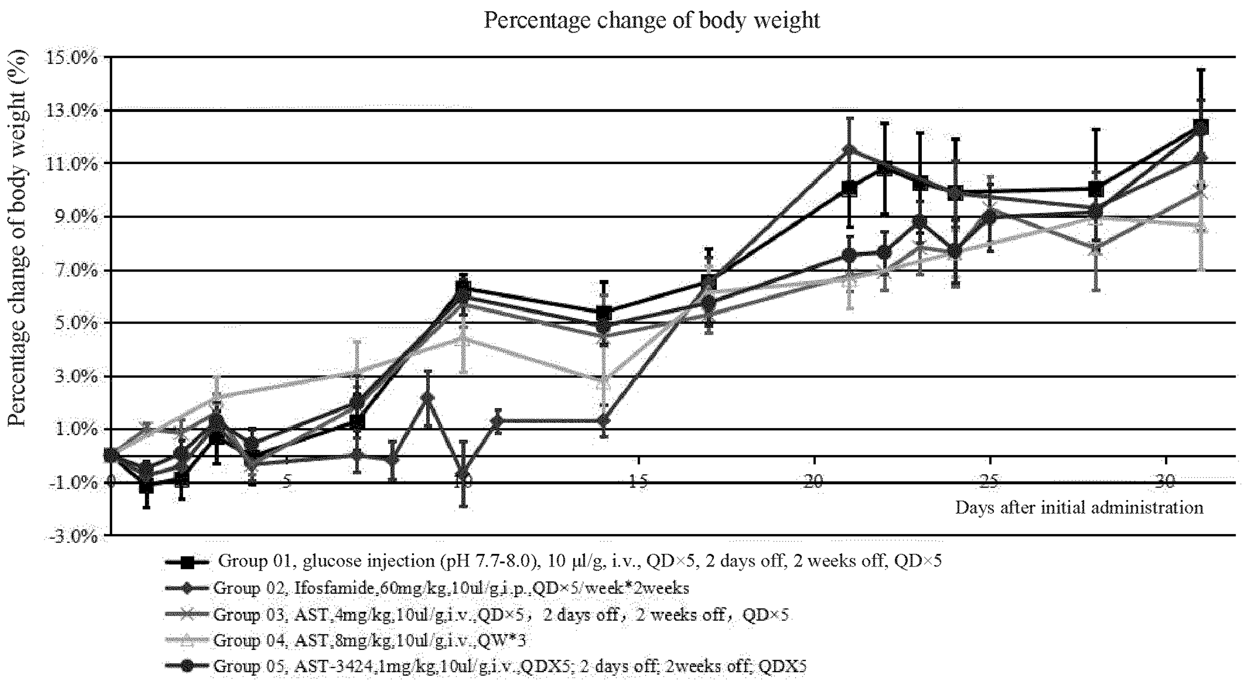
Percentage change of body weight
Percentage change of body weight (%)
15.0%
13.0%
11.0%
9.0%
7.0%
5.0%
3.0%
1.0%
-1.0%
-3.0%
0
5
10
15
20
25
30
Days after initial administration
Group 01, glucose injection (pH 7.7-8.0), 10 µl/g, i.v., QD×5, 2 days off, 2 weeks off, QD×5
Group 02, Ifosfamide,60mg/kg,10ul/g,i.p.,QD×5/week*2weeks
Group 03, AST,4mg/kg,10ul/g,i.v.,QD×5, 2 days off, 2 weeks off, QD×5
Group 04, AST,8mg/kg,10ul/g,i.v.,QW*3
Group 05, AST-3424,1mg/kg,10ul/g,i.v.,QDX5; 2 days off; 2weeks off; QDX5

Fig. 14

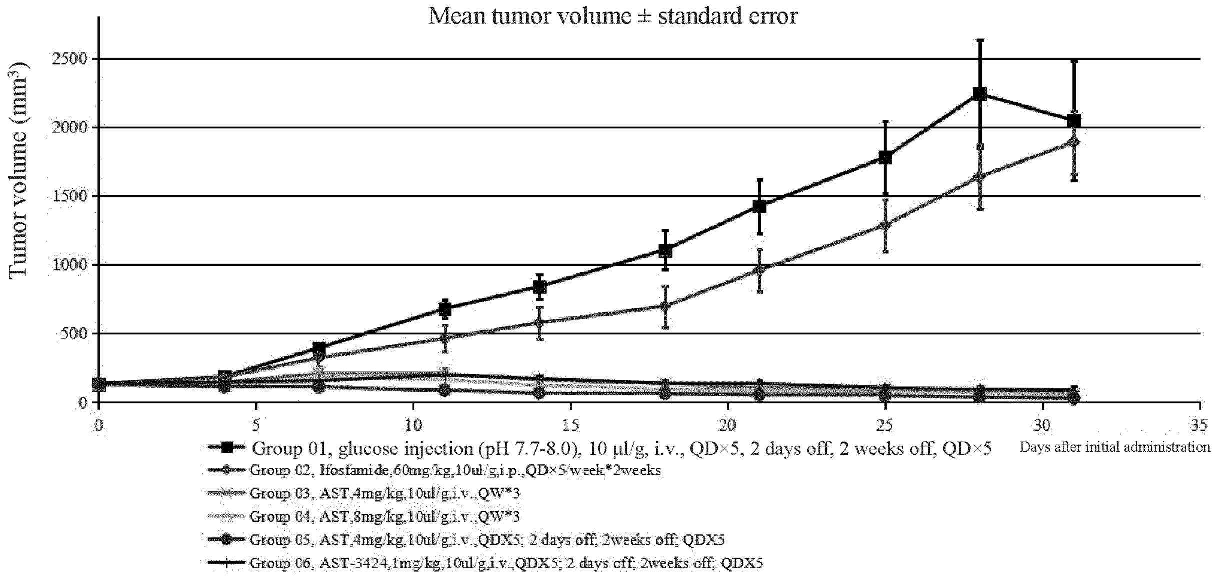
Mean tumor volume ± standard error
Tumor volume (mm3)
2500
2000
1500
1000
500
0
0
5
10
15
20
25
30
35
Days after initial administration
Group 01, glucose injection (pH 7.7-8.0), 10 μl/g, i.v., QD×5, 2 days off, 2 weeks off, QD×5
Group 02, Ifosfamide,60mg/kg,10ul/g,i.p.,QD×5/week*2weeks
Group 03, AST,4mg/kg,10ul/g,i.v.,QW*3
Group 04, AST,8mg/kg,10ul/g,i.v.,QW*3
Group 05, AST,4mg/kg,10ul/g,i.v.,QDX5; 2 days off; 2weeks off; QDX5
Group 06, AST-3424,1mg/kg,10ul/g,i.v.,QDX5; 2 days off; 2weeks off; QDX5

Fig. 15

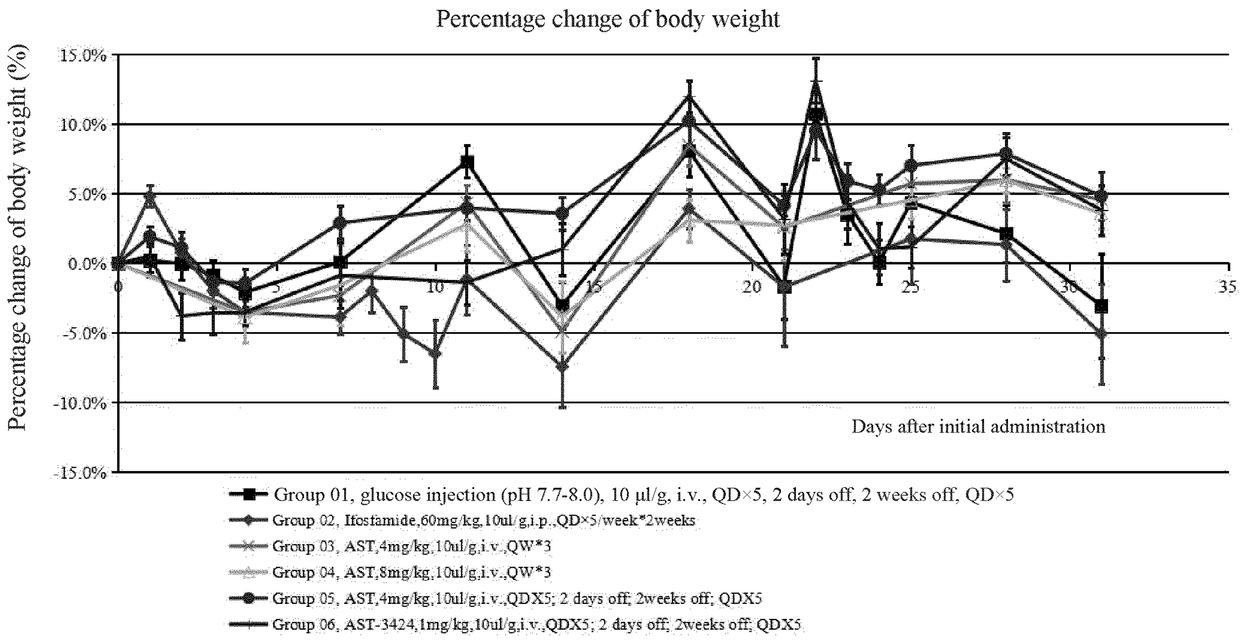
Percentage change of body weight
Percentage change of body weight (%)
15.0%
10.0%
5.0%
0.0%
-5.0%
-10.0%
-15.0%
0
5
10
15
20
25
30
35
Days after initial administration
Group 01, glucose injection (pH 7.7-8.0), 10 μl/g, i.v., QD×5, 2 days off, 2 weeks off, QD×5
Group 02, Ifosfamide,60mg/kg,10ul/g,i.p.,QD×5/week*2weeks
Group 03, AST,4mg/kg,10ul/g,i.v.,QW*3
Group 04, AST,8mg/kg,10ul/g,i.v.,QW*3
Group 05, AST,4mg/kg,10ul/g,i.v.,QDX5; 2 days off; 2weeks off; QDX5
Group 06, AST-3424,1mg/kg,10ul/g,i.v.,QDX5; 2 days off; 2weeks off; QDX5

Fig. 16

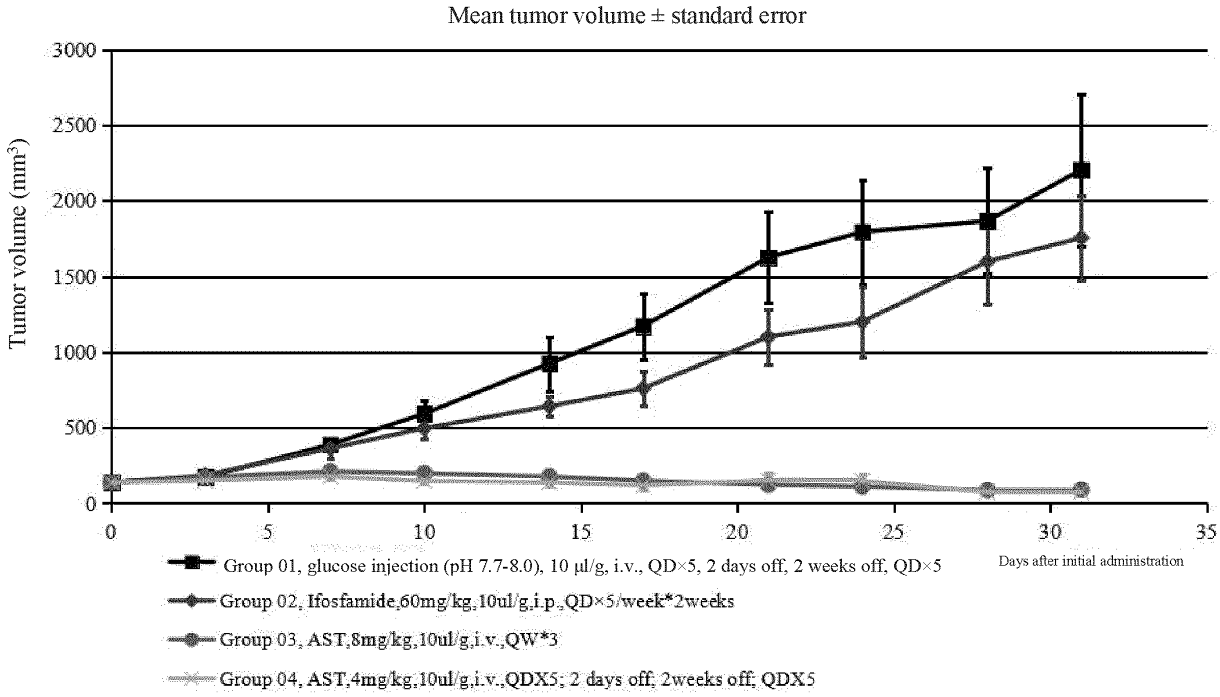

Mean tumor volume ± standard error
Tumor volume (mm³)
3000
2500
2000
1500
1000
500
0
0
5
10
15
20
25
30
35
Days after initial administration
Group 01, glucose injection (pH 7.7-8.0), 10 µl/g, i.v., QD×5, 2 days off, 2 weeks off, QD×5
Group 02, Ifosfamide,60mg/kg,10ul/g,i.p.,QD×5/week*2weeks
Group 03, AST,8mg/kg,10ul/g,i.v.,QW*3
Group 04, AST,4mg/kg,10ul/g,i.v.,QDX5; 2 days off; 2weeks off; QDX5


Fig. 17

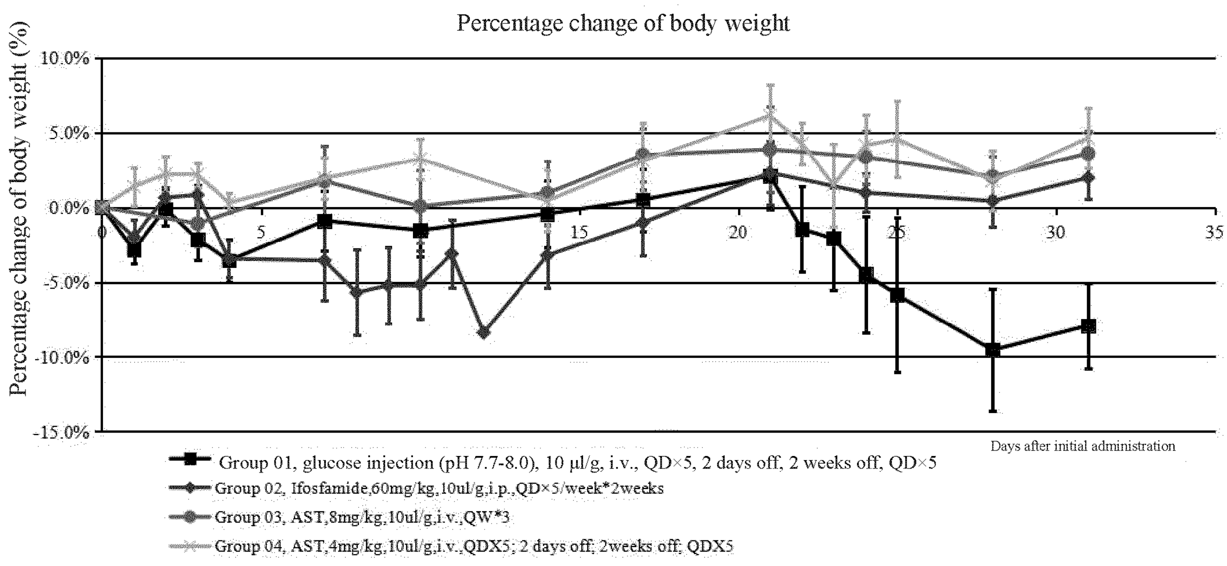

Percentage change of body weight
Percentage change of body weight (%)
10.0%
5.0%
0.0%
-5.0%
-10.0%
-15.0%
0
5
15
20
25
30
35
Days after initial administration
Group 01, glucose injection (pH 7.7-8.0), 10 µl/g, i.v., QD×5, 2 days off, 2 weeks off, QD×5
Group 02, Ifosfamide,60mg/kg,10ul/g,i.p.,QD×5/week*2weeks
Group 03, AST,8mg/kg,10ul/g,i.v.,QW*3
Group 04, AST,4mg/kg,10ul/g,i.v.,QDX5; 2 days off; 2weeks off; QDX5


Fig. 18

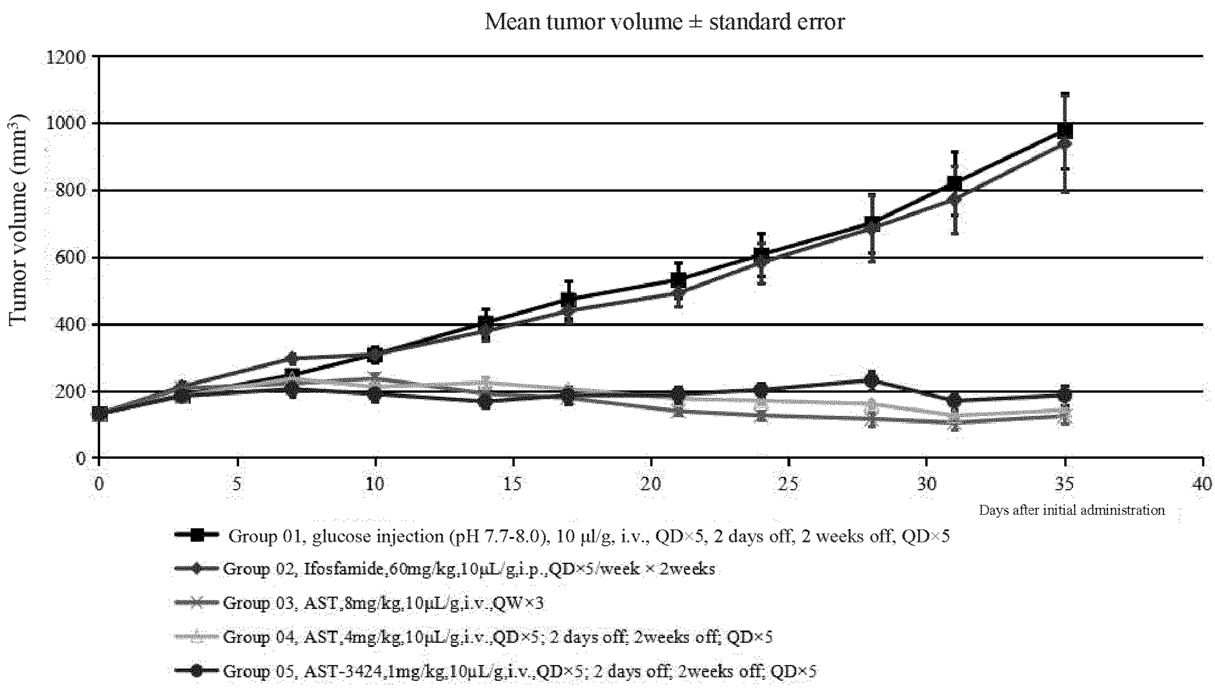

Mean tumor volume ± standard error
Tumor volume (mm³)
1200
1000
800
600
400
200
0
0
5
10
15
20
25
30
35
40
Days after initial administration
Group 01, glucose injection (pH 7.7-8.0), 10 µl/g, i.v., QD×5, 2 days off, 2 weeks off, QD×5
Group 02, Ifosfamide,60mg/kg,10µL/g,i.p.,QD×5/week × 2weeks
Group 03, AST,8mg/kg,10µL/g,i.v.,QW×3
Group 04, AST,4mg/kg,10µL/g,i.v.,QD×5; 2 days off; 2weeks off; QD×5
Group 05, AST-3424,1mg/kg,10µL/g,i.v.,QD×5; 2 days off; 2weeks off; QD×5


Fig. 19

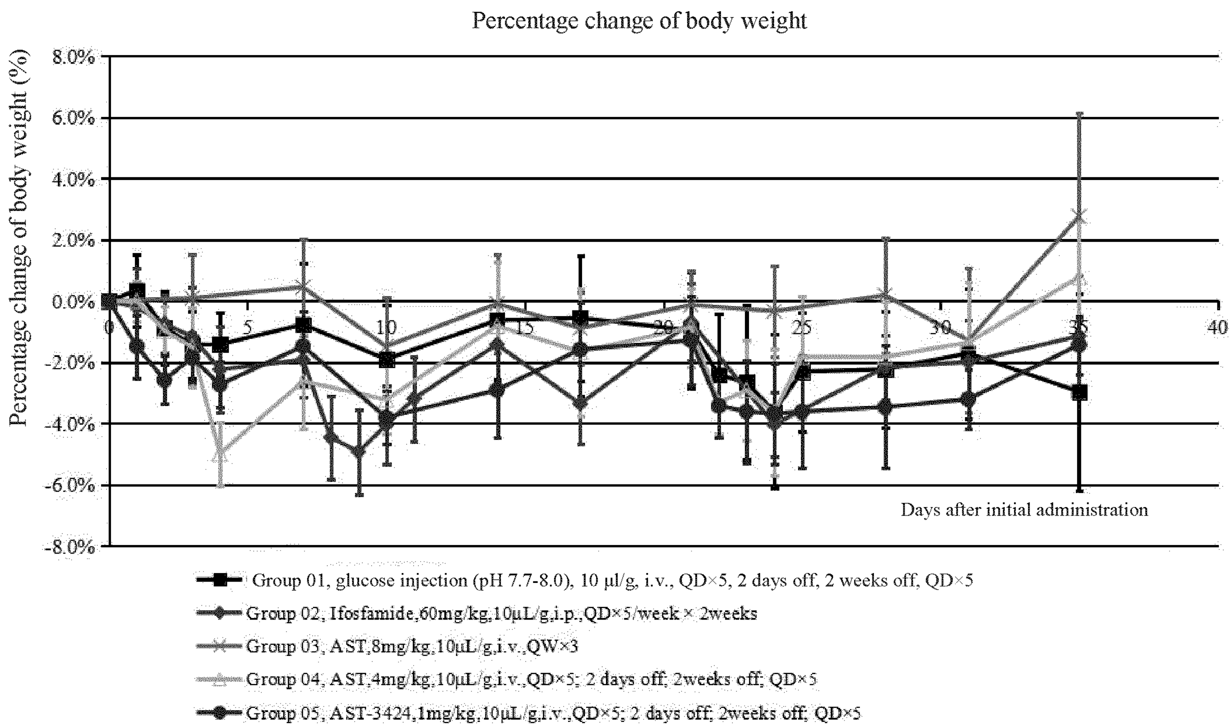

Percentage change of body weight
Percentage change of body weight (%)
8.0%
6.0%
4.0%
2.0%
0.0%
-2.0%
-4.0%
-6.0%
-8.0%
0
5
10
15
20
25
30
35
40
Days after initial administration
Group 01, glucose injection (pH 7.7-8.0), 10 µl/g, i.v., QD×5, 2 days off, 2 weeks off, QD×5
Group 02, Ifosfamide,60mg/kg,10µL/g,i.p.,QD×5/week × 2weeks
Group 03, AST,8mg/kg,10µL/g,i.v.,QW×3
Group 04, AST,4mg/kg,10µL/g,i.v.,QD×5; 2 days off; 2weeks off; QD×5
Group 05, AST-3424,1mg/kg,10µL/g,i.v.,QD×5; 2 days off; 2weeks off; QD×5


Fig. 20

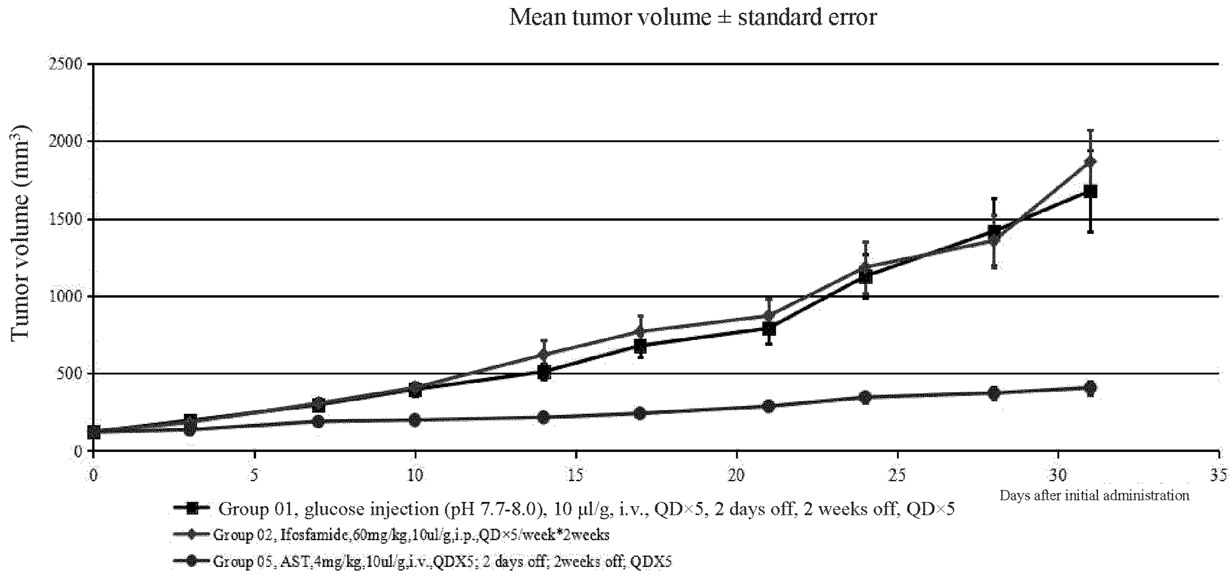
Mean tumor volume ± standard error
Tumor volume (mm³)
2500
2000
1500
1000
500
0
0
5
10
15
20
25
30
35
Days after initial administration
Group 01, glucose injection (pH 7.7-8.0), 10 μl/g, i.v., QD×5, 2 days off, 2 weeks off, QD×5
Group 02, Ifosfamide,60mg/kg,10ul/g,i.p.,QD×5/week*2weeks
Group 05, AST,4mg/kg,10ul/g,i.v.,QDX5; 2 days off; 2weeks off; QDX5

Fig. 21

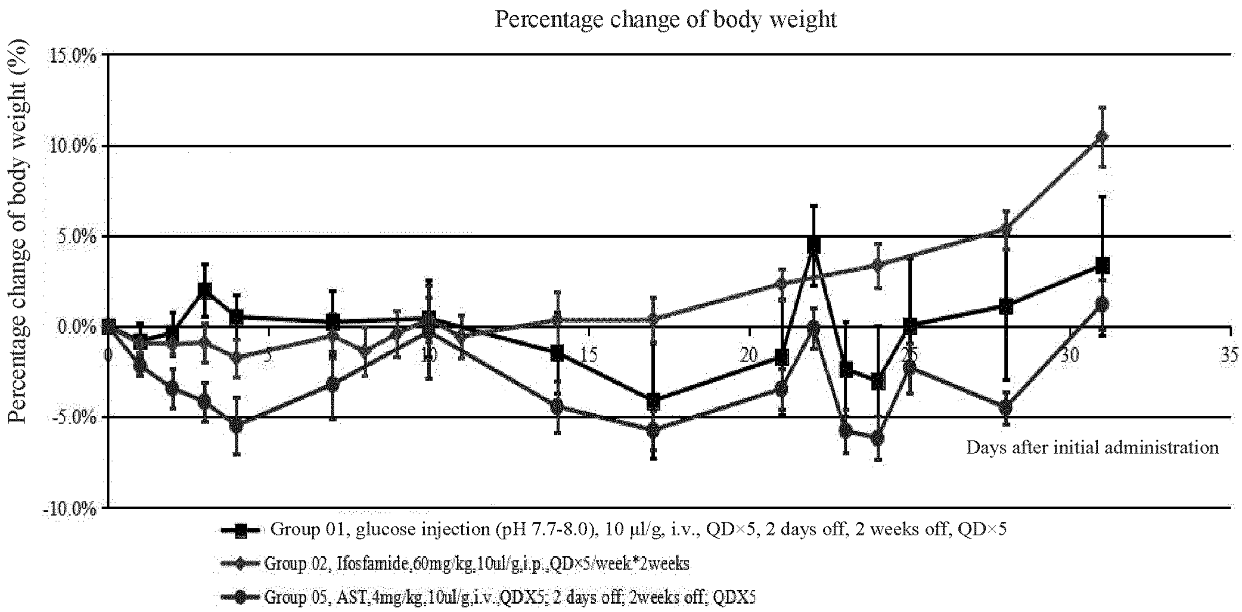
Percentage change of body weight
Percentage change of body weight (%)
15.0%
10.0%
5.0%
0.0%
-5.0%
-10.0%
0
5
10
15
20
25
30
35
Days after initial administration
Group 01, glucose injection (pH 7.7-8.0), 10 μl/g, i.v., QD×5, 2 days off, 2 weeks off, QD×5
Group 02, Ifosfamide,60mg/kg,10ul/g,i.p.,QD×5/week*2weeks
Group 05, AST,4mg/kg,10ul/g,i.v.,QDX5; 2 days off; 2weeks off; QDX5

Fig. 22

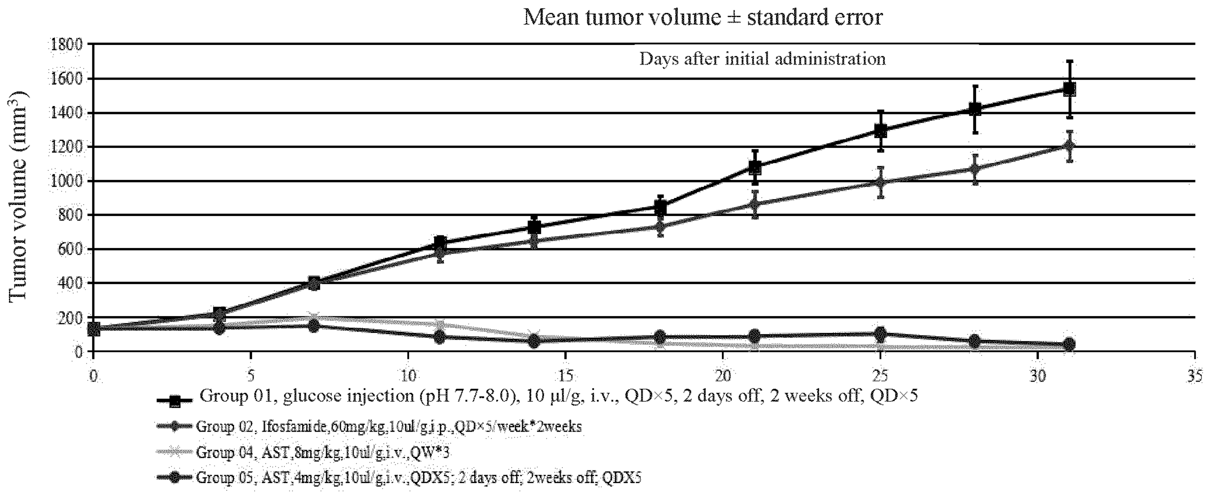
Mean tumor volume ± standard error
Days after initial administration
Tumor volume (mm3)
1800
1600
1400
1200
1000
800
600
400
200
0
0
5
10
15
20
25
30
35
Group 01, glucose injection (pH 7.7-8.0), 10 µl/g, i.v., QD×5, 2 days off, 2 weeks off, QD×5
Group 02, Ifosfamide,60mg/kg,10ul/g,i.p.,QD×5/week*2weeks
Group 04, AST,8mg/kg,10ul/g,i.v.,QW*3
Group 05, AST,4mg/kg,10ul/g,i.v.,QDX5; 2 days off; 2weeks off; QDX5

Fig. 23

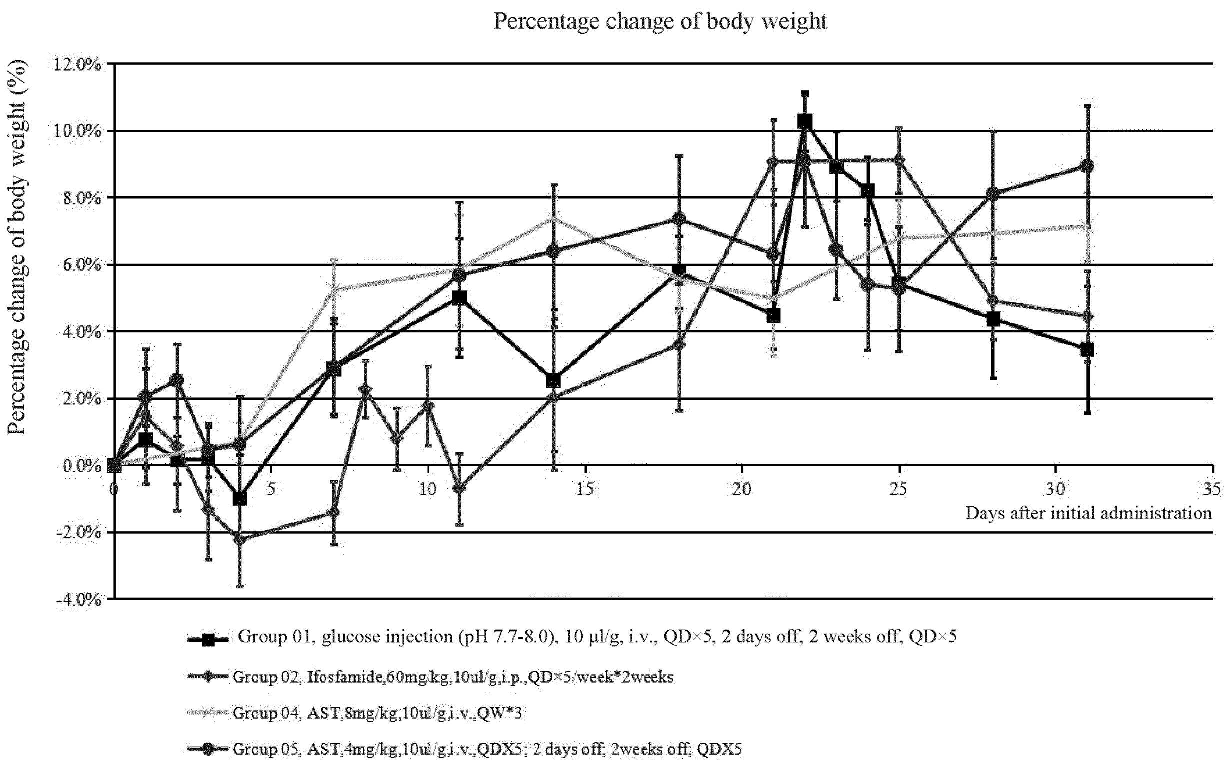
Percentage change of body weight
Percentage change of body weight (%)
12.0%
10.0%
8.0%
6.0%
4.0%
2.0%
0.0%
-2.0%
-4.0%
0
5
10
15
20
25
30
35
Days after initial administration
Group 01, glucose injection (pH 7.7-8.0), 10 µl/g, i.v., QD×5, 2 days off, 2 weeks off, QD×5
Group 02, Ifosfamide,60mg/kg,10ul/g,i.p.,QD×5/week*2weeks
Group 04, AST,8mg/kg,10ul/g,i.v.,QW*3
Group 05, AST,4mg/kg,10ul/g,i.v.,QDX5; 2 days off; 2weeks off; QDX5

Fig. 24

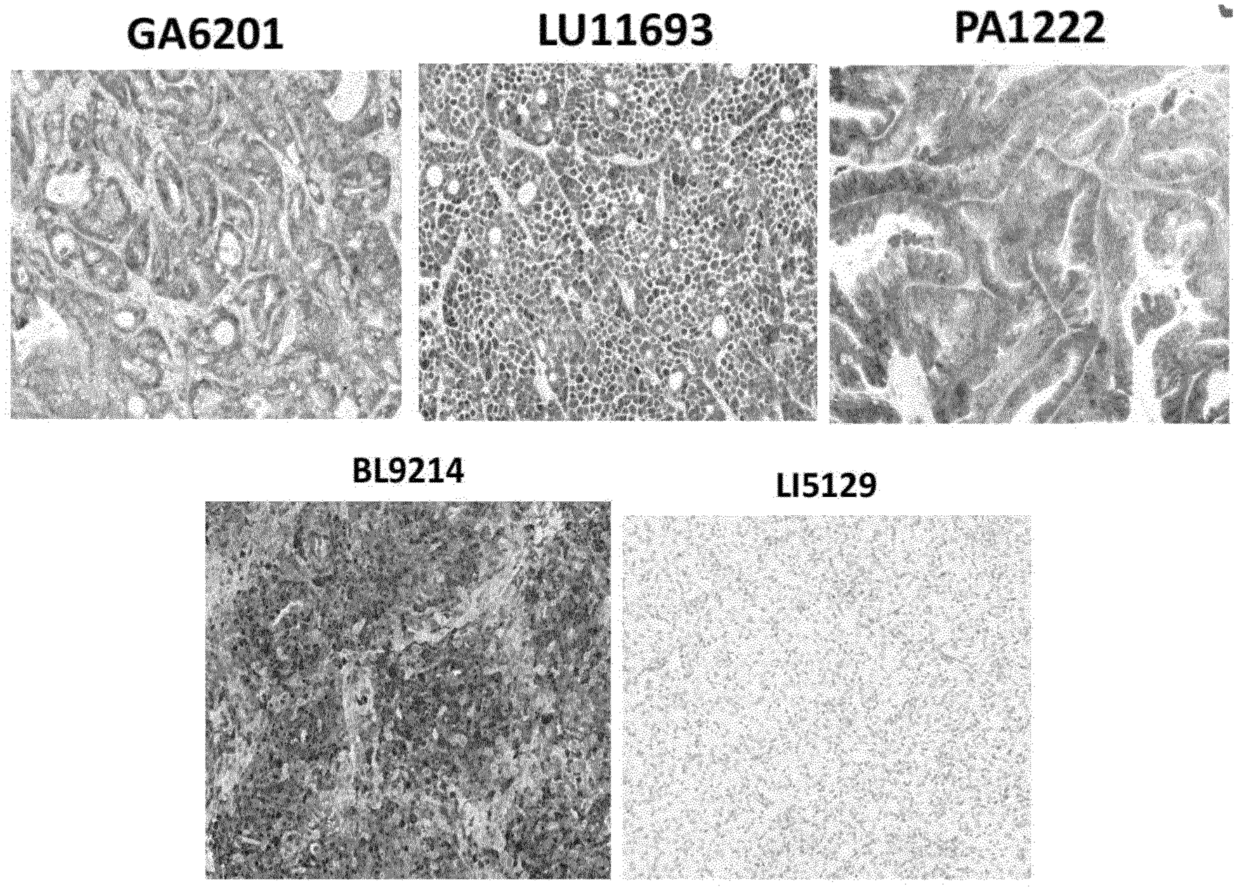


Fig. 25

## INTERNATIONAL SEARCH REPORT

International application No.
**PCT/CN2022/120817**

**A. CLASSIFICATION OF SUBJECT MATTER**

A61K 31/495(2006.01)i； A61K 31/496(2006.01)i； A61K 31/472(2006.01)i； A61K 31/551(2006.01)i； A61K 31/395(2006.01)i； A61K 31/4375(2006.01)i； A61K 31/445(2006.01)i； A61K 31/5375(2006.01)i； A61K 45/06(2006.01)i； A61P 35/00(2006.01)i； A61P 35/02(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, VEN, CNTXT, USTXT, EPTXT, JPTXT, KRTXT, CNKI, 读秀, DUXIU, ISI_Web of Science, Science direct, STN: 深圳艾欣达伟医药科技有限公司, 段建新, AKR1C3, DNA, 烷化剂, KRAS, 癌症, 肿瘤, 结构检索, structure search, alkylating, cancer

**C. DOCUMENTS CONSIDERED TO BE RELEVANT**

| Category* | Citation of document, with indication, where appropriate, of the relevant passages | Relevant to claim No. |
|---|---|---|
| Y | HIRAOKA, K. et al.,. "Inhibition of KRAS codon 12 mutants using a novel DNA-alkylating pyrrole-imidazole polyamide conjugate," *Nature Communications,* 27 April 2015 (2015-04-27), pp. 1-8 | 1-13 |
| Y | WO 2020228685 A1 (ASCENTAWITS PHARMACEUTICAL, LTD.) 19 November 2020 (2020-11-19) description, page 2, line 14 to page 3, line 20, and page 9, the second-to-last line to page 15, line 3, and claims 1-21 | 1-13 |
| Y | WO 2019190331 A1 (ACHILLES MEDICAL LIMITED) 03 October 2019 (2019-10-03) claims 1-17 | 1-13 |
| Y | WO 2021068952 A1 (MEDSHINE DISCOVERY INC.) 15 April 2021 (2021-04-15) see description, page 1, 4th-to-last paragraph, and page 2, line 1 to page 3, line 1, and claims 1-26 | 1-13 |
| Y | CN 108136214 A (THRESHOLD PHARMACEUTICALS, INC.) 08 June 2018 (2018-06-08) description, paragraphs 0008-0032 and 0217-0218, and claims 1-21 | 1-13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 December 2022** | **19 December 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/120817**

**C. DOCUMENTS CONSIDERED TO BE RELEVANT**

| Category* | Citation of document, with indication, where appropriate, of the relevant passages | Relevant to claim No. |
|---|---|---|
| Y | CN 106068266 A (CHIBA PREFECTURE) 02 November 2016 (2016-11-02) | 1-13 |
| Y | WO 2017087428 A1 (THRESHOLD PHARMACEUTICALS, INC.) 26 May 2017 (2017-05-26) | 1-13 |

**INTERNATIONAL SEARCH REPORT**

International application No.
**PCT/CN2022/120817**

**Box No. II** **Observations where certain claims were found unsearchable (Continuation of item 2 of first sheet)**

This international search report has not been established in respect of certain claims under Article 17(2)(a) for the following reasons:

1. ☑ Claims Nos.: **1-7**
because they relate to subject matter not required to be searched by this Authority, namely:

[1] Claims 1-7 set forth a treatment method, and said claims relate to a treatment method in which a human or animal body is a direct object. Therefore, claims 1-7 do not comply with PCT Rule 39.1(iv). The search for claims 1-7 is based on a pharmaceutical use of a AKR1C3-activated NDA alkylating agent precursor compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/120817**

| Patent document cited in search report | Publication date (day/month/year) | Patent family member(s) | Publication date (day/month/year) |
|---|---|---|---|
| WO 2020228685 A1 | 19 November 2020 | US 2022119429 A1 | 21 April 2022 |
| | | JP 2022533346 A | 22 July 2022 |
| | | ZA 202107920 B | 27 July 2022 |
| | | AU 2020275818 A1 | 25 November 2021 |
| | | CA 3140070 A1 | 19 November 2020 |
| | | EP 3971194 A1 | 23 March 2022 |
| | | KR 20220012274 A | 03 February 2022 |
| | | CN 113853379 A | 28 December 2021 |
| WO 2019190331 A1 | 03 October 2019 | EP 3774743 A1 | 17 February 2021 |
| | | US 2021115002 A1 | 22 April 2021 |
| | | CN 111918864 A | 10 November 2020 |
| WO 2021068952 A1 | 15 April 2021 | CN 114555574 A | 27 May 2022 |
| CN 108136214 A | 08 June 2018 | JP 2019178172 A | 17 October 2019 |
| | | US 2021017120 A1 | 21 January 2021 |
| | | KR 20170127463 A | 21 November 2017 |
| | | SG 10201913709 Q A | 30 March 2020 |
| | | WO 2016161342 A2 | 06 October 2016 |
| | | HK 1250959 A1 | 18 January 2019 |
| | | JP 2018511612 A | 26 April 2018 |
| | | US 2018086693 A1 | 29 March 2018 |
| | | CA 2981494 A1 | 06 October 2016 |
| | | EP 3277381 A2 | 07 February 2018 |
| | | SG CN 11201707375 U A | 30 October 2017 |
| | | IL 254769 D0 | 31 December 2017 |
| | | TW 201706267 A | 16 February 2017 |
| | | AU 2016244000 A1 | 12 October 2017 |
| | | CN 112142692 A | 29 December 2020 |
| | | KR 20190075145 A | 28 June 2019 |
| | | BR 112017021167 A2 | 03 July 2018 |
| CN 106068266 A | 02 November 2016 | EP 3078661 A1 | 12 October 2016 |
| | | US 2019290773 A1 | 26 September 2019 |
| | | JP WO2015053413 A1 | 09 March 2017 |
| | | WO 2015053413 A1 | 16 April 2015 |
| | | US 2016310605 A1 | 27 October 2016 |
| | | US 2020376133 A1 | 03 December 2020 |
| WO 2017087428 A1 | 26 May 2017 | JP 2018517710 A | 05 July 2018 |
| | | CN 108290911 A | 17 July 2018 |
| | | TW 201726695 A | 01 August 2017 |
| | | ES 2781398 T3 | 01 September 2020 |
| | | CA 2990696 A1 | 26 May 2017 |
| | | IL 256569 A | 28 February 2018 |
| | | KR 20170130615 A | 28 November 2017 |
| | | EP 3390415 A1 | 24 October 2018 |
| | | AU 2016357728 A1 | 30 November 2017 |
| | | BR 112017025778 A2 | 14 August 2018 |
| | | HK 1250988 A1 | 18 January 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2020089692 W
- WO 2020228685 A1
- NZ 2019050030 W
- WO 2019190331 A1
- CN 2019800234236
- CN 111918864 A
- CN 2020120281 W
- WO 2021068952 A1
- US 2016062114 W
- WO 2017087428 A1
- CN 2016800200132
- CN 108136214 A
- US 2016021581 W
- WO 2016145092 A1
- CN 2016800150788
- CN 107530556 A
- CN 2016800446081
- CN 108290911 A
- WO 2021008520 A1
- WO 2021043275 A1
- WO 2019062919 A1


### Non-patent literature cited in the description


- **LOONG HHF ; DU N ; CHENG C ; LIN H ; GUO J ; LIN G ; LI M ; JIANG T ; SHI Z ; CUI Y.** KRAS G12C mutations in Asia: A landscape analysis of 11,951 Chinese tumor samples. *Transl Lung Cancer Res,* 2020
- **MENG, F. ; LI, W. F. ; JUNG, D. ; WANG, C. C. ; QI, T. ; SHIA, C. S. ; HSU, R. Y ; HSIEH, Y C. ; DUAN, J.** A novel selective AKR1C3-activated prodrug AST-3424/OBI-3424 exhibits broad anti-tumor activity. *American journal of cancer research,* 2021, vol. 11 (7), 3645-3659
- **EVANS, K. ; DUAN, J. ; PRITCHARD, T. ; JONES, C. D. ; MCDERMOTT, L. ; GU, Z. ; TOSCAN, C. E. ; EL-ZEIN, N. ; MAYOH, C. ; ERICKSON, S. W.** OBI-3424, a Novel AKR1C3-Activated Prodrug, Exhibits Potent Efficacy against Preclinical Models of T-ALL. *Clinical cancer research: an official journal of the American Association for Cancer Research,* 2019, vol. 25 (14), 4493-4503
- **YANLAN WANG ; YUE LIU ; CHANGHUA ZHOU ; CHUNNIAN WANG ; NING ZHANG ; DONGLIN CAO ; QING LI ; ZHONG WANG.** An AKR1C3-specific prodrug with potent anti-tumor activities against T-ALL. *Leukemia & Lymphoma,* 2020, vol. 61 (7), 1660-1668